(19) Europäisches Patentamt
European Patent Office
Office européen des brevets

(11) **EP 2 470 905 B1**

(12) **EUROPEAN PATENT SPECIFICATION**

(45) Date of publication and mention
of the grant of the patent:
**07.01.2015 Bulletin 2015/02**

(51) Int Cl.:
*G01N 33/53* [(2006.01)]     *G01N 33/68* [(2006.01)]

(21) Application number: **10812639.2**

(22) Date of filing: **27.08.2010**

(86) International application number:
**PCT/US2010/046910**

(87) International publication number:
**WO 2011/025917 (03.03.2011 Gazette 2011/09)**

(54) **Methods and compositions for diagnosis and prognosis of renal injury and renal failure**

Verfahren und Zusammensetzungen zur Diagnose und Prognose von Nierenverletzungen und Niereninsuffizienz

Procédés et compositions pour le diagnostic et le pronostic d'une lésion rénale et d'une insuffisance rénale

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority:
28.08.2009 US 238134 P
18.09.2009 US 244002 P
28.08.2009 US 238120 P
28.08.2009 US 238129 P
28.08.2009 US 238118 P
28.08.2009 US 238123 P
28.08.2009 US 238127 P
18.09.2009 US 243993 P
18.09.2009 US 243991 P
28.08.2009 US 238121 P
28.08.2009 US 238128 P
28.08.2009 US 238125 P
28.08.2009 US 238115 P
18.09.2009 US 243997 P

(43) Date of publication of application:
**04.07.2012 Bulletin 2012/27**

(73) Proprietor: **Astute Medical, Inc.**
**San Diego, CA 92121 (US)**

(72) Inventors:
• **ANDERBERG, Joseph**
**Encinitas,CA 92024 (US)**
• **GRAY, Jeff**
**Solana Beach,CA 92075 (US)**
• **MCPHERSON, Paul**
**Encinitas,CA 92024 (US)**
• **NAKAMURA, Kevin**
**Cardiff by the Sea,CA 92007 (US)**
• **KAMPF, James, Patrick**
**San Diego,CA 92130 (US)**

(74) Representative: **Schiweck, Weinzierl & Koch**
**European Patent Attorneys**
**Landsberger Straße 98**
**80339 München (DE)**

(56) References cited:
**WO-A2-2008/122670     WO-A2-2010/048347**
**US-A1- 2007 248 989     US-B1- 7 141 382**

• C.J SONG ET AL: "Expression of TRAIL, DR4, and DR5 in kidney and serum from patients receiving renal transplantation", TRANSPLANTATION PROCEEDINGS, vol. 36, no. 5, 1 June 2004 (2004-06-01), pages 1340-1343, XP055069306, ISSN: 0041-1345, DOI: 10.1016/j.transproceed. 2004.05.005
• S G COCA ET AL: "Biomarkers for the diagnosis and risk stratification of acute kidney injury: A systematic review", KIDNEY INTERNATIONAL, vol. 73, no. 9, 19 December 2007 (2007-12-19), pages 1008-1016, XP055027810, ISSN: 0085-2538, DOI: 10.1038/sj.ki.5002729
• TAULAN ET AL.: 'Comprehensive analysis of the renal transcriptional response to acute uranyl nitrate exposure.' BMC GENOMICS vol. 7, no. 2, 11 January 2006, pages 1 - 14, XP008156021

- PARIKH ET AL.: 'Urinary IL-18 is an early predictive biomarker of acute kidney injury after cardiac surgery.' KIDNEY INTERNAT vol. 70, no. 1, 2006, pages 199 - 203, XP008156022
- KELLUM.: 'Acute Kidney Injury.' CRIT CARE MED vol. 36, no. 4, 2008, pages S141 - S145, XP008156025
- COCA ET AL.: 'Biomarkers for the diagnosis and risk stratification of acute kidney injury: A systematic review.' KIDNEY INTEMAT vol. 73, no. 9, 2008, pages 1008 - 1016, XP055027810
- MELNIKOV ET AL.: 'Impaired IL-18 processing protects caspase-1-deficient mice from ischemic acute renal failure.' J CLIN INVEST vol. 107, no. 9, 2001, pages 1145 - 1152, XP008156028

**Description**

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or bums), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinernic glomerulonephritis |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |

(continued)

| Intrinsic Renal | |
| --- | --- |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AK1 and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45,2008 and Ricci et al., Kidney Int. 73, 538-546, 2008, the RIFLE criteria provide a uniform definition of AKI which has been validated in numerous studies.

More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI. US 2007248989 (D1) discloses a method for identifying the presence of an early biomarker of impaired renal status following a renal event in a mammalian subject by detecting the presence of a protein selected from the group consisting of aprotinin, alpha-1-microglobulin (A1M), alpha-1-acid-glycoprotein (A1AG), microalbumin, and combinations thereof in a sample of bodily fluid.

BRIEF SUMMARY OF THE INVENTION

It is an object of the invention to provide methods and compositions for evaluating renal function in a subject. As described herein, measurement of one or more biomarkers comprising Tumor necrosis factor receptor superfamily member 10B and optionally comprising one or more biomarkers selected from the group consisting of Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 (the named biomarkers are referred to herein as a "kidney injury markers") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present invention may be used, individually or in panels comprising a plurality of kidney injury markers comprising Tumor necrosis factor receptor superfamly member 10B, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.*, hemodialysis, peritoneal dialysis, hemofiltration, and/or renal transplantation) a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more biomarkers comprising Tumor necrosis factor receptor superfamily member 10B and optionally comprising one or more biomarkers selected from the group consisting of Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 in a body fluid sample obtained from the subject. The assay result(s), for example measured concentration(s) of Tumor necrosis factor receptor superfamily member 10B and optionally one or more biomarkers selected from the group consisting of Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.*, is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.*, is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of Tumor necrosis factor receptor superfamily member 10B and optionally of one or more biomarkers selected from the group consisting of Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) Tumor necrosis factor receptor superfamily member 10B andcptionally of one or more biomarkers selected from the group consisting of Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of Tumor necrosis factor receptor superfamily member 10B and optionally one or more biomarkers selected from the group consisting of Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/-5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ec1 (electrochemical luminescence) labels, metal chelates, colloidal metal particles, *etc.)* as well as molecules that may be indirectly detected by production of a detectable reaction product (e.g., enzymes such as horseradish peroxidase, alkaline phosphatase, *etc.)* or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (*e.g.*, a diffraction grating, electrochemical sensor, etc) for generation of a signal, while in others, a signal is generated by a transducer that is spatially separate from the solid phase antibody (*e.g.*, a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of Tumor necrosis factor receptor superfamily member 10B and optionally one or more biomarkers selected from the group consisting of Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq 8.8$ μmol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq 26.4$ μmol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "tumor necrosis factor receptor superfamily member 10B" refers to one or more polypeptides present in a biological sample that are derived from the tumor necrosis factor receptor superfamily member 10B precursor (Swiss-Prot 014763 (SEQ ID NO: 1)).

```
        10          20          30          40          50          60
MEQRGQNAPA  ASGARKRHGP  GPREARGARP  GPRVPKTLVL  VVAAVLLLVS  AESALITQQD

        70          80          90         100         110         120
LAPQQRAAPQ  QKRSSPSEGL  CPPGHHISED  GRDCISCKYG  QDYSTHWNDL  LFCLRCTRCD

       130         140         150         160         170         180
SGEVELSPCT  TTRNTVCQCE  EGTFREEDSP  EMCRKCRTGC  PRGMVKVGDC  TPWSDIECVH

       190         200         210         220         230         240
KESGTKHSGE  APAVEETVTS  SPGTPASPCS  LSGIIIGVTV  AAVVLIVAVF  VCKSLLWKKV

       250         260         270         280         290         300
LPYLKGICSG  GGGDPERVDR  SSQRPGAEDN  VLNEIVSILQ  PTQVPEQEME  VQEPAEPTGV

       310         320         330         340         350         360
NMLSPGESEH  LLEPAEAERS  QRRRLLVPAN  EGDPTETLRQ  CFDDFADLVP  FDSWEPLMRK

       370         380         390         400         410         420
LGLMDNEIKV  AKAEAAGHRD  TLYTMLIKWV  NKTGRDASVH  TLLDALETLG  ERLAKQKIED

       430         440
HLLSSGKFMY  LEGNADSAMS
```

Most preferably, the tumor necrosis factor receptor superfamily member 10B assay detects one or more soluble forms of tumor necrosis factor receptor superfamily member 10B. Tumor necrosis factor receptor superfamily member 10B is a single-pass type I membrane protein having a large extracellular domain, most or all of which is present in soluble forms of tumor necrosis factor receptor superfamily member 10B generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in tumor necrosis factor receptor superfamily member 10B:

| Residues | Length | Domain ID |
| --- | --- | --- |
| 1-55 | 55 | signal sequence |
| 56-440 | 385 | tumor necrosis factor receptor superfamily member 10B |
| 56-210 | 155 | extracellular |
| 211-231 | 21 | transmembrane |
| 232-440 | 209 | cytoplasmic |

As used herein, the term "cadherin-16" refers to one or more polypeptides present in a biological sample that are derived from the cadherin-16 precursor (Swiss-Prot 075309 (SEQ ID NO: 2)).

|  | 10 | 20 | 30 | 40 | 50 | 60 |
|---|---|---|---|---|---|---|
|  | MVPAWLWLLC | VSVPQALPKA | QPAELSVEVP | ENYGGNFPLY | LTKLPLPREG | AEGQIVLSGD |
|  | 70 | 80 | 90 | 100 | 110 | 120 |
|  | SGKATEGPFA | MDPDSGFLLV | TRALDREEQA | EYQLQVTLEM | QDGHVLWGPQ | PVLVHVKDEN |
|  | 130 | 140 | 150 | 160 | 170 | 180 |
|  | DQVPHFSQAI | YRARLSRGTR | PGIPFLFLEA | SDRDEPGTAN | SDLRFHILSQ | APAQPSPDMF |
|  | 190 | 200 | 210 | 220 | 230 | 240 |
|  | QLEPRLGALA | LSPKGSTSLD | HALERTYQLL | VQVKDMGDQA | SGHQATATVE | VSIIESTWVS |
|  | 250 | 260 | 270 | 280 | 290 | 300 |
|  | LEPIHLAENL | KVLYPHHMAQ | VHWSGGDVHY | HLESHPPGPF | EVNAEGNLYV | TRELDREAQA |
|  | 310 | 320 | 330 | 340 | 350 | 360 |
|  | EYLLQVRAQN | SHGEDYAAPL | ELHVLVMDEN | DNVPICPPRD | PTVSIPELSP | PGTEVTRLSA |
|  | 370 | 380 | 390 | 400 | 410 | 420 |
|  | EDADAPGSPN | SHVVYQLLSP | EPEDGVEGRA | FQVDPTSGSV | TLGVLPLRAG | QNILLLVLAM |
|  | 430 | 440 | 450 | 460 | 470 | 480 |

```
DLAGAEGGFS   STCEVEVAVT   DINDHAPEFI   TSQIGPISLP   EDVEPGTLVA   MLTAIDADLE
      490          500          510          520          530          540

PAFRLMDFAI   ERGDTEGTFG   LDWEPDSGHV   RLRLCKNLSY   EAAPSHEVVV   VVQSVAKLVG
      550          560          570          580          590          600

PGPGPGATAT   VTVLVERVMP   PPKLDQESYE   ASVPISAPAG   SFLLTIQPSD   PISRTLRFSL
      610          620          630          640          650          660

VNDSEGWLCI   EKFSGEVHTA   QSLQGAQPGD   TYTVLVEAQD   TDEPRLSASA   PLVIHFLKAP
      670          680          690          700          710          720

PAPALTLAPV   PSQYLCTPRQ   DHGLIVSGPS   KDPDLASGHG   PYSFTLGPNP   TVQRDWRLQT
      730          740          750          760          770          780

LNGSHAYLTL   ALHWVEPREH   IIPVVVSHNA   QMWQLLVRVI   VCRCNVEGQC   MRKVGRMKGM
      790          800          810          820

PTKLSAVGIL   VGTLVAIGIF   LILIFTHWTM   SRKKDPDQPA   DSVPLKATV
```

Most preferably, the cadherin-16 assay detects one or more soluble forms of cadherin-16. Cadherin-16 is a single-pass type I membrane protein having a large extracellular domain, most or all of which is present in soluble forms of cadherin-16 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in cadherin-16:

| Residues | Length | Domain ID |
|---|---|---|
| 1-18 | 18 | signal sequence |
| 19 | 829 | cadherin-16 |
| 19-786 | 768 | extracellular |
| 787-807 | 22 | transmembrane |
| 808-829 | 22 | cytoplasmic |

As used herein, the term "caspase-9" refers to one or more polypeptides present in a biological sample that are derived from the caspase-9 precursor (Swiss-Prot P55211 (SEQ ID NO: 3)).

```
        10         20         30         40         50         60

MDEADRRLLR RCRLRLVEEL QVDQLWDALL SRELFRPHMI EDIQRAGSGS RRDQARQLII

        70         80         90        100        110        120

DLETRGSQAL PLFISCLEDT GQDMLASFLR TNRQAAKLSK PTLENLTPVV LRPEIRKPEV

       130        140        150        160        170        180

LRPETPRPVD IGSGGFGDVG ALESLRGNAD LAYILSMEPC GHCLIINNVN FCRESGLRTR

       190        200        210        220        230        240

TGSNIDCEKL RRRFSSLHFM VEVKGDLTAK KMVLALLELA QQDHGALDCC VVVILSHGCQ

       250        260        270        280        290        300

ASHLQFPGAV YGTDGCPVSV EKIVNIFNGT SCPSLGGKPK LFFIQACGGE QKDHGFEVAS

       310        320        330        340        350        360

TSPEDESPGS NPEPDATPFQ EGLRTFDQLD AISSLPTPSD IFVSYSTFPG FVSWRDPKSG

       370        380        390        400        410

SWYVETLDDI FEQWAHSEDL QSLLLRVANA VSVKGIYKQM PGCFNFLRKK LFFKTS
```

The following domains have been identified in caspase-9:

| Residues | Length | Domain ID |
|---|---|---|
| 1-315 | 315 | caspase-9 p35 subunit |
| 316-330 | 15 | pro-peptide |
| 331-416 | 86 | caspase-9 subunit p10 |

As used herein, the term "Bc12 antagonist of cell death" refers to one or more polypeptides present in a biological sample that are derived from the Bc12 antagonist of cell death precursor (Swiss-Prot Q92934 (SEQ ID NO: 4)).

```
        10         20         30         40         50         60

MFQIPEFEPS EQEDSSSAER GLGPSPAGDG PSGSGKHHRQ APGLLWDASH QQEQPTSSSH

        70         80         90        100        110        120

HGGAGAVEIR SRHSSYPAGT EDDEGMGEEP SPFRGRSRSA PPNLWAAQRY GRELRRMSDE

       130        140        150        160

FVDSFKKGLP RPKSAGTATQ MRQSSSWTRV FQSWWDRNLG RGSSAPSQ
```

As used herein, the term "caspase-1" refers to one or more polypeptides present in a biological sample that are derived from the caspase-1 precursor (Swiss-Prot P29466 (SEQ ID NO: 5)).

```
              10          20          30          40          50          60
MADKVLKEKR  KLFIRSMGEG  TINGLLDELL  QTRVLNKEEM  EKVKRENATV  MDKTRALIDS
              70          80          90         100         110         120
VIPKGAQACQ  ICITYICEED  SYLAGTLGLS  ADQTSGNYLN  MQDSQGVLSS  FPAPQAVQDN
             130         140         150         160         170         180
PAMPTSSGSE  GNVKLCSLEE  AQRIWKQKSA  EIYPIMDKSS  RTRLALIICN  EEFDSIPRRT
             190         200         210         220         230         240
GAEVDITGMT  MLLQNLGYSV  DVKKNLTASD  MTTELEAFAH  RPEHKTSDST  FLVFMSHGIR
             250         260         270         280         290         300
EGICGKKHSE  QVPDILQLNA  IFNMLNTKNC  PSLKDKPKVI  IIQACRGDSP  GVVWFKDSVG
             310         320         330         340         350         360
VSGNLSLPTT  EEFEDDAIKK  AHIEKDFIAF  CSSTPDNVSW  RHPTMGSVFI  GRLIEHMQEY
             370         380         390         400
ACSCDVEEIF  RKVRFSFEQP  DGRAQMPTTE  RVTLTRCFYL  FPGH
```

The following domains have been identified in caspase-1:

| Residues | Length | Domain ID |
|----------|--------|-----------|
| 1-119 | 119 | pro-peptide |
| 120-297 | 178 | caspase-1 p20 subunit |
| 298-316 | 19 | pro-peptide |
| 317-404 | 88 | caspase-1 p10 subunit |

As used herein, the terms "epithelial cadherin" or "Cadherin-1" refers to one or more polypeptides present in a biological sample that are derived from the epithelial cadherin precursor (Swiss-Prot P12830 (SEQ ID NO: 6)).

17

```
              10          20          30          40          50          60

MGPWSRSLSA  LLLLLQVSSW  LCQEPEPCHP  GFDAESYTFT  VPRRHLERGR  VLGRVNFEDC

              70          80          90         100         110         120

TGRQRTAYFS  LDTRFKVGTD  GVITVKRPLR  FHNPQIHFLV  YAWDSTYRKF  STKVTLNTVG

             130         140         150         160         170         180

HHHRPPPHQA  SVSGIQAELL  TFPNSSPGLR  RQKRDWVIPP  ISCPENEKGP  FPKNLVQIKS

             190         200         210         220         230         240

NKDKEGKVFY  SITGQGADTP  PVGVFIIERE  TGWLKVTEPL  DRERIATYTL  FSHAVSSNGN

             250         260         270         280         290         300

AVEDPMEILI  TVTDQNDNKP  EFTQEVFKGS  VMEGALPGTS  VMEVTATDAD  DDVNTYNAAI

             310         320         330         340         350         360

AYTILSQDPE  LPDKNMFTIN. RNTGVISVVT  TGLDRESFPT  YTLVVQAADL  QGEGLSTTAT

             370         380         390         400         410         420

AVITVTDTND  NPPIFNPTTY  KGQVPENEAN  VVITTLKVTD  ADAPNTPAWE  AVYTILNDDG

             430         440         450         460         470         480

GQFVVTTNPV  NNDGILKTAK  GLDFEAKQQY  ILHVAVTNVV  PFEVSLTTST  ATVTVDVLDV

             490         500         510         520         530         540

NEAPIFVPPE  KRVEVSEDFG  VGQEITSYTA  QEPDTFMEQK  ITYRIWRDTA  NWLEINPDTG

             550         560         570         580         590         600

AISTRAELDR  EDFEHVKNST  YTALIIATDN  GSPVATGTGT  LLLILSDVND  NAPIPEPRTI

             610         620         630         640         650         660

FFCERNPKPQ  VINIIDADLP  PNTSPFTAEL  THGASANWTI  QYNDPTQESI  ILKPKMALEV

             670         680         690         700         710         720

GDYKINLKLM  DNQNKDQVTT  LEVSVCDCEG  AAGVCRKAQP  VEAGLQIPAI  LGILGGILAL

             730         740         750         760         770         780

LILILLLLLF  LRRRAVVKEP  LLPPEDDTRD  NVYYYDEEGG  GEEDQDFDLS  QLHRGLDARP
```

18

```
      790         800         810         820         830         840

  EVTRNDVAPT  LMSVPRYLPR  PANPDEIGNF  IDENLKAADT  DPTAPPYDSL  LVFDYEGSGS

      850         860         870         880

  EAASLSSLNS  SESDKDQDYD  YLNEWGNRFK  KLADMYGGGE  DD
```

Most preferably, the epithelial cadherin assay detects one or more soluble forms of epithelial cadherin. Epithelial cadherin is a single-pass type I membrane protein having a large extracellular domain, most or all of which is present in soluble forms of epithelial cadherin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in epithelial cadherin:

| Residues | Length | Domain ID |
|---|---|---|
| 1-22 | 22 | signal sequence |
| 23-154 | 132 | pro-peptide |
| 155-882 | 728 | epithelial cadherin |
| 155-709 | 555 | extracellular |
| 710-730 | 21 | transmembrane |
| 731-882 | 152 | cytoplasmic |

As used herein, the term "poly [ADP-ribose] polymerase 1" refers to one or more polypeptides present in a biological sample that are derived from the poly [ADP-ribose] polymerase 1 precursor (Swiss-Prot P09874 (SEQ ID NO: 7)).

```
      10          20          30          40          50          60

  MAESSDKLYR  VEYAKSGRAS  CKKCSESIPK  DSLRMAIMVQ  SPMFDGKVPH  WYHFSCFWKV

      70          80          90         100         110         120

  GHSIRHPDVE  VDGFSELRWD  DQQKVKKTAE  AGGVTGKGQD  GIGSKAEKTL  GDFAAEYAKS

     130         140         150         160         170         180

  NRSTCKGCME  KIEKGQVRLS  KKMVDPEKPQ  LGMIDRWYHP  GCFVKNREEL  GFRPEYSASQ

     190         200         210         220         230         240
```

```
LKGFSLLATE DKEALKKQLP GVKSEGKRKG DEVDGVDEVA KKKSKKEKDK DSKLEKALKA
    250        260        270        280        290        300
QNDLIWNIKD ELKKVCSTND LKELLIFNKQ QVPSGESAIL DRVADGMVFG ALLPCEECSG
    310        320        330        340        350        360
QLVFKSDAYY CTGDVTAWTK CMVKTQTPNR KEWVTPKEFR EISYLKKLKV KKQDRIFPPE
    370        380        390        400        410        420
TSASVAATPP PSTASAPAAV NSSASADKPL SNMKILTLGK LSRNKDEVKA MIEKLGGKLT
    430        440        450        460        470        480
GTANKASLCI STKKEVEKMN KKMEEVKEAN IRVVSEDFLQ DVSASTKSLQ ELFLAHILSP
    490        500        510        520        530        540
WGAEVKAEPV EVVAPRGKSG AALSKKSKGQ VKEEGINKSE KRMKLTLKGG AAVDPDSGLE
    550        560        570        580        590        600
HSAHVLEKGG KVFSATLGLV DIVKGTNSYY KLQLLEDDKE NRYWIFRSWG RVGTVIGSNK
    610        620        630        640        650        660
LEQMPSKEDA IEHFMKLYEE KTGNAWHSKN FTKYPKKFYP LEIDYGQDEE AVKKLTVNPG
    670        680        690        700        710        720
TKSKLPKPVQ DLIKMIFDVE SMKKAMVEYE IDLQKMPLGK LSKRQIQAAY SILSEVQQAV
    730        740        750        760        770        780
SQGSSDSQIL DLSNRFYTLI PHDFGMKKPP LLNNADSVQA KVEMLDNLLD IEVAYSLLRG
    790        800        810        820        830        840
GSDDSSKDPI DVNYEKLKTD IKVVDRDSEE AEIIRKYVKN THATTHNAYD LEVIDIFKIE
    850        860        870        880        890        900
REGECQRYKP FKQLHNRRLL WHGSRTTNFA GILSQGLRIA PPEAPVTGYM FGKGIYFADM
    910        920        930        940        950        960
VSKSANYCHT SQGDPIGLIL LGEVALGNMY ELKHASHISK LPKGKHSVKG LGKTTPDPSA
    970        980        990       1000       1010
NISLDGVDVP LGTGISSGVN DTSLLYNEYI VYDIAQVNLK YLLKLKFNFK TSLW
```

The following domains have been identified in poly [ADP-ribose] polymerase 1:

| Residues | Length | Domain ID |
|---|---|---|
| 1 | 1 | initiator methionine |
| 2-1014 | 1013 | poly [ADP-ribose] polymerase 1 |

Poly [ADP-ribose] polymerase 1 can be cleaved by many caspases in vitro and is one of the main cleavage targets of caspase-3 in vivo. The cleavage occurs between Asp(214) and Gly(215), which separates PARP's N-terminal DNA binding domain (24 kDa) from its C-terminal catalytic domain (89 kDa). Suitable assays may recognize only the large fragment of poly [ADP-ribose] polymerase 1 (89 kDa) but not the full length poly [ADP-ribose] polymerase 1, may recognize only the small fragment of poly [ADP-ribose] polymerase 1 (24 kDa) but not the full length poly [ADP-ribose] polymerase 1, may recognize only full length poly [ADP-ribose] polymerase 1, or may recognize one fragment and the full length full length poly [ADP-ribose] polymerase 1.

As used herein, the term "cyclin-dependent kinase inhibitor 1" refers to one or more polypeptides present in a biological sample that are derived from the cyclin-dependent kinase inhibitor 1 precursor (Swiss-Prot P38936 (SEQ ID NO: 8)).

```
                10          20          30          40          50          60

    MSEPAGDVRQ  NPCGSKACRR  LFGPVDSEQL  SRDCDALMAG  CIQEARERWN  FDFVTETPLE

                70          80          90         100         110         120

    GDFAWERVRG  LGLPKLYLPT  GPRRGRDELG  GGRRPGTSPA  LLQGTAEEDH  VDLSLSCTLV

               130         140         150         160

    PRSGEQAEGS  PGGPGDSQGR  KRRQTSMTDF  YHSKRRLIFS  KRKP
```

The following domains have been identified in cyclin-dependent kinase inhibitor 1:

| Residues | Length | Domain |
|---|---|---|
| 1 | 1 | initiator methionine |
| 2-164 | 163 | cyclin-dependent kinase inhibitor 1 |

As used herein, the term "cadherin-5" refers to one or more polypeptides present in a biological sample that are derived from the cadherin-5 precursor (Swiss-Prot P33151 (SEQ ID NO: 9)).

MQRLMMLLAT SGACLGLLAV AAVAAAGANP AQRDTHSLLP THRRQKRDWI WNQMHIDEEK

NTSLPHHVGK IKSSVSRKNA KYLLKGEYVG KVFRVDAETG DVFAIERLDR ENISEYHLTA

VIVDKDTGEN LETPSSFTIK VHDVNDNWPV FTHRLFNASV PESSAVGTSV ISVTAVDADD

PTVGDHASVM YQILKGKEYF AIDNSGRIIT ITKSLDREKQ ARYEIVVEAR DAQGLRGDSG

TATVLVTLQD INDNFPFFTQ TKYTFVVPED TRVGTSVGSL FVEDPDEPQN RMTKYSILRG

DYQDAFTIET NPAHNEGIIK PMKPLDYEYI QQYSFIVEAT DPTIDLRYMS PPAGNRAQVI

INITDVDEPP IFQQPFYHFQ LKENQKKPLI GTVLAMDPDA ARHSIGYSIR RTSDKGQFFR

VTKKGDIYNE KELDREVYPW YNLTVEAKEL DSTGTPTGKE SIVQVHIEVL DENDNAPEFA

KPYQPKVCEN AVHGQLVLQI SAIDKDITPR NVKFKFTLNT ENNFTLTDNH DNTANITVKY

GQFDREHTKV HFLPVVISDN GMPSRTGTST LTVAVCKCNE QGEFTFCEDM AAQVGVSIQA

VVAILLCILT ITVITLLIFL RRRLRKQARA HGKSVPEIHE QLVTYDEEGG GEMDTTSYDV

SVLNSVRRGG AKPPRPALDA RPSLYAQVQK PPRHAPGAHG GPGEMAAMIE VKKDEADHDG

DGPPYDTLHI YGYEGSESIA ESLSSLGTDS SDSDVDYDFL NDWGPRFKML AELYGSDPRE

ELLY

Most preferably, the cadherin-5 assay detects one or more soluble forms of cadherin-5. Cadherin-5 is a single-pass type I membrane protein having a large extracellular domain, most or all of which is present in soluble forms of cadherin-5 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in cadherin-5:

| Residues | Length | Domain ID |
|---|---|---|
| 1-25 | 251 | signal sequence |
| 26-47 | 22 | pro-peptide |
| 48-784 | 737 | cadherin-5 |
| 48-599 | 522 | extracellular |
| 600-620 | 21 | transmembrane |
| 621-784 | 164 | cytoplasmic |

As used herein, the term "Myoglobin" refers to one or polypeptides present in a biological sample that are derived from the Myoglobin precursor (Swiss-Prot P02144 (SEQ ID NO: 10)).

```
          10         20         30         40         50         60

  MGLSDGEWQL VLNVWGKVEA DIPGHGQEVL IRLFKGHPET LEKFDKFKHL KSEDEMKASE

          70         80         90        100        110        120

  DLKKHGATVL TALGGILKKK GHHEAEIKPL AQSHATKHKI PVKYLEFISE CIIQVLQSKH

         130        140        150

  PGDFGADAQG AMNKALELFR KDMASNYKEL GFQG
```

The following domains have been identified in Myoglobin:

| Residues | Length | Domain ID |
|---|---|---|
| 1 | 1 | Initiator Methionine |
| 2-154 | 153 | Myoglobin |

As used herein, the term "Apolipoprotein A-II" refers to one or polypeptides present in a biological sample that are derived from the Apolipoprotein A-II precursor (Swiss-Prot P02652 (SEQ ID NO: 11)).

```
          10         20         30         40         50         60

  MKLLAATVLL LTICSLEGAL VRRQAKEPCV ESLVSQYFQT VTDYGKDLME KVKSPELQAE

          70         80         90        100

  AKSYFEKSKE QLTPLIKKAG TELVNFLSYF VELGTQPATQ
```

The following domains have been identified in Apolipoprotein A-II:

| Residues | Length | Domain ID |
|---|---|---|
| 1-18 | 18 | Signal sequence |
| 19-23 | 5 | Propeptide |
| 24-100 | 77 | Apolipoprotein A-II |

(continued)

| Residues | Length | Domain ID |
|----------|--------|-----------|
| 24-99 | 76 | Apolipoprotein A-II(1-76) |

As used herein, the term "Mucin-16" refers to one or polypeptides present in a biological sample that are derived from the Mucin-16 precursor (Swiss-Prot Q8WXI7 (SEQ ID NO: 12)).

```
              10          20          30          40          50          60

    MLKPSGLPGS  SSPTRSLMTG  SRSTKATPEM  DSGLTGATLS  PKTSTGAIVV  TEHTLPFTSP

              70          80          90         100         110         120

    DKTLASPTSS  VVGRTTQSLG  VMSSALPEST  SRGMTHSEQR  TSPSLSPQVN  GTPSRNYPAT

             130         140         150         160         170         180

    SMVSGLSSPR  TRTSSTEGNF  TKEASTYTLT  VETTSGPVTE  KYTVPTETST  TEGDSTETPW

             190         200         210         220         230         240

    DTRYIPVKIT  SPMKTFADST  ASKENAPVSM  TPAETTVTDS  HTPGRTNPSF  GTLYSSFLDL

             250         260         270         280         290         300

    SPKGTPNSRG  ETSLELILST  TGYPFSSPEP  GSAGHSRIST  SAPLSSSASV  LDNKISETSI
```

|  |  |  |  |  |  |
|---|---|---|---|---|---|
| 310 | 320 | 330 | 340 | 350 | 360 |
| FSGQSLTSPL | SPGVPEARAS | TMPNSAIPFS | MTLSNAETSA | ERVRSTISSL | GTPSISTKQT |
| 370 | 380 | 390 | 400 | 410 | 420 |
| AETILTFHAF | AETMDIPSTH | IAKTLASEWL | GSPGTLGGTS | TSALTTTSPS | TTLVSEETNT |
| 430 | 440 | 450 | 460 | 470 | 480 |
| HHSTSGKETE | GTLNTSMTPL | ETSAPGEESE | MTATLVPTLG | FTTLDSKIRS | PSQVSSSHPT |
| 490 | 500 | 510 | 520 | 530 | 540 |
| RELRTTGSTS | GRQSSSTAAH | GSSDILRATT | SSTSKASSWT | SESTAQQFSE | PQHTQWVETS |
| 550 | 560 | 570 | 580 | 590 | 600 |
| PSMKTERPPA | STSVAAPITT | SVPSVVSGFT | TLKTSSTKGI | WLEETSADTL | IGESTAGPTT |
| 610 | 620 | 630 | 640 | 650 | 660 |
| HQFAVPTGIS | MTGGSSTRGS | QGTTHLLTRA | TASSETSADL | TLATNGVPVS | VSPAVSKTAA |
| 670 | 680 | 690 | 700 | 710 | 720 |
| GSSPPGGTKP | SYTMVSSVIP | ETSSLQSSAF | REGTSLGLTP | LNTRHPFSSP | EPDSAGHTKI |
| 730 | 740 | 750 | 760 | 770 | 780 |
| STSIPLLSSA | SVLEDKVSAT | STFSHHKATS | SITTGTPEIS | TKTKPSSAVL | SSMTLSNAAT |
| 790 | 800 | 810 | 820 | 830 | 840 |
| SPERVRNATS | PLTHPSPSGE | ETAGSVLTLS | TSAETTDSPN | IHPTGTLTSE | SSESPSTLSL |
| 850 | 860 | 870 | 880 | 890 | 900 |
| PSVSGVKTTF | SSSTPSTHLF | TSGEETEETS | NPSVSQPETS | VSRVRTTLAS | TSVPTPVFPT |
| 910 | 920 | 930 | 940 | 950 | 960 |
| MDTWPTRSAQ | FSSSHLVSEL | RATSSTSVTN | STGSALPKIS | HLTGTATMSQ | TNRDTFNDSA |
| 970 | 980 | 990 | 1000 | 1010 | 1020 |
| APQSTTWPET | SPRFKTGLPS | ATTTVSTSAT | SLSATVMVSK | FTSPATSSME | ATSIREPSTT |
| 1030 | 1040 | 1050 | 1060 | 1070 | 1080 |
| ILTTETTNGP | GSMAVASTNI | PIGKGYITEG | RLDTSHLPIG | TTASSETSMD | FTMAKESVSM |
| 1090 | 1100 | 1110 | 1120 | 1130 | 1140 |
| SVSPSQSMDA | AGSSTPGRTS | QFVDTFSDDV | YHLTSREITI | PRDGTSSALT | PQMTATHPPS |

```
      1150       1160       1170       1180       1190       1200
PDPGSARSTW LGILSSSPSS PTPKVTMSST FSTQRVTTSM IMDTVETSRW NMPNLPSTTS

      1210       1220       1230       1240       1250       1260
LTPSNIPTSG AIGKSTLVPL DTPSPATSLE ASEGGLPTLS TYPESTNTPS IHLGAHASSE

      1270       1280       1290       1300       1310       1320
SPSTIKLTMA SVVKPGSYTP LTFPSIETHI HVSTARMAYS SGSSPEMTAP GETNTGSTWD

      1330       1340       1350       1360       1370       1380
PTTYITTTDP KDTSSAQVST PHSVRTLRTT ENHPKTESAT PAAYSGSPKI SSSPNLTSPA

      1390       1400       1410       1420       1430       1440
TKAWTITDTT EHSTQLHYTK LAEKSSGFET QSAPGPVSVV IPTSPTIGSS TLELTSDVPG

      1450       1460       1470       1480       1490       1500
EPLVLAPSEQ TTITLPMATW LSTSLTEEMA STDLDISSPS SPMSTFAIFP PMSTPSHELS

      1510       1520       1530       1540       1550       1560
KSEADTSAIR NTDSTTLDQH LGIRSLGRTG DLTTVPITPL TTTWTSVIEH STQAQDTLSA

      1570       1580       1590       1600       1610       1620
TMSPTHVTQS LKDQTSIPAS ASPSHLTEVY PELGTQGRSS SEATTFWKPS TDTLSREIET

      1630       1640       1650       1660       1670       1680
GPTNIQSTPP MDNTTTGSSS SGVTLGIAHL PIGTSSPAET STNMALERRS STATVSMAGT

      1690       1700       1710       1720       1730       1740
MGLLVTSAPG RSISQSLGRV SSVLSESTTE GVTDSSKGSS PRLNTQGNTA LSSSLEPSYA

      1750       1760       1770       1780       1790       1800
EGSQMSTSIP LTSSPTTPDV EFIGGSTFWT KEVTTVMTSD ISKSSARTES SSATLMSTAL

      1810       1820       1830       1840       1850       1860
GSTENTGKEK LRTASMDLPS PTPSMEVTPW ISLTLSNAPN TTDSLDLSHG VHTSSAGTLA

      1870       1880       1890       1900       1910       1920
TDRSLNTGVT RASRLENGSD TSSKSLSMGN STHTSMTDTE KSEVSSSIHP RPETSAPGAE

      1930       1940       1950       1960       1970       1980
TTLTSTPGNR AISLTLPFSS IPVEEVISTG ITSGPDINSA PMTHSPITPP TIVWTSTGTI
```

|      1990 |      2000 |      2010 |      2020 |      2030 |      2040 |
| EQSTQPLHAV | SSEKVSVQTQ | STPYVNSVAV | SASPTHENSV | SSGSSTSSPY | SSASLESLDS |

|      2050 |      2060 |      2070 |      2080 |      2090 |      2100 |
| TISRRNAITS | WLWDLTTSLP | TTTWPSTSLS | EALSSGHSGV | SNPSSTTTEF | PLFSAASTSA |

|      2110 |      2120 |      2130 |      2140 |      2150 |      2160 |
| AKQRNPETET | HGPQNTAAST | LNTDASSVTG | LSETPVGASI | SSEVPLPMAI | TSRSDVSGLT |

|      2170 |      2180 |      2190 |      2200 |      2210 |      2220 |
| SESTANPSLG | TASSAGTKLT | RTISLPTSES | LVSFRMNKDP | WTVSIPLGSH | PTTNTETSIP |

|      2230 |      2240 |      2250 |      2260 |      2270 |      2280 |
| VNSAGPPGLS | TVASDVIDTP | SDGAESIPTV | SFSPSPDTEV | TTISHFPEKT | THSFRTISSL |

|      2290 |      2300 |      2310 |      2320 |      2330 |      2340 |
| THELTSRVTP | IPGDWMSSAM | STKPTGASPS | ITLGERRTIT | SAAPTTSPIV | LTASFTETST |

|      2350 |      2360 |      2370 |      2380 |      2390 |      2400 |
| VSLDNETTVK | TSDILDARKT | NELPSDSSSS | SDLINTSIAS | STMDVTKTAS | ISPTSISGMT |

|      2410 |      2420 |      2430 |      2440 |      2450 |      2460 |
| ASSSPSLFSS | DRPQVPTSTT | ETNTATSPSV | SSNTYSLDGG | SNVGGTPSTL | PPFTITHPVE |

|      2470 |      2480 |      2490 |      2500 |      2510 |      2520 |
| TSSALLAWSR | PVRTFSTMVS | TDTASGENPT | SSNSVVTSVP | APGTWASVGS | TTDLPAMGFL |

|      2530 |      2540 |      2550 |      2560 |      2570 |      2580 |
| KTSPAGEAHS | LLASTIEPAT | AFTPHLSAAV | VTGSSATSEA | SLLTTSESKA | IHSSPQTPTT |

|      2590 |      2600 |      2610 |      2620 |      2630 |      2640 |
| PTSGANWETS | ATPESLLVVT | ETSDTTLTSK | ILVTDTILFS | TVSTPPSKFP | STGTLSGASF |

|      2650 |      2660 |      2670 |      2680 |      2690 |      2700 |
| PTLLPDTPAI | PLTATEPTSS | LATSFDSTPL | VTIASDSLGT | VPETTLTMSE | TSNGDALVLK |

|      2710 |      2720 |      2730 |      2740 |      2750 |      2760 |
| TVSNPDRSIP | GITIQGVTES | PLHPSSTSPS | KIVAPRNTTY | EGSITVALST | LPAGTTGSLV |

|      2770 |      2780 |      2790 |      2800 |      2810 |      2820 |
| FSQSSENSET | TALVDSSAGL | ERASVMPLTT | GSQGMASSGG | IRSGSTHSTG | TKTFSSLPLT |

|      | 2830 |      | 2840 |      | 2850 |      | 2860 |      | 2870 |      | 2880 |
|------|------|------|------|------|------|------|------|------|------|------|------|

MNPGEVTAMS EITTNRLTAT QSTAPKGIPV KPTSAESGLL TPVSASSSPS KAFASLTTAP

|      | 2890 |      | 2900 |      | 2910 |      | 2920 |      | 2930 |      | 2940 |

PSTWGIPQST LTFEFSEVPS LDTKSASLPT PGQSLNTIPD SDASTASSSL SKSPEKNPRA

|      | 2950 |      | 2960 |      | 2970 |      | 2980 |      | 2990 |      | 3000 |

RMMTSTKAIS ASSFQSTGFT ETPEGSASPS MAGHEPRVPT SGTGDPRYAS ESMSYPDPSK

|      | 3010 |      | 3020 |      | 3030 |      | 3040 |      | 3050 |      | 3060 |

ASSAMTSTSL ASKLTTLFST GQAARSGSSS SPISLSTEKE TSFLSPTAST SRKTSLFLGP

|      | 3070 |      | 3080 |      | 3090 |      | 3100 |      | 3110 |      | 3120 |

SMARQPNILV HLQTSALTLS PTSTLNMSQE EPPELTSSQT IAEEEGTTAE TQTLTFTPSE

|      | 3130 |      | 3140 |      | 3150 |      | 3160 |      | 3170 |      | 3180 |

TPTSLLPVSS PTEPTARRKS SPETWASSIS VPAKTSLVET TDGTLVTTIK MSSQAAQGNS

|      | 3190 |      | 3200 |      | 3210 |      | 3220 |      | 3230 |      | 3240 |

TWPAPAEETG TSPAGTSPGS PEVSTTLKIM SSKEPSISPE IRSTVRNSPW KTPETTVPME

|      | 3250 |      | 3260 |      | 3270 |      | 3280 |      | 3290 |      | 3300 |

TTVEPVTLQS TALGSGSTSI SHLPTGTTSP TKSPTENMLA TERVSLSPSP PEAWTNLYSG

|      | 3310 |      | 3320 |      | 3330 |      | 3340 |      | 3350 |      | 3360 |

TPGGTRQSLA TMSSVSLESP TARSITGTGQ QSSPELVSKT TGMEFSMWHG STGGTTGDTH

|      | 3370 |      | 3380 |      | 3390 |      | 3400 |      | 3410 |      | 3420 |

VSLSTSSNIL EDPVTSPNSV SSLTDKSKHK TETWVSTTAI PSTVLNNKIM AAEQQTSRSV

|      | 3430 |      | 3440 |      | 3450 |      | 3460 |      | 3470 |      | 3480 |

DEAYSSTSSW SDQTSGSDIT LGASPDVTNT LYITSTAQTT SLVSLPSGDQ GITSLTNPSG

|      | 3490 |      | 3500 |      | 3510 |      | 3520 |      | 3530 |      | 3540 |

GKTSSASSVT SPSIGLETLR ANVSAVKSDI APTAGHLSQT SSPAEVSILD VTTAPTPGIS

|      | 3550 |      | 3560 |      | 3570 |      | 3580 |      | 3590 |      | 3600 |

TTITTMGTNS ISTTTPNPEV GMSTMDSTPA TERRTTSTEH PSTWSSTAAS DSWTVTDMTS

|      | 3610 |      | 3620 |      | 3630 |      | 3640 |      | 3650 |      | 3660 |

NLKVARSPGT ISTMHTTSFL ASSTELDSMS TPHGRITVIG TSLVTPSSDA SAVKTETSTS

```
        3670       3680       3690       3700       3710       3720
ERTLSPSDTT ASTPISTFSR VQRMSISVPD ILSTSWTPSS TEAEDVPVSM VSTDHASTKT

        3730       3740       3750       3760       3770       3780
DPNTPLSTFL FDSLSTLDWD TGRSLSSATA TTSAPQGATT PQELTLETMI SPATSQLPFS

        3790       3800       3810       3820       3830       3840
IGHITSAVTP AAMARSSGVT FSRPDPTSKK AEQTSTQLPT TTSAHPGQVP RSAATTLDVI

        3850       3860       3870       3880       3890       3900
PHTAKTPDAT FQRQGQTALT TEARATSDSW NEKEKSTPSA PWITEMMNSV SEDTIKEVTS

        3910       3920       3930       3940       3950       3960
SSSVLKDPEY AGHKLGIWDD FIPKFGKAAH MRELPLLSPP QDKEAIHPST NTVETTGWVT

        3970    •  3980       3990       4000       4010       4020
SSEHASHSTI PAHSASSKLT SPVVTTSTRE QAIVSMSTTT WPESTRARTE PNSFLTIELR

        4030       4040       4050       4060       4070       4080
DVSPYMDTSS TTQTSIISSP GSTAITKGPR TEITSSKRIS SSFLAQSMRS SDSPSEAITR

        4090       4100       4110       4120       4130       4140
LSNFPAMTES GGMILAMQTS PPGATSLSAP TLDTSATASW TGTPLATTQR FTYSEKTTLF

        4150       4160       4170       4180       4190       4200
SKGPEDTSQP SPPSVEETSS SSSLVPIHAT TSPSNILLTS QGHSPSSTPP VTSVFLSETS

        4210       4220       4230       4240       4250       4260
GLGKTTDMSR ISLEPGTSLP PNLSSTAGEA LSTYEASRDT KAIHHSADTA VTNMEATSSE

        4270       4280       4290       4300       4310       4320
YSPIPGHTKP SKATSPLVTS HIMGDITSST SVFGSSETTE IETVSSVNQG LQERSTSQVA

        4330       4340       4350       4360       4370       4380
SSATETSTVI THVSSGDATT HVTKTQATFS SGTSISSPHQ FITSTNTFTD VSTNPSTSLI

        4390       4400       4410       4420       4430       4440
MTESSGVTIT TQTGPTGAAT QGPYLLDTST MPYLTETPLA VTPDFMQSEK TTLISKGPKD

        4450       4460       4470       4480       4490       4500
VTWTSPPSVA ETSYPSSLTP FLVTTIPPAT STLQGQHTSS PVSATSVLTS GLVKTTDMLN
```

29

```
        4510        4520        4530        4540        4550        4560

TSMEPVTNSP  QNLNNPSNEI  LATLAATTDI  ETIHPSINKA  VTNMGTASSA  HVLHSTLPVS

        4570        4580        4590        4600        4610        4620

SEPSTATSPM  VPASSMGDAL  ASISIPGSET  TDIEGEPTSS  LTAGRKENST  LQEMNSTTES

        4630        4640        4650        4660        4670        4680

NIILSNVSVG  AITEATKMEV  PSFDATFIPT  PAQSTKFPDI  FSVASSRLSN  SPPMTISTHM

        4690        4700        4710        4720        4730        4740

TTTQTGSSGA  TSKIPLALDT  STLETSAGTP  SVVTEGFAHS  KITTAMNNDV  KDVSQTNPPF

        4750        4760        4770        4780        4790        4800

QDEASSPSSQ  APVLVTTLPS  SVAFTPQWHS  TSSPVSMSSV  LTSSLVKTAG  KVDTSLETVT

        4810        4820        4830        4840        4850        4860

SSPQSMSNTL  DDISVTSAAT  TDIETTHPSI  NTVVTNVGTT  GSAFESHSTV  SAYPEPSKVT

        4870        4880        4890        4900        4910        4920

SPNVTTSTME  DTTISRSIPK  SSKTTRTETE  TTSSLTPKLR  ETSISQEITS  STETSTVPYK

        4930        4940        4950        4960        4970        4980

ELTGATTEVS  RTDVTSSSST  SFPGPDQSTV  SLDISTETNT  RLSTSPIMTE  SAEITITTQT

        4990        5000        5010        5020        5030        5040

GPHGATSQDT  FTMDPSNTTP  QAGIHSAMTH  GFSQLDVTTL  MSRIPQDVSW  TSPPSVDKTS

        5050        5060        5070        5080        5090        5100

SPSSFLSSPA  MTTPSLISST  LPEDKLSSPM  TSLLTSGLVK  ITDILRTRLE  PVTSSLPNFS

        5110        5120        5130        5140        5150        5160

STSDKILATS  KDSKDTKEIF  PSINTEETNV  KANNSGHESH  SPALADSETP  KATTQMVITT

        5170        5180        5190        5200        5210        5220

TVGDPAPSTS  MPVHGSSETT  NIKREPTYFL  TPRLRETSTS  QESSFPTDTS  FLLSKVPTGT

        5230        5240        5250        5260        5270        5280

ITEVSSTGVN  SSSKISTPDH  DKSTVPPDTF  TGEIPRVFTS  SIKTKSAEMT  ITTQASPPES

        5290        5300        5310        5320        5330        5340

ASHSTLPLDT  STTLSQGGTH  STVTQGFPYS  EVTTLMGMGP  GNVSWMTTPP  VEETSSVSSL
```

MSSPAMTSPS PVSSTSPQSI PSSPLPVTAL PTSVLVTTTD VLGTTSPESV TSSPPNLSSI

THERPATYKD TAHTEAAMHH STNTAVTNVG TSGSGHKSQS SVLADSETSK ATPLMSTTST

LGDTSVSTST PNISQTNQIQ TEPTASLSPR LRESSTSEKT SSTTETNTAF SYVPTGAITQ

ASRTEISSSR TSISDLDRPT IAPDISTGMI TRLFTSPIMT KSAEMTVTTQ TTTPGATSQG

ILPWDTSTTL FQGGTHSTVS QGFPHSEITT LRSRTPGDVS WMTTPPVEET SSGFSLMSPS

MTSPSPVSST SPESIPSSPL PVTALLTSVL VTTTNVLGTT SPETVTSSPP NLSSPTQERL

TTYKDTAHTE AMHASMHTNT AVANVGTSIS GHESQSSVPA DSHTSKATSP MGITFAMGDT

SVSTSTPAFF ETRIQTESTS SLIPGLRDTR TSEEINTVTE TSTVLSEVPT TTTTEVSRTE

VITSSRTTIS GPDHSKMSPY ISTETITRLS TFPFVTGSTE MAITNQTGPI GTISQATLTL

DTSSTASWEG THSPVTQRFP HSEETTTMSR STKGVSWQSP PSVEETSSPS SPVPLPAITS

HSSLYSAVSG SSPTSALPVT SLLTSGRRKT IDMLDTHSEL VTSSLPSASS FSGEILTSEA

STNTETIHFS ENTAETNMGT TNSMHKLHSS VSIHSQPSGH TPPKVTGSMM EDAIVSTSTP

GSPETKNVDR DSTSPLTPEL KEDSTALVMN STTESNTVFS SVSLDAATEV SRAEVTYYDP

TFMPASAQST KSPDISPEAS SSHSNSPPLT ISTHKTIATQ TGPSGVTSLG QLTLDTSTIA

```
        6190       6200       6210       6220       6230       6240

TSAGTPSART QDFVDSETTS VMNNDLNDVL KTSPFSAEEA NSLSSQAPLL VTTSPSPVTS

        6250       6260       6270       6280       6290       6300

TLQEHSTSSL VSVTSVPTPT LAKITDMDTN LEPVTRSPQN LRNTLATSEA TTDTHTMHPS

        6310       6320       6330       6340       6350       6360

INTAMANVGT TSSPNEFYFT VSPDSDPYKA TSAVVITSTS GDSIVSTSMP RSSAMKKIES

        6370       6380       6390       6400       6410       6420

ETTFSLIFRL RETSTSQKIG SSSDTSTVFD KAFTAATTEV SRTELTSSSR TSIQGTEKPT

        6430       6440       6450       6460       6470       6480

MSPDTSTRSV TMLSTFAGLT KSEERTIATQ TGPHRATSQG TLTWDTSITT SQAGTHSAMT

        6490       6500       6510       6520       6530       6540

HGFSQLDLST LTSRVPEYIS GTSPPSVEKT SSSSSLLSLP AITSPSPVPT TLPESRPSSP

        6550       6560       6570       6580       6590       6600

VHLTSLPTSG LVKTTDMLAS VASLPPNLGS TSHKIPTTSE DIKDTEKMYP STNIAVTNVG

        6610       6620       6630       6640       6650       6660

TTTSEKESYS SVPAYSEPPK VTSPMVTSFN IRDTIVSTSM PGSSEITRIE MESTFSVAHG

        6670       6680       6690       6700       6710       6720

LKGTSTSQDP IVSTEKSAVL HKLTTGATET SRTEVASSRR TSIPGPDHST ESPDISTEVI

        6730       6740       6750       6760       6770       6780

PSLPISLGIT ESSNMTIITR TGPPLGSTSQ GTFTLDTPTT SSRAGTHSMA TQEFPHSEMT

        6790       6800       6810       6820       6830       6840

TVMNKDPEIL SWTIPPSIEK TSFSSSLMPS PAMTSPPVSS TLPKTIHTTP SPMTSLLTPS

        6850       6860       6870       6880       6890       6900

LVMTTDTLGT SPEPTTSSPP NLSSTSHVIL TTDEDTTAIE AMHPSTSTAA TNVETTCSGH

        6910       6920       6930       6940       6950       6960

GSQSSVLTDS EKTKATAPMD TTSTMGHTTV STSMSVSSET TKIKRESTYS LTPGLRETSI

        6970       6980       6990       7000       7010       7020

SQNASFSTDT SIVLSEVPTG TTAEVSRTEV TSSGRTSIPG PSQSTVLPEI STRTMTRLFA
```

32

```
        7030       7040       7050       7060       7070       7080
SPTMTESAEM TIPTQTGPSG STSQDTLTLD TSTTKSQAKT HSTLTQRFPH SEMTTLMSRG

        7090       7100       7110       7120       7130       7140
PGDMSWQSSP SLENPSSLPS LLSLPATTSP PPISSTLPVT ISSSPLPVTS LLTSSPVTTT

        7150       7160       7170       7180       7190       7200
DMLHTSPELV TSSPPKLSHT SDERLTTGKD TTNTEAVHPS TNTAASNVEI PSFGHESPSS

        7210       7220       7230       7240       7250       7260
ALADSETSKA TSPMFITSTQ EDTTVAISTP HFLETSRIQK ESISSLSPKL RETGSSVETS

        7270       7280       7290       7300       7310       7320
SAIETSAVLS EVSIGATTEI SRTEVTSSSR TSISGSAEST MLPEISTTRK IIKFPTSPIL

        7330       7340       7350       7360       7370       7380
AESSEMTIKT QTSPPGSTSE STFTLDTSTT PSLVITHSTM TQRLPHSEIT TLVSRGAGDV

        7390       7400       7410       7420       7430       7440
PRPSSLPVEE TSPPSSQLSL SAMISPSPVS STLPASSHSS SASVTSPLTP GQVKTTEVLD

        7450       7460       7470       7480       7490       7500
ASAEPETSSP PSLSSTSVEI LATSEVTTDT EKIHPFPNTA VTKVGTSSSG HESPSSVLPD

        7510       7520       7530       7540       7550       7560
SETTKATSAM GTISIMGDTS VSTLTPALSN TRKIQSEPAS SLTTRLRETS TSEETSLATE

        7570       7580       7590       7600       7610       7620
ANTVLSKVST GATTEVSRTE AISFSRTSMS GPEQSTMSQD ISIGTIPRIS ASSVLTESAK

        7630       7640       7650       7660       7670       7680
MTITTQTGPS ESTLESTLNL NTATTPSWVE THSIVIQGFP HPEMTTSMGR GPGGVSWPSP

        7690       7700       7710       7720       7730       7740
PFVKETSPPS SPLSLPAVTS PHPVSTTFLA HIPPSPLPVT SLLTSGPATT TDILGTSTEP

        7750       7760       7770       7780       7790       7800
GTSSSSSLST TSHERLTTYK DTAHTEAVHP STNTGGTNVA TTSSGYKSQS SVLADSSPMC

        7810       7820       7830       7840       7850       7860
TTSTMGDTSV LTSTPAFLET RRIQTELASS LTPGLRESSG SEGTSSGTKM STVLSKVPTG
```

33

```
        7870       7880       7890       7900       7910       7920

ATTEISKEDV TSIPGPAQST ISPDISTRTV SWFSTSPVMT ESAEITMNTH TSPLGATTQG

        7930       7940       7950       7960       7970       7980

TSTLATSSTT SLTMTHSTIS QGFSHSQMST LMRRGPEDVS WMSPPLLEKT RPSFSLMSSP

        7990       8000       8010       8020       8030       8040

ATTSPSPVSS TLPESISSSP LPVTSLLTSG LAKTTDMLHK SSEPVTNSPA NLSSTSVEIL

        8050       8060       8070       8080       8090       8100

ATSEVTTDTE KTHPSSNRTV TDVGTSSSGH ESTSFVLADS QTSKVTSPMV ITSTMEDTSV

        8110       8120       8130       8140       8150       8160

STSTPGFFET SRIQTEPTSS LTLGLRKTSS SEGTSLATEM STVLSGVPTG ATAEVSRTEV

        8170       8180       8190       8200       8210       8220

TSSSRTSISG FAQLTVSPET STETITRLPT SSIMTESAEM MIKTQTDPPG STPESTHTVD

        8230       8240       8250       8260       8270       8280

ISTTPNWVET HSTVTQRFSH SEMTTLVSRS PGDMLWPSQS SVEETSSASS LLSLPATTSP

        8290       8300       8310       8320       8330       8340

SPVSSTLVED FPSASLPVTS LLTPGLVITT DRMGISREPG TSSTSNLSST SHERLTTLED

        8350       8360       8370       8380       8390       8400

TVDTEDMQPS THTAVTNVRT SISGHESQSS VLSDSETPKA TSPMGTTYTM GETSVSISTS

        8410       8420       8430       8440       8450       8460

DFFETSRIQI EPTSSLTSGL RETSSSERIS SATEGSTVLS EVPSGATTEV SRTEVISSRG

        8470       8480       8490       8500       8510       8520

TSMSGPDQFT ISPDISTEAI TRLSTSPIMT ESAESAITIE TGSPGATSEG TLTLDTSTTT

        8530       8540       8550       8560       8570       8580

FWSGTHSTAS PGFSHSEMTT LMSRTPGDVP WPSLPSVEEA SSVSSSLSSP AMTSTSFFSA

        8590       8600       8610       8620       8630       8640

LPESISSSPH PVTALLTLGP VKTTDMLRTS SEPETSSPPN LSSTSAEILA TSEVTKDREK

        8650       8660       8670       8680       8690       8700

IHPSSNTPVV NVGTVIYKHL SPSSVLADLV TTKPTSPMAT TSTLGNTSVS TSTPAFPETM
```

```
        8710       8720       8730       8740       8750       8760
MTQPTSSLTS GLREISTSQE TSSATERSAS LSGMPTGATT KVSRTEALSL GRTSTPGPAQ

        8770       8780       8790       8800       8810       8820
STISPEISTE TITRISTPLT TTGSAEMTIT PKTGHSGASS QGTFTLDTSS RASWPGTHSA

        8830       8840       8850       8860       8870       8880
ATHRSPHSGM TTPMSRGPED VSWPSRPSVE KTSPPSSLVS LSAVTSPSPL YSTPSESSHS

        8890       8900       8910       8920       8930       8940
SPLRVTSLFT PVMMKTTDML DTSLEPVTTS PPSMNITSDE SLATSKATME TEAIQLSENT

        8950       8960       8970       8980       8990       9000
AVTQMGTISA RQEFYSSYPG LPEPSKVTSP VVTSSTIKDI VSTTIPASSE ITRIEMESTS

        9010       9020       9030       9040       9050       9060
TLTPTPRETS TSQEIHSATK PSTVPYKALT SATIEDSMTQ VMSSSRGPSP DQSTMSQDIS

        9070       9080       9090       9100       9110       9120
SEVITRLSTS PIKAESTEMT ITTQTGSPGA TSRGTLTLDT STTFMSGTHS TASQGFSHSQ

        9130       9140       9150       9160       9170       9180
MTALMSRTPG DVPWLSHPSV EEASSASFSL SSPVMTSSSP VSSTLPDSIH SSSLPVTSLL

        9190       9200       9210       9220       9230       9240
TSGLVKTTEL LGTSSEPETS SPPNLSSTSA EILATTEVTT DTEKLEMTNV VTSGYTHESP

        9250       9260       9270       9280       9290       9300
SSVLADSVTT KATSSMGITY PTGDTNVLTS TPAFSDTSRI QTKSKLSLTP GLMETSISEE

        9310       9320       9330       9340       9350       9360
TSSATEKSTV LSSVPTGATT EVSRTEAISS SRTSIPGPAQ STMSSDTSME TITRISTPLT

        9370       9380       9390       9400       9410       9420
RKESTDMAIT PKTGPSGATS QGTFTLDSSS TASWPGTHSA TTQRFPQSVV TTPMSRGPED

        9430       9440       9450       9460       9470       9480
VSWPSPLSVE KNSPPSSLVS SSSVTSPSPL YSTPSGSSHS SPVPVTSLFT SIMMKATDML

        9490       9500       9510       9520       9530       9540
DASLEPETTS APNMNITSDE SLATSKATTE TEAIHVFENT AASHVETTSA TEELYSSSPG
```

```
          9550       9560       9570       9580       9590       9600
     FSEPTKVISP VVTSSSIRDN MVSTTMPGSS GITRIEIESM SSLTPGLRET RTSQDITSST

          9610       9620       9630       9640       9650       9660
     ETSTVLYKMS SGATPEVSRT EVMPSSRTSI PGPAQSTMSL DISDEVVTRL STSPIMTESA

          9670       9680       9690       9700       9710       9720
     EITITTQTGY SLATSQVTLP LGTSMTFLSG THSTMSQGLS HSEMTNLMSR GPESLSWTSP

          9730       9740       9750       9760       9770       9780
     RFVETTRSSS SLTSLPLTTS LSPVSSTLLD SSPSSPLPVT SLILPGLVKT TEVLDTSSEP

          9790       9800       9810       9820       9830       9840
     KTSSSPNLSS TSVEIPATSE IMTDTEKIHP SSNTAVAKVR TSSSVHESHS SVLADSETTI

          9850       9860       9870       9880       9890       9900
     TIPSMGITSA VDDTTVFTSN PAFSETRRIP TEPTFSLTPG FRETSTSEET TSITETSAVL

          9910       9920       9930       9940       9950       9960
     YGVPTSATTE VSMTEIMSSN RTHIPDSDQS TMSPDIITEV ITRLSSSSMM SESTQMTITT

          9970       9980       9990      10000      10010      10020
     QKSSPGATAQ STLTLATTTA PLARTHSTVP PRFLHSEMTT LMSRSPENPS WKSSPFVEKT

         10030      10040      10050      10060      10070      10080
     SSSSSLLSLP VTTSPSVSST LPQSIPSSSF SVTSLLTPGM VKTTDTSTEP GTSLSPNLSG

         10090      10100      10110      10120      10130      10140
     TSVEILAASE VTTDTEKIHP SSSMAVTNVG TTSSGHELYS SVSIHSEPSK ATYPVGTPSS

         10150      10160      10170      10180      10190      10200
     MAETSISTSM PANFETTGFE AEPFSHLTSG FRKTNMSLDT SSVTPTNTPS SPGSTHLLQS

         10210      10220      10230      10240      10250      10260
     SKTDFTSSAK TSSPDWPPAS QYTEIPVDII TPFNASPSIT ESTGITSFPE SRFTMSVTES

         10270      10280      10290      10300      10310      10320
     THHLSTDLLP SAETISTGTV MPSLSEAMTS FATTGVPRAI SGSGSPFSRT ESGPGDATLS

         10330      10340      10350      10360      10370      10380
     TIAESLPSST PVPFSSSTFT TTDSSTIPAL HEITSSSATP YRVDTSLGTE SSTTEGRLVM
```

|  10390 | 10400 | 10410 | 10420 | 10430 | 10440 |
|---|---|---|---|---|---|
| VSTLDTSSQP | GRTSSTPILD | TRMTESVELG | TVTSAYQVPS | LSTRLTRTDG | IMEHITKIPN |

|  10450 | 10460 | 10470 | 10480 | 10490 | 10500 |
|---|---|---|---|---|---|
| EAAHRGTIRP | VKGPQTSTSP | ASPKGLHTGG | TKRMETTTTA | LKTTTTALKT | TSRATLTTSV |

|  10510 | 10520 | 10530 | 10540 | 10550 | 10560 |
|---|---|---|---|---|---|
| YTPTLGTLTP | LNASRQMAST | ILTEMMITTP | YVFPDVPETT | SSLATSLGAE | TSTALPRTTP |

|  10570 | 10580 | 10590 | 10600 | 10610 | 10620 |
|---|---|---|---|---|---|
| SVLNRESETT | ASLVSRSGAE | RSPVIQTLDV | SSSEPDTTAS | WVIHPAETIP | TVSKTTPNFF |

|  10630 | 10640 | 10650 | 10660 | 10670 | 10680 |
|---|---|---|---|---|---|
| HSELDTVSST | ATSHGADVSS | AIPTNISPSE | LDALTPLVTI | SGTDTSTTFP | TLTKSPHETE |

|  10690 | 10700 | 10710 | 10720 | 10730 | 10740 |
|---|---|---|---|---|---|
| TRTTWLTHPA | ETSSTIPRTI | PNFSHHESDA | TPSIATSPGA | ETSSAIPIMT | VSPGAEDLVT |

|  10750 | 10760 | 10770 | 10780 | 10790 | 10800 |
|---|---|---|---|---|---|
| SQVTSSGTDR | NMTIPTLTLS | PGEPKTIASL | VTHPEAQTSS | AIPTSTISPA | VSRLVTSMVT |

|  10810 | 10820 | 10830 | 10840 | 10850 | 10860 |
|---|---|---|---|---|---|
| SLAAKTSTTN | RALTNSPGEP | ATTVSLVTHP | AQTSPTVPWT | TSIFFHSKSD | TTPSMTTSHG |

|  10870 | 10880 | 10890 | 10900 | 10910 | 10920 |
|---|---|---|---|---|---|
| AESSSAVPTP | TVSTEVPGVV | TPLVTSSRAV | ISTTIPILTL | SPGEPETTPS | MATSHGEEAS |

|  10930 | 10940 | 10950 | 10960 | 10970 | 10980 |
|---|---|---|---|---|---|
| SAIPTPTVSP | GVPGVVTSLV | TSSRAVTSTT | IPILTFSLGE | PETTPSMATS | HGTEAGSAVP |

|  10990 | 11000 | 11010 | 11020 | 11030 | 11040 |
|---|---|---|---|---|---|
| TVLPEVPGMV | TSLVASSRAV | TSTTLPTLTL | SPGEPETTPS | MATSHGAEAS | STVPTVSPEV |

|  11050 | 11060 | 11070 | 11080 | 11090 | 11100 |
|---|---|---|---|---|---|
| PGVVTSLVTS | SSGVNSTSIP | TLILSPGELE | TTPSMATSHG | AEASSAVPTP | TVSPGVSGVV |

|  11110 | 11120 | 11130 | 11140 | 11150 | 11160 |
|---|---|---|---|---|---|
| TPLVTSSRAV | TSTTIPILTL | SSSEPETTPS | MATSHGVEAS | SAVLTVSPEV | PGMVTSLVTS |

|  11170 | 11180 | 11190 | 11200 | 11210 | 11220 |
|---|---|---|---|---|---|
| SRAVTSTTIP | TLTISSDEPE | TTTSLVTHSE | AKMISAIPTL | AVSPTVQGLV | TSLVTSSGSE |

```
        11230      11240      11250      11260      11270      11280
TSAFSNLTVA SSQPETIDSW VAHPGTEASS VVPTLTVSTG EPFTNISLVT HPAESSSTLP

        11290      11300      11310      11320      11330      11340
RTTSRFSHSE LDTMPSTVTS PEAESSSAIS TTISPGIPGV LTSLVTSSGR DISATFPTVP

        11350      11360      11370      11380      11390      11400
ESPHESEATA SWVTHPAVTS TTVPRTTPNY SHSEPDTTPS IATSPGAEAT SDFPTITVSP

        11410      11420      11430      11440      11450      11460
DVPDMVTSQV TSSGTDTSIT IPTLTLSSGE PETTTSFITY SETHTSSAIP TLPVSPGASK

        11470      11480      11490      11500      11510      11520
MLTSLVISSG TDSTTTFPTL TETPYEPETT AIQLIHPAET NTMVPKTTPK FSHSKSDTTL

        11530      11540      11550      11560      11570      11580
PVAITSPGPE ASSAVSTTTI SPDMSDLVTS LVPSSGTDTS TTFPTLSETP YEPETTVTWL

        11590      11600      11610      11620      11630      11640
THPAETSTTV SGTIPNFSHR GSDTAPSMVT SPGVDTRSGV PTTTIPPSIP GVVTSQVTSS

        11650      11660      11670      11680      11690      11700
ATDTSTAIPT LTPSPGEPET TASSATHPGT QTGFTVPIRT VPSSEPDTMA SWVTHPPQTS

        11710      11720      11730      11740      11750      11760
TPVSRTTSSF SHSSPDATPV MATSPRTEAS SAVLTTISPG APEMVTSQIT SSGAATSTTV

        11770      11780      11790      11800      11810      11820
PTLTHSPGMP ETTALLSTHP RTGTSKTFPA STVFPQVSET TASLTIRPGA ETSTALPTQT

        11830      11840      11850      11860      11870      11880
TSSLFTLLVT GTSRVDLSPT ASPGVSAKTA PLSTHPGTET STMIPTSTLS LGLLETTGLL

        11890      11900      11910      11920      11930      11940
ATSSSAETST STLTLTVSPA VSGLSSASIT TDKPQTVTSW NTETSPSVTS VGPPEFSRTV

        11950      11960      11970      11980      11990      12000
TGTTMTLIPS EMPTPPKTSH GEGVSPTTIL RTTMVEATNL ATTGSSPTVA KTTTTFNTLA

        12010      12020      12030      12040      12050      12060
GSLFTPLTTP GMSTLASESV TSRTSYNHRS WISTTSSYNR RYWTPATSTP VTSTFSPGIS
```

|       |       |       |       |       |       |
|-------|-------|-------|-------|-------|-------|
| 12070 | 12080 | 12090 | 12100 | 12110 | 12120 |

TSSIPSSTAA TVPFMVPFTL NFTITNLQYE EDMRHPGSRK FNATERELQG LLKPLFRNSS

|       |       |       |       |       |       |
|-------|-------|-------|-------|-------|-------|
| 12130 | 12140 | 12150 | 12160 | 12170 | 12180 |

LEYLYSGCRL ASLRPEKDSS AMAVDAICTH RPDPEDLGLD RERLYWELSN LTNGIQELGP

|       |       |       |       |       |       |
|-------|-------|-------|-------|-------|-------|
| 12190 | 12200 | 12210 | 12220 | 12230 | 12240 |

YTLDRNSLYV NGFTHRSSMP TTSTPGTSTV DVGTSGTPSS SPSPTAAGPL LMPFTLNFTI

|       |       |       |       |       |       |
|-------|-------|-------|-------|-------|-------|
| 12250 | 12260 | 12270 | 12280 | 12290 | 12300 |

TNLQYEEDMR RTGSRKFNTM ESVLQGLLKP LFKNTSVGPL YSGCRLTLLR PEKDGAATGV

|       |       |       |       |       |       |
|-------|-------|-------|-------|-------|-------|
| 12310 | 12320 | 12330 | 12340 | 12350 | 12360 |

DAICTHRLDP KSPGLNREQL YWELSKLTND IEELGPYTLD RNSLYVNGFT HQSSVSTTST

|       |       |       |       |       |       |
|-------|-------|-------|-------|-------|-------|
| 12370 | 12380 | 12390 | 12400 | 12410 | 12420 |

PGTSTVDLRT SGTPSSLSSP TIMAAGPLLV PFTLNFTITN LQYGEDMGHP GSRKFNTTER

|       |       |       |       |       |       |
|-------|-------|-------|-------|-------|-------|
| 12430 | 12440 | 12450 | 12460 | 12470 | 12480 |

VLQGLLGPIF KNTSVGPLYS GCRLTSLRSE KDGAATGVDA ICIHHLDPKS PGLNRERLYW

|       |       |       |       |       |       |
|-------|-------|-------|-------|-------|-------|
| 12490 | 12500 | 12510 | 12520 | 12530 | 12540 |

ELSQLTNGIK ELGPYTLDRN SLYVNGFTHR TSVPTTSTPG TSTVDLGTSG TPFSLPSPAT

|       |       |       |       |       |       |
|-------|-------|-------|-------|-------|-------|
| 12550 | 12560 | 12570 | 12580 | 12590 | 12600 |

AGPLLVLFTL NFTITNLKYE EDMHRPGSRK FNTTERVLQT LLGPMFKNTS VGLLYSGCRL

|       |       |       |       |       |       |
|-------|-------|-------|-------|-------|-------|
| 12610 | 12620 | 12630 | 12640 | 12650 | 12660 |

TLLRSEKDGA ATGVDAICTH RLDPKSPGLD REQLYWELSQ LTNGIKELGP YTLDRNSLYV

|       |       |       |       |       |       |
|-------|-------|-------|-------|-------|-------|
| 12670 | 12680 | 12690 | 12700 | 12710 | 12720 |

NGFTHWIPVP TSSTPGTSTV DLGSGTPSSL PSPTAAGPLL VPFTLNFTIT NLQYEEDMHH

|       |       |       |       |       |       |
|-------|-------|-------|-------|-------|-------|
| 12730 | 12740 | 12750 | 12760 | 12770 | 12780 |

PGSRKFNTTE RVLQGLLGPM FKNTSVGLLY SGCRLTLLRS EKDGAATGVD AICTHRLDPK

|       |       |       |       |       |       |
|-------|-------|-------|-------|-------|-------|
| 12790 | 12800 | 12810 | 12820 | 12830 | 12840 |

SPGVDREQLY WELSQLTNGI KELGPYTLDR NSLYVNGFTH QTSAPNTSTP GTSTVDLGTS

|       |       |       |       |       |       |
|-------|-------|-------|-------|-------|-------|
| 12850 | 12860 | 12870 | 12880 | 12890 | 12900 |

GTPSSLPSPT SAGPLLVPFT LNFTITNLQY EEDMRHPGSR KFNTTERVLQ GLLKPLFKST

```
        12910      12920      12930      12940      12950      12960

SVGPLYSGCR LTLLRSEKDG AATGVDAICT HRLDPKSPGV DREQLYWELS QLTNGIKELG

        12970      12980      12990      13000      13010      13020

PYTLDRNSLY VNGFTHQTSA PNTSTPGTST VDLGTSGTPS SLPSPTSAGP LLVPFTLNFT

        13030      13040      13050      13060      13070      13080

ITNLQYEEDM HHPGSRKFNT TERVLQGLLG PMFKNTSVGL LYSGCRLTLL RPEKNGAATG

        13090      13100      13110      13120      13130      13140

MDAICSHRLD PKSPGLNREQ LYWELSQLTH GIKELGPYTL DRNSLYVNGF THRSSVAPTS

        13150      13160      13170      13180      13190      13200

TPGTSTVDLG TSGTPSSLPS PTTAVPLLVP FTLNFTITNL QYGEDMRHPG SRKFNTTERV

        13210      13220      13230      13240      13250      13260

LQGLLGPLFK NSSVGPLYSG CRLISLRSEK DGAATGVDAI CTHHLNPQSP GLDREQLYWQ

        13270      13280      13290      13300      13310      13320

LSQMTNGIKE LGPYTLDRNS LYVNGFTHRS SGLTTSTPWT STVDLGTSGT PSPVPSPTTA

        13330      13340      13350      13360      13370      13380

GPLLVPFTLN FTITNLQYEE DMHRPGSRKF NTTERVLQGL LSPIFKNSSV GPLYSGCRLT

        13390      13400      13410      13420      13430      13440

SLRPEKDGAA TGMDAVCLYH PNPKRPGLDR EQLYWELSQL THNITELGPY SLDRDSLYVN

        13450      13460      13470      13480      13490      13500

GFTHQNSVPT TSTPGTSTVY WATTGTPSSF PGHTEPGPLL IPFTFNFTIT NLHYEENMQH

        13510      13520      13530      13540      13550      13560

PGSRKFNTTE RVLQGLLKPL FKNTSVGPLY SGCRLTSLRP EKDGAATGMD AVCLYHPNPK

        13570      13580      13590      13600      13610      13620

RPGLDREQLY WELSQLTHNI TELGPYSLDR DSLYVNGFTH QNSVPTTSTP GTSTVYWATT

        13630      13640      13650      13660      13670      13680

GTPSSFPGHT EPGPLLIPFT FNFTITNLHY EENMQHPGSR KFNTTERVLQ GLLKPLFKNT

        13690      13700      13710      13720      13730      13740

SVGPLYSGCR LTLLRPEKHE AATGVDTICT HRVDPIGPGL DRERLYWELS QLTNSITELG
```

40

```
        13750       13760       13770       13780       13790       13800
   PYTLDRDSLY  VNGFNPRSSV  PTTSTPGTST  VHLATSGTPS  SLPGHTAPVP  LLIPFTLNFT

        13810       13820       13830       13840       13850       13860
   ITNLHYEENM  QHPGSRKFNT  TERVLQGLLK  PLFKNTSVGP  LYSGCRLTLL  RPEKHEAATG

        13870       13880       13890       13900       13910       13920
   VDTICTHRVD  PIGPGLXXEX  LYWELSXLTX  XIXELGPYTL  DRXSLYVNGF  THXXSXPTTS

        13930       13940       13950       13960       13970       13980
   TPGTSTVXXG  TSGTPSSXPX  XTSAGPLLVP  FTLNFTITNL  QYEEDMHHPG  SRKFNTTERV

        13990       14000       14010       14020       14030       14040
   LQGLLGPMFK  NTSVGLLYSG  CRLTLLRPEK  NGAATGMDAI  CSHRLDPKSP  GLDREQLYWE

        14050       14060       14070       14080       14090       14100
   LSQLTHGIKE  LGPYTLDRNS  LYVNGFTHRS  SVAPTSTPGT  STVDLGTSGT  PSSLPSPTTA

        14110       14120       14130       14140       14150       14160
   VPLLVPFTLN  FTITNLQYGE  DMRHPGSRKF  NTTERVLQGL  LGPLFKNSSV  GPLYSGCRLI

        14170       14180       14190       14200       14210       14220
   SLRSEKDGAA  TGVDAICTHH  LNPQSPGLDR  EQLYWQLSQM  TNGIKELGPY  TLDRNSLYVN

        14230       14240       14250       14260       14270       14280
   GFTHRSSGLT  TSTPWTSTVD  LGTSGTPSPV  PSPTTAGPLL  VPFTLNFTIT  NLQYEEDMHR

        14290       14300       14310       14320       14330       14340
   PGSRKFNATE  RVLQGLLSPI  FKNSSVGPLY  SGCRLTSLRP  EKDGAATGMD  AVCLYHPNPK

        14350       14360       14370       14380       14390       14400
   RPGLDREQLY  WELSQLTHNI  TELGPYSLDR  DSLYVNGFTH  QSSMTTTRTP  DTSTMHLATS

        14410       14420       14430       14440       14450       14460
   RTPASLSGPT  TASPLLVLFT  INCTITNLQY  EEDMRRTGSR  KFNTMESVLQ  GLLKPLFKNT

        14470       14480       14490       14500       14510       14520
   SVGPLYSGCR  LTLLRPKKDG  AATGVDAICT  HRLDPKSPGL  NREQLYWELS  KLTNDIEELG

        14530       14540       14550       14560       14570       14580
   PYTLDRNSLY  VNGFTHQSSV  STTSTPGTST  VDLRTSGTPS  SLSSPTIMXX  XPLLXPFTXN
```

```
            14590        14600        14610        14620        14630        14640

XTITNLXXXX XMXXPGSRKF NTTERVLQGL LRPLFKNTSV SSLYSGCRLT LLRPEKDGAA

            14650        14660        14670        14680        14690        14700

TRVDAACTYR PDPKSPGLDR EQLYWELSQL THSITELGPY TLDRVSLYVN GFNPRSSVPT

            14710        14720        14730        14740        14750        14760

TSTPGTSTVH LATSGTPSSL PGHTXXXPLL XPFTXNXTIT NLXXXXXMXX PGSRKFNTTE

            14770        14780        14790        14800        14810        14820

RVLQGLLKPL FRNSSLEYLY SGCRLASLRP EKDSSAMAVD AICTHRPDPE DLGLDRERLY

            14830        14840        14850        14860        14870        14880

WELSNLTNGI QELGPYTLDR NSLYVNGFTH RSSGLTTSTP WTSTVDLGTS GTPSPVPSPT

            14890        14900        14910        14920        14930        14940

TAGPLLVPFT LNFTITNLQY EEDMHRPGSR RFNTTERVLQ GLLTPLFKNT SVGPLYSGCR

            14950        14960        14970        14980        14990        15000

LTLLRPEKQE AATGVDTICT HRVDPIGPGL DRERLYWELS QLTNSITELG PYTLDRDSLY

            15010        15020        15030        15040        15050        15060

VNGFNPWSSV PTTSTPGTST VHLATSGTPS SLPGHTAPVP LLIPFTLNFT ITDLHYEENM

            15070        15080        15090        15100        15110        15120

QHPGSRKFNT TERVLQGLLK PLFKSTSVGP LYSGCRLTLL RPEKHGAATG VDAICTLRLD

            15130        15140        15150        15160        15170        15180

PTGPGLDRER LYWELSQLTN SVTELGPYTL DRDSLYVNGF THRSSVPTTS IPGTSAVHLE

            15190        15200        15210        15220        15230        15240

TSGTPASLPG HTAPGPLLVP FTLNFTITNL QYEEDMRHPG SRKFSTTERV LQGLLKPLFK

            15250        15260        15270        15280        15290        15300

NTSVSSLYSG CRLTLLRPEK DGAATRVDAV CTHRPDPKSP GLDRERLYWK LSQLTHGITE

            15310        15320        15330        15340        15350        15360

LGPYTLDRHS LYVNGFTHQS SMTTTRTPDT STMHLATSRT PASLSGPTTA SPLLVLFTIN

            15370        15380        15390        15400        15410        15420

FTITNLRYEE NMHHPGSRKF NTTERVLQGL LRPVFKNTSV GPLYSGCRLT TLRPKKDGAA
```

|  |  |  |  |  |  |
|---|---|---|---|---|---|
| TKVDAICTYR | PDPKSPGLDR | EQLYWELSQL | THSITELGPY | TQDRDSLYVN | GFTHRSSVPT |
| TSIPGTSAVH | LETSGTPASL | PGHTAPGPLL | VPFTLNFTIT | NLQYEEDMRH | PGSRKFNTTE |
| RVLQGLLKPL | FKSTSVGPLY | SGCRLTLLRP | EKRGAATGVD | TICTHRLDPL | NPGLDREQLY |
| WELSKLTRGI | IELGPYLLDR | GSLYVNGFTH | RTSVPTTSTP | GTSTVDLGTS | GTPFSLPSPA |
| XXXPLLXPFT | XNXTITNLXX | XXXMXXPGSR | KFNTTERVLQ | TLLGPMFKNT | SVGLLYSGCR |
| LTLLRSEKDG | AATGVDAICT | HRLDPKSPGV | DREQLYWELS | QLTNGIKELG | PYTLDRNSLY |
| VNGFTHWIPV | PTSSTPGTST | VDLGSGTPSS | LPSPTTAGPL | LVPFTLNFTI | TNLKYEEDMH |
| CPGSRKFNTT | ERVLQSLLGP | MFKNTSVGPL | YSGCRLTLLR | SEKDGAATGV | DAICTHRLDP |
| KSPGVDREQL | YWELSQLTNG | IKELGPYTLD | RNSLYVNGFT | HQTSAPNTST | PGTSTVDLGT |
| SGTPSSLPSP | TXXXPLLXPF | TXNXTITNLX | XXXXMXXPGS | RKFNTTEXVL | QGLLXPXFKN |
| XSVGXLYSGC | RLTXLRXEKX | GAATGXDAIC | XHXXXPKXPG | LXXEXLYWEL | SXLTXXIXEL |
| GPYTLDRXSL | YVNGFTHWIP | VPTSSTPGTS | TVDLGSGTPS | SLPSPTTAGP | LLVPFTLNFT |
| ITNLKYEEDM | HCPGSRKFNT | TERVLQSLLG | PMFKNTSVGP | LYSGCRLTSL | RSEKDGAATG |
| VDAICTHRVD | PKSPGVDREQ | LYWELSQLTN | GIKELGPYTL | DRNSLYVNGF | THQTSAPNTS |

TPGTSTVXXG TSGTPSSXPX XTSAGPLLVP FTLNFTITNL QYEEDMHHPG SRKFNTTERV

LQGLLGPMFK NTSVGLLYSG CRLTLLRPEK NGATTGMDAI CTHRLDPKSP GLXXEXLYWE

LSXLTXXIXE LGPYTLDRXS LYVNGFTHXX SXPTTSTPGT STVXXGTSGT PSSXPXXTXX

XPLLXPFTXN XTITNLXXXX XMXXPGSRKF NTTERVLQGL LKPLFRNSSL EYLYSGCRLA

SLRPEKDSSA MAVDAICTHR PDPEDLGLDR ERLYWELSNL TNGIQELGPY TLDRNSLYVN

GFTHRSSMPT TSTPGTSTVD VGTSGTPSSS PSPTTAGPLL IPFTLNFTIT NLQYGEDMGH

PGSRKFNTTE RVLQGLLGPI FKNTSVGPLY SGCRLTSLRS EKDGAATGVD AICIHHLDPK

SPGLNRERLY WELSQLTNGI KELGPYTLDR NSLYVNGFTH RTSVPTTSTP GTSTVDLGTS

GTPFSLPSPA TAGPLLVLFT LNFTITNLKY EEDMHRPGSR KFNTTERVLQ TLLGPMFKNT

SVGLLYSGCR LTLLRSEKDG AATGVDAICT HRLDPKSPGL XXEXLYWELS XLTXXIXELG

PYTLDRXSLY VNGFTHXXSX PTTSTPGTST VXXGTSGTPS SXPXXTXXXP LLXPFTXNXT

ITNLXXXXXM XXPGSRKFNT TERVLQGLLR PVFKNTSVGP LYSGCRLTLL RPKKDGAATK

VDAICTYRPD PKSPGLDREQ LYWELSQLTH SITELGPYTQ DRDSLYVNGF THRSSVPTTS

IPGTSAVHLE TTGTPSSFPG HTEPGPLLIP FTFNFTITNL RYEENMQHPG SRKFNTTERV

| 17110 | 17120 | 17130 | 17140 | 17150 | 17160 |
|-------|-------|-------|-------|-------|-------|
| LQGLLTPLFK | NTSVGPLYSG | CRLTLLRPEK | QEAATGVDTI | CTHRVDPIGP | GLDRERLYWE |

| 17170 | 17180 | 17190 | 17200 | 17210 | 17220 |
|-------|-------|-------|-------|-------|-------|
| LSQLTNSITE | LGPYTLDRDS | LYVDGFNPWS | SVPTTSTPGT | STVHLATSGT | PSPLPGHTAP |

| 17230 | 17240 | 17250 | 17260 | 17270 | 17280 |
|-------|-------|-------|-------|-------|-------|
| VPLLIPFTLN | FTITDLHYEE | NMQHPGSRKF | NTTERVLQGL | LKPLFKSTSV | GPLYSGCRLT |

| 17290 | 17300 | 17310 | 17320 | 17330 | 17340 |
|-------|-------|-------|-------|-------|-------|
| LLRPEKHGAA | TGVDAICTLR | LDPTGPGLDR | ERLYWELSQL | TNSITELGPY | TLDRDSLYVN |

| 17350 | 17360 | 17370 | 17380 | 17390 | 17400 |
|-------|-------|-------|-------|-------|-------|
| GFNPWSSVPT | TSTPGTSTVH | LATSGTPSSL | PGHTTAGPLL | VPFTLNFTIT | NLKYEEDMHC |

| 17410 | 17420 | 17430 | 17440 | 17450 | 17460 |
|-------|-------|-------|-------|-------|-------|
| PGSRKFNTTE | RVLQSLHGPM | FKNTSVGPLY | SGCRLTLLRS | EKDGAATGVD | AICTHRLDPK |

| 17470 | 17480 | 17490 | 17500 | 17510 | 17520 |
|-------|-------|-------|-------|-------|-------|
| SPGLXXEXLY | WELSXLTXXI | XELGPYTLDR | XSLYVNGFTH | XXSXPTTSTP | GTSTVXXGTS |

| 17530 | 17540 | 17550 | 17560 | 17570 | 17580 |
|-------|-------|-------|-------|-------|-------|
| GTPSSXPXXT | XXXPLLXPFT | XNXTITNLXX | XXXMXXPGSR | KFNTTEXVLQ | GLLXPXFKNX |

| 17590 | 17600 | 17610 | 17620 | 17630 | 17640 |
|-------|-------|-------|-------|-------|-------|
| SVGXLYSGCR | LTXLRXEKXG | AATGXDAICX | HXXXPKXPGL | XXEXLYWELS | XLTNSITELG |

| 17650 | 17660 | 17670 | 17680 | 17690 | 17700 |
|-------|-------|-------|-------|-------|-------|
| PYTLDRDSLY | VNGFTHRSSM | PTTSIPGTSA | VHLETSGTPA | SLPGHTAPGP | LLVPFTLNFT |

| 17710 | 17720 | 17730 | 17740 | 17750 | 17760 |
|-------|-------|-------|-------|-------|-------|
| ITNLQYEEDM | RHPGSRKFNT | TERVLQGLLK | PLFKSTSVGP | LYSGCRLTLL | RPEKRGAATG |

| 17770 | 17780 | 17790 | 17800 | 17810 | 17820 |
|-------|-------|-------|-------|-------|-------|
| VDTICTHRLD | PLNPGLXXEX | LYWELSXLTX | XIXELGPYTL | DRXSLYVNGF | THXXSXPTTS |

| 17830 | 17840 | 17850 | 17860 | 17870 | 17880 |
|-------|-------|-------|-------|-------|-------|
| TPGTSTVXXG | TSGTPSSXPX | XTXXXPLLXP | FTXNXTITNL | XXXXXMXXPG | SRKFNTTEXV |

| 17890 | 17900 | 17910 | 17920 | 17930 | 17940 |
|-------|-------|-------|-------|-------|-------|
| LQGLLXPXFK | NXSVGXLYSG | CRLTXLRXEK | XGAATGXDAI | CXHXXXPKXP | GLXXEXLYWE |

45

```
        17950      17960      17970      17980      17990      18000
   LSXLTXXIXE LGPYTLDRXS LYVNGFHPRS SVPTTSTPGT STVHLATSGT PSSLPGHTAP

        18010      18020      18030      18040      18050      18060
   VPLLIPFTLN FTITNLHYEE NMQHPGSRKF NTTERVLQGL LGPMFKNTSV GLLYSGCRLT

        18070      18080      18090      18100      18110      18120
   LLRPEKNGAA TGMDAICSHR LDPKSPGLXX EXLYWELSXL TXXIXELGPY TLDRXSLYVN

        18130      18140      18150      18160      18170      18180
   GFTHXXSXPT TSTPGTSTVX XGTSGTPSSX PXXTXXXPLL XPFTXNXTIT NLXXXXXMXX

        18190      18200      18210      18220      18230      18240
   PGSRKFNTTE XVLQGLLXPX FKNXSVGXLY SGCRLTXLRX EKXGAATGXD AICXHXXXPK

        18250      18260      18270      18280      18290      18300
   XPGLXXEXLY WELSXLTXXI XELGPYTLDR XSLYVNGFTH QNSVPTTSTP GTSTVYWATT

        18310      18320      18330      18340      18350      18360
   GTPSSFPGHT EPGPLLIPFT FNFTITNLHY EENMQHPGSR KFNTTERVLQ GLLTPLFKNT

        18370      18380      18390      18400      18410      18420
   SVGPLYSGCR LTLLRPEKQE AATGVDTICT HRVDPIGPGL XXEXLYWELS XLTXXIXELG

        18430      18440      18450      18460      18470      18480
   PYTLDRXSLY VNGFTHXXSX PTTSTPGTST VXXGTSGTPS SXPXXTXXXP LLXPFTXNXT

        18490      18500      18510      18520      18530      18540
   ITNLXXXXXM XXPGSRKFNT TEXVLQGLLX PXFKNXSVGX LYSGCRLTXL RXEKXGAATG

        18550      18560      18570      18580      18590      18600
   XDAICXHXXX PKXPGLXXEX LYWELSXLTX XIXELGPYTL DRXSLYVNGF THRSSVPTTS

        18610      18620      18630      18640      18650      18660
   SPGTSTVHLA TSGTPSSLPG HTAPVPLLIP FTLNFTITNL HYEENMQHPG SRKFNTTERV

        18670      18680      18690      18700      18710      18720
   LQGLLKPLFK STSVGPLYSG CRLTLLRPEK HGAATGVDAI CTLRLDPTGP GLXXEXLYWE

        18730      18740      18750      18760      18770      18780
   LSXLTXXIXE LGPYTLDRXS LYVNGFTHXX SXPTTSTPGT STVXXGTSGT PSSXPXXTXX
```

```
        18790       18800       18810       18820       18830       18840
XPLLXPFTXN  XTITNLXXXX  XMXXPGSRKF  NTTEXVLQGL  LXPXFKNXSV  GXLYSGCRLT

        18850       18860       18870       18880       18890       18900
XLRXEKXGAA  TGXDAICXHX  XXPKXPGLXX  EXLYWELSXL  TXXIXELGPY  TLDRXSLYVN

        18910       18920       18930       18940       18950       18960
GFTHRTSVPT  TSTPGTSTVH  LATSGTPSSL  PGHTAPVPLL  IPFTLNFTIT  NLQYEEDMHR

        18970       18980       18990       19000       19010       19020
PGSRKFNTTE  RVLQGLLSPI  FKNSSVGPLY  SGCRLTSLRP  EKDGAATGMD  AVCLYHPNPK

        19030       19040       19050       19060       19070       19080
RPGLDREQLY  CELSQLTHNI  TELGPYSLDR  DSLYVNGFTH  QNSVPTTSTP  GTSTVYWATT

        19090       19100       19110       19120       19130       19140
GTPSSFPGHT  XXXPLLXPFT  XNXTITNLXX  XXXMXXPGSR  KFNTTEXVLQ  GLLXPXFKNX

        19150       19160       19170       19180       19190       19200
SVGXLYSGCR  LTXLRXEKXG  AATGXDAICX  HXXXPKXPGL  XXEXLYWELS  XLTXXIXELG

        19210       19220       19230       19240       19250       19260
PYTLDRXSLY  VNGFTHWSSG  LTTSTPWTST  VDLGTSGTPS  PVPSPTTAGP  LLVPFTLNFT

        19270       19280       19290       19300       19310       19320
ITNLQYEEDM  HRPGSRKFNA  TERVLQGLLS  PIFKNTSVGP  LYSGCRLTLL  RPEKQEAATG

        19330       19340       19350       19360       19370       19380
VDTICTHRVD  PIGPGLXXEX  LYWELSXLTX  XIXELGPYTL  DRXSLYVNGF  THXXSXPTTS

        19390       19400       19410       19420       19430       19440
TPGTSTVXXG  TSGTPSSXPX  XTXXXPLLXP  FTXNXTITNL  XXXXXMXXPG  SRKFNTTEXV

        19450       19460       19470       19480       19490       19500
LQGLLXPXFK  NXSVGXLYSG  CRLTXLRXEK  XGAATGXDAI  CXHXXXPKXP  GLXXEXLYWE

        19510       19520       19530       19540       19550       19560
LSXLTXXIXE  LGPYTLDRXS  LYVNGFTHRS  FGLTTSTPWT  STVDLGTSGT  PSPVPSPTTA

        19570       19580       19590       19600       19610       19620
GPLLVPFTLN  FTITNLQYEE  DMHRPGSRKF  NTTERVLQGL  LTPLFRNTSV  SSLYSGCRLT
```

```
       19630      19640      19650      19660      19670      19680

LLRPEKDGAA TRVDAVCTHR PDPKSPGLXX EXLYWELSXL TXXIXELGPY TLDRXSLYVN

       19690      19700      19710      19720      19730      19740

GFTHXXSXPT TSTPGTSTVX XGTSGTPSSX PXXTXXXPLL XPFTXNXTIT NLXXXXXMXX

       19750      19760      19770      19780      19790      19800

PGSRKFNTTE XVLQGLLXPX FKNXSVGXLY SGCRLTXLRX EKXGAATGXD AICXHXXXPK

       19810      19820      19830      19840      19850      19860

XPGLXXEXLY WELSXLTXXI XELGPYTLDR XSLYVNGFTH WIPVPTSSTP GTSTVDLGSG

       19870      19880      19890      19900      19910      19920

TPSSLPSPTT AGPLLVPFTL NFTITNLQYG EDMGHPGSRK FNTTERVLQG LLGPIFKNTS

       19930      19940      19950      19960      19970      19980

VGPLYSGCRL TSLRSEKDGA ATGVDAICIH HLDPKSPGLX XEXLYWELSX LTXXIXELGP

       19990      20000      20010      20020      20030      20040

YTLDRXSLYV NGFTHXXSXP TTSTPGTSTV XXGTSGTPSS XPXXTXXXPL LXPFTXNXTI

       20050      20060      20070      20080      20090      20100

TNLXXXXXMX XPGSRKFNTT EXVLQGLLXP XFKNXSVGXL YSGCRLTXLR XEKXGAATGX

       20110      20120      20130      20140      20150      20160

DAICXHXXXP KXPGLXXEXL YWELSXLTXX IXELGPYTLD RXSLYVNGFT HQTFAPNTST

       20170      20180      20190      20200      20210      20220

PGTSTVDLGT SGTPSSLPSP TSAGPLLVPF TLNFTITNLQ YEEDMHHPGS RKFNTTERVL

       20230      20240      20250      20260      20270      20280

QGLLGPMFKN TSVGLLYSGC RLTLLRPEKN GAATRVDAVC THRPDPKSPG LXXEXLYWEL

       20290      20300      20310      20320      20330      20340

SXLTXXIXEL GPYTLDRXSL YVNGFTHXXS XPTTSTPGTS TVXXGTSGTP SSXPXXTAPV

       20350      20360      20370      20380      20390      20400

PLLIPFTLNF TITNLHYEEN MQHPGSRKFN TTERVLQGLL KPLFKSTSVG PLYSGCRLTL

       20410      20420      20430      20440      20450      20460

LRPEKHGAAT GVDAICTLRL DPTGPGLDRE RLYWELSQLT NSVTELGPYT LDRDSLYVNG
```

48

|       |       |       |       |       |       |
|-------|-------|-------|-------|-------|-------|
| 20470 | 20480 | 20490 | 20500 | 20510 | 20520 |

FTQRSSVPTT SIPGTSAVHL ETSGTPASLP GHTAPGPLLV PFTLNFTITN LQYEVDMRHP

|       |       |       |       |       |       |
|-------|-------|-------|-------|-------|-------|
| 20530 | 20540 | 20550 | 20560 | 20570 | 20580 |

GSRKFNTTER VLQGLLKPLF KSTSVGPLYS GCRLTLLRPE KRGAATGVDT ICTHRLDPLN

|       |       |       |       |       |       |
|-------|-------|-------|-------|-------|-------|
| 20590 | 20600 | 20610 | 20620 | 20630 | 20640 |

PGLDREQLYW ELSKLTRGII ELGPYLLDRG SLYVNGFTHR NFVPITSTPG TSTVHLGTSE

|       |       |       |       |       |       |
|-------|-------|-------|-------|-------|-------|
| 20650 | 20660 | 20670 | 20680 | 20690 | 20700 |

TPSSLPRPIV PGPLLVPFTL NFTITNLQYE EAMRHPGSRK FNTTERVLQG LLRPLFKNTS

|       |       |       |       |       |       |
|-------|-------|-------|-------|-------|-------|
| 20710 | 20720 | 20730 | 20740 | 20750 | 20760 |

IGPLYSSCRL TLLRPEKDKA ATRVDAICTH HPDPQSPGLN REQLYWELSQ LTHGITELGP

|       |       |       |       |       |       |
|-------|-------|-------|-------|-------|-------|
| 20770 | 20780 | 20790 | 20800 | 20810 | 20820 |

YTLDRDSLYV DGFTHWSPIP TTSTPGTSIV NLGTSGIPPS LPETTXXXPL LXPFTXNXTI

|       |       |       |       |       |       |
|-------|-------|-------|-------|-------|-------|
| 20830 | 20840 | 20850 | 20860 | 20870 | 20880 |

TNLXXXXXMX XPGSRKFNTT ERVLQGLLKP LFKSTSVGPL YSGCRLTLLR PEKDGVATRV

|       |       |       |       |       |       |
|-------|-------|-------|-------|-------|-------|
| 20890 | 20900 | 20910 | 20920 | 20930 | 20940 |

DAICTHRPDP KIPGLDRQQL YWELSQLTHS ITELGPYTLD RDSLYVNGFT QRSSVPTTST

|       |       |       |       |       |       |
|-------|-------|-------|-------|-------|-------|
| 20950 | 20960 | 20970 | 20980 | 20990 | 21000 |

PGTFTVQPET SETPSSLPGP TATGPVLLPF TLNFTITNLQ YEEDMHRPGS RKFNTTERVL

|       |       |       |       |       |       |
|-------|-------|-------|-------|-------|-------|
| 21010 | 21020 | 21030 | 21040 | 21050 | 21060 |

QGLLMPLFKN TSVSSLYSGC RLTLLRPEKD GAATRVDAVC THRPDPKSPG LDRERLYWKL

|       |       |       |       |       |       |
|-------|-------|-------|-------|-------|-------|
| 21070 | 21080 | 21090 | 21100 | 21110 | 21120 |

SQLTHGITEL GPYTLDRHSL YVNGFTHQSS MTTTRTPDTS TMHLATSRTP ASLSGPTTAS

|       |       |       |       |       |       |
|-------|-------|-------|-------|-------|-------|
| 21130 | 21140 | 21150 | 21160 | 21170 | 21180 |

PLLVLFTINF TITNLRYEEN MHHPGSRKFN TTERVLQGLL RPVFKNTSVG PLYSGCRLTL

|       |       |       |       |       |       |
|-------|-------|-------|-------|-------|-------|
| 21190 | 21200 | 21210 | 21220 | 21230 | 21240 |

LRPKKDGAAT KVDAICTYRP DPKSPGLDRE QLYWELSQLT HSITELGPYT LDRDSLYVNG

|       |       |       |       |       |       |
|-------|-------|-------|-------|-------|-------|
| 21250 | 21260 | 21270 | 21280 | 21290 | 21300 |

FTQRSSVPTT SIPGTPTVDL GTSGTPVSKP GPSAASPLLV LFTLNFTITN LRYEENMQHP

|  |  |  |  |  |  |
|---|---|---|---|---|---|
| 21310 | 21320 | 21330 | 21340 | 21350 | 21360 |
| GSRKFNTTER | VLQGLLRSLF | KSTSVGPLYS | GCRLTLLRPE | KDGTATGVDA | ICTHHPDPKS |
| 21370 | 21380 | 21390 | 21400 | 21410 | 21420 |
| PRLDREQLYW | ELSQLTHNIT | ELGHYALDND | SLFVNGFTHR | SSVSTTSTPG | TPTVYLGASK |
| 21430 | 21440 | 21450 | 21460 | 21470 | 21480 |
| TPASIFGPSA | ASHLLILFTL | NFTITNLRYE | ENMWPGSRKF | NTTERVLQGL | LRPLFKNTSV |
| 21490 | 21500 | 21510 | 21520 | 21530 | 21540 |
| GPLYSGSRLT | LLRPEKDGEA | TGVDAICTHR | PDPTGPGLDR | EQLYLELSQL | THSITELGPY |
| 21550 | 21560 | 21570 | 21580 | 21590 | 21600 |
| TLDRDSLYVN | GFTHRSSVPT | TSTGVVSEEP | FTLNFTINNL | RYMADMGQPG | SLKFNITDNV |
| 21610 | 21620 | 21630 | 21640 | 21650 | 21660 |
| MKHLLSPLFQ | RSSLGARYTG | CRVIALRSVK | NGAETRVDLL | CTYLQPLSGP | GLPIKQVFHE |
| 21670 | 21680 | 21690 | 21700 | 21710 | 21720 |
| LSQQTHGITR | LGPYSLDKDS | LYLNGYNEPG | LDEPPTTPKP | ATTFLPPLSE | ATTAMGYHLK |
| 21730 | 21740 | 21750 | 21760 | 21770 | 21780 |
| TLTLNFTISN | LQYSPDMGKG | SATFNSTEGV | LQHLLRPLFQ | KSSMGPFYLG | CQLISLRPEK |
| 21790 | 21800 | 21810 | 21820 | 21830 | 21840 |
| DGAATGVDTT | CTYHPDPVGP | GLDIQQLYWE | LSQLTHGVTQ | LGFYVLDRDS | LFINGYAPQN |
| 21850 | 21860 | 21870 | 21880 | 21890 | 21900 |
| LSIRGEYQIN | FHIVNWNLSN | PDPTSSEYIT | LLRDIQDKVT | TLYKGSQLHD | TFRFCLVTNL |
| 21910 | 21920 | 21930 | 21940 | 21950 | 21960 |
| TMDSVLVTVK | ALFSSNLDPS | LVEQVFLDKT | LNASFHWLGS | TYQLVDIHVT | EMESSVYQPT |
| 21970 | 21980 | 21990 | 22000 | 22010 | 22020 |
| SSSSTQHFYL | NFTITNLPYS | QDKAQPGTTN | YQRNKRNIED | ALNQLFRNSS | IKSYFSDCQV |
| 22030 | 22040 | 22050 | 22060 | 22070 | 22080 |
| STFRSVPNRH | HTGVDSLCNF | SPLARRVDRV | AIYEEFLRMT | RNGTQLQNFT | LDRSSVLVDG |
| 22090 | 22100 | 22110 | 22120 | 22130 | 22140 |
| YSPNRNEPLT | GNSDLPFWAV | ILIGLAGLLG | LITCLICGVL | VTTRRRKKEG | EYNVQQQCPG |

22150

YYQSHLDLED LQ

Most preferably, the Mucin-16 assay detects one or more soluble forms of Mucin-16. Mucin-16 is a single-pass type I membrane protein having a large extracellular domain, most or all of which is present in soluble forms of Mucin-16 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Mucin-16:

| Residues | Length | Domain ID |
|---|---|---|
| 1-22152 | 22152 | Mucin-16 |
| 1-22096 | 22906 | extracellular |
| 22907-22117 | 21 | transmembrane |
| 22128-22152 | 35 | cytoplasmic |

As used herein, the term "Carcinoembryonic antigen-related cell adhesion molecule 5" refers to one or polypeptides present in a biological sample that are derived from the Carcinoembryonic antigen-related cell adhesion molecule 5 precursor (Swiss-Prot P06731 (SEQ ID NO: 13)).

```
                10          20          30          40          50          60

    MESPSAPPHR  WCIPWQRLLL  TASLLTFWNP  PTTAKLTIES  TPFNVAEGKE  VLLLVHNLPQ

                70          80          90         100         110         120

    HLFGYSWYKG  ERVDGNRQII  GYVIGTQQAT  PGPAYSGREI  IYPNASLLIQ  NIIQNDTGFY

               130         140         150         160         170         180

    TLHVIKSDLV  NEEATGQFRV  YPELPKPSIS  SNNSKPVEDK  DAVAFTCEPE  TQDATYLWWV

               190         200         210         220         230         240

    NNQSLPVSPR  LQLSNGNRTL  TLFNVTRNDT  ASYKCETQNP  VSARRSDSVI  LNVLYGPDAP

               250         260         270         280         290         300

    TISPLNTSYR  SGENLNLSCH  AASNPPAQYS  WFVNGTFQQS  TQELFIPNIT  VNNSGSYTCQ

               310         320         330         340         350         360
```

```
AHNSDTGLNR TTVTTITVYA EPPKPFITSN NSNPVEDEDA VALTCEPEIQ NTTYLWWVNN

    370        380        390        400        410        420

QSLPVSPRLQ LSNDNRTLTL LSVTRNDVGP YECGIQNELS VDHSDPVILN VLYGPDDPTI

    430        440        450        460        470        480

SPSYTYYRPG VNLSLSCHAA SNPPAQYSWL IDGNIQQHTQ ELFISNITEK NSGLYTCQAN

    490        500        510        520        530        540

NSASGHSRTT VKTITVSAEL PKPSISSNNS KPVEDKDAVA FTCEPEAQNT TYLWWVNGQS

    550        560        570        580        590        600

LPVSPRLQLS NGNRTLTLFN VTRNDARAYV CGIQNSVSAN RSDPVTLDVL YGPDTPIISP

    610        620        630        640        650        660

PDSSYLSGAN LNLSCHSASN PSPQYSWRIN GIPQQHTQVL FIAKITPNNN GTYACFVSNL

    670        680        690        700

ATGRNNSIVK SITVSASGTS PGLSAGATVG IMIGVLVGVA LI
```

The following domains have been identified in Carcinoembryonic antigen-related cell adhesion molecule 5:

| Residues | Length | Domain ID |
|----------|--------|-----------|
| 1-34 | 34 | Signal sequence |
| 35-685 | 5 | Carcinoembryonic antigen-related cell adhesion molecule 5 |
| 686-702 | 17 | Propeptide |

As used herein, the term "cellular tumor antigen p53" refers to one or more polypeptides present in a biological sample that are derived from the cellular tumor antigen p53 precursor (Swiss-Prot P04637 (SEQ ID NO: 14)).

```
    10         20         30         40         50         60

MEEPQSDPSV EPPLSQETFS DLWKLLPENN VLSPLPSQAM DDLMLSPDDI EQWFTEDPGP

    70         80         90        100        110        120

DEAPRMPEAA PRVAPAPAAP TPAAPAPAPS WPLSSSVPSQ KTYQGSYGFR LGFLHSGTAK

   130        140        150        160        170        180

SVTCTYSPAL NKMFCQLAKT CPVQLWVDST PPPGTRVRAM AIYKQSQHMT EVVRRCPHHE
```

|     |     |     |     |     |     |
| 190 | 200 | 210 | 220 | 230 | 240 |

RCSDSDGLAP PQHLIRVEGN LRVEYLDDRN TFRHSVVVPY EPPEVGSDCT TIHYNYMCNS

|     |     |     |     |     |     |
| 250 | 260 | 270 | 280 | 290 | 300 |

SCMGGMNRRP ILTIITLEDS SGNLLGRNSF EVRVCACPGR DRRTEEENLR KKGEPHHELP

|     |     |     |     |     |     |
| 310 | 320 | 330 | 340 | 350 | 360 |

PGSTKRALPN NTSSSPQPKK KPLDGEYFTL QIRGRERFEM FRELNEALEL KDAQAGKEPG

|     |     |     |
| 370 | 380 | 390 |

GSRAHSSHLK SKKGQSTSRH KKLMFKTEGP DSD

Isoform 2 of cellular tumor antigen p53 has the following changes from this isoform 1 sequence:

332-341: IRGRERFEMF (SEQ ID NO: 15) → DGTSFQKENC (SEQ ID NO: 16)

342-393: Missing

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, *etc.*

In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.*) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody or other binding species. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.)* as well as molecules that may be indirectly detected by production of a detectable reaction product (*e.g.*, enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theroy developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Betal (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q 15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, O00458); Isovaleryl-CoA dehydrogenase (IVD, P26440); I-TAC/CXCL11 (O14625); Keratin 19 (P08727); Kim-1 (Hepatitis A virus cellular receptor 1, O43656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, O15244); Osteoprotegerin (O14788); P8 protein (O60356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-1, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anticoagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Tumor necrosis factor receptor superfamily member 10B, Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 were each measured by standard immunoassay methods using commercially available assay reagents in the urine samples and the plasma component of the blood samples collected. Concentrations were reported as follows: Tumor necrosis factor receptor superfamily member 10B - ng/ml, Cadherin-16 - ng/ml, Caspase-9 - ng/ml, Bc12 antagonist of cell death - absorbance units, Caspase-1- pg/ml, Cadherin-1 - pg/ml, Poly [ADP-ribose] polymerase 1 - ng/ml, Cyclin-dependent kinase inhibitor 1 - pg/ml, Cadherin-5 - ng/ml, Myoglobin - ng/ml, Apolipoprotein A-II - ng/ml, Mucin-16, Carcinoembryonic antigen-related cell adhesion molecule 5, and Cellular tumor antigen p53 - U/ml, Carcinoembryonic antigen-related cell adhesion molecule 5 - ng/ml, and Cellular tumor antigen p53 - ng/ml.

Two cohorts were defined as described in the introduction to each of the following tables. In the following tables, the time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) was determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may have included patients adjudicated to stage R, I, or F on the basis of urine output; for those patients adjudicated to stage R, I, or F on the basis of urine output alone, the stage 0 cohort may have included patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage was used.

The ability to distinguish cohort 1 from Cohort 2 was determined using ROC analysis. SE is the standard error of the AUC, n is the number of sample or individual patients ("pts," as indicated). Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test, and are reported as p<0.05 in tables 1-6 and p<0.10 in tables 7-14. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 were determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

## Apolipoprotein A-II

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 71.6 | 85.6 | 71.6 | 72.2 | 71.6 | 75.2 |
| Average | 199 | 234 | 199 | 394 | 199 | 136 |
| Stdev | 723 | 763 | 723 | 2590 | 723 | 224 |
| p(t-test) | | 0.70 | | 0.21 | | 0.58 |
| Min | 2.08 | 4.67 | 2.08 | 9.26 | 2.08 | 1.00E-9 |
| Max | 6400 | 6400 | 6400 | 24300 | 6400 | 1420 |
| n (Samp) | 366 | 74 | 366 | 88 | 366 | 41 |
| n (Patient) | 196 | 74 | 196 | 88 | 196 | 41 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 75.8 | 85.0 | 75.8 | 85.9 | 75.8 | 75.2 |
| Average | 201 | 358 | 201 | 131 | 201 | 120 |
| Stdev | 1050 | 1220 | 1050 | 145 | 1050 | 127 |
| p(t-test) | | 0.45 | | 0.69 | | 0.71 |
| Min | 1.00E-9 | 4.67 | 1.00E-9 | 9.44 | 1.00E-9 | 5.58 |
| Max | 24300 | 6400 | 24300 | 677 | 24300 | 488 |
| n (Samp) | 750 | 27 | 750 | 37 | 750 | 23 |
| n (Patient) | 294 | 27 | 294 | 37 | 294 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.9 | 95.4 | 72.9 | 82.5 | 72.9 | 117 |
| Average | 219 | 249 | 219 | 483 | 219 | 174 |
| Stdev | 774 | 808 | 774 | 2820 | 774 | 260 |
| p(t-test) | | 0.78 | | 0.16 | | 0.73 |
| Min | 2.08 | 18.9 | 2.08 | 9.26 | 2.08 | 1.00E-9 |
| Max | 6400 | 6400 | 6400 | 24300 | 6400 | 1420 |
| n (Samp) | 297 | 65 | 297 | 74 | 297 | 35 |
| n (Patient) | 132 | 65 | 132 | 74 | 132 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.54 | 0.59 | 0.53 | 0.54 | 0.56 | 0.53 | 0.51 | 0.60 |
| SE | 0.038 | 0.058 | 0.040 | 0.035 | 0.050 | 0.038 | 0.048 | 0.062 | 0.053 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.066 | 0.54 | 0.019 | 0.47 | 0.45 | 0.11 | 0.57 | 0.82 | 0.068 |
| nCohort 1 | 366 | 750 | 297 | 366 | 750 | 297 | 366 | 750 | 297 |
| nCohort 2 | 74 | 27 | 65 | 88 | 37 | 74 | 41 | 23 | 35 |
| Cutoff 1 | 53.4 | 58.0 | 64.3 | 46.8 | 58.2 | 56.1 | 46.8 | 47.2 | 68.6 |
| Sens 1 | 70% | 70% | 71% | 70% | 70% | 70% | 71% | 74% | 71% |
| Spec 1 | 38% | 36% | 45% | 32% | 37% | 38% | 32% | 28% | 48% |
| Cutoff 2 | 36.3 | 34.3 | 48.8 | 40.5 | 33.2 | 44.5 | 28.5 | 41.3 | 37.4 |
| Sens 2 | 81% | 81% | 80% | 81% | 81% | 81% | 80% | 83% | 80% |
| Spec 2 | 20% | 16% | 31% | 24% | 15% | 25% | 12% | 23% | 19% |
| Cutoff 3 | 33.5 | 26.3 | 36.2 | 28.5 | 26.3 | 33.2 | 21.0 | 27.1 | 23.1 |
| Sens 3 | 91% | 93% | 91% | 91% | 92% | 91% | 90% | 91% | 91% |
| Spec 3 | 17% | 9% | 16% | 12% | 9% | 14% | 7% | 10% | 8% |
| Cutoff 4 | 107 | 114 | 113 | 107 | 114 | 113 | 107 | 114 | 113 |
| Sens 4 | 39% | 41% | 42% | 34% | 41% | 41% | 39% | 35% | 54% |
| Spec 4 | 70% | 70% | 70% | 70% | 70% | 70% | 70% | 70% | 70% |
| Cutoff 5 | 135 | 148 | 144 | 135 | 148 | 144 | 135 | 148 | 144 |
| Sens 5 | 32% | 26% | 34% | 26% | 30% | 31% | 32% | 17% | 46% |
| Spec 5 | 80% | 80% | 80% | 80% | 80% | 80% | 80% | 80% | 80% |
| Cutoff 6 | 226 | 227 | 292 | 226 | 227 | 292 | 226 | 227 | 292 |
| Sens 6 | 18% | 15% | 14% | 10% | 11% | 9% | 12% | 13% | 9% |
| Spec 6 | 90% | 90% | 90% | 90% | 90% | 90% | 90% | 90% | 90% |
| OR Quart 2 | 0.67 | 0.42 | 1.2 | 1.4 | 0.58 | 1.1 | 0.46 | 1.4 | 0.60 |
| p Value | 0.32 | 0.21 | 0.68 | 0.33 | 0.31 | 0.87 | 0.14 | 0.56 | 0.39 |
| 95% CI of OR Quart2 | 0.30 1.5 | 0.11 1.6 | 0.51 2.8 | 0.72 2.7 | 0.21 1.6 | 0.50 2.3 | 0.17 1.3 | 0.44 4.5 | 0.19 1.9 |
| OR Quart 3 | 1.2 | 1.1 | 1.7 | 0.88 | 0.79 | 0.99 | 0.72 | 0.80 | 0.60 |
| p Value | 0.59 | 0.79 | 0.22 | 0.72 | 0.62 | 0.97 | 0.48 | 0.74 | 0.39 |
| 95% CI of OR Quart3 | 0.60 2.5 | 0.41 3.2 | 0.73 3.8 | 0.43 1.8 | 0.30 2.0 | 0.46 2.1 | 0.29 1.8 | 0.21 3.0 | 0.19 1.9 |
| OR Quart 4 | 1.6 | 1.3 | 2.6 | 1.5 | 1.3 | 1.7 | 1.2 | 1.4 | 2.4 |
| p Value | 0.17 | 0.62 | 0.018 | 0.20 | 0.53 | 0.12 | 0.69 | 0.57 | 0.056 |
| 95% CI of OR Quart4 | 0.81 3.2 | 0.47 3.5 | 1.2 5.6 | 0.80 3.0 | 0.56 3.1 | 0.86 3.5 | 0.52 2.7 | 0.44 4.5 | 0.98 6.0 |

## Bcl2 antagonist of cell death

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0412 | 0.00246 | nd | nd | nd | nd |
| Stdev | 0.150 | 0.00254 | nd | nd | nd | nd |
| p(t-test) | | 0.38 | nd | nd | nd | nd |
| Min | 0.00173 | 0.00173 | nd | nd | nd | nd |
| Max | 0.833 | 0.0105 | nd | nd | nd | nd |
| n (Samp) | 54 | 12 | nd | nd | nd | nd |
| n (Patient) | 36 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0279 | 0.00319 | nd | nd | nd | nd |
| Stdev | 0.122 | 0.00359 | nd | nd | nd | nd |
| p(t-test) | | 0.62 | nd | nd | nd | nd |
| Min | 0.00173 | 0.00173 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 0.833 | 0.0105 | nd | nd | nd | nd |
| n (Samp) | 84 | 6 | nd | nd | nd | nd |
| n (Patient) | 59 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00173 | 0.00173 | nd | nd | nd | nd |
| Average | 0.0457 | 0.00173 | nd | nd | nd | nd |
| Stdev | 0.172 | 0 | nd | nd | nd | nd |
| p(t-test) | | 0.38 | nd | nd | nd | nd |
| Min | 0.00173 | 0.00173 | nd | nd | nd | nd |
| Max | 0.833 | 0.00173 | nd | nd | nd | nd |
| n (Samp) | 40 | 12 | nd | nd | nd | nd |
| n (Patient) | 26 | 12 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.38 | 0.43 | 0.39 | nd | nd | nd | nd | nd | nd |
| SE | 0.094 | 0.13 | 0.097 | nd | nd | nd | nd | nd | nd |
| p | 0.20 | 0.60 | 0.24 | nd | nd | nd | nd | nd | nd |
| nCohort 1 | 54 | 84 | 40 | nd | nd | nd | nd | nd | nd |
| nCohort 2 | 12 | 6 | 12 | nd | nd | nd | nd | nd | nd |
| Cutoff 1 | 0 | 0 | 0 | nd | nd | nd | nd | nd | nd |
| Sens 1 | 100% | 100% | 100% | nd | nd | nd | nd | nd | nd |
| Spec 1 | 0% | 0% | 0% | nd | nd | nd | nd | nd | nd |
| Cutoff 2 | 0 | 0 | 0 | nd | nd | nd | nd | nd | nd |
| Sens 2 | 100% | 100% | 100% | nd | nd | nd | nd | nd | nd |
| Spec 2 | 0% | 0% | 0% | nd | nd | nd | nd | nd | nd |
| Cutoff 3 | 0 | 0 | 0 | nd | nd | nd | nd | nd | nd |
| Sens 3 | 100% | 100% | 100% | nd | nd | nd | nd | nd | nd |
| Spec 3 | 0% | 0% | 0% | nd | nd | nd | nd | nd | nd |
| Cutoff 4 | 0.00509 | 0.00173 | 0.00173 | nd | nd | nd | nd | nd | nd |
| Sens 4 | 8% | 17% | 0% | nd | nd | nd | nd | nd | nd |
| Spec 4 | 70% | 71% | 78% | nd | nd | nd | nd | nd | nd |
| Cutoff 5 | 0.0148 | 0.00943 | 0.00509 | nd | nd | nd | nd | nd | nd |
| Sens 5 | 0% | 17% | 0% | nd | nd | nd | nd | nd | nd |
| Spec 5 | 83% | 82% | 80% | nd | nd | nd | nd | nd | nd |
| Cutoff 6 | 0.0354 | 0.0235 | 0.0148 | nd | nd | nd | nd | nd | nd |
| Sens 6 | 0% | 0% | 0% | nd | nd | nd | nd | nd | nd |
| Spec 6 | 91% | 92% | 90% | nd | nd | nd | nd | nd | nd |
| OR Quart 2 | 9.6 | 4.9 | >0 | nd | nd | nd | nd | nd | nd |
| p Value | 0.050 | 0.17 | <na | nd | nd | nd | nd | nd | nd |
| 95% CI of OR Quart2 | 1.0 | 0.50 | >na | nd | nd | nd | nd | nd | nd |
| | 92 | 48 | na | nd | nd | nd | nd | nd | nd |
| OR Quart 3 | 3.4 | 1.0 | >160 | nd | nd | nd | nd | nd | nd |
| p Value | 0.31 | 1.0 | <5.9E-4 | nd | nd | nd | nd | nd | nd |
| 95% CI of OR Quart3 | 0.32 | 0.059 | >8.8 | nd | nd | nd | nd | nd | nd |
| | 37 | 17 | na | nd | nd | nd | nd | nd | nd |
| OR Quart 4 | 2.3 | 0 | >0 | nd | nd | nd | nd | nd | nd |
| p Value | 0.52 | na | <na | nd | nd | nd | nd | nd | nd |
| 95% CI of OR Quart4 | 0.19 | na | >na | nd | nd | nd | nd | nd | nd |
| | 28 | na | na | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.955 | nd | nd | nd | nd |
| Average | 1.11 | 0.937 | nd | nd | nd | nd |
| Stdev | 2.89 | 1.01 | nd | nd | nd | nd |
| p(t-test) | | 0.84 | nd | nd | nd | nd |
| Min | 0.0360 | 0.0360 | nd | nd | nd | nd |
| Max | 19.5 | 3.69 | nd | nd | nd | nd |
| n (Samp) | 54 | 12 | nd | nd | nd | nd |
| n (Patient) | 36 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 1.05 | nd | nd | nd | nd |
| Average | 1.08 | 1.44 | nd | nd | nd | nd |
| Stdev | 2.44 | 1.42 | nd | nd | nd | nd |
| p(t-test) | | 0.73 | nd | nd | nd | nd |
| Min | 0.0360 | 0.0360 | nd | nd | nd | nd |
| Max | 19.5 | 3.92 | nd | nd | nd | nd |
| n (Samp) | 86 | 6 | nd | nd | nd | nd |
| n (Patient) | 60 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.586 | 0.790 | nd | nd | nd | nd |
| Average | 1.35 | 0.929 | nd | nd | nd | nd |
| Stdev | 3.31 | 0.998 | nd | nd | nd | nd |
| p(t-test) | | 0.67 | nd | nd | nd | nd |
| Min | 0.0360 | 0.0360 | nd | nd | nd | nd |
| Max | 19.5 | 3.69 | nd | nd | nd | nd |
| n (Samp) | 40 | 12 | nd | nd | nd | nd |
| n (Patient) | 26 | 12 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.66 | 0.56 | nd | nd | nd | nd | nd | nd |
| SE | 0.094 | 0.13 | 0.097 | nd | nd | nd | nd | nd | nd |
| p | 0.32 | 0.20 | 0.52 | nd | nd | nd | nd | nd | nd |
| nCohort 1 | 54 | 86 | 40 | nd | nd | nd | nd | nd | nd |
| nCohort 2 | 12 | 6 | 12 | nd | nd | nd | nd | nd | nd |
| Cutoff 1 | 0.208 | 0.330 | 0.208 | nd | nd | nd | nd | nd | nd |
| Sens 1 | 75% | 83% | 83% | nd | nd | nd | nd | nd | nd |
| Spec 1 | 37% | 38% | 28% | nd | nd | nd | nd | nd | nd |
| Cutoff 2 | 0 | 0.330 | 0.208 | nd | nd | nd | nd | nd | nd |
| Sens 2 | 100% | 83% | 83% | nd | nd | nd | nd | nd | nd |
| Spec 2 | 0% | 38% | 28% | nd | nd | nd | nd | nd | nd |
| Cutoff 3 | 0 | 0 | 0 | nd | nd | nd | nd | nd | nd |
| Sens 3 | 100% | 100% | 100% | nd | nd | nd | nd | nd | nd |
| Spec 3 | 0% | 0% | 0% | nd | nd | nd | nd | nd | nd |
| Cutoff 4 | 0.703 | 0.811 | 0.781 | nd | nd | nd | nd | nd | nd |
| Sens 4 | 58% | 67% | 50% | nd | nd | nd | nd | nd | nd |
| Spec 4 | 70% | 71% | 72% | nd | nd | nd | nd | nd | nd |
| Cutoff 5 | 1.04 | 1.04 | 0.868 | nd | nd | nd | nd | nd | nd |
| Sens 5 | 33% | 50% | 50% | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 81% | 80% | 80% | nd | nd | nd | nd | nd | nd |
| Cutoff 6 | 1.76 | 2.17 | 2.17 | nd | nd | nd | nd | nd | nd |
| Sens 6 | 8% | 17% | 8% | nd | nd | nd | nd | nd | nd |
| Spec 6 | 91% | 91% | 90% | nd | nd | nd | nd | nd | nd |
| OR Quart 2 | 0.58 | 1.0 | 1.6 | nd | nd | nd | nd | nd | nd |
| p Value | 0.58 | 1.0 | 0.62 | nd | nd | nd | nd | nd | nd |
| 95% CI of OR Quart2 | 0.083 4.0 | 0.059 17 | 0.23 12 | nd | nd | nd | nd | nd | nd |
| OR Quart 3 | 0.29 | 1.0 | 1.0 | nd | nd | nd | nd | nd | nd |
| p Value | 0.31 | 1.0 | 1.0 | nd | nd | nd | nd | nd | nd |
| 95% CI of OR Quart3 | 0.027 3.1 | 0.059 17 | 0.12 8.4 | nd | nd | nd | nd | nd | nd |
| OR Quart 4 | 2.4 | 3.3 | 3.4 | nd | nd | nd | nd | nd | nd |
| p Value | 0.29 | 0.32 | 0.20 | nd | nd | nd | nd | nd | nd |
| 95% CI of OR Quart4 | 0.48 12 | 0.32 34 | 0.53 22 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48800 | 32700 | 48800 | 111000 | nd | nd |
| Average | 84900 | 79300 | 84900 | 143000 | nd | nd |
| Stdev | 121000 | 100000 | 121000 | 147000 | nd | nd |
| p(t-test) | | 0.86 | | 0.091 | nd | nd |
| Min | 160 | 1620 | 160 | 1660 | nd | nd |
| Max | 744000 | 363000 | 744000 | 543000 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 37500 | 50200 | 37500 | 111000 | nd | nd |
| Average | 75900 | 91900 | 75900 | 146000 | nd | nd |
| Stdev | 124000 | 107000 | 124000 | 140000 | nd | nd |
| p(t-test) | | 0.65 | | 0.046 | nd | nd |
| Min | 160 | 1620 | 160 | 2220 | nd | nd |
| Max | 744000 | 363000 | 744000 | 543000 | nd | nd |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | nd | 0.55 | 0.64 | nd | 0.71 | nd | nd | nd |
| SE | 0.077 | nd | 0.086 | 0.076 | nd | 0.073 | nd | nd | nd |
| p | 0.55 | nd | 0.58 | 0.064 | nd | 0.0041 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 10500 | nd | 21400 | 51100 | nd | 54200 | nd | nd | nd |
| Sens 1 | 70% | nd | 75% | 70% | nd | 71% | nd | nd | nd |
| Spec 1 | 15% | nd | 36% | 54% | nd | 60% | nd | nd | nd |
| Cutoff 2 | 9800 | nd | 10400 | 28500 | nd | 36500 | nd | nd | nd |
| Sens 2 | 80% | nd | 81% | 80% | nd | 81% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 13% | nd | 24% | 38% | nd | 50% | nd | nd | nd |
| Cutoff 3 | 4310 | nd | 3350 | 8380 | nd | 11700 | nd | nd | nd |
| Sens 3 | 90% | nd | 94% | 90% | nd | 90% | nd | nd | nd |
| Spec 3 | 8% | nd | 10% | 12% | nd | 29% | nd | nd | nd |
| Cutoff 4 | 94000 | nd | 80700 | 94000 | nd | 80700 | nd | nd | nd |
| Sens 4 | 25% | nd | 38% | 55% | nd | 62% | nd | nd | nd |
| Spec 4 | 71% | nd | 71% | 71% | nd | 71% | nd | nd | nd |
| Cutoff 5 | 134000 | nd | 122000 | 134000 | nd | 122000 | nd | nd | nd |
| Sens 5 | 20% | nd | 31% | 40% | nd | 48% | nd | nd | nd |
| Spec 5 | 81% | nd | 81% | 81% | nd | 81% | nd | nd | nd |
| Cutoff 6 | 152000 | nd | 144000 | 152000 | nd | 144000 | nd | nd | nd |
| Sens 6 | 15% | nd | 19% | 35% | nd | 33% | nd | nd | nd |
| Spec 6 | 90% | nd | 90% | 90% | nd | 90% | nd | nd | nd |
| OR Quart 2 | 0.74 | nd | 0.62 | 1.4 | nd | 0.92 | nd | nd | nd |
| p Value | 0.70 | nd | 0.59 | 0.67 | nd | 0.93 | nd | nd | nd |
| 95% CI of OR Quart2 | 0.16 | nd | 0.11 | 0.27 | nd | 0.16 | nd | nd | nd |
| | 3.4 | nd | 3.5 | 7.5 | nd | 5.5 | nd | nd | nd |
| OR Quart 3 | 0.74 | nd | 0.68 | 1.9 | nd | 1.8 | nd | nd | nd |
| p Value | 0.70 | nd | 0.66 | 0.43 | nd | 0.48 | nd | nd | nd |
| 95% CI of OR Quart3 | 0.16 | nd | 0.12 | 0.38 | nd | 0.35 | nd | nd | nd |
| | 3.4 | nd | 3.8 | 9.6 | nd | 9.5 | nd | nd | nd |
| OR Quart 4 | 1.7 | nd | 1.7 | 4.0 | nd | 6.7 | nd | nd | nd |
| p Value | 0.48 | nd | 0.52 | 0.080 | nd | 0.022 | nd | nd | nd |
| 95% CI of OR Quart4 | 0.41 | nd | 0.35 | 0.85 | nd | 1.3 | nd | nd | nd |
| | 6.7 | nd | 7.9 | 19 | nd | 34 | nd | nd | nd |

## Cadherin-5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00224 | 0.00224 | nd | nd | nd | nd |
| Average | 0.0957 | 0.0142 | nd | nd | nd | nd |
| Stdev | 0.210 | 0.0183 | nd | nd | nd | nd |
| p(t-test) | | 0.28 | nd | nd | nd | nd |
| Min | 0.00224 | 0.00224 | nd | nd | nd | nd |
| Max | 1.10 | 0.0502 | nd | nd | nd | nd |
| n (Samp) | 47 | 8 | nd | nd | nd | nd |
| n (Patient) | 30 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00224 | 0.00224 | nd | nd | nd | nd |
| Average | 0.0806 | 0.00753 | nd | nd | nd | nd |
| Stdev | 0.218 | 0.0108 | nd | nd | nd | nd |
| p(t-test) | | 0.32 | nd | nd | nd | nd |
| Min | 0.00224 | 0.00224 | nd | nd | nd | nd |
| Max | 1.10 | 0.0309 | nd | nd | nd | nd |
| n (Samp) | 34 | 9 | nd | nd | nd | nd |
| n (Patient) | 20 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.44 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.11 | nd | 0.11 | nd | nd | nd | nd | nd | nd |
| p | 0.72 | nd | 0.62 | nd | nd | nd | nd | nd | nd |
| nCohort 1 | 47 | nd | 34 | nd | nd | nd | nd | nd | nd |
| nCohort 2 | 8 | nd | 9 | nd | nd | nd | nd | nd | nd |
| Cutoff 1 | 0 | nd | 0 | nd | nd | nd | nd | nd | nd |
| Sens 1 | 100% | nd | 100% | nd | nd | nd | nd | nd | nd |
| Spec 1 | 0% | nd | 0% | nd | nd | nd | nd | nd | nd |
| Cutoff 2 | 0 | nd | 0 | nd | nd | nd | nd | nd | nd |
| Sens 2 | 100% | nd | 100% | nd | nd | nd | nd | nd | nd |
| Spec 2 | 0% | nd | 0% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.0405 | nd | 0.00224 | nd | nd | nd | nd | nd | nd |
| Sens 4 | 12% | nd | 22% | nd | nd | nd | nd | nd | nd |
| Spec 4 | 72% | nd | 71% | nd | nd | nd | nd | nd | nd |
| Cutoff 5 | 0.137 | nd | 0.127 | nd | nd | nd | nd | nd | nd |
| Sens 5 | 0% | nd | 0% | nd | nd | nd | nd | nd | nd |
| Spec 5 | 81% | nd | 82% | nd | nd | nd | nd | nd | nd |
| Cutoff 6 | 0.329 | nd | 0.239 | nd | nd | nd | nd | nd | nd |
| Sens 6 | 0% | nd | 0% | nd | nd | nd | nd | nd | nd |
| Spec 6 | 91% | nd | 91% | nd | nd | nd | nd | nd | nd |
| OR Quart 2 | 2.2 | nd | 0 | nd | nd | nd | nd | nd | nd |
| p Value | 0.55 | nd | na | nd | nd | nd | nd | nd | nd |
| 95% CI of OR Quart2 | 0.17 | nd | na | nd | nd | nd | nd | nd | nd |
| | 27 | nd | na | nd | nd | nd | nd | nd | nd |
| OR Quart 3 | 3.5 | nd | 5.4 | nd | nd | nd | nd | nd | nd |
| p Value | 0.30 | nd | 0.088 | nd | nd | nd | nd | nd | nd |
| 95% CI of OR Quart3 | 0.32 | nd | 0.78 | nd | nd | nd | nd | nd | nd |
| | 39 | nd | 38 | nd | nd | nd | nd | nd | nd |
| OR Quart 4 | 2.4 | nd | 0.50 | nd | nd | nd | nd | nd | nd |
| p Value | 0.51 | nd | 0.60 | nd | nd | nd | nd | nd | nd |
| 95% CI of OR Quart4 | 0.19 | nd | 0.038 | nd | nd | nd | nd | nd | nd |
| | 30 | nd | 6.5 | nd | nd | nd | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.713 | 0.116 | 0.713 | 1.98 | 0.713 | 1.09 |
| Average | 103 | 8.88 | 103 | 19.5 | 103 | 5.00 |
| Stdev | 670 | 19.5 | 670 | 49.0 | 670 | 9.16 |
| p(t-test) | | 0.33 | | 0.35 | | 0.47 |
| Min | 1.14E-14 | 1.70E-14 | 1.14E-14 | 1.70E-14 | 1.14E-14 | 1.70E-14 |
| Max | 6950 | 112 | 6950 | 327 | 6950 | 42.0 |
| n (Samp) | 165 | 48 | 165 | 56 | 165 | 25 |
| n (Patient) | 102 | 48 | 102 | 56 | 102 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 5.28 | 1.09 | 10.7 | 1.09 | 2.45 |
| Average | 62.4 | 15.7 | 62.4 | 19.5 | 62.4 | 6.76 |

70

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 482 | 28.6 | 482 | 21.2 | 482 | 12.3 |
| p(t-test) | | 0.70 | | 0.69 | | 0.68 |
| Min | 1.14E-14 | 0.116 | 1.14E-14 | 1.70E-14 | 1.14E-14 | 1.70E-14 |
| Max | 6950 | 112 | 6950 | 71.0 | 6950 | 44.1 |
| n (Samp) | 325 | 16 | 325 | 20 | 325 | 13 |
| n (Patient) | 168 | 16 | 168 | 20 | 168 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.850 | 0.116 | 0.850 | 2.45 | 0.850 | 1.09 |
| Average | 119 | 24.9 | 119 | 23.7 | 119 | 10.5 |
| Stdev | 729 | 97.5 | 729 | 60.2 | 729 | 24.6 |
| p(t-test) | | 0.39 | | 0.35 | | 0.48 |
| Min | 1.14E-14 | 1.70E-14 | 1.14E-14 | 0.116 | 1.14E-14 | 0.116 |
| Max | 6950 | 630 | 6950 | 327 | 6950 | 112 |
| n (Samp) | 139 | 46 | 139 | 51 | 139 | 23 |
| n (Patient) | 85 | 46 | 85 | 51 | 85 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.61 | 0.45 | 0.53 | 0.62 | 0.54 | 0.46 | 0.46 | 0.49 |
| SE | 0.048 | 0.077 | 0.050 | 0.045 | 0.069 | 0.048 | 0.063 | 0.084 | 0.065 |
| p | 0.35 | 0.16 | 0.28 | 0.51 | 0.088 | 0.44 | 0.58 | 0.60 | 0.89 |
| nCohort 1 | 165 | 325 | 139 | 165 | 325 | 139 | 165 | 325 | 139 |
| nCohort 2 | 48 | 16 | 46 | 56 | 20 | 51 | 25 | 13 | 23 |
| Cutoff 1 | 1.70E-14 | 0.850 | 1.70E-14 | 1.70E-14 | 0.850 | 1.14E-14 | 1.70E-14 | 1.70E-14 | 1.14E-14 |
| Sens 1 | 98% | 75% | 98% | 96% | 75% | 100% | 96% | 92% | 100% |
| Spec 1 | 2% | 50% | 2% | 2% | 50% | 2% | 2% | 2% | 2% |
| Cutoff 2 | 1.70E-14 | 0.116 | 1.70E-14 | 1.70E-14 | 1.70E-14 | 1.14E-14 | 1.70E-14 | 1.70E-14 | 1.14E-14 |
| Sens 2 | 98% | 81% | 98% | 96% | 90% | 100% | 96% | 92% | 100% |
| Spec 2 | 2% | 43% | 2% | 2% | 2% | 2% | 2% | 2% | 2% |
| Cutoff 3 | 1.70E-14 | 1.14E-14 | 1.70E-14 | 1.70E-14 | 1.70E-14 | 1.14E-14 | 1.70E-14 | 1.70E-14 | 1.14E-14 |
| Sens 3 | 98% | 100% | 98% | 96% | 90% | 100% | 96% | 92% | 100% |
| Spec 3 | 2% | 2% | 2% | 2% | 2% | 2% | 2% | 2% | 2% |
| Cutoff 4 | 7.84 | 9.28 | 9.28 | 7.84 | 9.28 | 9.28 | 7.84 | 9.28 | 9.28 |
| Sens 4 | 23% | 25% | 26% | 38% | 50% | 35% | 24% | 15% | 22% |
| Spec 4 | 70% | 71% | 71% | 70% | 71% | 71% | 70% | 71% | 71% |
| Cutoff 5 | 18.3 | 15.5 | 22.7 | 18.3 | 15.5 | 22.7 | 18.3 | 15.5 | 22.7 |
| Sens 5 | 17% | 25% | 15% | 25% | 45% | 20% | 4% | 15% | 13% |
| Spec 5 | 80% | 80% | 81% | 80% | 80% | 81% | 80% | 80% | 81% |
| Cutoff 6 | 60.3 | 44.8 | 73.1 | 60.3 | 44.8 | 73.1 | 60.3 | 44.8 | 73.1 |
| Sens 6 | 2% | 6% | 4% | 7% | 10% | 6% | 0% | 0% | 4% |
| Spec 6 | 90% | 90% | 91% | 90% | 90% | 91% | 90% | 90% | 91% |
| OR Quart 2 | 1.3 | 0.33 | 1.0 | 0.25 | 0.19 | 0.60 | 4.6 | 2.6 | 2.7 |
| p Value | 0.60 | 0.34 | 0.96 | 0.0079 | 0.13 | 0.31 | 0.027 | 0.26 | 0.13 |
| 95% CI of OR Quart2 | 0.50 | 0.033 | 0.38 | 0.091 | 0.022 | 0.23 | 1.2 | 0.50 | 0.75 |
| | 3.3 | 3.2 | 2.8 | 0.70 | 1.7 | 1.6 | 18 | 14 | 9.6 |
| OR Quart 3 | 0.78 | 2.8 | 1.2 | 0.77 | 0.79 | 1.1 | 0 | 0 | 0.23 |
| p Value | 0.64 | 0.13 | 0.76 | 0.53 | 0.73 | 0.82 | na | na | 0.20 |
| 95% CI of OR Quart3 | 0.28 | 0.73 | 0.44 | 0.34 | 0.20 | 0.45 | na | na | 0.025 |
| | 2.2 | 11 | 3.1 | 1.7 | 3.0 | 2.7 | na | na | 2.2 |
| OR Quart 4 | 2.3 | 1.3 | 1.8 | 0.90 | 2.1 | 1.2 | 4.6 | 3.2 | 2.7 |
| p Value | 0.073 | 0.71 | 0.22 | 0.79 | 0.19 | 0.70 | 0.027 | 0.16 | 0.13 |
| 95% CI of OR Quart4 | 0.93 | 0.29 | 0.71 | 0.40 | 0.69 | 0.49 | 1.2 | 0.63 | 0.75 |
| | 5.5 | 6.1 | 4.5 | 2.0 | 6.4 | 2.9 | 18 | 16 | 9.6 |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.646 | 1.40 | 0.646 | 1.30 | 0.646 | 1.38 |
| Average | 2.04 | 4.66 | 2.04 | 4.98 | 2.04 | 4.81 |
| Stdev | 4.06 | 7.77 | 4.06 | 10.7 | 4.06 | 9.92 |
| p(t-test) | | 6.7E-4 | | 7.2E-4 | | 0.0064 |
| Min | 0.00336 | 0.00336 | 0.00336 | 0.0411 | 0.00336 | 0.0844 |
| Max | 43.4 | 29.4 | 43.4 | 54.4 | 43.4 | 47.4 |
| n (Samp) | 253 | 48 | 253 | 57 | 253 | 26 |
| n (Patient) | 102 | 48 | 102 | 57 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.947 | 1.48 | 0.947 | 1.66 | 0.947 | 2.29 |
| Average | 12.1 | 3.83 | 12.1 | 7.05 | 12.1 | 3.90 |
| Stdev | 192 | 5.63 | 192 | 12.5 | 192 | 4.53 |
| p(t-test) | | 0.86 | | 0.90 | | 0.88 |
| Min | 0.00336 | 0.00336 | 0.00336 | 0.0411 | 0.00336 | 0.173 |
| Max | 4070 | 21.1 | 4070 | 51.3 | 4070 | 15.3 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.625 | 1.44 | 0.625 | 1.25 | 0.625 | 1.35 |
| Average | 2.35 | 4.89 | 2.35 | 4.76 | 2.35 | 4.02 |
| Stdev | 4.80 | 7.57 | 4.80 | 10.6 | 4.80 | 9.89 |
| p(t-test) | | 0.0038 | | 0.015 | | 0.16 |
| Min | 0.00336 | 0.00336 | 0.00336 | 0.0946 | 0.00336 | 0.00336 |
| Max | 43.4 | 29.4 | 43.4 | 54.4 | 43.4 | 47.4 |
| n (Samp) | 212 | 47 | 212 | 52 | 212 | 25 |
| n (Patient) | 85 | 47 | 85 | 52 | 85 | 25 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.56 | 0.64 | 0.61 | 0.62 | 0.61 | 0.60 | 0.65 | 0.55 |
| SE | 0.046 | 0.076 | 0.047 | 0.043 | 0.067 | 0.045 | 0.061 | 0.084 | 0.062 |
| p | 0.012 | 0.44 | 0.0036 | 0.0089 | 0.081 | 0.014 | 0.11 | 0.068 | 0.41 |
| nCohort 1 | 253 | 447 | 212 | 253 | 447 | 212 | 253 | 447 | 212 |
| nCohort 2 | 48 | 16 | 47 | 57 | 21 | 52 | 26 | 13 | 25 |
| Cutoff 1 | 0.540 | 0.414 | 0.550 | 0.607 | 1.10 | 0.607 | 0.456 | 1.06 | 0.456 |
| Sens 1 | 71% | 75% | 70% | 70% | 71% | 71% | 73% | 77% | 72% |
| Spec 1 | 45% | 32% | 46% | 48% | 53% | 49% | 42% | 53% | 43% |
| Cutoff 2 | 0.383 | 0.271 | 0.485 | 0.394 | 0.394 | 0.444 | 0.303 | 0.849 | 0.303 |
| Sens 2 | 81% | 81% | 81% | 81% | 81% | 81% | 81% | 85% | 80% |
| Spec 2 | 37% | 23% | 43% | 38% | 31% | 42% | 31% | 47% | 32% |
| Cutoff 3 | 0.146 | 0.102 | 0.148 | 0.173 | 0.0990 | 0.211 | 0.146 | 0.211 | 0.0990 |
| Sens 3 | 92% | 94% | 91% | 91% | 90% | 90% | 92% | 92% | 92% |
| Spec 3 | 13% | 7% | 12% | 16% | 7% | 20% | 13% | 17% | 8% |
| Cutoff 4 | 1.54 | 1.91 | 1.54 | 1.54 | 1.91 | 1.54 | 1.54 | 1.91 | 1.54 |
| Sens 4 | 38% | 44% | 43% | 44% | 48% | 42% | 42% | 54% | 32% |
| Spec 4 | 70% | 70% | 70% | 70% | 70% | 70% | 70% | 70% | 70% |
| Cutoff 5 | 2.53 | 3.14 | 2.77 | 2.53 | 3.14 | 2.77 | 2.53 | 3.14 | 2.77 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 35% | 44% | 34% | 28% | 33% | 25% | 27% | 31% | 16% |
| Spec 5 | 80% | 80% | 80% | 80% | 80% | 80% | 80% | 80% | 80% |
| Cutoff 6 | 5.50 | 8.51 | 6.40 | 5.50 | 8.51 | 6.40 | 5.50 | 8.51 | 6.40 |
| Sens 6 | 21% | 12% | 23% | 18% | 19% | 17% | 19% | 15% | 12% |
| Spec 6 | 90% | 90% | 90% | 90% | 90% | 90% | 90% | 90% | 90% |
| OR Quart 2 | 2.2 | 0.74 | 1.8 | 1.6 | 0.66 | 1.3 | 0.57 | 0.50 | 0.58 |
| p Value | 0.14 | 0.69 | 0.30 | 0.36 | 0.65 | 0.61 | 0.46 | 0.57 | 0.47 |
| 95% CI of OR Quart2 | 0.78 | 0.16 | 0.60 | 0.60 | 0.11 | 0.48 | 0.13 | 0.044 | 0.13 |
| | 6.2 | 3.4 | 5.2 | 4.1 | 4.0 | 3.5 | 2.5 | 5.5 | 2.5 |
| OR Quart 3 | 2.4 | 0.49 | 2.2 | 3.0 | 2.8 | 3.2 | 2.1 | 2.6 | 2.8 |
| p Value | 0.093 | 0.41 | 0.14 | 0.015 | 0.14 | 0.013 | 0.19 | 0.27 | 0.074 |
| 95% CI of OR Quart3 | 0.86 | 0.087 | 0.77 | 1.2 | 0.72 | 1.3 | 0.69 | 0.49 | 0.90 |
| | 6.7 | 2.7 | 6.2 | 7.4 | 11 | 7.8 | 6.6 | 14 | 8.4 |
| OR Quart 4 | 3.3 | 1.8 | 4.0 | 2.4 | 2.8 | 2.0 | 1.7 | 2.6 | 0.98 |
| p Value | 0.018 | 0.37 | 0.0064 | 0.058 | 0.14 | 0.17 | 0.40 | 0.27 | 0.98 |
| 95% CI of OR Quart4 | 1.2 | 0.51 | 1.5 | 0.97 | 0.72 | 0.76 | 0.51 | 0.49 | 0.27 |
| | 8.9 | 6.3 | 11 | 6.0 | 11 | 5.0 | 5.3 | 14 | 3.6 |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.21 | 0.965 | 1.21 | 0.546 | 1.21 | 0.330 |
| Average | 26.0 | 38.3 | 26.0 | 70.8 | 26.0 | 47.2 |
| Stdev | 82.5 | 97.7 | 82.5 | 151 | 82.5 | 131 |
| p(t-test) | | 0.36 | | 0.0021 | | 0.24 |
| Min | 0.000105 | 0.000105 | 0.000105 | 0.0276 | 0.000105 | 0.0254 |
| Max | 618 | 469 | 618 | 618 | 618 | 469 |
| n (Samp) | 253 | 48 | 253 | 57 | 253 | 26 |
| n (Patient) | 102 | 48 | 102 | 57 | 102 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.608 | 0.186 | 0.608 | 0.375 | 0.608 | 0.349 |
| Average | 32.4 | 66.7 | 32.4 | 58.7 | 32.4 | 62.5 |
| Stdev | 99.0 | 156 | 99.0 | 131 | 99.0 | 173 |
| p(t-test) | | 0.18 | | 0.24 | | 0.29 |
| Min | 0.000105 | 0.000105 | 0.000105 | 0.0375 | 0.000105 | 0.0567 |
| Max | 618 | 469 | 618 | 442 | 618 | 618 |
| n (Samp) | 447 | 16 | 447 | 21 | 447 | 13 |
| n (Patient) | 170 | 16 | 170 | 21 | 170 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.23 | 1.00 | 1.23 | 0.855 | 1.23 | 0.366 |
| Average | 29.6 | 29.5 | 29.6 | 76.5 | 29.6 | 68.8 |
| Stdev | 90.0 | 76.4 | 90.0 | 160 | 90.0 | 155 |
| p(t-test) | | 0.99 | | 0.0051 | | 0.062 |
| Min | 0.00616 | 0.0266 | 0.00616 | 0.000105 | 0.00616 | 0.0254 |
| Max | 618 | 469 | 618 | 618 | 618 | 469 |
| n (Samp) | 212 | 47 | 212 | 52 | 212 | 25 |
| n (Patient) | 85 | 47 | 85 | 52 | 85 | 25 |

| | 0hr prior to AKI stage | 24hr prior to AKI stage | 48hr prior to AKI stage |
|---|---|---|---|

| | sCr or UO | sCr only | UO only | sCr or UO | sCr only | UO only | sCr or UO | sCr only | UO only |
|---|---|---|---|---|---|---|---|---|---|
| AUC | 0.51 | 0.48 | 0.51 | 0.50 | 0.53 | 0.51 | 0.45 | 0.52 | 0.50 |
| SE | 0.046 | 0.074 | 0.047 | 0.042 | 0.066 | 0.045 | 0.061 | 0.082 | 0.061 |
| p | 0.85 | 0.84 | 0.87 | 0.94 | 0.66 | 0.84 | 0.44 | 0.76 | 0.97 |
| nCohort 1 | 253 | 447 | 212 | 253 | 447 | 212 | 253 | 447 | 212 |
| nCohort 2 | 48 | 16 | 47 | 57 | 21 | 52 | 26 | 13 | 25 |
| Cutoff 1 | 0.0819 | 0.0422 | 0.0832 | 0.0855 | 0.0849 | 0.0855 | 0.0877 | 0.132 | 0.118 |
| Sens 1 | 71% | 75% | 70% | 70% | 71% | 71% | 73% | 77% | 72% |
| Spec 1 | 19% | 16% | 19% | 19% | 26% | 19% | 19% | 33% | 24% |
| Cutoff 2 | 0.0394 | 0.0333 | 0.0485 | 0.0746 | 0.0556 | 0.0767 | 0.0667 | 0.0667 | 0.0832 |
| Sens 2 | 81% | 81% | 81% | 81% | 81% | 81% | 81% | 85% | 80% |
| Spec 2 | 11% | 12% | 11% | 18% | 21% | 18% | 17% | 23% | 19% |
| Cutoff 3 | 0.0329 | 0 | 0.0333 | 0.0337 | 0.0399 | 0.0333 | 0.0296 | 0.0584 | 0.0296 |
| Sens 3 | 92% | 100% | 91% | 91% | 90% | 90% | 92% | 92% | 92% |
| Spec 3 | 8% | 0% | 7% | 8% | 15% | 7% | 6% | 22% | 5% |
| Cutoff 4 | 6.79 | 5.77 | 7.09 | 6.79 | 5.77 | 7.09 | 6.79 | 5.77 | 7.09 |
| Sens 4 | 44% | 44% | 43% | 35% | 38% | 37% | 23% | 31% | 32% |
| Spec 4 | 70% | 70% | 70% | 70% | 70% | 70% | 70% | 70% | 70% |
| Cutoff 5 | 16.7 | 16.8 | 18.6 | 16.7 | 16.8 | 18.6 | 16.7 | 16.8 | 18.6 |
| Sens 5 | 31% | 38% | 26% | 32% | 29% | 31% | 19% | 15% | 28% |
| Spec 5 | 80% | 80% | 80% | 80% | 80% | 80% | 80% | 80% | 80% |
| Cutoff 6 | 45.5 | 63.4 | 54.1 | 45.5 | 63.4 | 54.1 | 45.5 | 63.4 | 54.1 |
| Sens 6 | 19% | 12% | 13% | 21% | 19% | 23% | 12% | 15% | 16% |
| Spec 6 | 90% | 90% | 90% | 90% | 90% | 90% | 90% | 90% | 90% |
| OR Quart 2 | 0.33 | 0 | 0.33 | 0.47 | 1.0 | 0.42 | 0.65 | 1.7 | 0.47 |
| p Value | 0.020 | na | 0.025 | 0.068 | 1.0 | 0.056 | 0.51 | 0.48 | 0.24 |
| 95% CI of OR Quart2 | 0.13 | na | 0.13 | 0.21 | 0.31 | 0.17 | 0.17 | 0.40 | 0.13 |
| | 0.84 | na | 0.87 | 1.1 | 3.2 | 1.0 | 2.4 | 7.3 | 1.7 |
| OR Quart 3 | 0.33 | 0.41 | 0.39 | 0.33 | 0.32 | 0.37 | 1.4 | 0.66 | 0.60 |
| p Value | 0.020 | 0.21 | 0.045 | 0.015 | 0.17 | 0.032 | 0.57 | 0.65 | 0.40 |
| 95% CI of OR Quart3 | 0.13 | 0.10 | 0.15 | 0.14 | 0.064 | 0.15 | 0.45 | 0.11 | 0.18 |
| | 0.84 | 1.6 | 0.98 | 0.81 | 1.6 | 0.92 | 4.2 | 4.0 | 2.0 |
| OR Quart 4 | 0.84 | 0.86 | 0.83 | 0.86 | 1.2 | 0.93 | 1.4 | 1.0 | 1.0 |
| p Value | 0.66 | 0.79 | 0.65 | 0.68 | 0.78 | 0.84 | 0.56 | 1.0 | 0.97 |
| 95% CI of OR Quart4 | 0.39 | 0.28 | 0.37 | 0.41 | 0.38 | 0.43 | 0.46 | 0.20 | 0.36 |
| | 1.8 | 2.6 | 1.8 | 1.8 | 3.6 | 2.0 | 4.3 | 5.1 | 2.9 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.619 | 0.753 | 0.619 | 0.547 | nd | nd |
| Average | 1.06 | 1.12 | 1.06 | 23.1 | nd | nd |
| Stdev | 1.28 | 0.983 | 1.28 | 96.4 | nd | nd |
| p(t-test) | | 0.85 | | 0.10 | nd | nd |
| Min | 0.141 | 1.00E-9 | 0.141 | 0.223 | nd | nd |
| Max | 6.37 | 3.40 | 6.37 | 433 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.547 | 0.654 | 0.547 | 0.684 | nd | nd |
| Average | 0.951 | 1.00 | 0.951 | 22.0 | nd | nd |
| Stdev | 1.18 | 0.898 | 1.18 | 94.1 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.87 | | 0.15 | nd | nd |
| Min | 0.0565 | 1.00E-9 | 0.0565 | 0.223 | nd | nd |
| Max | 6.37 | 2.82 | 6.37 | 433 | nd | nd |
| n (Samp) | 42 | 16 | 42 | 21 | nd | nd |
| n (Patient) | 33 | 16 | 33 | 21' | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.54 | 0.49 | nd | 0.56 | nd | nd | nd |
| SE | 0.077 | nd | 0.086 | 0.077 | nd | 0.078 | nd | nd | nd |
| p | 0.61 | nd | 0.67 | 0.87 | nd | 0.48 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 0.479 | nd | 0.412 | 0.344 | nd | 0.384 | nd | nd | nd |
| Sens 1 | 70% | nd | 75% | 70% | nd | 71% | nd | nd | nd |
| Spec 1 | 37% | nd | 36% | 17% | nd | 29% | nd | nd | nd |
| Cutoff 2 | 0.223 | nd | 0.223 | 0.278 | nd | 0.278 | nd | nd | nd |
| Sens 2 | 85% | nd | 81% | 90% | nd | 90% | nd | nd | nd |
| Spec 2 | 4% | nd | 7% | 10% | nd | 12% | nd | nd | nd |
| Cutoff 3 | 0.146 | nd | 0.0565 | 0.278 | nd | 0.278 | nd | nd | nd |
| Sens 3 | 90% | nd | 94% | 90% | nd | 90% | nd | nd | nd |
| Spec 3 | 4% | nd | 2% | 10% | nd | 12% | nd | nd | nd |
| Cutoff 4 | 0.937 | nd | 0.859 | 0.937 | nd | 0.859 | nd | nd | nd |
| Sens 4 | 45% | nd | 38% | 30% | nd | 43% | nd | nd | nd |
| Spec 4 | 71% | nd | 71% | 71% | nd | 71% | nd | nd | nd |
| Cutoff 5 | 1.24 | nd | 1.09 | 1.24 | nd | 1.09 | nd | nd | nd |
| Sens 5 | 40% | nd | 38% | 30% | nd | 33% | nd | nd | nd |
| Spec 5 | 81% | nd | 81% | 81% | nd | 81% | nd | nd | nd |
| Cutoff 6 | 1.87 | nd | 1.68 | 1.87 | nd | 1.68 | nd | nd | nd |
| Sens 6 | 20% | nd | 19% | 25% | nd | 24% | nd | nd | nd |
| Spec 6 | 90% | nd | 90% | 90% | nd | 90% | nd | nd | nd |
| OR Quart 2 | 0.74 | nd | 0.62 | 0.40 | nd | 0.50 | nd | nd | nd |
| p Value | 0.70 | nd | 0.59 | 0.26 | nd | 0.38 | nd | nd | nd |
| 95% CI of OR Quart2 | 0.16 – 3.4 | nd | 0.11 – 3.5 | 0.082 – 1.9 | nd | 0.11 – 2.3 | nd | nd | nd |
| OR Quart 3 | 0.52 | nd | 0.68 | 0.77 | nd | 0.50 | nd | nd | nd |
| p Value | 0.43 | nd | 0.66 | 0.72 | nd | 0.38 | nd | nd | nd |
| 95% CI of OR Quart3 | 0.10 – 2.6 | nd | 0.12 – 3.8 | 0.19 – 3.2 | nd | 0.11 – 2.3 | nd | nd | nd |
| OR Quart 4 | 2.1 | nd | 1.7 | 1.0 | nd | 1.2 | nd | nd | nd |
| p Value | 0.30 | nd | 0.52 | 1.0 | nd | 0.83 | nd | nd | nd |
| 95% CI of OR Quart4 | 0.52 – 8.3 | nd | 0.35 – 7.9 | 0.25 – 4.0 | nd | 0.28 – 4.9 | nd | nd | nd |

## Poly [ADP-ribose] polymerase 1 (cleaved)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 0.00439 |
| Average | 0.00474 | 0.00294 | 0.00474 | 0.00432 | 0.00474 | 0.00271 |
| Stdev | 0.00688 | 0.00538 | 0.00688 | 0.00586 | 0.00688 | 0.00261 |
| p(t-test) | | 0.11 | | 0.70 | | 0.16 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0183 | 0.0357 | 0.00723 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 118 | 47 | 118 | 54 | 118 | 24 |
| n (Patient) | 97 | 47 | 97 | 54 | 97 | 24 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 1.00E-9 |
| Average | 0.00375 | 0.00338 | 0.00375 | 0.00301 | 0.00375 | 0.00350 |
| Stdev | 0.00582 | 0.00600 | 0.00582 | 0.00648 | 0.00582 | 0.00540 |
| p(t-test) | | 0.82 | | 0.60 | | 0.89 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0253 | 0.0357 | 0.0144 |
| n (Samp) | 263 | 14 | 263 | 19 | 263 | 12 |
| n (Patient) | 159 | 14 | 159 | 19 | 159 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 0.00340 | 1.00E-9 | 0.00471 |
| Average | 0.00356 | 0.00258 | 0.00356 | 0.00527 | 0.00356 | 0.00448 |
| Stdev | 0.00628 | 0.00494 | 0.00628 | 0.00622 | 0.00628 | 0.00455 |
| p(t-test) | | 0.36 | | 0.12 | | 0.51 |
| Min | 1.00E-9 | 1.00E-9 | 1.00E-9 | 1.00E-9 | 1.00E-9 | 1.00E-9 |
| Max | 0.0357 | 0.0144 | 0.0357 | 0.0183 | 0.0357 | 0.0144 |
| n (Samp) | 105 | 45 | 105 | 49 | 105 | 23 |
| n (Patient) | 84 | 45 | 84 | 49 | 84 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | 0.46 | 0.47 | 0.51 | 0.45 | 0.59 | 0.50 | 0.50 | 0.62 |
| SE | 0.050 | 0.081 | 0.052 | 0.048 | 0.070 | 0.050 | 0.065 | 0.086 | 0.068 |
| p | 0.18 | 0.63 | 0.51 | 0.85 | 0.52 | 0.060 | 0.97 | 0.97 | 0.071 |
| nCohort 1 | 118 | 263 | 105 | 118 | 263 | 105 | 118 | 263 | 105 |
| nCohort 2 | 47 | 14 | 45 | 54 | 19 | 49 | 24 | 12 | 23 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.00598 | 0.00471 | 0.00406 | 0.00598 | 0.00471 | 0.00406 | 0.00598 | 0.00471 | 0.00406 |
| Sens 4 | 17% | 21% | 24% | 24% | 16% | 47% | 4% | 17% | 61% |
| Spec 4 | 70% | 74% | 70% | 70% | 74% | 70% | 70% | 74% | 70% |
| Cutoff 5 | 0.0141 | 0.00723 | 0.00681 | 0.0141 | 0.00723 | 0.00681 | 0.0141 | 0.00723 | 0.00681 |
| Sens 5 | 11% | 21% | 13% | 19% | 11% | 31% | 0% | 17% | 17% |
| Spec 5 | 81% | 81% | 80% | 81% | 81% | 80% | 81% | 81% | 80% |
| Cutoff 6 | 0.0144 | 0.0144 | 0.0144 | 0.0144 | 0.0144 | 0.0144 | 0.0144 | 0.0144 | 0.0144 |
| Sens 6 | 0% | 0% | 0% | 2% | 5% | 2% | 0% | 0% | 0% |
| Spec 6 | 95% | 97% | 99% | 95% | 97% | 99% | 95% | 97% | 99% |
| OR Quart 2 | 0.59 | 0.33 | 1.3 | 2.3 | 1.0 | 1.3 | 18 | 1.5 | 1.0 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.39 | 0.34 | 0.56 | 0.073 | 0.99 | 0.63 | 0.0069 | 0.65 | 1.0 |
| 95% CI of | 0.18 | 0.033 | 0.50 | 0.93 | 0.20 | 0.45 | 2.2 | 0.25 | 0.23 |
| OR Quart2 | 2.0 | 3.2 | 3.6 | 5.7 | 5.2 | 3.7 | 150 | 9.4 | 4.4 |
| OR Quart 3 | 7.4 | 3.4 | 1.0 | 1.3 | 4.6 | 2.2 | 5.6 | 3.8 | 2.3 |
| p Value | 8.9E-5 | 0.080 | 1.0 | 0.63 | 0.022 | 0.13 | 0.12 | 0.11 | 0.21 |
| 95% CI of | 2.7 | 0.87 | 0.36 | 0.49 | 1.2 | 0.79 | 0.62 | 0.76 | 0.62 |
| OR Quart3 | 20 | 13 | 2.8 | 3.2 | 17 | 6.1 | 51 | 19 | 8.7 |
| OR Quart 4 | 1.0 | 0.33 | 1.5 | 1.0 | 0.33 | 2.9 | 7.2 | 0 | 2.0 |
| p Value | 0.96 | 0.34 | 0.41 | 1.0 | 0.34 | 0.038 | 0.074 | na | 0.33 |
| 95% CI of | 0.35 | 0.033 | 0.56 | 0.38 | 0.033 | 1.1 | 0.82 | na | 0.51 |
| OR Quart4 | 3.1 | 3.2 | 4.1 | 2.6 | 3.2 | 7.9 | 64 | na | 7.5 |

## KSP-Cadherin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.03 | 1.24 | 1.03 | 1.15 | nd | nd |
| Average | 1.47 | 1.58 | 1.47 | 1.90 | nd | nd |
| Stdev | 1.59 | 1.15 | 1.59 | 1.74 | nd | nd |
| p(t-test) | | 0.71 | | 0.25 | nd | nd |
| Min | 0.00263 | 0.0646 | 0.00263 | 0.291 | nd | nd |
| Max | 11.9 | 4.63 | 11.9 | 7.51 | nd | nd |
| n (Samp) | 85 | 32 | 85 | 25 | nd | nd |
| n (Patient) | 68 | 32 | 68 | 25 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.10 | 0.683 | 1.10 | 1.04 | nd | nd |
| Average | 1.58 | 1.36 | 1.58 | 1.60 | nd | nd |
| Stdev | 1.55 | 1.28 | 1.55 | 1.45 | nd | nd |
| p(t-test) | | 0.66 | | 0.98 | nd | nd |
| Min | 0.00263 | 0.0646 | 0.00263 | 0.291 | nd | nd |
| Max | 11.9 | 3.49 | 11.9 | 4.08 | nd | nd |
| n (Samp) | 152 | 10 | 152 | 6 | nd | nd |
| n (Patient) | 114 | 10 | 114 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.02 | 1.40 | 1.02 | 1.61 | nd | nd |
| Average | 1.55 | 1.73 | 1.55 | 2.07 | nd | nd |
| Stdev | 1.72 | 1.11 | 1.72 | 1.77 | nd | nd |
| p(t-test) | | 0.61 | | 0.19 | nd | nd |
| Min | 0.00263 | 0.557 | 0.00263 | 0.371 | nd | nd |
| Max | 11.9 | 4.63 | 11.9 | 7.51 | nd | nd |
| n (Samp) | 73 | 27 | 73 | 25 | nd | nd |
| n (Patient) | 59 | 27 | 59 | 25 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.44 | 0.62 | 0.58 | 0.50 | 0.60 | nd | nd | nd |
| SE | 0.061 | 0.097 | 0.065 | 0.067 | 0.12 | 0.068 | nd | nd | nd |
| p | 0.25 | 0.53 | 0.068 | 0.26 | 1.00 | 0.12 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 85 | 152 | 73 | 85 | 152 | 73 | nd | nd | nd |
| nCohort 2 | 32 | 10 | 27 | 25 | 6 | 25 | nd | nd | nd |
| Cutoff 1 | 0.946 | 0.622 | 0.996 | 0.845 | 0.569 | 0.845 | nd | nd | nd |
| Sens 1 | 72% | 70% | 70% | 72% | 83% | 72% | nd | nd | nd |
| Spec 1 | 46% | 25% | 48% | 40% | 22% | 41% | nd | nd | nd |
| Cutoff 2 | 0.734 | 0.380 | 0.935 | 0.569 | 0.569 | 0.723 | nd | nd | nd |
| Sens 2 | 81% | 80% | 81% | 80% | 83% | 80% | nd | nd | nd |
| Spec 2 | 35% | 14% | 47% | 22% | 22% | 34% | nd | nd | nd |
| Cutoff 3 | 0.555 | 0.0646 | 0.739 | 0.398 | 0.252 | 0.426 | nd | nd | nd |
| Sens 3 | 91% | 90% | 93% | 92% | 100% | 92% | nd | nd | nd |
| Spec 3 | 21% | 3% | 36% | 15% | 8% | 16% | nd | nd | nd |
| Cutoff 4 | 1.66 | 1.69 | 1.71 | 1.66 | 1.69 | 1.71 | nd | nd | nd |
| Sens 4 | 28% | 40% | 30% | 40% | 33% | 40% | nd | nd | nd |
| Spec 4 | 71% | 70% | 71% | 71% | 70% | 71% | nd | nd | nd |
| Cutoff 5 | 2.10 | 2.45 | 2.19 | 2.10 | 2.45 | 2.19 | nd | nd | nd |
| Sens 5 | 25% | 30% | 26% | 36% | 33% | 36% | nd | nd | nd |
| Spec 5 | 80% | 80% | 81% | 80% | 80% | 81% | nd | nd | nd |
| Cutoff 6 | 2.74 | 3.39 | 3.17 | 2.74 | 3.39 | 3.17 | nd | nd | nd |
| Sens 6 | 16% | 10% | 11% | 16% | 17% | 20% | nd | nd | nd |
| Spec 6 | 91% | 90% | 90% | 91% | 90% | 90% | nd | nd | nd |
| OR Quart 2 | 2.2 | 0.32 | 16 | 0.76 | 0.47 | 0.95 | nd | nd | nd |
| p Value | 0.22 | 0.34 | 0.012 | 0.69 | 0.55 | 0.94 | nd | nd | nd |
| 95% CI of OR Quart2 | 0.62 7.5 | 0.032 3.3 | 1.9 140 | 0.20 2.9 | 0.041 5.5 | 0.24 3.8 | nd | nd | nd |
| OR Quart 3 | 2.5 | 1.0 | 14 | 0.80 | 0.49 | 1.3 | nd | nd | nd |
| p Value | 0.14 | 1.0 | 0.018 | 0.74 | 0.56 | 0.73 | nd | nd | nd |
| 95% CI of OR Quart3 | 0.74 8.6 | 0.19 5.3 | 1.6 120 | 0.21 3.0 | 0.042 5.6 | 0.33 4.9 | nd | nd | nd |
| OR Quart 4 | 1.7 | 1.0 | 9.3 | 1.7 | 0.97 | 2.1 | nd | nd | nd |
| p Value | 0.39 | 0.97 | 0.045 | 0.41 | 0.98 | 0.24 | nd | nd | nd |
| 95% CI of OR Quart4 | 0.50 6.1 | 0.19 5.4 | 1.1 83 | 0.50 5.5 | 0.13 7.3 | 0.59 7.7 | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.124 | 0.573 | 0.124 | 0.395 | nd | nd |
| Average | 0.891 | 1.21 | 0.891 | 1.09 | nd | nd |
| Stdev | 1.49 | 1.55 | 1.49 | 1.82 | nd | nd |
| p(t-test) | | 0.43 | | 0.64 | nd | nd |
| Min | 0.00360 | 0.0182 | 0.00360 | 0.00464 | nd | nd |
| Max | 6.71 | 4.65 | 6.71 | 6.48 | nd | nd |
| n (Samp) | 52 | 20 | 52 | 20 | nd | nd |
| n (Patient) | 41 | 20 | 41 | 20 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.106 | 0.709 | 0.106 | 0.464 | nd | nd |
| Average | 0.595 | 1.40 | 0.595 | 1.25 | nd | nd |
| Stdev | 1.03 | 1.68 | 1.03 | 1.96 | nd | nd |
| p(t-test) | | 0.031 | | 0.087 | nd | nd |
| Min | 0.00360 | 0.0573 | 0.00360 | 0.0172 | nd | nd |
| Max | 4.38 | 4.65 | 4.38 | 6.48 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 42 | 16 | 42 | 21 | nd | nd |
| n (Patient) | 33 | 16 | 33 | 21 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | nd | 0.71 | 0.62 | nd | 0.68 | nd | nd | nd |
| SE | 0.076 | nd | 0.081 | 0.076 | nd | 0.074 | nd | nd | nd |
| p | 0.064 | nd | 0.0099 | 0.12 | nd | 0.014 | nd | nd | nd |
| nCohort 1 | 52 | nd | 42 | 52 | nd | 42 | nd | nd | nd |
| nCohort 2 | 20 | nd | 16 | 20 | nd | 21 | nd | nd | nd |
| Cutoff 1 | 0.182 | nd | 0.107 | 0.154 | nd | 0.154 | nd | nd | nd |
| Sens 1 | 70% | nd | 75% | 70% | nd | 71% | nd | nd | nd |
| Spec 1 | 54% | nd | 52% | 52% | nd | 55% | nd | nd | nd |
| Cutoff 2 | 0.0761 | nd | 0.0761 | 0.141 | nd | 0.141 | nd | nd | nd |
| Sens 2 | 80% | nd | 81% | 80% | nd | 81% | nd | nd | nd |
| Spec 2 | 40% | nd | 40% | 52% | nd | 55% | nd | nd | nd |
| Cutoff 3 | 0.0591 | nd | 0.0591 | 0.0796 | nd | 0.101 | nd | nd | nd |
| Sens 3 | 90% | nd | 94% | 90% | nd | 90% | nd | nd | nd |
| Spec 3 | 38% | nd | 38% | 44% | nd | 48% | nd | nd | nd |
| Cutoff 4 | 0.786 | nd | 0.398 | 0.786 | nd | 0.398 | nd | nd | nd |
| Sens 4 | 45% | nd | 56% | 30% | nd | 52% | nd | nd | nd |
| Spec 4 | 71% | nd | 71% | 71% | nd | 71% | nd | nd | nd |
| Cutoff 5 | 1.56 | nd | 0.819 | 1.56 | nd | 0.819 | nd | nd | nd |
| Sens 5 | 25% | nd | 50% | 15% | nd | 33% | nd | nd | nd |
| Spec 5 | 81% | nd | 81% | 81% | nd | 81% | nd | nd | nd |
| Cutoff 6 | 3.11 | nd | 2.19 | 3.11 | nd | 2.19 | nd | nd | nd |
| Sens 6 | 15% | nd | 25% | 10% | nd | 14% | nd | nd | nd |
| Spec 6 | 90% | nd | 90% | 90% | nd | 90% | nd | nd | nd |
| OR Quart 2 | 8.5 | nd | >7.0 | 11 | nd | 11 | nd | nd | nd |
| p Value | 0.061 | nd | <0.097 | 0.036 | nd | 0.038 | nd | nd | nd |
| 95% CI of OR Quart2 | 0.90 80 | nd | >0.71 na | 1.2 100 | nd | 1.1 100 | nd | nd | nd |
| OR Quart 3 | 11 | nd | >3.8 | 11 | nd | 8.4 | nd | nd | nd |
| p Value | 0.036 | nd | <0.27 | 0.036 | nd | 0.066 | nd | nd | nd |
| 95% CI of OR Quart3 | 1.2 100 | nd | >0.35 na | 1.2 100 | nd | 0.87 81 | nd | nd | nd |
| OR Quart 4 | 8.5 | nd | >16 | 6.5 | nd | 11 | nd | nd | nd |
| p Value | 0.061 | nd | <0.017 | 0.10 | nd | 0.038 | nd | nd | nd |
| 95% CI of OR Quart4 | 0.90 80 | nd | >1.7 na | 0.68 63 | nd | 1.1 100 | nd | nd | nd |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 73.2 | 102 | 73.2 | 116 | 73.2 | 81.7 |
| Average | 185 | 272 | 185 | 697 | 185 | 87.4 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 645 | 1020 | 645 | 3530 | 645 | 69.4 |
| p(t-test) | | 0.44 | | 0.0018 | | 0.44 |
| Min | 2.08 | 8.69 | 2.08 | 10.8 | 2.08 | 1.00E-9 |
| Max | 6400 | 6400 | 6400 | 24300 | 6400 | 301 |
| n (Samp) | 685 | 38 | 685 | 47 | 685 | 26 |
| n (Patient) | 283 | 38 | 283 | 47 | 283 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 76.6 | 106 | 76.6 | 127 | 76.6 | 98.8 |
| Average | 198 | 891 | 198 | 176 | 198 | 108 |
| Stdev | 990 | 2230 | 990 | 158 | 990 | 84.1 |
| p(t-test) | | 0.053 | | 0.93 | | 0.74 |
| Min | 1.00E-9 | 34.5 | 1.00E-9 | 24.0 | 1.00E-9 | 6.18 |
| Max | 24300 | 6400 | 24300 | 613 | 24300 | 288 |
| n (Samp) | 891 | 8 | 891 | 13 | 891 | 13 |
| n (Patient) | 334 | 8 | 334 | 13 | 334 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 76.6 | 107 | 76.6 | 131 | 76.6 | 98.7 |
| Average | 195 | 297 | 195 | 789 | 195 | 105 |
| Stdev | 649 | 1060 | 649 | 3780 | 649 | 74.2 |
| p(t-test) | | 0.39 | | 0.0017 | | 0.52 |
| Min | 2.08 | 8.69 | 2.08 | 10.8 | 2.08 | 1.00E-9 |
| Max | 6400 | 6400 | 6400 | 24300 | 6400 | 301 |
| n (Samp) | 553 | 35 | 553 | 41 | 553 | 22 |
| n (Patient) | 202 | 35 | 202 | 41 | 202 | 22 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.60 | 0.60 | 0.66 | 0.66 | 0.67 | 0.47 | 0.51 | 0.53 |
| SE | 0.050 | 0.11 | 0.052 | 0.045 | 0.083 | 0.048 | 0.059 | 0.081 | 0.064 |
| p | 0.069 | 0.36 | 0.053 | 3.3E-4 | 0.055 | 2.3E-4 | 0.60 | 0.87 | 0.61 |
| nCohort 1 | 685 | 891 | 553 | 685 | 891 | 553 | 685 | 891 | 553 |
| nCohort 2 | 38 | 8 | 35 | 47 | 13 | 41 | 26 | 13 | 22 |
| Cutoff 1 | 70.0 | 59.9 | 76.9 | 85.8 | 85.8 | 99.4 | 39.3 | 39.3 | 56.5 |
| Sens 1 | 71% | 75% | 71% | 70% | 77% | 71% | 73% | 77% | 73% |
| Spec 1 | 48% | 37% | 50% | 59% | 56% | 63% | 22% | 21% | 35% |
| Cutoff 2 | 56.5 | 49.4 | 69.8 | 66.5 | 66.5 | 72.6 | 31.3 | 31.3 | 43.2 |
| Sens 2 | 82% | 88% | 80% | 81% | 85% | 80% | 81% | 85% | 82% |
| Spec 2 | 38% | 30% | 45% | 46% | 43% | 47% | 14% | 13% | 22% |
| Cutoff 3 | 33.5 | 34.3 | 33.5 | 43.2 | 43.2 | 44.8 | 11.0 | 16.2 | 27.1 |
| Sens 3 | 92% | 100% | 91% | 91% | 92% | 90% | 92% | 92% | 91% |
| Spec 3 | 16% | 16% | 14% | 26% | 24% | 24% | 2% | 3% | 9% |
| Cutoff 4 | 110 | 122 | 118 | 110 | 122 | 118 | 110 | 122 | 118 |
| Sens 4 | 42% | 50% | 46% | 53% | 62% | 54% | 31% | 38% | 36% |
| Spec 4 | 70% | 70% | 70% | 70% | 70% | 70% | 70% | 70% | 70% |
| Cutoff 5 | 148 | 154 | 154 | 148 | 154 | 154 | 148 | 154 | 154 |
| Sens 5 | 18% | 38% | 20% | 38% | 31% | 41% | 19% | 23% | 14% |
| Spec 5 | 80% | 80% | 80% | 80% | 80% | 80% | 80% | 80% | 80% |
| Cutoff 6 | 226 | 229 | 248 | 226 | 229 | 248 | 226 | 229 | 248 |
| Sens 6 | 11% | 12% | 11% | 21% | 31% | 24% | 4% | 8% | 5% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 6 | 90% | 90% | 90% | 90% | 90% | 90% | 90% | 90% | 90% |
| OR Quart 2 | 1.2 | 2.0 | 1.2 | 1.4 | 0.50 | 0.49 | 1.3 | 0 | 0.79 |
| p Value | 0.79 | 0.57 | 0.76 | 0.56 | 0.57 | 0.31 | 0.59 | na | 0.73 |
| 95% CI of OR Quart2 | 0.38 | 0.18 | 0.36 | 0.44 | 0.045 | 0.12 | 0.46 | na | 0.21 |
| | 3.5 | 22 | 4.1 | 4.5 | 5.5 | 2.0 | 4.0 | na | 3.0 |
| OR Quart 3 | 2.2 | 2.0 | 2.5 | 3.2 | 2.5 | 2.3 | 0.49 | 0.59 | 1.2 |
| p Value | 0.11 | 0.57 | 0.090 | 0.028 | 0.27 | 0.10 | 0.32 | 0.48 | 0.77 |
| 95% CI of OR Quart3 | 0.83 | 0.18 | 0.87 | 1.1 | 0.49 | 0.84 | 0.12 | 0.14 | 0.36 |
| | 6.0 | 22 | 7.4 | 8.9 | 13 | 6.2 | 2.0 | 2.5 | 4.0 |
| OR Quart 4 | 2.1 | 3.0 | 2.5 | 4.4 | 2.5 | 3.5 | 1.5 | 1.0 | 1.4 |
| p Value | 0.16 | 0.34 | 0.090 | 0.0040 | 0.27 | 0.010 | 0.43 | 1.0 | 0.57 |
| 95% CI of OR Quart4 | 0.76 | 0.31 | 0.87 | 1.6 | 0.49 | 1.3 | 0.53 | 0.29 | 0.44 |
| | 5.6 | 29 | 7.4 | 12 | 13 | 8.9 | 4.4 | 3.5 | 4.6 |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.02 | 0.826 | nd | nd | nd | nd |
| Average | 1.20 | 0.896 | nd | nd | nd | nd |
| Stdev | 1.22 | 0.687 | nd | nd | nd | nd |
| p(t-test) | | 0.49 | nd | nd | nd | nd |
| Min | 0.0223 | 0.0223 | nd | nd | nd | nd |
| Max | 4.68 | 2.33 | nd | nd | nd | nd |
| n (Samp) | 55 | 8 | nd | nd | nd | nd |
| n (Patient) | 35 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 0.826 | nd | nd | nd | nd |
| Average | 1.20 | 0.896 | nd | nd | nd | nd |
| Stdev | 1.19 | 0.687 | nd | nd | nd | nd |
| p(t-test) | | 0.50 | nd | nd | nd | nd |
| Min | 0.0223 | 0.0223 | nd | nd | nd | nd |
| Max | 4.46 | 2.33 | nd | nd | nd | nd |
| n (Samp) | 41 | 8 | nd | nd | nd | nd |
| n (Patient) | 24 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.46 | nd | nd | nd | nd | nd | nd |
| SE | 0.11 | nd | 0.11 | nd | nd | nd | nd | nd | nd |
| p | 0.74 | nd | 0.76 | nd | nd | nd | nd | nd | nd |
| nCohort 1 | 55 | nd | 41 | nd | nd | nd | nd | nd | nd |
| nCohort 2 | 8 | nd | 8 | nd | nd | nd | nd | nd | nd |
| Cutoff 1 | 0.496 | nd | 0.496 | nd | nd | nd | nd | nd | nd |
| Sens 1 | 75% | nd | 75% | nd | nd | nd | nd | nd | nd |
| Spec 1 | 40% | nd | 41% | nd | nd | nd | nd | nd | nd |
| Cutoff 2 | 0.298 | nd | 0.298 | nd | nd | nd | nd | nd | nd |
| Sens 2 | 88% | nd | 88% | nd | nd | nd | nd | nd | nd |
| Spec 2 | 36% | nd | 37% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 0% | nd | 0% | nd | nd | nd | nd | nd | nd |
| Cutoff 4 | 1.54 | nd | 1.54 | nd | nd | nd | nd | nd | nd |
| Sens 4 | 12% | nd | 12% | nd | nd | nd | nd | nd | nd |
| Spec 4 | 71% | nd | 71% | nd | nd | nd | nd | nd | nd |
| Cutoff 5 | 2.07 | nd | 2.07 | nd | nd | nd | nd | nd | nd |
| Sens 5 | 12% | nd | 12% | nd | nd | nd | nd | nd | nd |
| Spec 5 | 82% | nd | 85% | nd | nd | nd | nd | nd | nd |
| Cutoff 6 | 2.59 | nd | 2.59 | nd | nd | nd | nd | nd | nd |
| Sens 6 | 0% | nd | 0% | nd | nd | nd | nd | nd | nd |
| Spec 6 | 91% | nd | 90% | nd | nd | nd | nd | nd | nd |
| OR Quart 2 | 1.0 | nd | 2.4 | nd | nd | nd | nd | nd | nd |
| p Value | 1.0 | nd | 0.50 | nd | nd | nd | nd | nd | nd |
| 95% CI of OR Quart2 | 0.057 18 | nd | 0.19 31 | nd | nd | nd | nd | nd | nd |
| OR Quart 3 | 6.8 | nd | 6.0 | nd | nd | nd | nd | nd | nd |
| p Value | 0.099 | nd | 0.14 | nd | nd | nd | nd | nd | nd |
| 95% CI of OR Quart3 | 0.69 67 | nd | 0.56 64 | nd | nd | nd | nd | nd | nd |
| OR Quart 4 | 1.1 | nd | 1.1 | nd | nd | nd | nd | nd | nd |
| p Value | 0.96 | nd | 0.95 | nd | nd | nd | nd | nd | nd |
| 95% CI of OR Quart4 | 0.061 19 | nd | 0.061 20 | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.547 | nd | nd | nd | nd |
| Average | 0.965 | 0.998 | nd | nd | nd | nd |
| Stdev | 2.44 | 1.52 | nd | nd | nd | nd |
| p(t-test) | | 0.97 | nd | nd | nd | nd |
| Min | 0.0360 | 0.0360 | nd | nd | nd | nd |
| Max | 19.5 | 4.64 | nd | nd | nd | nd |
| n (Samp) | 78 | 8 | nd | nd | nd | nd |
| n (Patient) | 56 | 8 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.469 | 0.547 | nd | nd | nd | nd |
| Average | 1.12 | 0.998 | nd | nd | nd | nd |
| Stdev | 2.73 | 1.52 | nd | nd | nd | nd |
| p(t-test) | | 0.90 | nd | nd | nd | nd |
| Min | 0.0360 | 0.0360 | nd | nd | nd | nd |
| Max | 19.5 | 4.64 | nd | nd | nd | nd |
| n (Samp) | 61 | 8 | nd | nd | nd | nd |
| n (Patient) | 43 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | nd | 0.50 | nd | nd | nd | nd | nd | nd |
| SE | 0.11 | nd | 0.11 | nd | nd | nd | nd | nd | nd |
| p | 0.73 | nd | 0.98 | nd | nd | nd | nd | nd | nd |
| nCohort 1 | 78 | nd | 61 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 8 | nd | 8 | nd | nd | nd | nd | nd | nd |
| Cutoff 1 | 0.208 | nd | 0.208 | nd | nd | nd | nd | nd | nd |
| Sens 1 | 75% | nd | 75% | nd | nd | nd | nd | nd | nd |
| Spec 1 | 36% | nd | 28% | nd | nd | nd | nd | nd | nd |
| Cutoff 2 | 0 | nd | 0 | nd | nd | nd | nd | nd | nd |
| Sens 2 | 100% | nd | 100% | nd | nd | nd | nd | nd | nd |
| Spec 2 | 0% | nd | 0% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.781 | nd | 0.781 | nd | nd | nd | nd | nd | nd |
| Sens 4 | 38% | nd | 38% | nd | nd | nd | nd | nd | nd |
| Spec 4 | 72% | nd | 70% | nd | nd | nd | nd | nd | nd |
| Cutoff 5 | 0.955 | nd | 0.955 | nd | nd | nd | nd | nd | nd |
| Sens 5 | 25% | nd | 25% | nd | nd | nd | nd | nd | nd |
| Spec 5 | 81% | nd | 80% | nd | nd | nd | nd | nd | nd |
| Cutoff 6 | 1.66 | nd | 1.49 | nd | nd | nd | nd | nd | nd |
| Sens 6 | 12% | nd | 12% | nd | nd | nd | nd | nd | nd |
| Spec 6 | 91% | nd | 90% | nd | nd | nd | nd | nd | nd |
| OR Quart 2 | 0.95 | nd | 0.47 | nd | nd | nd | nd | nd | nd |
| p Value | 0.96 | nd | 0.55 | nd | nd | nd | nd | nd | nd |
| 95% CI of OR Quart2 | 0.12 7.4 | nd | 0.038 5.7 | nd | nd | nd | nd | nd | nd |
| OR Quart 3 | 1.0 | nd | 1.0 | nd | nd | nd | nd | nd | nd |
| p Value | 1.0 | nd | 1.0 | nd | nd | nd | nd | nd | nd |
| 95% CI of OR Quart3 | 0.13 7.9 | nd | 0.12 8.1 | nd | nd | nd | nd | nd | nd |
| OR Quart 4 | 0.95 | nd | 1.5 | nd | nd | nd | nd | nd | nd |
| p Value | 0.96 | nd | 0.68 | nd | nd | nd | nd | nd | nd |
| 95% CI of OR Quart4 | 0.12 7.4 | nd | 0.22 10 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 57300 | 62400 | nd | nd |
| Average | nd | nd | 102000 | 78500 | nd | nd |
| Stdev | nd | nd | 128000 | 81700 | nd | nd |
| p(t-test) | nd | nd | | 0.47 | nd | nd |
| Min | nd | nd | 160 | 1620 | nd | nd |
| Max | nd | nd | 744000 | 260000 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 58600 | 59500 | nd | nd |
| Average | nd | nd | 104000 | 82000 | nd | nd |
| Stdev | nd | nd | 134000 | 82800 | nd | nd |
| p(t-test) | nd | nd | | 0.54 | nd | nd |
| Min | nd | nd | 160 | 1620 | nd | nd |
| Max | nd | nd | 744000 | 260000 | nd | nd |
| n (Samp) | nd | nd | 81 | 15 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.44 | nd | 0.47 | nd | nd | nd |
| SE | nd | nd | nd | 0.080 | nd | 0.082 | nd | nd | nd |
| p | nd | nd | nd | 0.49 | nd | 0.72 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 12500 | nd | 12900 | nd | nd | nd |
| Sens 1 | nd | nd | nd | 75% | nd | 73% | nd | nd | nd |
| Spec 1 | nd | nd | nd | 19% | nd | 22% | nd | nd | nd |
| Cutoff 2 | nd | nd | nd | 9460 | nd | 11700 | nd | nd | nd |
| Sens 2 | nd | nd | nd | 81% | nd | 80% | nd | nd | nd |
| Spec 2 | nd | nd | nd | 11% | nd | 22% | nd | nd | nd |
| Cutoff 3 | nd | nd | nd | 1660 | nd | 1660 | nd | nd | nd |
| Sens 3 | nd | nd | nd | 94% | nd | 93% | nd | nd | nd |
| Spec 3 | nd | nd | nd | 2% | nd | 2% | nd | nd | nd |
| Cutoff 4 | nd | nd | nd | 128000 | nd | 125000 | nd | nd | nd |
| Sens 4 | nd | nd | nd | 25% | nd | 27% | nd | nd | nd |
| Spec 4 | nd | nd | nd | 71% | nd | 70% | nd | nd | nd |
| Cutoff 5 | nd | nd | nd | 141000 | nd | 144000 | nd | nd | nd |
| Sens 5 | nd | nd | nd | 25% | nd | 27% | nd | nd | nd |
| Spec 5 | nd | nd | nd | 80% | nd | 80% | nd | nd | nd |
| Cutoff 6 | nd | nd | nd | 219000 | nd | 219000 | nd | nd | nd |
| Sens 6 | nd | nd | nd | 6% | nd | 7% | nd | nd | nd |
| Spec 6 | nd | nd | nd | 91% | nd | 90% | nd | nd | nd |
| OR Quart 2 | nd | nd | nd | 1.3 | nd | 1.0 | nd | nd | nd |
| p Value | nd | nd | nd | 0.72 | nd | 1.0 | nd | nd | nd |
| 95% CI of OR Quart2 | nd | nd | nd | 0.31 | nd | 0.22 | nd | nd | nd |
| | nd | nd | nd | 5.5 | nd | 4.6 | nd | nd | nd |
| OR Quart 3 | nd | nd | nd | 0 | nd | 0.45 | nd | nd | nd |
| p Value | nd | nd | nd | na | nd | 0.39 | nd | nd | nd |
| 95% CI of OR Quart3 | nd | nd | nd | na | nd | 0.075 | nd | nd | nd |
| | nd | nd | nd | na | nd | 2.8 | nd | nd | nd |
| OR Quart 4 | nd | nd | nd | 2.0 | nd | 1.3 | nd | nd | nd |
| p Value | nd | nd | nd | 0.32 | nd | 0.71 | nd | nd | nd |
| 95% CI of OR Quart4 | nd | nd | nd | 0.51 | nd | 0.31 | nd | nd | nd |
| | nd | nd | nd | 7.8 | nd | 5.6 | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.09 | 3.35 | 1.09 | 4.76 | 1.09 | 2.45 |
| Average | 62.1 | 42.8 | 62.1 | 57.1 | 62.1 | 13.0 |
| Stdev | 497 | 133 | 497 | 173 | 497 | 18.2 |
| p(t-test) | | 0.85 | | 0.95 | | 0.68 |
| Min | 1.14E-14 | 1.70E-14 | 1.14E-14 | 0.116 | 1.14E-14 | 0.116 |
| Max | 6950 | 630 | 6950 | 907 | 6950 | 52.9 |
| n (Samp) | 302 | 24 | 302 | 35 | 302 | 17 |
| n (Patient) | 164 | 24 | 164 | 35 | 164 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.23 | 7.84 | 1.23 | 9.80 |
| Average | nd | nd | 58.3 | 15.2 | 58.3 | 15.4 |
| Stdev | nd | nd | 447 | 17.6 | 447 | 16.5 |
| p(t-test) | nd | nd | | 0.80 | | 0.80 |
| Min | nd | nd | 1.14E-14 | 0.116 | 1.14E-14 | 0.116 |
| Max | nd | nd | 6950 | 42.1 | 6950 | 39.2 |
| n (Samp) | nd | nd | 380 | 7 | 380 | 7 |
| n (Patient) | nd | nd | 196 | 7 | 196 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.850 | 3.35 | 0.850 | 2.87 | 0.850 | 2.66 |
| Average | 70.1 | 42.8 | 70.1 | 62.4 | 70.1 | 12.3 |
| Stdev | 538 | 133 | 538 | 183 | 538 | 17.6 |
| p(t-test) | | 0.80 | | 0.94 | | 0.67 |
| Min | 1.14E-14 | 1.70E-14 | 1.14E-14 | 0.116 | 1.14E-14 | 0.116 |
| Max | 6950 | 630 | 6950 | 907 | 6950 | 52.9 |
| n (Samp) | 257 | 24 | 257 | 31 | 257 | 16 |
| n (Patient) | 134 | 24 | 134 | 31 | 134 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | 0.54 | 0.59 | 0.64 | 0.58 | 0.55 | 0.60 | 0.59 |
| SE | 0.063 | nd | 0.063 | 0.053 | 0.11 | 0.056 | 0.074 | 0.11 | 0.077 |
| p | 0.46 | nd | 0.50 | 0.080 | 0.21 | 0.16 | 0.49 | 0.36 | 0.24 |
| nCohort 1 | 302 | nd | 257 | 302 | 380 | 257 | 302 | 380 | 257 |
| nCohort 2 | 24 | nd | 24 | 35 | 7 | 31 | 17 | 7 | 16 |
| Cutoff 1 | 1.70E-14 | nd | 1.70E-14 | 0.713 | 6.55 | 1.70E-14 | 1.70E-14 | 2.02 | 0.116 |
| Sens 1 | 96% | nd | 96% | 71% | 71% | 100% | 100% | 71% | 75% |
| Spec 1 | 2% | nd | 2% | 47% | 65% | 2% | 2% | 52% | 45% |
| Cutoff 2 | 1.70E-14 | nd | 1.70E-14 | 1.70E-14 | 0.850 | 1.70E-14 | 1.70E-14 | 1.70E-14 | 1.70E-14 |
| Sens 2 | 96% | nd | 96% | 100% | 86% | 100% | 100% | 100% | 100% |
| Spec 2 | 2% | nd | 2% | 2% | 47% | 2% | 2% | 2% | 2% |
| Cutoff 3 | 1.70E-14 | nd | 1.70E-14 | 1.70E-14 | 1.70E-14 | 1.70E-14 | 1.70E-14 | 1.70E-14 | 1.70E-14 |
| Sens 3 | 96% | nd | 96% | 100% | 100% | 100% | 100% | 100% | 100% |
| Spec 3 | 2% | nd | 2% | 2% | 2% | 2% | 2% | 2% | 2% |
| Cutoff 4 | 7.84 | nd | 7.29 | 7.84 | 9.35 | 7.29 | 7.84 | 9.35 | 7.29 |
| Sens 4 | 38% | nd | 38% | 43% | 29% | 42% | 41% | 57% | 44% |
| Spec 4 | 70% | nd | 70% | 70% | 71% | 70% | 70% | 71% | 70% |
| Cutoff 5 | 16.1 | nd | 13.9 | 16.1 | 18.3 | 13.9 | 16.1 | 18.3 | 13.9 |
| Sens 5 | 21% | nd | 21% | 29% | 29% | 32% | 24% | 43% | 31% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | nd | 80% | 80% | 80% | 80% | 80% | 80% | 80% |
| Cutoff 6 | 43.4 | nd | 44.8 | 43.4 | 48.9 | 44.8 | 43.4 | 48.9 | 44.8 |
| Sens 6 | 12% | nd | 12% | 11% | 0% | 13% | 12% | 0% | 12% |
| Spec 6 | 90% | nd | 90% | 90% | 90% | 90% | 90% | 90% | 90% |
| OR Quart 2 | 0.23 | nd | 0 | 1.4 | 0.99 | 2.9 | >7.6 | 0 | 0.24 |
| p Value | 0.067 | nd | na | 0.55 | 0.99 | 0.13 | <0.061 | na | 0.21 |
| 95% CI of | 0.047 | nd | na | 0.44 | 0.061 | 0.73 | >0.91 | na | 0.026 |
| OR Quart2 | 1.1 | nd | na | 4.7 | 16 | 11 | na | na | 2.2 |
| OR Quart 3 | 0.86 | nd | 0.87 | 2.6 | 3.0 | 3.3 | >3.1 | 0.99 | 1.3 |
| p Value | 0.79 | nd | 0.80 | 0.082 | 0.34 | 0.084 | <0.33 | 0.99 | 0.73 |
| 95% CI of | 0.30 | nd | 0.32 | 0.88 | 0.31 | 0.85 | >0.31 | 0.14 | 0.33 |
| OR Quart3 | 2.5 | nd | 2.4 | 7.8 | 30 | 13 | na | 7.2 | 4.9 |
| OR Quart 4 | 0.85 | nd | 0.74 | 2.3 | 2.0 | 4.1 | >7.6 | 1.5 | 1.5 |
| p Value | 0.77 | nd | 0.58 | 0.13 | 0.57 | 0.035 | <0.061 | 0.66 | 0.53 |
| 95% CI of | 0.29 | nd | 0.26 | 0.78 | 0.18 | 1.1 | >0.91 | 0.25 | 0.41 |
| OR Quart4 | 2.5 | nd | 2.1 | 7.1 | 22 | 16 | na | 9.2 | 5.7 |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.852 | 1.70 | 0.852 | 2.56 | 0.852 | 1.46 |
| Average | 12.6 | 4.03 | 12.6 | 5.68 | 12.6 | 4.91 |
| Stdev | 199 | 4.99 | 199 | 9.51 | 199 | 11.3 |
| p(t-test) | | 0.83 | | 0.84 | | 0.87 |
| Min | 0.00336 | 0.00336 | 0.00336 | 0.0950 | 0.00336 | 0.148 |
| Max | 4070 | 19.5 | 4070 | 50.6 | 4070 | 47.4 |
| n (Samp) | 419 | 26 | 419 | 34 | 419 | 17 |
| n (Patient) | 164 | 26 | 164 | 34 | 164 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.978 | 3.62 | 0.978 | 5.87 |
| Average | nd | nd | 11.1 | 5.25 | 11.1 | 6.39 |
| Stdev | nd | nd | 180 | 7.06 | 180 | 4.92 |
| p(t-test) | nd | nd | | 0.93 | | 0.94 |
| Min | nd | nd | 0.00336 | 0.102 | 0.00336 | 0.173 |
| Max | nd | nd | 4070 | 22.0 | 4070 | 14.6 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.852 | 1.48 | 0.852 | 2.42 | 0.852 | 1.12 |
| Average | 14.6 | 3.87 | 14.6 | 5.34 | 14.6 | 4.41 |
| Stdev | 216 | 5.02 | 216 | 9.63 | 216 | 11.3 |
| p(t-test) | | 0.80 | | 0.81 | | 0.85 |
| Min | 0.00336 | 0.0573 | 0.00336 | 0.00336 | 0.00336 | 0.148 |
| Max | 4070 | 19.5 | 4070 | 50.6 | 4070 | 47.4 |
| n (Samp) | 355 | 26 | 355 | 30 | 355 | 17 |
| n (Patient) | 134 | 26 | 134 | 30 | 134 | 17 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.63 | nd | 0.61 | 0.67 | 0.65 | 0.66 | 0.57 | 0.76 | 0.54 |
| SE | 0.060 | nd | 0.061 | 0.052 | 0.11 | 0.056 | 0.074 | 0.11 | 0.073 |
| p | 0.029 | nd | 0.059 | 0.0014 | 0.16 | 0.0056 | 0.34 | 0.016 | 0.58 |
| nCohort 1 | 419 | nd | 355 | 419 | 511 | 355 | 419 | 511 | 355 |
| nCohort 2 | 26 | nd | 26 | 34 | 8 | 30 | 17 | 7 | 17 |
| Cutoff 1 | 0.969 | nd | 0.816 | 0.942 | 2.26 | 0.942 | 0.607 | 3.47 | 0.491 |
| Sens 1 | 73% | nd | 73% | 71% | 75% | 70% | 71% | 71% | 71% |
| Spec 1 | 53% | nd | 50% | 52% | 73% | 51% | 43% | 80% | 39% |
| Cutoff 2 | 0.590 | nd | 0.590 | 0.546 | 0.214 | 0.684 | 0.279 | 2.55 | 0.279 |
| Sens 2 | 81% | nd | 81% | 82% | 88% | 80% | 82% | 86% | 82% |
| Spec 2 | 42% | nd | 43% | 41% | 17% | 47% | 25% | 74% | 25% |
| Cutoff 3 | 0.150 | nd | 0.150 | 0.214 | 0.0990 | 0.444 | 0.150 | 0.172 | 0.150 |
| Sens 3 | 92% | nd | 92% | 91% | 100% | 90% | 94% | 100% | 94% |
| Spec 3 | 12% | nd | 12% | 18% | 7% | 37% | 12% | 14% | 12% |
| Cutoff 4 | 1.76 | nd | 1.84 | 1.76 | 1.97 | 1.84 | 1.76 | 1.97 | 1.84 |
| Sens 4 | 50% | nd | 46% | 59% | 75% | 57% | 41% | 86% | 29% |
| Spec 4 | 70% | nd | 70% | 70% | 70% | 70% | 70% | 70% | 70% |
| Cutoff 5 | 3.16 | nd | 3.25 | 3.16 | 3.51 | 3.25 | 3.16 | 3.51 | 3.25 |
| Sens 5 | 38% | nd | 35% | 44% | 50% | 40% | 24% | 57% | 18% |
| Spec 5 | 80% | nd | 80% | 80% | 80% | 80% | 80% | 80% | 80% |
| Cutoff 6 | 8.38 | nd | 8.86 | 8.38 | 8.51 | 8.86 | 8.38 | 8.51 | 8.86 |
| Sens 6 | 15% | nd | 12% | 15% | 12% | 13% | 12% | 29% | 6% |
| Spec 6 | 90% | nd | 90% | 90% | 90% | 90% | 90% | 90% | 90% |
| OR Quart 2 | 1.3 | nd | 1.7 | 1.5 | 0 | 2.4 | 0.74 | 0 | 1.7 |
| p Value | 0.70 | nd | 0.47 | 0.52 | na | 0.21 | 0.70 | na | 0.47 |
| 95% CI of OR Quart2 | 0.29 | nd | 0.40 | 0.42 | na | 0.61 | 0.16 | na | 0.40 |
|  | 6.2 | nd | 7.3 | 5.6 | na | 9.7 | 3.4 | na | 7.3 |
| OR Quart 3 | 3.2 | nd | 3.2 | 2.1 | 0.99 | 2.4 | 1.3 | 1.0 | 1.7 |
| p Value | 0.090 | nd | 0.088 | 0.24 | 0.99 | 0.21 | 0.73 | 1.0 | 0.47 |
| 95% CI of OR Quart3 | 0.84 | nd | 0.84 | 0.61 | 0.14 | 0.61 | 0.33 | 0.062 | 0.40 |
|  | 12 | nd | 12 | 7.1 | 7.2 | 9.7 | 4.8 | 16 | 7.3 |
| OR Quart 4 | 3.5 | nd | 3.2 | 4.4 | 2.0 | 4.8 | 1.3 | 5.1 | 1.3 |
| p Value | 0.061 | nd | 0.091 | 0.0096 | 0.42 | 0.017 | 0.73 | 0.14 | 0.70 |
| 95% CI of OR Quart4 | 0.94 | nd | 0.83 | 1.4 | 0.36 | 1.3 | 0.33 | 0.59 | 0.29 |
|  | 13 | nd | 12 | 14 | 11 | 17 | 4.8 | 44 | 6.2 |

## Myoglobin

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.860 | 0.477 | 0.860 | 0.126 | 0.860 | 0.176 |
| Average | 35.5 | 18.1 | 35.5 | 44.2 | 35.5 | 6.85 |
| Stdev | 103 | 38.1 | 103 | 130 | 103 | 15.5 |
| p(t-test) |  | 0.39 |  | 0.64 |  | 0.25 |
| Min | 0.000105 | 0.0151 | 0.000105 | 0.000105 | 0.000105 | 0.0254 |
| Max | 618 | 163 | 618 | 469 | 618 | 49.1 |
| n (Samp) | 419 | 26 | 419 | 34 | 419 | 17 |
| n (Patient) | 164 | 26 | 164 | 34 | 164 | 17 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.599 | 4.97 | 0.599 | 0.349 |
| Average | nd | nd | 37.0 | 95.0 | 37.0 | 19.7 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | nd | nd | 109 | 171 | 109 | 28.5 |
| p(t-test) | nd | nd | | 0.14 | | 0.68 |
| Min | nd | nd | 0.000105 | 0.0561 | 0.000105 | 0.0683 |
| Max | nd | nd | 618 | 420 | 618 | 71.6 |
| n (Samp) | nd | nd | 511 | 8 | 511 | 7 |
| n (Patient) | nd | nd | 198 | 8 | 198 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.885 | 0.354 | 0.885 | 0.108 | 0.885 | 0.176 |
| Average | 40.5 | 17.6 | 40.5 | 36.1 | 40.5 | 5.29 |
| Stdev | 112 | 38.2 | 112 | 119 | 112 | 12.1 |
| p(t-test) | | 0.30 | | 0.84 | | 0.20 |
| Min | 0.000105 | 0.0151 | 0.000105 | 0.000105 | 0.000105 | 0.0254 |
| Max | 618 | 163 | 618 | 469 | 618 | 49.1 |
| n (Samp) | 355 | 26 | 355 | 30 | 355 | 17 |
| n (Patient) | 134 | 26 | 134 | 30 | 134 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | nd | 0.45 | 0.39 | 0.57 | 0.38 | 0.40 | 0.55 | 0.39 |
| SE | 0.059 | nd | 0.060 | 0.053 | 0.11 | 0.057 | 0.074 | 0.11 | 0.074 |
| p | 0.72 | nd | 0.36 | 0.045 | 0.53 | 0.031 | 0.16 | 0.67 | 0.15 |
| nCohort 1 | 419 | nd | 355 | 419 | 511 | 355 | 419 | 511 | 355 |
| nCohort 2 | 26 | nd | 26 | 34 | 8 | 30 | 17 | 7 | 17 |
| Cutoff 1 | 0.0772 | nd | 0.0667 | 0.0584 | 0.118 | 0.0562 | 0.0849 | 0.132 | 0.0834 |
| Sens 1 | 73% | nd | 73% | 71% | 75% | 70% | 71% | 71% | 71% |
| Spec 1 | 22% | nd | 20% | 19% | 32% | 18% | 23% | 32% | 22% |
| Cutoff 2 | 0.0511 | nd | 0.0422 | 0.0375 | 0.0589 | 0.0366 | 0.0667 | 0.0849 | 0.0630 |
| Sens 2 | 81% | nd | 81% | 82% | 88% | 80% | 82% | 86% | 82% |
| Spec 2 | 18% | nd | 13% | 12% | 22% | 11% | 21% | 26% | 19% |
| Cutoff 3 | 0.0375 | nd | 0.0366 | 0.0331 | 0.0556 | 0.0331 | 0.0337 | 0.0667 | 0.0337 |
| Sens 3 | 92% | nd | 92% | 91% | 100% | 90% | 94% | 100% | 94% |
| Spec 3 | 12% | nd | 11% | 10% | 21% | 9% | 11% | 23% | 10% |
| Cutoff 4 | 7.09 | nd | 7.24 | 7.09 | 6.05 | 7.24 | 7.09 | 6.05 | 7.24 |
| Sens 4 | 31% | nd | 27% | 21% | 50% | 20% | 18% | 43% | 24% |
| Spec 4 | 70% | nd | 70% | 70% | 70% | 70% | 70% | 70% | 70% |
| Cutoff 5 | 19.0 | nd | 23.8 | 19.0 | 17.9 | 23.8 | 19.0 | 17.9 | 23.8 |
| Sens 5 | 19% | nd | 19% | 15% | 25% | 13% | 12% | 43% | 6% |
| Spec 5 | 80% | nd | 80% | 80% | 80% | 80% | 80% | 80% | 80% |
| Cutoff 6 | 66.4 | nd | 79.4 | 66.4 | 70.5 | 79.4 | 66.4 | 70.5 | 79.4 |
| Sens 6 | 12% | nd | 8% | 12% | 25% | 7% | 0% | 14% | 0% |
| Spec 6 | 90% | nd | 90% | 90% | 90% | 90% | 90% | 90% | 90% |
| OR Quart 2 | 0.83 | nd | 0.66 | 0.83 | 0.99 | 0.66 | 0.66 | 3.0 | 2.0 |
| p Value | 0.77 | nd | 0.53 | 0.77 | 0.99 | 0.53 | 0.65 | 0.34 | 0.42 |
| 95% CI of OR Quart2 | 0.25 | nd | 0.18 | 0.25 | 0.14 | 0.18 | 0.11 | 0.31 | 0.37 |
| | 2.8 | nd | 2.4 | 2.8 | 7.2 | 2.4 | 4.0 | 29 | 11 |
| OR Quart 3 | 1.0 | nd | 1.0 | 1.2 | 0.49 | 0.83 | 2.1 | 0 | 2.0 |
| p Value | 0.99 | nd | 0.99 | 0.76 | 0.56 | 0.77 | 0.32 | na | 0.42 |
| 95% CI of OR Quart3 | 0.32 | nd | 0.31 | 0.39 | 0.044 | 0.25 | 0.50 | na | 0.37 |
| | 3.2 | nd | 3.3 | 3.7 | 5.5 | 2.8 | 8.4 | na | 11 |
| OR Quart 4 | 1.6 | nd | 1.8 | 3.0 | 1.5 | 2.8 | 2.1 | 3.0 | 3.7 |
| p Value | 0.42 | nd | 0.29 | 0.029 | 0.66 | 0.042 | 0.32 | 0.34 | 0.11 |
| 95% CI of | 0.54 | nd | 0.61 | 1.1 | 0.25 | 1.0 | 0.50 | 0.31 | 0.75 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart4 | 4.5 | nd | 5.1 | 7.9 | 9.1 | 7.6 | 8.4 | 29 | 18 |

## Mucin-16

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.684 | 0.822 | nd | nd |
| Average | nd | nd | 1.24 | 28.1 | nd | nd |
| Stdev | nd | nd | 1.59 | 108 | nd | nd |
| p(t-test) | nd | nd |  | 0.014 | nd | nd |
| Min | nd | nd | 1.00E-9 | 0.304 | nd | nd |
| Max | nd | nd | 10.1 | 433 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 0.623 | 0.822 | nd | nd |
| Average | nd | nd | 1.23 | 29.9 | nd | nd |
| Stdev | nd | nd | 1.64 | 111 | nd | nd |
| p(t-test) | nd | nd |  | 0.020 | nd | nd |
| Min | nd | nd | 1.00E-9 | 0.304 | nd | nd |
| Max | nd | nd | 10.1 | 433 | nd | nd |
| n (Samp) | nd | nd | 81 | 15 | nd | nd |
| n (Patient) | nd | nd | 61 | 15 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | nd | nd | nd | 0.55 | nd | 0.56 | nd | nd | nd |
| SE | nd | nd | nd | 0.080 | nd | 0.083 | nd | nd | nd |
| p | nd | nd | nd | 0.49 | nd | 0.45 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0.412 | nd | 0.412 | nd | nd | nd |
| Sens 1 | nd | nd | nd | 75% | nd | 73% | nd | nd | nd |
| Spec 1 | nd | nd | nd | 32% | nd | 36% | nd | nd | nd |
| Cutoff 2 | nd | nd | nd | 0.344 | nd | 0.344 | nd | nd | nd |
| Sens 2 | nd | nd | nd | 88% | nd | 87% | nd | nd | nd |
| Spec 2 | nd | nd | nd | 21% | nd | 26% | nd | nd | nd |
| Cutoff 3 | nd | nd | nd | 0.278 | nd | 0.278 | nd | nd | nd |
| Sens 3 | nd | nd | nd | 100% | nd | 100% | nd | nd | nd |
| Spec 3 | nd | nd | nd | 11% | nd | 15% | nd | nd | nd |
| Cutoff 4 | nd | nd | nd | 1.17 | nd | 1.09 | nd | nd | nd |
| Sens 4 | nd | nd | nd | 25% | nd | 33% | nd | nd | nd |
| Spec 4 | nd | nd | nd | 71% | nd | 70% | nd | nd | nd |
| Cutoff 5 | nd | nd | nd | 1.41 | nd | 1.44 | nd | nd | nd |
| Sens 5 | nd | nd | nd | 19% | nd | 20% | nd | nd | nd |
| Spec 5 | nd | nd | nd | 80% | nd | 80% | nd | nd | nd |
| Cutoff 6 | nd | nd | nd | 3.40 | nd | 3.26 | nd | nd | nd |
| Sens 6 | nd | nd | nd | 12% | nd | 13% | nd | nd | nd |
| Spec 6 | nd | nd | nd | 91% | nd | 90% | nd | nd | nd |
| OR Quart 2 | nd | nd | nd | 0.72 | nd | 1.0 | nd | nd | nd |
| p Value | nd | nd | nd | 0.69 | nd | 1.0 | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | nd | nd | nd | 0.15 | nd | 0.18 | nd | nd | nd |
| | nd | nd | nd | 3.6 | nd | 5.5 | nd | nd | nd |
| OR Quart 3 | nd | nd | nd | 1.3 | nd | 1.8 | nd | nd | nd |
| p Value | nd | nd | nd | 0.72 | nd | 0.44 | nd | nd | nd |
| 95% CI of OR Quart3 | nd | nd | nd | 0.31 | nd | 0.39 | nd | nd | nd |
| | nd | nd | nd | 5.5 | nd | 8.8 | nd | nd | nd |
| OR Quart 4 | nd | nd | nd | 1.0 | nd | 1.4 | nd | nd | nd |
| p Value | nd | nd | nd | 1.0 | nd | 0.68 | nd | nd | nd |
| 95% CI of OR Quart4 | nd | nd | nd | 0.22 | nd | 0.28 | nd | nd | nd |
| | nd | nd | nd | 4.5 | nd | 7.1 | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.344 | 0.306 | nd | nd |
| Average | nd | nd | 0.956 | 1.45 | nd | nd |
| Stdev | nd | nd | 1.45 | 1.97 | nd | nd |
| p(t-test) | nd | nd | | 0.24 | nd | nd |
| Min | nd | nd | 0.00360 | 0.0172 | nd | nd |
| Max | nd | nd | 6.71 | 5.86 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.343 | 0.287 | nd | nd |
| Average | nd | nd | 0.805 | 1.42 | nd | nd |
| Stdev | nd | nd | 1.26 | 2.00 | nd | nd |
| p(t-test) | nd | nd | | 0.12 | nd | nd |
| Min | nd | nd | 0.00360 | 0.0172 | nd | nd |
| Max | nd | nd | 6.48 | 5.86 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.56 | nd | 0.55 | nd | nd | nd |
| SE | nd | nd | nd | 0.080 | nd | 0.083 | nd | nd | nd |
| p | nd | nd | nd | 0.46 | nd | 0.53 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0.109 | nd | 0.109 | nd | nd | nd |
| Sens 1 | nd | nd | nd | 75% | nd | 73% | nd | nd | nd |
| Spec 1 | nd | nd | nd | 35% | nd | 36% | nd | nd | nd |
| Cutoff 2 | nd | nd | nd | 0.101 | nd | 0.101 | nd | nd | nd |
| Sens 2 | nd | nd | nd | 81% | nd | 80% | nd | nd | nd |
| Spec 2 | nd | nd | nd | 31% | nd | 32% | nd | nd | nd |
| Cutoff 3 | nd | nd | nd | 0.0198 | nd | 0.0182 | nd | nd | nd |
| Sens 3 | nd | nd | nd | 94% | nd | 93% | nd | nd | nd |
| Spec 3 | nd | nd | nd | 11% | nd | 11% | nd | nd | nd |
| Cutoff 4 | nd | nd | nd | 0.855 | nd | 0.819 | nd | nd | nd |
| Sens 4 | nd | nd | nd | 38% | nd | 33% | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | nd | nd | nd | 71% | nd | 70% | nd | nd | nd |
| Cutoff 5 | nd | nd | nd | 1.56 | nd | 0.979 | nd | nd | nd |
| Sens 5 | nd | nd | nd | 31% | nd | 33% | nd | nd | nd |
| Spec 5 | nd | nd | nd | 80% | nd | 80% | nd | nd | nd |
| Cutoff 6 | nd | nd | nd | 3.11 | nd | 2.25 | nd | nd | nd |
| Sens 6 | nd | nd | nd | 19% | nd | 27% | nd | nd | nd |
| Spec 6 | nd | nd | nd | 91% | nd | 90% | nd | nd | nd |
| OR Quart 2 | nd | nd | nd | 4.3 | nd | 3.7 | nd | nd | nd |
| p Value | nd | nd | nd | 0.086 | nd | 0.14 | nd | nd | nd |
| 95% CI of OR Quart2 | nd | nd | nd | 0.81 23 | nd | 0.66 20 | nd | nd | nd |
| OR Quart 3 | nd | nd | nd | 0.48 | nd | 1.0 | nd | nd | nd |
| p Value | nd | nd | nd | 0.56 | nd | 1.0 | nd | nd | nd |
| 95% CI of OR Quart3 | nd | nd | nd | 0.041 5.6 | nd | 0.13 7.7 | nd | nd | nd |
| OR Quart 4 | nd | nd | nd | 3.5 | nd | 2.9 | nd | nd | nd |
| p Value | nd | nd | nd | 0.14 | nd | 0.23 | nd | nd | nd |
| 95% CI of OR Quart4 | nd | nd | nd | 0.65 19 | nd | 0.50 17 | nd | nd | nd |

## Cellular tumor antigen p53

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 0.000165 | nd | nd |
| Average | nd | nd | 0.00163 | 0.00180 | nd | nd |
| Stdev | nd | nd | 0.00319 | 0.00245 | nd | nd |
| p(t-test) | nd | nd | | 0.84 | nd | nd |
| Min | nd | nd | 1.00E-9 | 1.00E-9 | nd | nd |
| Max | nd | nd | 0.0202 | 0.00839 | nd | nd |
| n (Samp) | nd | nd | 96 | 16 | nd | nd |
| n (Patient) | nd | nd | 73 | 16 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.00E-9 | 3.53E-5 | nd | nd |
| Average | nd | nd | 0.00186 | 0.00145 | nd | nd |
| Stdev | nd | nd | 0.00355 | 0.00190 | nd | nd |
| p(t-test) | nd | nd | | 0.66 | nd | nd |
| Min | nd | nd | 1.00E-9 | 1.00E-9 | nd | nd |
| Max | nd | nd | 0.0202 | 0.00470 | nd | nd |
| n (Samp) | nd | nd | 81 | 15 | nd | nd |
| n (Patient) | nd | nd | 61 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.58 | nd | 0.55 | nd | nd | nd |
| SE | nd | nd | nd | 0.080 | nd | 0.083 | nd | nd | nd |
| p | nd | nd | nd | 0.30 | nd | 0.56 | nd | nd | nd |
| nCohort 1 | nd | nd | nd | 96 | nd | 81 | nd | nd | nd |
| nCohort 2 | nd | nd | nd | 16 | nd | 15 | nd | nd | nd |
| Cutoff 1 | nd | nd | nd | 0 | nd | 0 | nd | nd | nd |
| Sens 1 | nd | nd | nd | 100% | nd | 100% | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 1 | nd | nd | nd | 0% | nd | 0% | nd | nd | nd |
| Cutoff 2 | nd | nd | nd | 0 | nd | 0 | nd | nd | nd |
| Sens 2 | nd | nd | nd | 100% | nd | 100% | nd | nd | nd |
| Spec 2 | nd | nd | nd | 0% | nd | 0% | nd | nd | nd |
| Cutoff 3 | nd | nd | nd | 0 | nd | 0 | nd | nd | nd |
| Sens 3 | nd | nd | nd | 100% | nd | 100% | nd | nd | nd |
| Spec 3 | nd | nd | nd | 0% | nd | 0% | nd | nd | nd |
| Cutoff 4 | nd | nd | nd | 0.00160 | nd | 0.00160 | nd | nd | nd |
| Sens 4 | nd | nd | nd | 38% | nd | 33% | nd | nd | nd |
| Spec 4 | nd | nd | nd | 73% | nd | 72% | nd | nd | nd |
| Cutoff 5 | nd | nd | nd | 0.00313 | nd | 0.00343 | nd | nd | nd |
| Sens 5 | nd | nd | nd | 38% | nd | 20% | nd | nd | nd |
| Spec 5 | nd | nd | nd | 81% | nd | 80% | nd | nd | nd |
| Cutoff 6 | nd | nd | nd | 0.00470 | nd | 0.00599 | nd | nd | nd |
| Sens 6 | nd | nd | nd | 6% | nd | 0% | nd | nd | nd |
| Spec 6 | nd | nd | nd | 92% | nd | 91% | nd | nd | nd |
| OR Quart 2 | nd | nd | nd | 0.46 | nd | 0.17 | nd | nd | nd |
| p Value | nd | nd | nd | 0.40 | nd | 0.11 | nd | nd | nd |
| 95% CI of OR Quart2 | nd | nd | nd | 0.077 | nd | 0.018 | nd | nd | nd |
|  | nd | nd | nd | 2.8 | nd | 1.5 | nd | nd | nd |
| OR Quart 3 | nd | nd | nd | 1.0 | nd | 0.76 | nd | nd | nd |
| p Value | nd | nd | nd | 1.0 | nd | 0.71 | nd | nd | nd |
| 95% CI of OR Quart3 | nd | nd | nd | 0.22 | nd | 0.18 | nd | nd | nd |
|  | nd | nd | nd | 4.5 | nd | 3.3 | nd | nd | nd |
| OR Quart 4 | nd | nd | nd | 1.6 | nd | 1.0 | nd | nd | nd |
| p Value | nd | nd | nd | 0.49 | nd | 1.0 | nd | nd | nd |
| 95% CI of OR Quart4 | nd | nd | nd | 0.41 | nd | 0.25 | nd | nd | nd |
|  | nd | nd | nd | 6.6 | nd | 4.0 | nd | nd | nd |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Apolipoprotein A-II**

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| Median | 78.2 | 125 | 77.5 | 247 | 85.8 | 106 |
| Average | 156 | 187 | 167 | 307 | 149 | 132 |
| Stdev | 303 | 193 | 368 | 295 | 238 | 77.5 |
| p(t-test) |  | 0.58 |  | 0.30 |  | 0.74 |
| Min | 4.67 | 18.9 | 4.67 | 23.0 | 21.4 | 18.9 |
| Max | 2000 | 845 | 2000 | 845 | 1790 | 323 |
| n (Samp) | 77 | 33 | 29 | 9 | 61 | 24 |
| n (Patient) | 77 | 33 | 29 | 9 | 61 | 24 |

|  | At Enrollment | | |
| --- | --- | --- | --- |
|  | sCr or UO | sCr only | UO only |
| AUC | 0.64 | 0.76 | 0.60 |
| SE | 0.059 | 0.10 | 0.070 |
| p | 0.016 | 0.011 | 0.16 |
| nCohort 1 | 77 | 29 | 61 |
| nCohort 2 | 33 | 9 | 24 |

| | At Enrollment | | |
|---|---|---|---|
| | sCr or UO | sCr only | UO only |
| Cutoff 1 | 82.3 | 125 | 85.8 |
| Sens 1 | 73% | 78% | 71% |
| Spec 1 | 53% | 72% | 51% |
| Cutoff 2 | 67.3 | 78.5 | 65.3 |
| Sens 2 | 82% | 89% | 83% |
| Spec 2 | 47% | 55% | 43% |
| Cutoff 3 | 58.1 | 21.6 | 62.3 |
| Sens 3 | 91% | 100% | 92% |
| Spec 3 | 42% | 10% | 41% |
| Cutoff 4 | 126 | 125 | 148 |
| Sens 4 | 48% | 78% | 33% |
| Spec 4 | 70% | 72% | 70% |
| Cutoff 5 | 166 | 166 | 180 |
| Sens 5 | 33% | 56% | 21% |
| Spec 5 | 81% | 83% | 80% |
| Cutoff 6 | 314 | 195 | 314 |
| Sens 6 | 12% | 56% | 4% |
| Spec 6 | 91% | 93% | 90% |
| OR Quart 2 | 3.2 | 0.89 | 12 |
| p Value | 0.12 | 0.94 | 0.025 |
| 95% CI of | 0.75 | 0.047 | 1.4 |
| OR Quart2 | 14 | 17 | 110 |
| OR Quart 3 | 5.5 | 2.3 | 15 |
| p Value | 0.019 | 0.53 | 0.015 |
| 95% CI of | 1.3 | 0.17 | 1.7 |
| OR Quart3 | 23 | 31 | 130 |
| OR Quart 4 | 5.2 | 8.0 | 7.5 |
| p Value | 0.023 | 0.092 | 0.075 |
| 95% CI of | 1.3 | 0.71 | 0.82 |
| OR Quart4 | 21 | 90 | 69 |

## Myoglobin

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.515 | 0.0847 | nd | nd | 0.758 | 0.0561 |
| Average | 42.9 | 26.9 | nd | nd | 49.3 | 3.71 |
| Stdev | 122 | 94.2 | nd | nd | 131 | 12.0 |
| p(t-test) | | 0.57 | nd | nd | | 0.16 |
| Min | 0.000105 | 0.000105 | nd | nd | 0.0176 | 0.0145 |
| Max | 618 | 460 | nd | nd | 618 | 49.1 |
| n (Samp) | 52 | 24 | nd | nd | 44 | 17 |
| n (Patient) | 52 | 24 | nd | nd | 44 | 17 |

| | At Enrollment | | |
|---|---|---|---|
| | sCr or UO | sCr only | UO only |
| AUC | 0.40 | nd | 0.30 |
| SE | 0.072 | nd | 0.079 |
| p | 0.17 | nd | 0.011 |
| nCohort 1 | 52 | nd | 44 |
| nCohort 2 | 24 | nd | 17 |
| Cutoff 1 | 0.0365 | nd | 0.0347 |
| Sens 1 | 71% | nd | 71% |
| Spec 1 | 15% | nd | 14% |
| Cutoff 2 | 0.0324 | nd | 0.0324 |

| | At Enrollment | | |
|---|---|---|---|
| | sCr or UO | sCr only | UO only |
| Sens 2 | 83% | nd | 82% |
| Spec 2 | 12% | nd | 11% |
| Cutoff 3 | 0.0176 | nd | 0.0176 |
| Sens 3 | 92% | nd | 94% |
| Spec 3 | 4% | nd | 2% |
| Cutoff 4 | 13.5 | nd | 13.9 |
| Sens 4 | 21% | nd | 6% |
| Spec 4 | 71% | nd | 70% |
| Cutoff 5 | 33.1 | nd | 34.7 |
| Sens 5 | 12% | nd | 6% |
| Spec 5 | 81% | nd | 82% |
| Cutoff 6 | 63.7 | nd | 85.7 |
| Sens 6 | 8% | nd | 0% |
| Spec 6 | 90% | nd | 91% |
| OR Quart 2 | 0.75 | nd | 1.8 |
| p Value | 0.70 | nd | 0.57 |
| 95% CI of | 0.17 | nd | 0.25 |
| OR Quart2 | 3.4 | nd | 12 |
| OR Quart 3 | 1.6 | nd | 3.5 |
| p Value | 0.49 | nd | 0.18 |
| 95% CI of | 0.41 | nd | 0.56 |
| OR Quart3 | 6.5 | nd | 22 |
| OR Quart 4 | 2.0 | nd | 6.1 |
| p Value | 0.31 | nd | 0.048 |
| 95% CI of | 0.52 | nd | 1.0 |
| OR Quart4 | 8.0 | nd | 37 |

## KSP-Cadherin

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 1.48 | 0.809 | nd | nd | 1.46 | 0.996 |
| Average | 1.74 | 1.25 | nd | nd | 1.73 | 1.18 |
| Stdev | 1.18 | 1.36 | nd | nd | 1.10 | 1.26 |
| p(t-test) | | 0.18 | nd | nd | | 0.21 |
| Min | 0.131 | 0.00263 | nd | nd | 0.196 | 0.00263 |
| Max | 4.63 | 4.23 | nd | nd | 4.63 | 3.88 |
| n (Samp) | 40 | 16 | nd | nd | 31 | 9 |
| n (Patient) | 40 | 16 | nd | nd | 31 | 9 |

| | At Enrollment | | |
|---|---|---|---|
| | sCr or UO | sCr only | UO only |
| AUC | 0.33 | nd | 0.31 |
| SE | 0.084 | nd | 0.11 |
| p | 0.037 | nd | 0.081 |
| nCohort 1 | 40 | nd | 31 |
| nCohort 2 | 16 | nd | 9 |
| Cutoff 1 | 0.258 | nd | 0.196 |
| Sens 1 | 75% | nd | 78% |
| Spec 1 | 5% | nd | 3% |
| Cutoff 2 | 0.196 | nd | 0.00263 |
| Sens 2 | 81% | nd | 89% |
| Spec 2 | 5% | nd | 0% |
| Cutoff 3 | 0.00263 | nd | 0 |
| Sens 3 | 94% | nd | 100% |

|  | At Enrollment | | |
|---|---|---|---|
|  | sCr or UO | sCr only | UO only |
| Spec 3 | 0% | nd | 0% |
| Cutoff 4 | 1.84 | nd | 1.84 |
| Sens 4 | 25% | nd | 22% |
| Spec 4 | 70% | nd | 71% |
| Cutoff 5 | 2.57 | nd | 2.54 |
| Sens 5 | 19% | nd | 11% |
| Spec 5 | 80% | nd | 81% |
| Cutoff 6 | 3.39 | nd | 3.11 |
| Sens 6 | 12% | nd | 11% |
| Spec 6 | 90% | nd | 90% |
| OR Quart 2 | 0 | nd | 0 |
| p Value | na | nd | na |
| 95% CI of | na | nd | na |
| OR Quart2 | na | nd | na |
| OR Quart 3 | 1.0 | nd | 1.7 |
| p Value | 1.0 | nd | 0.61 |
| 95% CI of | 0.19 | nd | 0.22 |
| OR Quart3 | 5.2 | nd | 13 |
| OR Quart 4 | 3.3 | nd | 2.7 |
| p Value | 0.13 | nd | 0.34 |
| 95% CI of | 0.69 | nd | 0.36 |
| OR Quart4 | 16 | nd | 20 |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 76.0 | 265 | 76.0 | 217 | 76.0 | 157 |
| Average | 241 | 1760 | 241 | 1460 | 241 | 225 |
| Stdev | 841 | 5360 | 841 | 5260 | 841 | 193 |
| p(t-test) | | 3.5E-4 | | 0.0035 | | 0.95 |
| Min | 5.33 | 27.6 | 5.33 | 9.51 | 5.33 | 55.6 |
| Max | 6400 | 24300 | 6400 | 24300 | 6400 | 764 |
| n (Samp) | 196 | 21 | 196 | 21 | 196 | 11 |
| n (Patient) | 196 | 21 | 196 | 21 | 196 | 11 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 97.8 | 268 | 97.8 | 265 | 97.8 | 216 |
| Average | 342 | 954 | 342 | 393 | 342 | 213 |
| Stdev | 1610 | 1880 | 1610 | 510 | 1610 | 68.6 |
| p(t-test) | | 0.22 | | 0.92 | | 0.84 |
| Min | 5.33 | 27.6 | 5.33 | 9.51 | 5.33 | 131 |
| Max | 24300 | 6400 | 24300 | 1860 | 24300 | 288 |
| n (Samp) | 294 | 11 | 294 | 11 | 294 | 6 |
| n (Patient) | 294 | 11 | 294 | 11 | 294 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 87.2 | 174 | 87.2 | 174 | 87.2 | 149 |
| Average | 294 | 2240 | 294 | 1850 | 294 | 227 |
| Stdev | 972 | 6320 | 972 | 6230 | 972 | 214 |
| p(t-test) | | 0.0012 | | 0.0086 | | 0.84 |
| Min | 5.33 | 55.6 | 5.33 | 45.8 | 5.33 | 55.6 |
| Max | 6400 | 24300 | 6400 | 24300 | 6400 | 764 |
| n (Samp) | 132 | 15 | 132 | 15 | 132 | 9 |
| n (Patient) | 132 | 15 | 132 | 15 | 132 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.78 | 0.79 | 0.77 | 0.76 | 0.76 | 0.78 | 0.78 | 0.73 |
| SE | 0.059 | 0.084 | 0.072 | 0.062 | 0.086 | 0.075 | 0.084 | 0.11 | 0.098 |
| p | 2.8E-7 | 9.4E-4 | 4.7E-5 | 8.7E-6 | 0.0029 | 5.7E-4 | 0.0010 | 0.014 | 0.019 |
| nCohort 1 | 196 | 294 | 132 | 196 | 294 | 132 | 196 | 294 | 132 |
| nCohort 2 | 21 | 11 | 15 | 21 | 11 | 15 | 11 | 6 | 9 |
| Cutoff 1 | 148 | 165 | 146 | 148 | 165 | 146 | 146 | 157 | 111 |
| Sens 1 | 71% | 73% | 73% | 71% | 73% | 73% | 73% | 83% | 78% |
| Spec 1 | 80% | 74% | 77% | 80% | 74% | 77% | 79% | 71% | 63% |
| Cutoff 2 | 129 | 157 | 129 | 129 | 157 | 129 | 111 | 157 | 100 |
| Sens 2 | 81% | 82% | 80% | 81% | 82% | 80% | 82% | 83% | 89% |
| Spec 2 | 74% | 71% | 73% | 74% | 71% | 73% | 67% | 71% | 58% |
| Cutoff 3 | 100 | 135 | 100 | 54.2 | 129 | 54.3 | 100 | 129 | 54.3 |
| Sens 3 | 90% | 91% | 93% | 90% | 91% | 93% | 91% | 100% | 100% |
| Spec 3 | 62% | 65% | 58% | 37% | 64% | 32% | 62% | 64% | 32% |
| Cutoff 4 | 124 | 155 | 127 | 124 | 155 | 127 | 124 | 155 | 127 |
| Sens 4 | 81% | 82% | 80% | 81% | 82% | 80% | 73% | 83% | 67% |
| Spec 4 | 70% | 70% | 70% | 70% | 70% | 70% | 70% | 70% | 70% |
| Cutoff 5 | 153 | 200 | 164 | 153 | 200 | 164 | 153 | 200 | 164 |
| Sens 5 | 67% | 64% | 53% | 67% | 64% | 53% | 55% | 50% | 33% |
| Spec 5 | 80% | 80% | 80% | 80% | 80% | 80% | 80% | 80% | 80% |
| Cutoff 6 | 296 | 335 | 312 | 296 | 335 | 312 | 296 | 335 | 312 |
| Sens 6 | 29% | 27% | 20% | 24% | 18% | 20% | 9% | 0% | 11% |
| Spec 6 | 90% | 90% | 90% | 90% | 90% | 90% | 90% | 90% | 90% |
| OR Quart 2 | 1.0 | 0 | >1.0 | 2.0 | 0 | 0.97 | >1.0 | >0 | >1.0 |
| p Value | 1.0 | na | <1.0 | 0.57 | na | 0.98 | <1.0 | <na | <0.98 |
| 95% CI of OR Quart2 | 0.061 | na | >0.060 | 0.18 | na | 0.058 | >0.061 | >na | >0.062 |
| | 16 | na | na | 23 | na | 16 | na | na | na |
| OR Quart 3 | 4.2 | 3.1 | >5.6 | 3.1 | 3.1 | 4.2 | >2.0 | >3.1 | >2.1 |
| p Value | 0.20 | 0.33 | <0.12 | 0.33 | 0.33 | 0.21 | <0.57 | <0.33 | <0.55 |
| 95% CI of OR Quart3 | 0.46 | 0.31 | >0.62 | 0.31 | 0.31 | 0.45 | >0.18 | >0.32 | >0.18 |
| | 39 | 30 | na | 31 | 30 | 40 | na | na | na |
| OR Quart 4 | 20 | 7.5 | >12 | 20 | 7.5 | 11 | >9.3 | >3.1 | >7.0 |
| p Value | 0.0046 | 0.063 | <0.024 | 0.0046 | 0.063 | 0.026 | <0.039 | <0.33 | <0.079 |
| 95% CI of OR Quart4 | 2.5 | 0.90 | >1.4 | 2.5 | 0.90 | 1.3 | >1.1 | >0.32 | >0.80 |
| | 160 | 63 | na | 160 | 63 | 94 | na | na | na |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.28 | 4.81 | 1.28 | 2.33 | 1.28 | 0.988 |
| Average | 1.56 | 10.5 | 1.56 | 4.46 | 1.56 | 1.77 |
| Stdev | 1.38 | 15.7 | 1.38 | 6.02 | 1.38 | 2.36 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.0057 | | 0.027 | | 0.78 |
| Min | 0.0223 | 0.0223 | 0.0223 | 0.0223 | 0.0223 | 0.0223 |
| Max | 4.68 | 47.1 | 4.68 | 17.3 | 4.68 | 6.25 |
| n (Samp) | 26 | 8 | 26 | 7 | 26 | 6 |
| n (Patient) | 26 | 8 | 26 | 7 | 26 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | nd | nd | 0.64 | nd | nd | 0.45 | nd | nd |
| SE | 0.11 | nd | nd | 0.12 | nd | nd | 0.13 | nd | nd |
| p | 0.021 | nd | nd | 0.25 | nd | nd | 0.72 | nd | nd |
| nCohort 1 | 26 | nd | nd | 26 | nd | nd | 26 | nd | nd |
| nCohort 2 | 8 | nd | nd | 7 | nd | nd | 6 | nd | nd |
| Cutoff 1 | 2.08 | nd | nd | 1.02 | nd | nd | 0 | nd | nd |
| Sens 1 | 75% | nd | nd | 71% | nd | nd | 100% | nd | nd |
| Spec 1 | 69% | nd | nd | 46% | nd | nd | 0% | nd | nd |
| Cutoff 2 | 0.496 | nd | nd | 0.496 | nd | nd | 0 | nd | nd |
| Sens 2 | 88% | nd | nd | 86% | nd | nd | 100% | nd | nd |
| Spec 2 | 31% | nd | nd | 31% | nd | nd | 0% | nd | nd |
| Cutoff 3 | 0 | nd | nd | 0 | nd | nd | 0 | nd | nd |
| Sens 3 | 100% | nd | nd | 100% | nd | nd | 100% | nd | nd |
| Spec 3 | 0% | nd | nd | 0% | nd | nd | 0% | nd | nd |
| Cutoff 4 | 2.33 | nd | nd | 2.33 | nd | nd | 2.33 | nd | nd |
| Sens 4 | 62% | nd | nd | 43% | nd | nd | 17% | nd | nd |
| Spec 4 | 73% | nd | nd | 73% | nd | nd | 73% | nd | nd |
| Cutoff 5 | 2.59 | nd | nd | 2.59 | nd | nd | 2.59 | nd | nd |
| Sens 5 | 62% | nd | nd | 43% | nd | nd | 17% | nd | nd |
| Spec 5 | 85% | nd | nd | 85% | nd | nd | 85% | nd | nd |
| Cutoff 6 | 3.90 | nd | nd | 3.90 | nd | nd | 3.90 | nd | nd |
| Sens 6 | 50% | nd | nd | 29% | nd | nd | 17% | nd | nd |
| Spec 6 | 92% | nd | nd | 92% | nd | nd | 92% | nd | nd |
| OR Quart 2 | 0.88 | nd | nd | 1.0 | nd | nd | 1.0 | nd | nd |
| p Value | 0.93 | nd | nd | 1.0 | nd | nd | 1.0 | nd | nd |
| 95% CI of OR Quart2 | 0.046 17 | nd | nd | 0.052 19 | nd | nd | 0.052 19 | nd | nd |
| OR Quart 3 | 1.0 | nd | nd | 2.3 | nd | nd | 2.3 | nd | nd |
| p Value | 1.0 | nd | nd | 0.53 | nd | nd | 0.53 | nd | nd |
| 95% CI of OR Quart3 | 0.052 19 | nd | nd | 0.17 33 | nd | nd | 0.17 33 | nd | nd |
| OR Quart 4 | 8.8 | nd | nd | 3.5 | nd | nd | 2.3 | nd | nd |
| p Value | 0.086 | nd | nd | 0.33 | nd | nd | 0.53 | nd | nd |
| 95% CI of OR Quart4 | 0.74 100 | nd | nd | 0.28 43 | nd | nd | 0.17 33 | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.549 | 3.00 | 0.549 | 2.16 | 0.549 | 1.09 |
| Average | 1.25 | 2.65 | 1.25 | 1.79 | 1.25 | 1.35 |
| Stdev | 3.21 | 1.71 | 3.21 | 1.60 | 3.21 | 1.25 |
| p(t-test) | | 0.24 | | 0.67 | | 0.94 |
| Min | 0.0360 | 0.146 | 0.0360 | 0.146 | 0.0360 | 0.146 |
| Max | 19.5 | 4.64 | 19.5 | 4.64 | 19.5 | 3.33 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 36 | 8 | 36 | 7 | 36 | 6 |
| n (Patient) | 36 | 8 | 36 | 7 | 36 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | nd | nd | 0.69 | nd | nd | 0.64 | nd | nd |
| SE | 0.10 | nd | nd | 0.12 | nd | nd | 0.13 | nd | nd |
| p | 0.0034 | nd | nd | 0.11 | nd | nd | 0.27 | nd | nd |
| nCohort 1 | 36 | nd | nd | 36 | nd | nd | 36 | nd | nd |
| nCohort 2 | 8 | nd | nd | 7 | nd | nd | 6 | nd | nd |
| Cutoff 1 | 1.76 | nd | nd | 0.473 | nd | nd | 0.208 | nd | nd |
| Sens 1 | 75% | nd | nd | 71% | nd | nd | 83% | nd | nd |
| Spec 1 | 89% | nd | nd | 50% | nd | nd | 33% | nd | nd |
| Cutoff 2 | 0.208 | nd | nd | 0.208 | nd | nd | 0.208 | nd | nd |
| Sens 2 | 88% | nd | nd | 86% | nd | nd | 83% | nd | nd |
| Spec 2 | 33% | nd | nd | 33% | nd | nd | 33% | nd | nd |
| Cutoff 3 | 0.135 | nd | nd | 0.135 | nd | nd | 0.135 | nd | nd |
| Sens 3 | 100% | nd | nd | 100% | nd | nd | 100% | nd | nd |
| Spec 3 | 28% | nd | nd | 28% | nd | nd | 28% | nd | nd |
| Cutoff 4 | 0.868 | nd | nd | 0.868 | nd | nd | 0.868 | nd | nd |
| Sens 4 | 75% | nd | nd | 57% | nd | nd | 50% | nd | nd |
| Spec 4 | 72% | nd | nd | 72% | nd | nd | 72% | nd | nd |
| Cutoff 5 | 1.22 | nd | nd | 1.22 | nd | nd | 1.22 | nd | nd |
| Sens 5 | 75% | nd | nd | 57% | nd | nd | 50% | nd | nd |
| Spec 5 | 81% | nd | nd | 81% | nd | nd | 81% | nd | nd |
| Cutoff 6 | 2.17 | nd | nd | 2.17 | nd | nd | 2.17 | nd | nd |
| Sens 6 | 62% | nd | nd | 29% | nd | nd | 17% | nd | nd |
| Spec 6 | 92% | nd | nd | 92% | nd | nd | 92% | nd | nd |
| OR Quart 2 | >2.4 | nd | nd | >2.2 | nd | nd | >2.2 | nd | nd |
| p Value | <0.49 | nd | nd | <0.54 | nd | nd | <0.54 | nd | nd |
| 95% CI of OR Quart2 | >0.19 na | nd | nd | >0.17 na | nd | nd | >0.17 na | nd | nd |
| OR Quart 3 | >0 | nd | nd | >1.0 | nd | nd | >1.1 | nd | nd |
| p Value | <na | nd | nd | <1.0 | nd | nd | <0.94 | nd | nd |
| 95% CI of OR Quart3 | >na na | nd | nd | >0.055 na | nd | nd | >0.060 na | nd | nd |
| OR Quart 4 | >13 | nd | nd | >5.7 | nd | nd | >3.8 | nd | nd |
| p Value | <0.033 | nd | nd | <0.15 | nd | nd | <0.29 | nd | nd |
| 95% CI of OR Quart4 | >1.2 na | nd | nd | >0.52 na | nd | nd | >0.32 na | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 66800 | 66800 | 66800 | 66800 | nd | nd |
| Average | 99700 | 95000 | 99700 | 95000 | nd | nd |
| Stdev | 131000 | 95400 | 131000 | 95400 | nd | nd |
| p(t-test) | | 0.92 | | 0.92 | nd | nd |
| Min | 3290 | 2220 | 3290 | 2220 | nd | nd |
| Max | 744000 | 260000 | 744000 | 260000 | nd | nd |
| n (Samp) | 41 | 8 | 41 | 8 | nd | nd |
| n (Patient) | 41 | 8 | 41 | 8 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | nd | 0.49 | nd | nd | nd | nd | nd |
| SE | 0.11 | nd | nd | 0.11 | nd | nd | nd | nd | nd |
| p | 0.91 | nd | nd | 0.91 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 16300 | nd | nd | 16300 | nd | nd | nd | nd | nd |
| Sens 1 | 75% | nd | nd | 75% | nd | nd | nd | nd | nd |
| Spec 1 | 17% | nd | nd | 17% | nd | nd | nd | nd | nd |
| Cutoff 2 | 8380 | nd | nd | 8380 | nd | nd | nd | nd | nd |
| Sens 2 | 88% | nd | nd | 88% | nd | nd | nd | nd | nd |
| Spec 2 | 10% | nd | nd | 10% | nd | nd | nd | nd | nd |
| Cutoff 3 | 0 | nd | nd | 0 | nd | nd | nd | nd | nd |
| Sens 3 | 100% | nd | nd | 100% | nd | nd | nd | nd | nd |
| Spec 3 | 0% | nd | nd | 0% | nd | nd | nd | nd | nd |
| Cutoff 4 | 123000 | nd | nd | 123000 | nd | nd | nd | nd | nd |
| Sens 4 | 38% | nd | nd | 38% | nd | nd | nd | nd | nd |
| Spec 4 | 71% | nd | nd | 71% | nd | nd | nd | nd | nd |
| Cutoff 5 | 138000 | nd | nd | 138000 | nd | nd | nd | nd | nd |
| Sens 5 | 25% | nd | nd | 25% | nd | nd | nd | nd | nd |
| Spec 5 | 80% | nd | nd | 80% | nd | nd | nd | nd | nd |
| Cutoff 6 | 155000 | nd | nd | 155000 | nd | nd | nd | nd | nd |
| Sens 6 | 25% | nd | nd | 25% | nd | nd | nd | nd | nd |
| Spec 6 | 90% | nd | nd | 90% | nd | nd | nd | nd | nd |
| OR Quart 2 | 0.30 | nd | nd | 0.30 | nd | nd | nd | nd | nd |
| p Value | 0.33 | nd | nd | 0.33 | nd | nd | nd | nd | nd |
| 95% CI of | 0.027 | nd | nd | 0.027 | nd | nd | nd | nd | nd |
| OR Quart2 | 3.4 | nd | nd | 3.4 | nd | nd | nd | nd | nd |
| OR Quart 3 | 0.30 | nd | nd | 0.30 | nd | nd | nd | nd | nd |
| p Value | 0.33 | nd | nd | 0.33 | nd | nd | nd | nd | nd |
| 95% CI of | 0.027 | nd | nd | 0.027 | nd | nd | nd | nd | nd |
| OR Quart3 | 3.4 | nd | nd | 3.4 | nd | nd | nd | nd | nd |
| OR Quart 4 | 1.1 | nd | nd | 1.1 | nd | nd | nd | nd | nd |
| p Value | 0.91 | nd | nd | 0.91 | nd | nd | nd | nd | nd |
| 95% CI of | 0.18 | nd | nd | 0.18 | nd | nd | nd | nd | nd |
| OR Quart4 | 7.0 | nd | nd | 7.0 | nd | nd | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.09 | 14.9 | 3.09 | 9.35 | 3.09 | 4.76 |
| Average | 88.7 | 37.1 | 88.7 | 36.6 | 88.7 | 15.2 |
| Stdev | 688 | 81.6 | 688 | 81.8 | 688 | 16.5 |
| p(t-test) | | 0.77 | | 0.77 | | 0.75 |
| Min | 0.116 | 0.116 | 0.116 | 0.116 | 0.116 | 1.64 |
| Max | 6950 | 327 | 6950 | 327 | 6950 | 44.1 |
| n (Samp) | 102 | 15 | 102 | 15 | 102 | 9 |
| n (Patient) | 102 | 15 | 102 | 15 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.90 | 18.8 | 4.90 | 15.7 | nd | nd |
| Average | 69.2 | 21.5 | 69.2 | 20.7 | nd | nd |
| Stdev | 541 | 18.2 | 541 | 18.6 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.80 | | 0.80 | nd | nd |
| Min | 1.14E-14 | 0.116 | 1.14E-14 | 0.116 | nd | nd |
| Max | 6950 | 44.1 | 6950 | 44.1 | nd | nd |
| n (Samp) | 168 | 8 | 168 | 8 | nd | nd |
| n (Patient) | 168 | 8 | 168 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.29 | 14.9 | 3.29 | 14.9 | 3.29 | 4.76 |
| Average | 101 | 50.0 | 101 | 50.0 | 101 | 14.4 |
| Stdev | 753 | 105 | 753 | 105 | 753 | 16.4 |
| p(t-test) | | 0.84 | | 0.84 | | 0.76 |
| Min | 0.116 | 2.45 | 0.116 | 2.45 | 0.116 | 2.45 |
| Max | 6950 | 327 | 6950 | 327 | 6950 | 44.1 |
| n (Samp) | 85 | 9 | 85 | 9 | 85 | 7 |
| n (Patient) | 85 | 9 | 85 | 9 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.66 | 0.69 | 0.65 | 0.65 | 0.68 | 0.63 | nd | 0.63 |
| SE | 0.081 | 0.11 | 0.10 | 0.081 | 0.11 | 0.10 | 0.10 | nd | 0.12 |
| p | 0.050 | 0.15 | 0.067 | 0.062 | 0.18 | 0.078 | 0.22 | nd | 0.28 |
| nCohort 1 | 102 | 168 | 85 | 102 | 168 | 85 | 102 | nd | 85 |
| nCohort 2 | 15 | 8 | 9 | 15 | 8 | 9 | 9 | nd | 7 |
| Cutoff 1 | 2.45 | 7.35 | 2.45 | 2.45 | 7.35 | 2.45 | 2.02 | nd | 2.45 |
| Sens 1 | 73% | 75% | 78% | 73% | 75% | 78% | 89% | nd | 71% |
| Spec 1 | 46% | 59% | 47% | 46% | 59% | 47% | 46% | nd | 47% |
| Cutoff 2 | 2.02 | 1.36 | 2.02 | 2.02 | 1.36 | 2.02 | 2.02 | nd | 2.02 |
| Sens 2 | 87% | 88% | 100% | 87% | 88% | 100% | 89% | nd | 100% |
| Spec 2 | 46% | 40% | 47% | 46% | 40% | 47% | 46% | nd | 47% |
| Cutoff 3 | 1.09 | 1.14E-14 | 2.02 | 1.09 | 1.14E-14 | 2.02 | 1.09 | nd | 2.02 |
| Sens 3 | 93% | 100% | 100% | 93% | 100% | 100% | 100% | nd | 100% |
| Spec 3 | 44% | 1% | 47% | 44% | 1% | 47% | 44% | nd | 47% |
| Cutoff 4 | 12.3 | 13.6 | 12.3 | 12.3 | 13.6 | 12.3 | 12.3 | nd | 12.3 |
| Sens 4 | 53% | 62% | 56% | 47% | 50% | 56% | 44% | nd | 43% |
| Spec 4 | 71% | 70% | 71% | 71% | 70% | 71% | 71% | nd | 71% |
| Cutoff 5 | 25.7 | 26.7 | 25.7 | 25.7 | 26.7 | 25.7 | 25.7 | nd | 25.7 |
| Sens 5 | 33% | 38% | 33% | 33% | 38% | 33% | 33% | nd | 29% |
| Spec 5 | 80% | 80% | 80% | 80% | 80% | 80% | 80% | nd | 80% |
| Cutoff 6 | 55.7 | 56.1 | 44.8 | 55.7 | 56.1 | 44.8 | 55.7 | nd | 44.8 |
| Sens 6 | 7% | 0% | 11% | 7% | 0% | 11% | 0% | nd | 0% |
| Spec 6 | 90% | 90% | 91% | 90% | 90% | 91% | 90% | nd | 91% |
| OR Quart 2 | 4.5 | >2.1 | >3.3 | 4.5 | >2.1 | >3.3 | >4.5 | nd | >3.4 |
| p Value | 0.19 | <0.55 | <0.32 | 0.19 | <0.55 | <0.32 | <0.19 | nd | <0.30 |
| 95% CI of | 0.47 | >0.18 | >0.32 | 0.47 | >0.18 | >0.32 | >0.47 | nd | >0.33 |
| OR Quart2 | 43 | na | na | 43 | na | na | na | nd | na |
| OR Quart 3 | 4.5 | >3.2 | >3.4 | 4.5 | >3.2 | >3.4 | >2.1 | nd | >2.2 |
| p Value | 0.19 | <0.32 | <0.30 | 0.19 | <0.32 | <0.30 | <0.56 | nd | <0.53 |
| 95% CI of | 0.47 | >0.32 | >0.33 | 0.47 | >0.32 | >0.33 | >0.18 | nd | >0.18 |
| OR Quart3 | 43 | na | na | 43 | na | na | na | nd | na |
| OR Quart 4 | 7.0 | >3.2 | >3.3 | 7.0 | >3.2 | >3.3 | >3.2 | nd | >2.2 |
| p Value | 0.081 | <0.32 | <0.32 | 0.081 | <0.32 | <0.32 | <0.32 | nd | <0.53 |
| 95% CI of | 0.79 | >0.32 | >0.32 | 0.79 | >0.32 | >0.32 | >0.32 | nd | >0.18 |
| OR Quart4 | 62 | na | na | 62 | na | na | na | nd | na |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.14 | 5.45 | 1.14 | 2.86 | 1.14 | 2.98 |
| Average | 3.19 | 7.89 | 3.19 | 7.48 | 3.19 | 5.77 |
| Stdev | 5.64 | 11.7 | 5.64 | 12.1 | 5.64 | 4.90 |
| p(t-test) | | 0.0094 | | 0.021 | | 0.19 |
| Min | 0.00336 | 0.282 | 0.00336 | 0.219 | 0.00336 | 0.852 |
| Max | 43.4 | 50.1 | 43.4 | 50.1 | 43.4 | 14.6 |
| n (Samp) | 102 | 17 | 102 | 16 | 102 | 9 |
| n (Patient) | 102 | 17 | 102 | 16 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.57 | 4.46 | 1.57 | 3.11 | nd | nd |
| Average | 28.3 | 4.33 | 28.3 | 3.91 | nd | nd |
| Stdev | 312 | 3.44 | 312 | 3.51 | nd | nd |
| p(t-test) | | 0.83 | | 0.83 | nd | nd |
| Min | 0.00336 | 0.282 | 0.00336 | 0.219 | nd | nd |
| Max | 4070 | 10.9 | 4070 | 10.9 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.19 | 6.79 | 1.19 | 4.88 | 1.19 | 2.98 |
| Average | 3.63 | 10.4 | 3.63 | 10.3 | 3.63 | 6.19 |
| Stdev | 6.41 | 14.1 | 6.41 | 14.7 | 6.41 | 5.50 |
| p(t-test) | | 0.0070 | | 0.011 | | 0.31 |
| Min | 0.00336 | 0.356 | 0.00336 | 0.852 | 0.00336 | 0.852 |
| Max | 43.4 | 50.1 | 43.4 | 50.1 | 43.4 | 14.6 |
| n (Samp) | 85 | 11 | 85 | 10 | 85 | 7 |
| n (Patient) | 85 | 11 | 85 | 10 | 85 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.62 | 0.75 | 0.69 | 0.57 | 0.77 | 0.76 | nd | 0.74 |
| SE | 0.075 | 0.11 | 0.089 | 0.078 | 0.11 | 0.091 | 0.096 | nd | 0.11 |
| p | 0.0072 | 0.28 | 0.0055 | 0.015 | 0.50 | 0.0030 | 0.0080 | nd | 0.034 |
| nCohort 1 | 102 | 170 | 85 | 102 | 170 | 85 | 102 | nd | 85 |
| nCohort 2 | 17 | 8 | 11 | 16 | 8 | 10 | 9 | nd | 7 |
| Cutoff 1 | 2.56 | 2.59 | 2.88 | 2.26 | 2.26 | 2.55 | 2.53 | nd | 2.53 |
| Sens 1 | 71% | 75% | 73% | 75% | 75% | 70% | 78% | nd | 71% |
| Spec 1 | 71% | 65% | 71% | 67% | 61% | 68% | 70% | nd | 68% |
| Cutoff 2 | 0.816 | 0.450 | 1.40 | 1.44 | 0.238 | 2.53 | 1.44 | nd | 1.40 |
| Sens 2 | 82% | 88% | 82% | 81% | 88% | 80% | 89% | nd | 86% |
| Spec 2 | 42% | 20% | 56% | 58% | 8% | 68% | 58% | nd | 56% |
| Cutoff 3 | 0.339 | 0.281 | 0.816 | 0.238 | 0.214 | 1.40 | 0.816 | nd | 0.816 |
| Sens 3 | 94% | 100% | 91% | 94% | 100% | 90% | 100% | nd | 100% |
| Spec 3 | 19% | 10% | 39% | 9% | 5% | 56% | 42% | nd | 39% |
| Cutoff 4 | 2.56 | 3.33 | 2.88 | 2.56 | 3.33 | 2.88 | 2.56 | nd | 2.88 |
| Sens 4 | 71% | 62% | 73% | 56% | 50% | 60% | 67% | nd | 57% |
| Spec 4 | 71% | 70% | 71% | 71% | 70% | 71% | 71% | nd | 71% |
| Cutoff 5 | 4.03 | 5.77 | 5.67 | 4.03 | 5.77 | 5.67 | 4.03 | nd | 5.67 |
| Sens 5 | 53% | 25% | 55% | 44% | 25% | 50% | 44% | nd | 43% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | nd | 80% |
| Cutoff 6 | 8.86 | 11.0 | 9.55 | 8.86 | 11.0 | 9.55 | 8.86 | nd | 9.55 |
| Sens 6 | 29% | 0% | 45% | 25% | 0% | 40% | 33% | nd | 43% |
| Spec 6 | 90% | 90% | 91% | 90% | 90% | 91% | 90% | nd | 91% |
| OR Quart 2 | 0.30 | 0 | 1.0 | 0.47 | 0 | >1.0 | >1.0 | nd | >1.0 |
| p Value | 0.31 | na | 1.0 | 0.54 | na | <1.0 | <1.0 | nd | <0.98 |
| 95% CI of OR Quart2 | 0.029 | na | 0.059 | 0.040 | na | >0.059 | >0.059 | nd | >0.062 |
| | 3.1 | na | 17 | 5.4 | na | na | na | nd | na |
| OR Quart 3 | 1.3 | 1.0 | 3.3 | 3.5 | 1.5 | >4.6 | >4.5 | nd | >3.4 |
| p Value | 0.72 | 1.0 | 0.32 | 0.15 | 0.65 | <0.19 | <0.19 | nd | <0.30 |
| 95% CI of OR Quart3 | 0.27 | 0.13 | 0.32 | 0.65 | 0.24 | >0.47 | >0.47 | nd | >0.33 |
| | 6.6 | 7.4 | 34 | 19 | 9.7 | na | na | nd | na |
| OR Quart 4 | 3.7 | 2.0 | 7.7 | 4.1 | 1.5 | >6.1 | >4.5 | nd | >3.4 |
| p Value | 0.072 | 0.42 | 0.070 | 0.097 | 0.67 | <0.11 | <0.19 | nd | <0.30 |
| 95% CI of OR Quart4 | 0.89 | 0.36 | 0.85 | 0.78 | 0.24 | >0.65 | >0.47 | nd | >0.33 |
| | 15 | 12 | 70 | 22 | 9.4 | na | na | nd | na |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.42 | 18.9 | 5.42 | 11.9 | 5.42 | 44.9 |
| Average | 51.4 | 149 | 51.4 | 155 | 51.4 | 174 |
| Stdev | 121 | 203 | 121 | 208 | 121 | 213 |
| p(t-test) | | 0.0066 | | 0.0052 | | 0.0076 |
| Min | 0.00616 | 0.0439 | 0.00616 | 0.0439 | 0.00616 | 0.0772 |
| Max | 618 | 469 | 618 | 469 | 618 | 469 |
| n (Samp) | 102 | 17 | 102 | 16 | 102 | 9 |
| n (Patient) | 102 | 17 | 102 | 16 | 102 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.65 | 31.9 | 3.65 | 29.4 | nd | nd |
| Average | 63.1 | 142 | 63.1 | 141 | nd | nd |
| Stdev | 142 | 195 | 142 | 196 | nd | nd |
| p(t-test) | | 0.13 | | 0.14 | nd | nd |
| Min | 0.00616 | 0.0439 | 0.00616 | 0.0439 | nd | nd |
| Max | 618 | 469 | 618 | 469 | nd | nd |
| n (Samp) | 170 | 8 | 170 | 8 | nd | nd |
| n (Patient) | 170 | 8 | 170 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.83 | 0.377 | 3.83 | 2.87 | 3.83 | 5.36 |
| Average | 56.6 | 143 | 56.6 | 152 | 56.6 | 150 |
| Stdev | 128 | 203 | 128 | 212 | 128 | 207 |
| p(t-test) | | 0.055 | | 0.042 | | 0.081 |
| Min | 0.00616 | 0.0696 | 0.00616 | 0.0772 | 0.00616 | 0.0772 |
| Max | 618 | 469 | 618 | 469 | 618 | 469 |
| n (Samp) | 85 | 11 | 85 | 10 | 85 | 7 |
| n (Patient) | 85 | 11 | 85 | 10 | 85 | 7 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.57 | 0.64 | 0.49 | 0.57 | 0.63 | 0.51 | 0.62 | nd | 0.54 |
| SE | 0.078 | 0.11 | 0.093 | 0.080 | 0.11 | 0.097 | 0.10 | nd | 0.12 |
| p | 0.36 | 0.21 | 0.95 | 0.36 | 0.24 | 0.93 | 0.25 | nd | 0.73 |
| nCohort 1 | 102 | 170 | 85 | 102 | 170 | 85 | 102 | nd | 85 |
| nCohort 2 | 17 | 8 | 11 | 16 | 8 | 10 | 9 | nd | 7 |
| Cutoff 1 | 0.288 | 10.2 | 0.117 | 0.288 | 9.39 | 0.288 | 0.288 | nd | 0.288 |
| Sens 1 | 71% | 75% | 73% | 75% | 75% | 70% | 78% | nd | 71% |
| Spec 1 | 21% | 61% | 14% | 21% | 61% | 20% | 21% | nd | 20% |
| Cutoff 2 | 0.0772 | 0.0780 | 0.0772 | 0.117 | 0.0780 | 0.117 | 0.117 | nd | 0.117 |
| Sens 2 | 82% | 88% | 82% | 81% | 88% | 80% | 89% | nd | 86% |
| Spec 2 | 13% | 14% | 11% | 16% | 14% | 14% | 16% | nd | 14% |
| Cutoff 3 | 0.0606 | 0.0411 | 0.0696 | 0.0606 | 0.0411 | 0.0772 | 0.0606 | nd | 0.0661 |
| Sens 3 | 94% | 100% | 91% | 94% | 100% | 90% | 100% | nd | 100% |
| Spec 3 | 13% | 9% | 11% | 13% | 9% | 11% | 13% | nd | 11% |
| Cutoff 4 | 17.2 | 18.6 | 17.9 | 17.2 | 18.6 | 17.9 | 17.2 | nd | 17.9 |
| Sens 4 | 53% | 62% | 45% | 44% | 50% | 40% | 56% | nd | 43% |
| Spec 4 | 71% | 70% | 71% | 71% | 70% | 71% | 71% | nd | 71% |
| Cutoff 5 | 38.9 | 45.3 | 55.8 | 38.9 | 45.3 | 55.8 | 38.9 | nd | 55.8 |
| Sens 5 | 47% | 38% | 36% | 44% | 38% | 40% | 56% | nd | 43% |
| Spec 5 | 80% | 80% | 80% | 80% | 80% | 80% | 80% | nd | 80% |
| Cutoff 6 | 222 | 234 | 232 | 222 | 234 | 232 | 222 | nd | 232 |
| Sens 6 | 29% | 25% | 27% | 31% | 25% | 30% | 33% | nd | 29% |
| Spec 6 | 90% | 90% | 91% | 90% | 90% | 91% | 90% | nd | 91% |
| OR Quart 2 | 0.13 | 0 | 0 | 0.13 | 0 | 0.21 | 0.30 | nd | 0 |
| p Value | 0.070 | na | na | 0.070 | na | 0.17 | 0.31 | nd | na |
| 95% CI of OR Quart2 | 0.015 | na | na | 0.015 | na | 0.021 | 0.029 | nd | na |
|  | 1.2 | na | na | 1.2 | na | 2.0 | 3.0 | nd | na |
| OR Quart 3 | 0.27 | 1.0 | 0.17 | 0.28 | 1.0 | 0.21 | 0 | nd | 0.30 |
| p Value | 0.13 | 1.0 | 0.11 | 0.15 | 1.0 | 0.17 | na | nd | 0.32 |
| 95% CI of OR Quart3 | 0.050 | 0.13 | 0.018 | 0.052 | 0.13 | 0.021 | na | nd | 0.029 |
|  | 1.5 | 7.4 | 1.5 | 1.5 | 7.4 | 2.0 | na | nd | 3.2 |
| OR Quart 4 | 1.4 | 2.0 | 1.0 | 1.2 | 2.0 | 0.95 | 1.7 | nd | 1.0 |
| p Value | 0.59 | 0.42 | 1.0 | 0.81 | 0.42 | 0.95 | 0.48 | nd | 1.0 |
| 95% CI of OR Quart4 | 0.42 | 0.36 | 0.25 | 0.34 | 0.36 | 0.21 | 0.37 | nd | 0.18 |
|  | 4.7 | 12 | 4.0 | 4.0 | 12 | 4.4 | 8.1 | nd | 5.6 |

## Mucin-16

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.684 | 1.03 | 0.684 | 1.03 | nd | nd |
| Average | 1.18 | 54.9 | 1.18 | 54.9 | nd | nd |
| Stdev | 1.41 | 153 | 1.41 | 153 | nd | nd |
| p(t-test) |  | 0.023 |  | 0.023 | nd | nd |
| Min | 0.141 | 0.304 | 0.141 | 0.304 | nd | nd |
| Max | 6.37 | 433 | 6.37 | 433 | nd | nd |
| n (Samp) | 41 | 8 | 41 | 8 | nd | nd |
| n (Patient) | 41 | 8 | 41 | 8 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.65 | nd | nd | 0.65 | nd | nd | nd | nd | nd |

106

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| SE | 0.11 | nd | nd | 0.11 | nd | nd | nd | nd | nd |
| p | 0.20 | nd | nd | 0.20 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 0.781 | nd | nd | 0.781 | nd | nd | nd | nd | nd |
| Sens 1 | 75% | nd | nd | 75% | nd | nd | nd | nd | nd |
| Spec 1 | 61% | nd | nd | 61% | nd | nd | nd | nd | nd |
| Cutoff 2 | 0.464 | nd | nd | 0.464 | nd | nd | nd | nd | nd |
| Sens 2 | 88% | nd | nd | 88% | nd | nd | nd | nd | nd |
| Spec 2 | 32% | nd | nd | 32% | nd | nd | nd | nd | nd |
| Cutoff 3 | 0.278 | nd | nd | 0.278 | nd | nd | nd | nd | nd |
| Sens 3 | 100% | nd | nd | 100% | nd | nd | nd | nd | nd |
| Spec 3 | 10% | nd | nd | 10% | nd | nd | nd | nd | nd |
| Cutoff 4 | 0.961 | nd | nd | 0.961 | nd | nd | nd | nd | nd |
| Sens 4 | 50% | nd | nd | 50% | nd | nd | nd | nd | nd |
| Spec 4 | 71% | nd | nd | 71% | nd | nd | nd | nd | nd |
| Cutoff 5 | 1.31 | nd | nd | 1.31 | nd | nd | nd | nd | nd |
| Sens 5 | 25% | nd | nd | 25% | nd | nd | nd | nd | nd |
| Spec 5 | 80% | nd | nd | 80% | nd | nd | nd | nd | nd |
| Cutoff 6 | 3.42 | nd | nd | 3.42 | nd | nd | nd | nd | nd |
| Sens 6 | 12% | nd | nd | 12% | nd | nd | nd | nd | nd |
| Spec 6 | 90% | nd | nd | 90% | nd | nd | nd | nd | nd |
| OR Quart 2 | 1.0 | nd | nd | 1.0 | nd | nd | nd | nd | nd |
| p Value | 1.0 | nd | nd | 1.0 | nd | nd | nd | nd | nd |
| 95% CI of OR Quart2 | 0.055 | nd | nd | 0.055 | nd | nd | nd | nd | nd |
|  | 18 | nd | nd | 18 | nd | nd | nd | nd | nd |
| OR Quart 3 | 3.7 | nd | nd | 3.7 | nd | nd | nd | nd | nd |
| p Value | 0.29 | nd | nd | 0.29 | nd | nd | nd | nd | nd |
| 95% CI of OR Quart3 | 0.32 | nd | nd | 0.32 | nd | nd | nd | nd | nd |
|  | 42 | nd | nd | 42 | nd | nd | nd | nd | nd |
| OR Quart 4 | 3.3 | nd | nd | 3.3 | nd | nd | nd | nd | nd |
| p Value | 0.33 | nd | nd | 0.33 | nd | nd | nd | nd | nd |
| 95% CI of OR Quart4 | 0.29 | nd | nd | 0.29 | nd | nd | nd | nd | nd |
|  | 37 | nd | nd | 37 | nd | nd | nd | nd | nd |

## Poly [ADP-ribose] polymerase 1 (cleaved)

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 0.0140 | 1.00E-9 | 0.0140 | 1.00E-9 | 1.00E-9 |
| Average | 0.00479 | 0.0101 | 0.00479 | 0.00956 | 0.00479 | 0.00605 |
| Stdev | 0.00701 | 0.00857 | 0.00701 | 0.00900 | 0.00701 | 0.00754 |
| p(t-test) |  | 0.018 |  | 0.034 |  | 0.65 |
| Min | 1.00E-9 | 1.00E-9 | 1.00E-9 | 1.00E-9 | 1.00E-9 | 1.00E-9 |
| Max | 0.0357 | 0.0253 | 0.0357 | 0.0253 | 0.0357 | 0.0144 |
| n (Samp) | 97 | 12 | 97 | 12 | 97 | 7 |
| n (Patient) | 97 | 12 | 97 | 12 | 97 | 7 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 0.00990 | 1.00E-9 | 0.00691 | nd | nd |
| Average | 0.00475 | 0.00992 | 0.00475 | 0.00892 | nd | nd |
| Stdev | 0.00651 | 0.00984 | 0.00651 | 0.0106 | nd | nd |
| p(t-test) |  | 0.063 |  | 0.13 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 1.00E-9 | 1.00E-9 | 1.00E-9 | 1.00E-9 | nd | nd |
| Max | 0.0357 | 0.0253 | 0.0357 | 0.0253 | nd | nd |
| n (Samp) | 159 | 6 | 159 | 6 | nd | nd |
| n (Patient) | 159 | 6 | 159 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.0140 | 1.00E-9 | 0.0140 | 1.00E-9 | 0.00691 |
| Average | 0.00380 | 0.00938 | 0.00380 | 0.00938 | 0.00380 | 0.00706 |
| Stdev | 0.00658 | 0.00789 | 0.00658 | 0.00789 | 0.00658 | 0.00773 |
| p(t-test) | | 0.027 | | 0.027 | | 0.25 |
| Min | 1.00E-9 | 1.00E-9 | 1.00E-9 | 1.00E-9 | 1.00E-9 | 1.00E-9 |
| Max | 0.0357 | 0.0183 | 0.0357 | 0.0183 | 0.0357 | 0.0144 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.65 | 0.69 | 0.64 | 0.58 | 0.69 | 0.52 | nd | 0.60 |
| SE | 0.090 | 0.12 | 0.11 | 0.091 | 0.12 | 0.11 | 0.11 | nd | 0.13 |
| p | 0.059 | 0.23 | 0.081 | 0.13 | 0.51 | 0.081 | 0.87 | nd | 0.43 |
| nCohort 1 | 97 | 159 | 84 | 97 | 159 | 84 | 97 | nd | 84 |
| nCohort 2 | 12 | 6 | 8 | 12 | 6 | 8 | 7 | nd | 6 |
| Cutoff 1 | 0 | 0 | 0 | 0 | 0 | 0 | 0 | nd | 0 |
| Sens 1 | 100% | 100% | 100% | 100% | 100% | 100% | 100% | nd | 100% |
| Spec 1 | 0% | 0% | 0% | 0% | 0% | 0% | 0% | nd | 0% |
| Cutoff 2 | 0 | 0 | 0 | 0 | 0 | 0 | 0 | nd | 0 |
| Sens 2 | 100% | 100% | 100% | 100% | 100% | 100% | 100% | nd | 100% |
| Spec 2 | 0% | 0% | 0% | 0% | 0% | 0% | 0% | nd | 0% |
| Cutoff 3 | 0 | 0 | 0 | 0 | 0 | 0 | 0 | nd | 0 |
| Sens 3 | 100% | 100% | 100% | 100% | 100% | 100% | 100% | nd | 100% |
| Spec 3 | 0% | 0% | 0% | 0% | 0% | 0% | 0% | nd | 0% |
| Cutoff 4 | 0.00598 | 0.00598 | 0.00471 | 0.00598 | 0.00598 | 0.00471 | 0.00598 | nd | 0.00471 |
| Sens 4 | 58% | 50% | 62% | 58% | 50% | 62% | 43% | nd | 50% |
| Spec 4 | 70% | 71% | 74% | 70% | 71% | 74% | 70% | nd | 74% |
| Cutoff 5 | 0.0144 | 0.0144 | 0.0120 | 0.0144 | 0.0144 | 0.0120 | 0.0144 | nd | 0.0120 |
| Sens 5 | 17% | 17% | 62% | 17% | 17% | 62% | 0% | nd | 50% |
| Spec 5 | 94% | 96% | 81% | 94% | 96% | 81% | 94% | nd | 81% |
| Cutoff 6 | 0.0144 | 0.0144 | 0.0144 | 0.0144 | 0.0144 | 0.0144 | 0.0144 | nd | 0.0144 |
| Sens 6 | 17% | 17% | 12% | 17% | 17% | 12% | 0% | nd | 0% |
| Spec 6 | 94% | 96% | 99% | 94% | 96% | 99% | 94% | nd | 99% |
| OR Quart 2 | 1.0 | 1.0 | 2.1 | 1.6 | 2.1 | 2.1 | 3.3 | nd | 2.0 |
| p Value | 1.0 | 1.0 | 0.56 | 0.64 | 0.56 | 0.56 | 0.32 | nd | 0.58 |
| 95% CI of OR Quart2 | 0.13 7.7 | 0.060 17 | 0.18 25 | 0.24 10 | 0.18 24 | 0.18 25 | 0.32 34 | nd nd | 0.17 24 |
| OR Quart 3 | 1.0 | 1.0 | 0 | 0 | 0 | 0 | 1.0 | nd | 0 |
| p Value | 1.0 | 1.0 | na | na | na | na | 1.0 | nd | na |
| 95% CI of OR Quart3 | 0.13 7.7 | 0.060 17 | na na | na na | na na | na na | 0.059 17 | nd nd | na na |
| OR Quart 4 | 3.4 | 3.1 | 6.1 | 4.2 | 3.1 | 6.1 | 2.1 | nd | 3.1 |
| p Value | 0.16 | 0.34 | 0.11 | 0.095 | 0.34 | 0.11 | 0.56 | nd | 0.34 |
| 95% CI of OR Quart4 | 0.62 19 | 0.31 31 | 0.65 57 | 0.78 22 | 0.31 31 | 0.65 57 | 0.18 25 | nd nd | 0.30 33 |

## KSP-Cadherin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.23 | 2.26 | 1.23 | 2.26 | nd | nd |
| Average | 1.66 | 2.48 | 1.66 | 2.48 | nd | nd |
| Stdev | 1.71 | 1.19 | 1.71 | 1.19 | nd | nd |
| p(t-test) | | 0.17 | | 0.17 | nd | nd |
| Min | 0.00263 | 0.562 | 0.00263 | 0.562 | nd | nd |
| Max | 11.9 | 4.23 | 11.9 | 4.23 | nd | nd |
| n (Samp) | 68 | 9 | 68 | 9 | nd | nd |
| n (Patient) | 68 | 9 | 68 | 9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.17 | 1.87 | 1.17 | 1.87 | nd | nd |
| Average | 1.76 | 2.07 | 1.76 | 2.07 | nd | nd |
| Stdev | 1.84 | 1.22 | 1.84 | 1.22 | nd | nd |
| p(t-test) | | 0.69 | | 0.69 | nd | nd |
| Min | 0.00263 | 0.562 | 0.00263 | 0.562 | nd | nd |
| Max | 11.9 | 4.23 | 11.9 | 4.23 | nd | nd |
| n (Samp) | 59 | 6 | 59 | 6 | nd | nd |
| n (Patient) | 59 | 6 | 59 | 6 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | nd | 0.64 | 0.74 | nd | 0.64 | nd | nd | nd |
| SE | 0.099 | nd | 0.13 | 0.099 | nd | 0.13 | nd | nd | nd |
| p | 0.017 | nd | 0.29 | 0.017 | nd | 0.29 | nd | nd | nd |
| nCohort 1 | 68 | nd | 59 | 68 | nd | 59 | nd | nd | nd |
| nCohort 2 | 9 | nd | 6 | 9 | nd | 6 | nd | nd | nd |
| Cutoff 1 | 1.61 | nd | 1.51 | 1.61 | nd | 1.51 | nd | nd | nd |
| Sens 1 | 78% | nd | 83% | 78% | nd | 83% | nd | nd | nd |
| Spec 1 | 60% | nd | 59% | 60% | nd | 59% | nd | nd | nd |
| Cutoff 2 | 1.56 | nd | 1.51 | 1.56 | nd | 1.51 | nd | nd | nd |
| Sens 2 | 89% | nd | 83% | 89% | nd | 83% | nd | nd | nd |
| Spec 2 | 60% | nd | 59% | 60% | nd | 59% | nd | nd | nd |
| Cutoff 3 | 0.466 | nd | 0.494 | 0.466 | nd | 0.494 | nd | nd | nd |
| Sens 3 | 100% | nd | 100% | 100% | nd | 100% | nd | nd | nd |
| Spec 3 | 19% | nd | 19% | 19% | nd | 19% | nd | nd | nd |
| Cutoff 4 | 1.99 | nd | 2.08 | 1.99 | nd | 2.08 | nd | nd | nd |
| Sens 4 | 67% | nd | 50% | 67% | nd | 50% | nd | nd | nd |
| Spec 4 | 71% | nd | 71% | 71% | nd | 71% | nd | nd | nd |
| Cutoff 5 | 2.24 | nd | 2.54 | 2.24 | nd | 2.54 | nd | nd | nd |
| Sens 5 | 56% | nd | 17% | 56% | nd | 17% | nd | nd | nd |
| Spec 5 | 81% | nd | 81% | 81% | nd | 81% | nd | nd | nd |
| Cutoff 6 | 3.17 | nd | 3.77 | 3.17 | nd | 3.77 | nd | nd | nd |
| Sens 6 | 22% | nd | 17% | 22% | nd | 17% | nd | nd | nd |
| Spec 6 | 91% | nd | 92% | 91% | nd | 92% | nd | nd | nd |
| OR Quart 2 | 0 | nd | 0 | 0 | nd | 0 | nd | nd | nd |
| p Value | na | nd | na | na | nd | na | nd | nd | nd |
| 95% CI of OR Quart2 | na | nd | na | na | nd | na | nd | nd | nd |
| OR Quart 3 | 3.4 | nd | 3.5 | 3.4 | nd | 3.5 | nd | nd | nd |
| p Value | 0.31 | nd | 0.31 | 0.31 | nd | 0.31 | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of OR Quart3 | 0.32 36 | nd | 0.32 37 | 0.32 36 | nd | 0.32 37 | nd | nd | nd |
| OR Quart 4 | 6.0 | nd | 2.0 | 6.0 | nd | 2.0 | nd | nd | nd |
| p Value | 0.12 | nd | 0.59 | 0.12 | nd | 0.59 | nd | nd | nd |
| 95% CI of OR Quart4 | 0.63 57 | nd | 0.16 24 | 0.63 57 | nd | 0.16 24 | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.141 | 0.904 | 0.141 | 0.904 | nd | nd |
| Average | 0.957 | 1.95 | 0.957 | 1.95 | nd | nd |
| Stdev | 1.61 | 2.64 | 1.61 | 2.64 | nd | nd |
| p(t-test) |  | 0.16 |  | 0.16 | nd | nd |
| Min | 0.00360 | 0.104 | 0.00360 | 0.104 | nd | nd |
| Max | 6.71 | 7.54 | 6.71 | 7.54 | nd | nd |
| n (Samp) | 41 | 8 | 41 | 8 | nd | nd |
| n (Patient) | 41 | 8 | 41 | 8 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.69 | nd | nd | 0.69 | nd | nd | nd | nd | nd |
| SE | 0.11 | nd | nd | 0.11 | nd | nd | nd | nd | nd |
| p | 0.092 | nd | nd | 0.092 | nd | nd | nd | nd | nd |
| nCohort 1 | 41 | nd | nd | 41 | nd | nd | nd | nd | nd |
| nCohort 2 | 8 | nd | nd | 8 | nd | nd | nd | nd | nd |
| Cutoff 1 | 0.141 | nd | nd | 0.141 | nd | nd | nd | nd | nd |
| Sens 1 | 75% | nd | nd | 75% | nd | nd | nd | nd | nd |
| Spec 1 | 51% | nd | nd | 51% | nd | nd | nd | nd | nd |
| Cutoff 2 | 0.107 | nd | nd | 0.107 | nd | nd | nd | nd | nd |
| Sens 2 | 88% | nd | nd | 88% | nd | nd | nd | nd | nd |
| Spec 2 | 49% | nd | nd | 49% | nd | nd | nd | nd | nd |
| Cutoff 3 | 0.0796 | nd | nd | 0.0796 | nd | nd | nd | nd | nd |
| Sens 3 | 100% | nd | nd | 100% | nd | nd | nd | nd | nd |
| Spec 3 | 41% | nd | nd | 41% | nd | nd | nd | nd | nd |
| Cutoff 4 | 0.693 | nd | nd | 0.693 | nd | nd | nd | nd | nd |
| Sens 4 | 50% | nd | nd | 50% | nd | nd | nd | nd | nd |
| Spec 4 | 71% | nd | nd | 71% | nd | nd | nd | nd | nd |
| Cutoff 5 | 1.56 | nd | nd | 1.56 | nd | nd | nd | nd | nd |
| Sens 5 | 38% | nd | nd | 38% | nd | nd | nd | nd | nd |
| Spec 5 | 80% | nd | nd | 80% | nd | nd | nd | nd | nd |
| Cutoff 6 | 3.31 | nd | nd | 3.31 | nd | nd | nd | nd | nd |
| Sens 6 | 25% | nd | nd | 25% | nd | nd | nd | nd | nd |
| Spec 6 | 90% | nd | nd | 90% | nd | nd | nd | nd | nd |
| OR Quart 2 | >4.0 | nd | nd | >4.0 | nd | nd | nd | nd | nd |
| p Value | <0.26 | nd | nd | <0.26 | nd | nd | nd | nd | nd |
| 95% CI of OR Quart2 | >0.35 na | nd | nd | >0.35 na | nd | nd | nd | nd | nd |
| OR Quart 3 | >1.1 | nd | nd | >1.1 | nd | nd | nd | nd | nd |
| p Value | <0.95 | nd | nd | <0.95 | nd | nd | nd | nd | nd |
| 95% CI of OR Quart3 | >0.061 na | nd | nd | >0.061 na | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 4 | >5.3 | nd | nd | >5.3 | nd | nd | nd | nd | nd |
| p Value | <0.16 | nd | nd | <0.16 | nd | nd | nd | nd | nd |
| 95% CI of | >0.51 | nd | nd | >0.51 | nd | nd | nd | nd | nd |
| OR Quart4 | na | nd | nd | na | nd | nd | nd | nd | nd |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

## Apolipoprotein A-II

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 50600 | 49600 | 50600 | 44300 | 50600 | 54300 |
| Average | 51700 | 52000 | 51700 | 50700 | 51700 | 57500 |
| Stdev | 22200 | 21100 | 22200 | 36000 | 22200 | 32700 |
| p(t-test) | | 0.94 | | 0.83 | | 0.29 |
| Min | 5450 | 7970 | 5450 | 7680 | 5450 | 8560 |
| Max | 105000 | 97000 | 105000 | 253000 | 105000 | 152000 |
| n (Samp) | 105 | 45 | 105 | 50 | 105 | 24 |
| n (Patient) | 97 | 45 | 97 | 50 | 97 | 24 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 49500 | 59900 | 49500 | 50300 | 49500 | 57100 |
| Average | 51700 | 60100 | 51700 | 64100 | 51700 | 67500 |
| Stdev | 26400 | 18100 | 26400 | 55400 | 26400 | 44100 |
| p(t-test) | | 0.26 | | 0.097 | | 0.062 |
| Min | 1810 | 34500 | 1810 | 12100 | 1810 | 10900 |
| Max | 253000 | 95300 | 253000 | 251000 | 253000 | 176000 |
| n (Samp) | 246 | 13 | 246 | 16 | 246 | 11 |
| n (Patient) | 160 | 13 | 160 | 16 | 160 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 50200 | 49600 | 50200 | 43800 | 50200 | 51400 |
| Average | 52500 | 51300 | 52500 | 51600 | 52500 | 58800 |
| Stdev | 20200 | 21600 | 20200 | 38000 | 20200 | 32900 |
| p(t-test) | | 0.76 | | 0.86 | | 0.25 |
| Min | 8680 | 7970 | 8680 | 7680 | 8680 | 8560 |
| Max | 123000 | 97000 | 123000 | 253000 | 123000 | 152000 |
| n (Samp) | 96 | 40 | 96 | 44 | 96 | 21 |
| n (Patient) | 84 | 40 | 84 | 44 | 84 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.63 | 0.48 | 0.45 | 0.55 | 0.44 | 0.55 | 0.60 | 0.55 |
| SE | 0.052 | 0.085 | 0.055 | 0.050 | 0.076 | 0.053 | 0.066 | 0.092 | 0.071 |
| p | 0.89 | 0.12 | 0.75 | 0.35 | 0.55 | 0.24 | 0.50 | 0.27 | 0.49 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| nCohort 1 | 105 | 246 | 96 | 105 | 246 | 96 | 105 | 246 | 96 |
| nCohort 2 | 45 | 13 | 40 | 50 | 16 | 44 | 24 | 11 | 21 |
| Cutoff 1 | 39500 | 43000 | 39500 | 35500 | 37700 | 35000 | 40700 | 46100 | 40900 |
| Sens 1 | 71% | 77% | 70% | 70% | 75% | 70% | 71% | 73% | 71% |
| Spec 1 | 33% | 40% | 28% | 24% | 31% | 19% | 35% | 46% | 31% |
| Cutoff 2 | 33000 | 42600 | 32500 | 29600 | 35500 | 25400 | 29600 | 37000 | 35000 |
| Sens 2 | 80% | 85% | 80% | 80% | 81% | 82% | 83% | 82% | 81% |
| Spec 2 | 22% | 39% | 16% | 19% | 24% | 6% | 19% | 29% | 19% |
| Cutoff 3 | 23400 | 37000 | 23400 | 20800 | 14000 | 20800 | 14000 | 36200 | 21600 |
| Sens 3 | 91% | 92% | 90% | 90% | 94% | 91% | 92% | 91% | 90% |
| Spec 3 | 10% | 29% | 5% | 6% | 5% | 3% | 4% | 27% | 4% |
| Cutoff 4 | 61300 | 61500 | 61500 | 61300 | 61500 | 61500 | 61300 | 61500 | 61500 |
| Sens 4 | 33% | 46% | 30% | 32% | 44% | 34% | 46% | 36% | 48% |
| Spec 4 | 70% | 70% | 71% | 70% | 70% | 71% | 70% | 70% | 71% |
| Cutoff 5 | 69000 | 69400 | 67400 | 69000 | 69400 | 67400 | 69000 | 69400 | 67400 |
| Sens 5 | 24% | 38% | 22% | 18% | 38% | 16% | 33% | 36% | 43% |
| Spec 5 | 80% | 80% | 80% | 80% | 80% | 80% | 80% | 80% | 80% |
| Cutoff 6 | 82000 | 82800 | 77900 | 82000 | 82800 | 77900 | 82000 | 82800 | 77900 |
| Sens 6 | 9% | 8% | 12% | 8% | 12% | 11% | 21% | 36% | 19% |
| Spec 6 | 90% | 90% | 91% | 90% | 90% | 91% | 90% | 90% | 91% |
| OR Quart 2 | 1.6 | 4.1 | 0.74 | 0.60 | 0.98 | 0.76 | 0.80 | 4.2 | 0.61 |
| p Value | 0.36 | 0.21 | 0.58 | 0.32 | 0.98 | 0.60 | 0.74 | 0.21 | 0.49 |
| 95% CI of OR Quart2 | 0.59–4.2 | 0.45–38 | 0.25–2.2 | 0.22–1.6 | 0.24–4.1 | 0.27–2.1 | 0.22–3.0 | 0.46–39 | 0.15–2.5 |
| OR Quart 3 | 0.87 | 3.0 | 1.1 | 1.1 | 0.48 | 0.65 | 0.62 | 2.0 | 0.28 |
| p Value | 0.79 | 0.34 | 0.79 | 0.81 | 0.41 | 0.42 | 0.49 | 0.57 | 0.15 |
| 95% CI of OR Quart3 | 0.31–2.5 | 0.31–30 | 0.41–3.2 | 0.44–2.8 | 0.086–2.7 | 0.22–1.9 | 0.16–2.4 | 0.18–23 | 0.052–1.5 |
| OR Quart 4 | 1.2 | 5.2 | 1.1 | 1.2 | 1.5 | 1.8 | 1.6 | 4.1 | 1.6 |
| p Value | 0.67 | 0.14 | 0.79 | 0.75 | 0.53 | 0.22 | 0.42 | 0.21 | 0.41 |
| 95% CI of OR Quart4 | 0.46–3.4 | 0.60–46 | 0.41–3.2 | 0.46–3.0 | 0.41–5.7 | 0.69–4.9 | 0.50–5.2 | 0.45–38 | 0.50–5.4 |

## Bcl2 antagonist of cell death

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.432 | 0.221 | nd | nd | nd | nd |
| Average | 0.590 | 0.255 | nd | nd | nd | nd |
| Stdev | 0.478 | 0.204 | nd | nd | nd | nd |
| p(t-test) |  | 0.10 | nd | nd | nd | nd |
| Min | 0.0873 | 0.0786 | nd | nd | nd | nd |
| Max | 2.49 | 0.651 | nd | nd | nd | nd |
| n (Samp) | 32 | 6 | nd | nd | nd | nd |
| n (Patient) | 20 | 6 | nd | nd | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.596 | 0.213 | nd | nd | nd | nd |
| Average | 0.643 | 0.243 | nd | nd | nd | nd |
| Stdev | 0.503 | 0.174 | nd | nd | nd | nd |
| p(t-test) |  | 0.035 | nd | nd | nd | nd |
| Min | 0.0873 | 0.0786 | nd | nd | nd | nd |
| Max | 2.49 | 0.651 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 27 | 8 | nd | nd | nd | nd |
| n (Patient) | 17 | 8 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.21 | nd | 0.19 | nd | nd | nd | nd | nd | nd |
| SE | 0.12 | nd | 0.098 | nd | nd | nd | nd | nd | nd |
| p | 0.012 | nd | 0.0013 | nd | nd | nd | nd | nd | nd |
| nCohort 1 | 32 | nd | 27 | nd | nd | nd | nd | nd | nd |
| nCohort 2 | 6 | nd | 8 | nd | nd | nd | nd | nd | nd |
| Cutoff 1 | 0.114 | nd | 0.181 | nd | nd | nd | nd | nd | nd |
| Sens 1 | 83% | nd | 75% | nd | nd | nd | nd | nd | nd |
| Spec 1 | 6% | nd | 15% | nd | nd | nd | nd | nd | nd |
| Cutoff 2 | 0.114 | nd | 0.114 | nd | nd | nd | nd | nd | nd |
| Sens 2 | 83% | nd | 88% | nd | nd | nd | nd | nd | nd |
| Spec 2 | 6% | nd | 7% | nd | nd | nd | nd | nd | nd |
| Cutoff 3 | 0 | nd | 0 | nd | nd | nd | nd | nd | nd |
| Sens 3 | 100% | nd | 100% | nd | nd | nd | nd | nd | nd |
| Spec 3 | 0% | nd | 0% | nd | nd | nd | nd | nd | nd |
| Cutoff 4 | 0.703 | nd | 0.771 | nd | nd | nd | nd | nd | nd |
| Sens 4 | 0% | nd | 0% | nd | nd | nd | nd | nd | nd |
| Spec 4 | 72% | nd | 70% | nd | nd | nd | nd | nd | nd |
| Cutoff 5 | 0.991 | nd | 1.01 | nd | nd | nd | nd | nd | nd |
| Sens 5 | 0% | nd | 0% | nd | nd | nd | nd | nd | nd |
| Spec 5 | 81% | nd | 81% | nd | nd | nd | nd | nd | nd |
| Cutoff 6 | 1.05 | nd | 1.13 | nd | nd | nd | nd | nd | nd |
| Sens 6 | 0% | nd | 0% | nd | nd | nd | nd | nd | nd |
| Spec 6 | 91% | nd | 93% | nd | nd | nd | nd | nd | nd |
| OR Quart 2 | >1.2 | nd | >1.1 | nd | nd | nd | nd | nd | nd |
| p Value | <0.88 | nd | <0.94 | nd | nd | nd | nd | nd | nd |
| 95% CI of OR Quart2 | >0.067 na | nd | >0.060 na | nd | nd | nd | nd | nd | nd |
| OR Quart 3 | >2.5 | nd | >4.5 | nd | nd | nd | nd | nd | nd |
| p Value | <0.49 | nd | <0.24 | nd | nd | nd | nd | nd | nd |
| 95% CI of OR Quart3 | >0.19 na | nd | >0.37 na | nd | nd | nd | nd | nd | nd |
| OR Quart 4 | >5.0 | nd | >9.0 | nd | nd | nd | nd | nd | nd |
| p Value | <0.20 | nd | <0.083 | nd | nd | nd | nd | nd | nd |
| 95% CI of OR Quart4 | >0.42 na | nd | >0.75 na | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.5 | 14.8 | nd | nd | nd | nd |
| Average | 44.5 | 24.6 | nd | nd | nd | nd |
| Stdev | 60.6 | 24.9 | nd | nd | nd | nd |
| p(t-test) | | 0.27 | nd | nd | nd | nd |
| Min | 0.400 | 4.59 | nd | nd | nd | nd |
| Max | 366 | 78.6 | nd | nd | nd | nd |
| n (Samp) | 57 | 12 | nd | nd | nd | nd |
| n (Patient) | 37 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.8 | 13.2 | nd | nd | nd | nd |
| Average | 42.5 | 12.5 | nd | nd | nd | nd |
| Stdev | 58.2 | 7.24 | nd | nd | nd | nd |
| p(t-test) | | 0.21 | nd | nd | nd | nd |
| Min | 0.400 | 4.32 | nd | nd | nd | nd |
| Max | 366 | 21.7 | nd | nd | nd | nd |
| n (Samp) | 89 | 6 | nd | nd | nd | nd |
| n (Patient) | 61 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.1 | 9.06 | nd | nd | nd | nd |
| Average | 50.3 | 20.6 | nd | nd | nd | nd |
| Stdev | 67.6 | 24.4 | nd | nd | nd | nd |
| p(t-test) | | 0.12 | nd | nd | nd | nd |
| Min | 6.05 | 3.79 | nd | nd | nd | nd |
| Max | 366 | 78.6 | nd | nd | nd | nd |
| n (Samp) | 42 | 14 | nd | nd | nd | nd |
| n (Patient) | 27 | 14 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.39 | 0.28 | 0.26 | nd | nd | nd | nd | nd | nd |
| SE | 0.094 | 0.12 | 0.083 | nd | nd | nd | nd | nd | nd |
| p | 0.25 | 0.077 | 0.0038 | nd | nd | nd | nd | nd | nd |
| nCohort 1 | 57 | 89 | 42 | nd | nd | nd | nd | nd | nd |
| nCohort 2 | 12 | 6 | 14 | nd | nd | nd | nd | nd | nd |
| Cutoff 1 | 8.91 | 3.65 | 6.05 | nd | nd | nd | nd | nd | nd |
| Sens 1 | 75% | 100% | 71% | nd | nd | nd | nd | nd | nd |
| Spec 1 | 21% | 7% | 2% | nd | nd | nd | nd | nd | nd |
| Cutoff 2 | 8.22 | 3.65 | 4.59 | nd | nd | nd | nd | nd | nd |
| Sens 2 | 83% | 100% | 86% | nd | nd | nd | nd | nd | nd |
| Spec 2 | 19% | 7% | 0% | nd | nd | nd | nd | nd | nd |
| Cutoff 3 | 4.59 | 3.65 | 3.79 | nd | nd | nd | nd | nd | nd |
| Sens 3 | 92% | 100% | 93% | nd | nd | nd | nd | nd | nd |
| Spec 3 | 9% | 7% | 0% | nd | nd | nd | nd | nd | nd |
| Cutoff 4 | 46.1 | 45.3 | 49.3 | nd | nd | nd | nd | nd | nd |
| Sens 4 | 17% | 0% | 14% | nd | nd | nd | nd | nd | nd |
| Spec 4 | 70% | 71% | 71% | nd | nd | nd | nd | nd | nd |
| Cutoff 5 | 54.5 | 57.5 | 54.5 | nd | nd | nd | nd | nd | nd |
| Sens 5 | 17% | 0% | 14% | nd | nd | nd | nd | nd | nd |
| Spec 5 | 81% | 81% | 81% | nd | nd | nd | nd | nd | nd |
| Cutoff 6 | 90.6 | 94.1 | 106 | nd | nd | nd | nd | nd | nd |
| Sens 6 | 0% | 0% | 0% | nd | nd | nd | nd | nd | nd |
| Spec 6 | 91% | 91% | 90% | nd | nd | nd | nd | nd | nd |
| OR Quart 2 | 1.1 | >1.0 | 1.0 | nd | nd | nd | nd | nd | nd |
| p Value | 0.95 | <0.98 | 1.0 | nd | nd | nd | nd | nd | nd |
| 95% CI of OR Quart2 | 0.13 | >0.062 | 0.12 | nd | nd | nd | nd | nd | nd |
| | 8.6 | na | 8.3 | nd | nd | nd | nd | nd | nd |
| OR Quart 3 | 1.7 | >3.4 | 1.0 | nd | nd | nd | nd | nd | nd |
| p Value | 0.58 | <0.30 | 1.0 | nd | nd | nd | nd | nd | nd |
| 95% CI of OR Quart3 | 0.25 | >0.33 | 0.12 | nd | nd | nd | nd.| nd | nd |
| | 12 | na | 8.3 | nd | nd | nd | nd | nd | nd |
| OR Quart 4 | 3.3 | >2.3 | 8.0 | nd | nd | nd | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.19 | <0.51 | 0.026 | nd | nd | nd | nd | nd | nd |
| 95% CI of | 0.55 | >0.19 | 1.3 | nd | nd | nd | nd | nd | nd |
| OR Quart4 | 20 | na | 50 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 72700 | 88200 | 72700 | 92800 | 72700 | 98500 |
| Average | 102000 | 129000 | 102000 | 127000 | 102000 | 134000 |
| Stdev | 106000 | 88400 | 106000 | 90200 | 106000 | 91700 |
| p(t-test) |  | 0.26 |  | 0.28 |  | 0.28 |
| Min | 14500 | 34900 | 14500 | 3320 | 14500 | 25800 |
| Max | 621000 | 334000 | 621000 | 340000 | 621000 | 283000 |
| n (Samp) | 52 | 28 | 52 | 31 | 52 | 16 |
| n (Patient) | 50 | 28 | 50 | 31 | 50 | 16 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 86800 | 130000 | 86800 | 73000 | 86800 | 109000 |
| Average | 116000 | 145000 | 116000 | 120000 | 116000 | 116000 |
| Stdev | 94600 | 90700 | 94600 | 94900 | 94600 | 73700 |
| p(t-test) |  | 0.35 |  | 0.87 |  | 0.99 |
| Min | 3320 | 34900 | 3320 | 30700 | 3320 | 56100 |
| Max | 621000 | 334000 | 621000 | 340000 | 621000 | 283000 |
| n (Samp) | 123 | 10 | 123 | 16 | 123 | 8 |
| n (Patient) | 96 | 10 | 96 | 16 | 96 | 8 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 81200 | 88200 | 81200 | 103000 | 81200 | 89400 |
| Average | 119000 | 122000 | 119000 | 122000 | 119000 | 139000 |
| Stdev | 114000 | 85000 | 114000 | 78600 | 114000 | 94300 |
| p(t-test) |  | 0.89 |  | 0.89 |  | 0.59 |
| Min | 39700 | 38100 | 39700 | 3320 | 39700 | 25800 |
| Max | 621000 | 314000 | 621000 | 300000 | 621000 | 277000 |
| n (Samp) | 51 | 22 | 51 | 27 | 51 | 11 |
| n (Patient) | 44 | 22 | 44 | 27 | 44 | 11 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.62 | 0.63 | 0.54 | 0.59 | 0.47 | 0.57 | 0.62 | 0.56 | 0.59 |
| SE | 0.067 | 0.098 | 0.075 | 0.065 | 0.078 | 0.069 | 0.083 | 0.11 | 0.098 |
| p | 0.066 | 0.18 | 0.57 | 0.15 | 0.72 | 0.32 | 0.13 | 0.57 | 0.38 |
| nCohort 1 | 52 | 123 | 51 | 52 | 123 | 51 | 52 | 123 | 51 |
| nCohort 2 | 28 | 10 | 22 | 31 | 16 | 27 | 16 | 8 | 11 |
| Cutoff 1 | 78500 | 79100 | 75400 | 62800 | 40700 | 67700 | 61700 | 61700 | 79100 |
| Sens 1 | 71% | 70% | 73% | 71% | 75% | 70% | 75% | 75% | 73% |
| Spec 1 | 56% | 46% | 41% | 42% | 9% | 37% | 40% | 31% | 49% |
| Cutoff 2 | 52700 | 78500 | 52700 | 47600 | 39700 | 54300 | 57000 | 57000 | 70100 |
| Sens 2 | 82% | 80% | 82% | 81% | 81% | 81% | 81% | 88% | 82% |
| Spec 2 | 21% | 46% | 12% | 17% | 8% | 14% | 29% | 26% | 39% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 3 | 40700 | 72100 | 47600 | 39700 | 31800 | 39700 | 48100 | 54700 | 47600 |
| Sens 3 | 93% | 90% | 91% | 90% | 94% | 93% | 94% | 100% | 91% |
| Spec 3 | 10% | 41% | 10% | 8% | 6% | 2% | 19% | 23% | 10% |
| Cutoff 4 | 97000 | 131000 | 100000 | 97000 | 131000 | 100000 | 97000 | 131000 | 100000 |
| Sens 4 | 46% | 50% | 32% | 48% | 38% | 52% | 50% | 25% | 45% |
| Spec 4 | 71% | 71% | 71% | 71% | 71% | 71% | 71% | 71% | 71% |
| Cutoff 5 | 114000 | 165000 | 138000 | 114000 | 165000 | 138000 | 114000 | 165000 | 138000 |
| Sens 5 | 36% | 40% | 27% | 42% | 38% | 33% | 38% | 12% | 36% |
| Spec 5 | 81% | 80% | 80% | 81% | 80% | 80% | 81% | 80% | 80% |
| Cutoff 6 | 153000 | 243000 | 242000 | 153000 | 243000 | 242000 | 153000 | 243000 | 242000 |
| Sens 6 | 32% | 20% | 18% | 39% | 6% | 11% | 31% | 12% | 27% |
| Spec 6 | 90% | 90% | 90% | 90% | 90% | 90% | 90% | 90% | 90% |
| OR Quart 2 | 0.78 | 3.2 | 0.74 | 0.47 | 0.14 | 0.54 | 1.0 | 2.0 | 0.93 |
| p Value | 0.72 | 0.33 | 0.70 | 0.27 | 0.079 | 0.41 | 1.0 | 0.58 | 0.94 |
| 95% CI of OR Quart2 | 0.19–3.1 | 0.32–32 | 0.16–3.4 | 0.12–1.8 | 0.016–1.3 | 0.13–2.3 | 0.17–5.8 | 0.17–23 | 0.11–7.6 |
| OR Quart 3 | 1.3 | 2.1 | 1.7 | 0.60 | 0.62 | 1.6 | 1.4 | 4.3 | 1.6 |
| p Value | 0.74 | 0.56 | 0.48 | 0.44 | 0.50 | 0.50 | 0.67 | 0.21 | 0.63 |
| 95% CI of OR Quart3 | 0.33–4.7 | 0.18–24 | 0.41–6.7 | 0.16–2.2 | 0.16–2.4 | 0.42–5.9 | 0.27–7.7 | 0.45–41 | 0.23–11 |
| OR Quart 4 | 2.3 | 4.3 | 1.2 | 2.0 | 0.83 | 1.8 | 2.5 | 0.97 | 2.2 |
| p Value | 0.20 | 0.21 | 0.80 | 0.27 | 0.78 | 0.39 | 0.25 | 0.98 | 0.42 |
| 95% CI of OR Quart4 | 0.64–8.5 | 0.45–40 | 0.29–4.9 | 0.58–6.9 | 0.23–3.0 | 0.48–6.6 | 0.52–13 | 0.058–16 | 0.33–14 |

## Cadherin-5

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 20.4 | 21.2 | nd | nd | nd | nd |
| Average | 20.5 | 23.9 | nd | nd | nd | nd |
| Stdev | 5.01 | 5.05 | nd | nd | nd | nd |
| p(t-test) |  | 0.065 | nd | nd | nd | nd |
| Min | 12.4 | 18.0 | nd | nd | nd | nd |
| Max | 35.9 | 32.1 | nd | nd | nd | nd |
| n (Samp) | 48 | 9 | nd | nd | nd | nd |
| n (Patient) | 30 | 9 | nd | nd | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 20.5 | 21.2 | nd | nd | nd | nd |
| Average | 20.7 | 23.3 | nd | nd | nd | nd |
| Stdev | 4.09 | 5.02 | nd | nd | nd | nd |
| p(t-test) |  | 0.080 | nd | nd | nd | nd |
| Min | 12.4 | 17.0 | nd | nd | nd | nd |
| Max | 29.3 | 32.1 | nd | nd | nd | nd |
| n (Samp) | 34 | 11 | nd | nd | nd | nd |
| n (Patient) | 20 | 11 | nd | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.69 | nd | 0.64 | nd | nd | nd | nd | nd | nd |
| SE | 0.10 | nd | 0.10 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.077 | nd | 0.16 | nd | nd | nd | nd | nd | nd |
| nCohort 1 | 48 | nd | 34 | nd | nd | nd | nd | nd | nd |
| nCohort 2 | 9 | nd | 11 | nd | nd | nd | nd | nd | nd |
| Cutoff 1 | 20.6 | nd | 19.5 | nd | nd | nd | nd | nd | nd |
| Sens 1 | 78% | nd | 73% | nd | nd | nd | nd | nd | nd |
| Spec 1 | 54% | nd | 44% | nd | nd | nd | nd | nd | nd |
| Cutoff 2 | 18.9 | nd | 18.9 | nd | nd | nd | nd | nd | nd |
| Sens 2 | 89% | nd | 82% | nd | nd | nd | nd | nd | nd |
| Spec 2 | 42% | nd | 38% | nd | nd | nd | nd | nd | nd |
| Cutoff 3 | 17.1 | nd | 17.1 | nd | nd | nd | nd | nd | nd |
| Sens 3 | 100% | nd | 91% | nd | nd | nd | nd | nd | nd |
| Spec 3 | 33% | nd | 26% | nd | nd | nd | nd | nd | nd |
| Cutoff 4 | 22.6 | nd | 22.7 | nd | nd | nd | nd | nd | nd |
| Sens 4 | 44% | nd | 45% | nd | nd | nd | nd | nd | nd |
| Spec 4 | 71% | nd | 71% | nd | nd | nd | nd | nd | nd |
| Cutoff 5 | 24.0 | nd | 24.0 | nd | nd | nd | nd | nd | nd |
| Sens 5 | 44% | nd | 45% | nd | nd | nd | nd | nd | nd |
| Spec 5 | 81% | nd | 82% | nd | nd | nd | nd | nd | nd |
| Cutoff 6 | 26.5 | nd | 26.2 | nd | nd | nd | nd | nd | nd |
| Sens 6 | 33% | nd | 27% | nd | nd | nd | nd | nd | nd |
| Spec 6 | 92% | nd | 91% | nd | nd | nd | nd | nd | nd |
| OR Quart 2 | >2.3 | nd | 3.8 | nd | nd | nd | nd | nd | nd |
| p Value | <0.51 | nd | 0.29 | nd | nd | nd | nd | nd | nd |
| 95% CI of | >0.19 | nd | 0.32 | nd | nd | nd | nd | nd | nd |
| OR Quart2 | na | nd | 43 | nd | nd | nd | nd | nd | nd |
| OR Quart 3 | >3.8 | nd | 2.2 | nd | nd | nd | nd | nd | nd |
| p Value | <0.27 | nd | 0.54 | nd | nd | nd | nd | nd | nd |
| 95% CI of | >0.35 | nd | 0.17 | nd | nd | nd | nd | nd | nd |
| OR Quart3 | na | nd | 29 | nd | nd | nd | nd | nd | nd |
| OR Quart 4 | >5.1 | nd | 7.1 | nd | nd | nd | nd | nd | nd |
| p Value | <0.17 | nd | 0.10 | nd | nd | nd | nd | nd | nd |
| 95% CI of | >0.50 | nd | 0.68 | nd | nd | nd | nd | nd | nd |
| OR Quart4 | na | nd | 75 | nd | nd | nd | nd | nd | nd |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 208 | 138 | nd | nd | nd | nd |
| Average | 1230 | 1120 | nd | nd | nd | nd |
| Stdev | 2090 | 1790 | nd | nd | nd | nd |
| p(t-test) | | 0.87 | nd | nd | nd | nd |
| Min | 0.116 | 28.5 | nd | nd | nd | nd |
| Max | 6840 | 5430 | nd | nd | nd | nd |
| n (Samp) | 56 | 12 | nd | nd | nd | nd |
| n (Patient) | 37 | 12 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 197 | 171 | nd | nd | nd | nd |
| Average | 1140 | 463 | nd | nd | nd | nd |
| Stdev | 1940 | 716 | nd | nd | nd | nd |
| p(t-test) | | 0.40 | nd | nd | nd | nd |
| Min | 0.116 | 73.9 | nd | nd | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 6840 | 1910 | nd | nd | nd | nd |
| n (Samp) | 88 | 6 | nd | nd | nd | nd |
| n (Patient) | 61 | 6 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 381 | 151 | nd | nd | nd | nd |
| Average | 1460 | 1310 | nd | nd | nd | nd |
| Stdev | 2310 | 2210 | nd | nd | nd | nd |
| p(t-test) | | 0.84 | nd | nd | nd | nd |
| Min | 0.116 | 28.5 | nd | nd | nd | nd |
| Max | 6840 | 6400 | nd | nd | nd | nd |
| n (Samp) | 41 | 14 | nd | nd | nd | nd |
| n (Patient) | 27 | 14 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.46 | 0.44 | nd | nd | nd | nd | nd | nd |
| SE | 0.093 | 0.12 | 0.091 | nd | nd | nd | nd | nd | nd |
| p | 0.91 | 0.78 | 0.52 | nd | nd | nd | nd | nd | nd |
| nCohort 1 | 56 | 88 | 41 | nd | nd | nd | nd | nd | nd |
| nCohort 2 | 12 | 6 | 14 | nd | nd | nd | nd | nd | nd |
| Cutoff 1 | 116 | 123 | 108 | nd | nd | nd | nd | nd | nd |
| Sens 1 | 75% | 83% | 71% | nd | nd | nd | nd | nd | nd |
| Spec 1 | 32% | 33% | 27% | nd | nd | nd | nd | nd | nd |
| Cutoff 2 | 96.8 | 123 | 73.1 | nd | nd | nd | nd | nd | nd |
| Sens 2 | 83% | 83% | 86% | nd | nd | nd | nd | nd | nd |
| Spec 2 | 30% | 33% | 17% | nd | nd | nd | nd | nd | nd |
| Cutoff 3 | 47.1 | 73.1 | 42.3 | nd | nd | nd | nd | nd | nd |
| Sens 3 | 92% | 100% | 93% | nd | nd | nd | nd | nd | nd |
| Spec 3 | 12% | 17% | 12% | nd | nd | nd | nd | nd | nd |
| Cutoff 4 | 715 | 715 | 902 | nd | nd | nd | nd | nd | nd |
| Sens 4 | 33% | 17% | 21% | nd | nd | nd | nd | nd | nd |
| Spec 4 | 71% | 70% | 71% | nd | nd | nd | nd | nd | nd |
| Cutoff 5 | 1430 | 1430 | 1760 | nd | nd | nd | nd | nd | nd |
| Sens 5 | 25% | 17% | 21% | nd | nd | nd | nd | nd | nd |
| Spec 5 | 80% | 82% | 80% | nd | nd | nd | nd | nd | nd |
| Cutoff 6 | 5560 | 5160 | 6560 | nd | nd | nd | nd | nd | nd |
| Sens 6 | 0% | 0% | 0% | nd | nd | nd | nd | nd | nd |
| Spec 6 | 91% | 91% | 90% | nd | nd | nd | nd | nd | nd |
| OR Quart 2 | 0.62 | 2.2 | 1.0 | nd | nd | nd | nd | nd | nd |
| p Value | 0.63 | 0.53 | 1.0 | nd | nd | nd | nd | nd | nd |
| 95% CI of OR Quart2 | 0.090 4.3 | 0.18 26 | 0.16 6.1 | nd | nd | nd | nd | nd | nd |
| OR Quart 3 | 1.9 | 2.1 | 2.0 | nd | nd | nd | nd | nd | nd |
| p Value | 0.42 | 0.56 | 0.41 | nd | nd | nd | nd | nd | nd |
| 95% CI of OR Quart3 | 0.38 9.9 | 0.18 25 | 0.38 11 | nd | nd | nd | nd | nd | nd |
| OR Quart 4 | 0.62 | 1.0 | 1.1 | nd | nd | nd | nd | nd | nd |
| p Value | 0.63 | 0.98 | 0.92 | nd | nd | nd | nd | nd | nd |
| 95% CI of OR Quart4 | 0.090 4.3 | 0.062 18 | 0.18 6.8 | nd | nd | nd | nd | nd | nd |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.66 | 2.13 | 1.66 | 2.36 | 1.66 | 2.26 |
| Average | 2.32 | 3.66 | 2.32 | 3.88 | 2.32 | 3.00 |
| Stdev | 2.32 | 4.90 | 2.32 | 5.35 | 2.32 | 2.72 |
| p(t-test) | | 0.0028 | | 6.3E-4 | | 0.17 |
| Min | 0.183 | 0.453 | 0.183 | 0.562 | 0.183 | 0.363 |
| Max | 20.8 | 30.1 | 20.8 | 33.6 | 20.8 | 12.5 |
| n (Samp) | 260 | 51 | 260 | 56 | 260 | 25 |
| n (Patient) | 110 | 51 | 110 | 56 | 110 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.77 | 1.68 | 1.77 | 2.00 | 1.77 | 1.91 |
| Average | 2.76 | 3.12 | 2.76 | 3.22 | 2.76 | 1.68 |
| Stdev | 3.35 | 4.09 | 3.35 | 3.30 | 3.35 | 0.833 |
| p(t-test) | | 0.66 | | 0.54 | | 0.25 |
| Min | 0.183 | 0.245 | 0.183 | 0.355 | 0.183 | 0.363 |
| Max | 33.6 | 17.1 | 33.6 | 14.5 | 33.6 | 2.69 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.62 | 2.22 | 1.62 | 2.36 | 1.62 | 2.26 |
| Average | 2.47 | 3.81 | 2.47 | 3.82 | 2.47 | 3.39 |
| Stdev | 2.58 | 4.85 | 2.58 | 5.27 | 2.58 | 2.98 |
| p(t-test) | | 0.0067 | | 0.0083 | | 0.11 |
| Min | 0.183 | 0.622 | 0.183 | 0.562 | 0.183 | 0.591 |
| Max | 20.8 | 30.1 | 20.8 | 33.6 | 20.8 | 12.5 |
| n (Samp) | 213 | 51 | 213 | 53 | 213 | 23 |
| n (Patient) | 89 | 51 | 89 | 53 | 89 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.48 | 0.63 | 0.63 | 0.54 | 0.62 | 0.60 | 0.43 | 0.62 |
| SE | 0.045 | 0.070 | 0.046 | 0.043 | 0.066 | 0.045 | 0.062 | 0.084 | 0.065 |
| p | 0.026 | 0.73 | 0.0052 | 0.0029 | 0.51 | 0.0056 | 0.12 | 0.44 | 0.073 |
| nCohort 1 | 260 | 466 | 213 | 260 | 466 | 213 | 260 | 466 | 213 |
| nCohort 2 | 51 | 18 | 51 | 56 | 21 | 53 | 25 | 13 | 23 |
| Cutoff 1 | 1.35 | 1.17 | 1.52 | 1.70 | 1.22 | 1.77 | 1.43 | 1.05 | 1.57 |
| Sens 1 | 71% | 72% | 71% | 71% | 71% | 72% | 72% | 77% | 74% |
| Spec 1 | 40% | 31% | 47% | 53% | 32% | 58% | 43% | 26% | 49% |
| Cutoff 2 | 1.10 | 0.591 | 1.11 | 1.02 | 1.01 | 1.02 | 0.855 | 0.971 | 0.855 |
| Sens 2 | 80% | 83% | 80% | 80% | 81% | 81% | 80% | 85% | 83% |
| Spec 2 | 32% | 8% | 32% | 30% | 25% | 30% | 23% | 23% | 22% |
| Cutoff 3 | 0.893 | 0.448 | 0.919 | 0.787 | 0.731 | 0.787 | 0.679 | 0.417 | 0.699 |
| Sens 3 | 90% | 94% | 90% | 91% | 90% | 91% | 92% | 92% | 91% |
| Spec 3 | 25% | 4% | 23% | 20% | 13% | 18% | 16% | 3% | 15% |
| Cutoff 4 | 2.42 | 2.71 | 2.52 | 2.42 | 2.71 | 2.52 | 2.42 | 2.71 | 2.52 |
| Sens 4 | 39% | 28% | 45% | 45% | 43% | 42% | 48% | 0% | 48% |
| Spec 4 | 70% | 70% | 71% | 70% | 70% | 71% | 70% | 70% | 71% |
| Cutoff 5 | 3.34 | 3.88 | 3.74 | 3.34 | 3.88 | 3.74 | 3.34 | 3.88 | 3.74 |
| Sens 5 | 31% | 22% | 31% | 32% | 29% | 28% | 32% | 0% | 30% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | 80% |
| Cutoff 6 | 4.93 | 5.28 | 5.88 | 4.93 | 5.28 | 5.88 | 4.93 | 5.28 | 5.88 |
| Sens 6 | 20% | 17% | 12% | 18% | 14% | 11% | 12% | 0% | 17% |
| Spec 6 | 90% | 90% | 90% | 90% | 90% | 90% | 90% | 90% | 90% |
| OR Quart 2 | 2.0 | 0.59 | 1.1 | 0.67 | 0.99 | 0.45 | 0.48 | >7.4 | 0.79 |
| p Value | 0.17 | 0.48 | 0.80 | 0.44 | 0.99 | 0.17 | 0.31 | <0.063 | 0.73 |
| 95% CI of OR Quart2 | 0.75 5.3 | 0.14 2.5 | 0.41 3.2 | 0.24 1.9 | 0.28 3.5 | 0.15 1.4 | 0.11 2.0 | >0.90 na | 0.20 3.1 |
| OR Quart 3 | 2.2 | 1.0 | 2.5 | 2.3 | 0.58 | 2.4 | 1.2 | >3.1 | 1.0 |
| p Value | 0.11 | 1.0 | 0.050 | 0.046 | 0.47 | 0.041 | 0.77 | <0.33 | 1.0 |
| 95% CI of OR Quart3 | 0.83 5.8 | 0.28 3.5 | 1.0 6.3 | 1.0 5.4 | 0.14 2.5 | 1.0 5.7 | 0.38 3.7 | >0.32 na | 0.27 3.7 |
| OR Quart 4 | 2.8 | 1.0 | 2.5 | 2.2 | 1.6 | 2.1 | 1.5 | >3.1 | 1.9 |
| p Value | 0.033 | 1.0 | 0.050 | 0.068 | 0.40 | 0.10 | 0.43 | <0.33 | 0.26 |
| 95% CI of OR Quart4 | 1.1 7.2 | 0.28 3.5 | 1.0 6.3 | 0.94 5.1 | 0.52 5.1 | 0.87 4.9 | 0.52 4.6 | >0.32 na | 0.61 6.2 |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26.8 | 36.2 | 26.8 | 41.7 | 26.8 | 54.3 |
| Average | 66.0 | 106 | 66.0 | 168 | 66.0 | 113 |
| Stdev | 145 | 166 | 145 | 301 | 145 | 194 |
| p(t-test) | | 0.078 | | 1.8E-4 | | 0.13 |
| Min | 4.60 | 6.86 | 4.60 | 4.40 | 4.60 | 7.57 |
| Max | 1720 | 737 | 1720 | 1470 | 1720 | 988 |
| n (Samp) | 260 | 51 | 260 | 56 | 260 | 25 |
| n (Patient) | 110 | 51 | 110 | 56 | 110 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.0 | 62.6 | 33.0 | 64.7 | 33.0 | 69.7 |
| Average | 83.7 | 266 | 83.7 | 272 | 83.7 | 94.9 |
| Stdev | 191 | 465 | 191 | 433 | 191 | 93.0 |
| p(t-test) | | 2.8E-4 | | 5.2E-5 | | 0.83 |
| Min | 3.68 | 8.01 | 3.68 | 4.40 | 3.68 | 7.57 |
| Max | 2130 | 1880 | 2130 | 1470 | 2130 | 308 |
| n (Samp) | 466 | 18 | 466 | 21 | 466 | 13 |
| n (Patient) | 180 | 18 | 180 | 21 | 180 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 27.4 | 37.3 | 27.4 | 41.0 | 27.4 | 62.1 |
| Average | 72.4 | 102 | 72.4 | 147 | 72.4 | 165 |
| Stdev | 153 | 158 | 153 | 260 | 153 | 320 |
| p(t-test) | | 0.22 | | 0.0071 | | 0.017 |
| Min | 4.60 | 6.86 | 4.60 | 8.81 | 4.60 | 8.40 |
| Max | 1720 | 737 | 1720 | 1410 | 1720 | 1310 |
| n (Samp) | 213 | 51 | 213 | 53 | 213 | 23 |
| n (Patient) | 89 | 51 | 89 | 53 | 89 | 23 |

| | 0hr prior to AKI stage | 24hr prior to AKI stage | 48hr prior to AKI stage |
|---|---|---|---|
| | | | |

| | sCr or UO | sCr only | UO only | sCr or UO | sCr only | UO only | sCr or UO | sCr only | UO only |
|---|---|---|---|---|---|---|---|---|---|
| AUC | 0.60 | 0.65 | 0.59 | 0.65 | 0.61 | 0.64 | 0.63 | 0.62 | 0.63 |
| SE | 0.045 | 0.072 | 0.046 | 0.043 | 0.067 | 0.045 | 0.062 | 0.084 | 0.065 |
| p | 0.034 | 0.041 | 0.039 | 7.0E-4 | 0.086 | 0.0019 | 0.036 | 0.15 | 0.051 |
| nCohort 1 | 260 | 466 | 213 | 260 | 466 | 213 | 260 | 466 | 213 |
| nCohort 2 | 51 | 18 | 51 | 56 | 21 | 53 | 25 | 13 | 23 |
| Cutoff 1 | 21.3 | 26.6 | 20.9 | 27.5 | 24.5 | 27.5 | 20.6 | 25.6 | 20.4 |
| Sens 1 | 71% | 72% | 71% | 71% | 71% | 72% | 72% | 77% | 74% |
| Spec 1 | 40% | 43% | 40% | 52% | 38% | 50% | 39% | 41% | 40% |
| Cutoff 2 | 18.6 | 25.4 | 18.6 | 18.6 | 19.2 | 18.9 | 17.4 | 17.4 | 16.3 |
| Sens 2 | 80% | 83% | 80% | 80% | 81% | 81% | 80% | 85% | 83% |
| Spec 2 | 33% | 41% | 34% | 33% | 31% | 35% | 30% | 25% | 29% |
| Cutoff 3 | 12.5 | 8.56 | 14.8 | 12.3 | 12.9 | 13.2 | 9.64 | 8.39 | 10.6 |
| Sens 3 | 90% | 94% | 90% | 91% | 90% | 91% | 92% | 92% | 91% |
| Spec 3 | 18% | 8% | 26% | 18% | 17% | 21% | 13% | 8% | 15% |
| Cutoff 4 | 50.2 | 60.4 | 56.0 | 50.2 | 60.4 | 56.0 | 50.2 | 60.4 | 56.0 |
| Sens 4 | 43% | 50% | 41% | 46% | 52% | 45% | 52% | 54% | 52% |
| Spec 4 | 70% | 70% | 70% | 70% | 70% | 70% | 70% | 70% | 70% |
| Cutoff 5 | 74.3 | 93.4 | 84.8 | 74.3 | 93.4 | 84.8 | 74.3 | 93.4 | 84.8 |
| Sens 5 | 35% | 39% | 35% | 43% | 38% | 42% | 44% | 31% | 43% |
| Spec 5 | 80% | 80% | 80% | 80% | 80% | 80% | 80% | 80% | 80% |
| Cutoff 6 | 138 | 171 | 174 | 138 | 171 | 174 | 138 | 171 | 174 |
| Sens 6 | 22% | 33% | 16% | 29% | 29% | 26% | 20% | 15% | 13% |
| Spec 6 | 90% | 90% | 90% | 90% | 90% | 90% | 90% | 90% | 90% |
| OR Quart 2 | 1.6 | 2.6 | 2.9 | 0.88 | 0.99 | 2.0 | 1.0 | 0.99 | 0.74 |
| p Value | 0.36 | 0.27 | 0.038 | 0.80 | 0.99 | 0.16 | 1.0 | 0.99 | 0.70 |
| 95% CI of | 0.60 | 0.49 | 1.1 | 0.32 | 0.24 | 0.75 | 0.24 | 0.14 | 0.16 |
| OR Quart2 | 4.1 | 13 | 8.1 | 2.4 | 4.1 | 5.5 | 4.2 | 7.2 | 3.4 |
| OR Quart 3 | 1.7 | 1.5 | 2.0 | 1.8 | 0.99 | 1.7 | 1.5 | 1.5 | 1.3 |
| p Value | 0.26 | 0.65 | 0.20 | 0.19 | 0.99 | 0.31 | 0.51 | 0.66 | 0.73 |
| 95% CI of | 0.67 | 0.25 | 0.69 | 0.75 | 0.24 | 0.61 | 0.42 | 0.25 | 0.32 |
| OR Quart3 | 4.4 | 9.2 | 5.8 | 4.5 | 4.1 | 4.7 | 5.7 | 9.1 | 5.0 |
| OR Quart 4 | 2.6 | 4.2 | 4.0 | 3.4 | 2.3 | 4.1 | 3.0 | 3.1 | 3.2 |
| p Value | 0.039 | 0.073 | 0.0059 | 0.0045 | 0.17 | 0.0030 | 0.070 | 0.17 | 0.063 |
| 95% CI of | 1.1 | 0.88 | 1.5 | 1.5 | 0.70 | 1.6 | 0.91 | 0.61 | 0.94 |
| OR Quart4 | 6.4 | 20 | 11 | 7.9 | 7.8 | 10 | 10.0 | 16 | 11 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.11 | 1.51 | 1.11 | 2.02 | 1.11 | 1.59 |
| Average | 3.53 | 10.4 | 3.53 | 13.9 | 3.53 | 3.72 |
| Stdev | 14.4 | 41.9 | 14.4 | 46.3 | 14.4 | 6.27 |
| p(t-test) | | 0.19 | | 0.063 | | 0.95 |
| Min | 0.117 | 0.204 | 0.117 | 0.122 | 0.117 | 0.122 |
| Max | 131 | 253 | 131 | 248 | 131 | 31.2 |
| n (Samp) | 82 | 36 | 82 | 45 | 82 | 26 |
| n (Patient) | 75 | 36 | 75 | 45 | 75 | 26 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.41 | 1.27 | 1.41 | 1.83 | 1.41 | 1.24 |
| Average | 8.87 | 1.81 | 8.87 | 5.69 | 8.87 | 1.52 |
| Stdev | 35.3 | 1.57 | 35.3 | 9.55 | 35.3 | 1.06 |
| p(t-test) | | 0.49 | | 0.74 | | 0.53 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.117 | 0.481 | 0.117 | 0.631 | 0.117 | 0.631 |
| Max | 263 | 5.56 | 263 | 37.0 | 263 | 4.16 |
| n (Samp) | 197 | 12 | 197 | 14 | 197 | 9 |
| n (Patient) | 131 | 12 | 131 | 14 | 131 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.24 | 1.60 | 1.24 | 2.52 | 1.24 | 2.44 |
| Average | 3.82 | 12.7 | 3.82 | 14.8 | 3.82 | 4.48 |
| Stdev | 15.1 | 46.6 | 15.1 | 47.3 | 15.1 | 6.81 |
| p(t-test) | | 0.14 | | 0.067 | | 0.84 |
| Min | 0.122 | 0.204 | 0.122 | 0.122 | 0.122 | 0.122 |
| Max | 131 | 253 | 131 | 248 | 131 | 31.2 |
| n (Samp) | 75 | 29 | 75 | 43 | 75 | 21 |
| n (Patient) | 62 | 29 | 62 | 43 | 62 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.47 | 0.61 | 0.65 | 0.61 | 0.64 | 0.62 | 0.45 | 0.66 |
| SE | 0.058 | 0.087 | 0.064 | 0.052 | 0.082 | 0.054 | 0.066 | 0.10 | 0.071 |
| p | 0.036 | 0.72 | 0.084 | 0.0055 | 0.18 | 0.0073 | 0.068 | 0.64 | 0.024 |
| nCohort 1 | 82 | 197 | 75 | 82 | 197 | 75 | 82 | 197 | 75 |
| nCohort 2 | 36 | 12 | 29 | 45 | 14 | 43 | 26 | 9 | 21 |
| Cutoff 1 | 1.06 | 1.09 | 0.999 | 1.06 | 1.33 | 1.12 | 1.06 | 1.01 | 1.25 |
| Sens 1 | 72% | 75% | 72% | 71% | 71% | 72% | 73% | 78% | 71% |
| Spec 1 | 50% | 41% | 44% | 50% | 47% | 47% | 50% | 38% | 52% |
| Cutoff 2 | 0.803 | 0.821 | 0.761 | 0.821 | 1.02 | 0.803 | 0.999 | 0.754 | 1.06 |
| Sens 2 | 81% | 83% | 83% | 80% | 86% | 81% | 85% | 89% | 81% |
| Spec 2 | 46% | 36% | 33% | 46% | 38% | 43% | 48% | 29% | 47% |
| Cutoff 3 | 0.464 | 0.481 | 0.379 | 0.334 | 1.01 | 0.334 | 0.630 | 0.630 | 0.999 |
| Sens 3 | 92% | 92% | 93% | 91% | 93% | 91% | 92% | 100% | 90% |
| Spec 3 | 16% | 16% | 11% | 10% | 38% | 8% | 24% | 22% | 44% |
| Cutoff 4 | 2.09 | 3.28 | 1.96 | 2.09 | 3.28 | 1.96 | 2.09 | 3.28 | 1.96 |
| Sens 4 | 39% | 17% | 45% | 49% | 43% | 56% | 38% | 11% | 52% |
| Spec 4 | 72% | 70% | 71% | 72% | 70% | 71% | 72% | 70% | 71% |
| Cutoff 5 | 2.78 | 5.21 | 2.98 | 2.78 | 5.21 | 2.98 | 2.78 | 5.21 | 2.98 |
| Sens 5 | 36% | 8% | 41% | 44% | 29% | 47% | 27% | 0% | 38% |
| Spec 5 | 80% | 80% | 80% | 80% | 80% | 80% | 80% | 80% | 80% |
| Cutoff 6 | 4.88 | 8.80 | 5.21 | 4.88 | 8.80 | 5.21 | 4.88 | 8.80 | 5.21 |
| Sens 6 | 28% | 0% | 31% | 29% | 14% | 28% | 12% | 0% | 14% |
| Spec 6 | 90% | 90% | 91% | 90% | 90% | 91% | 90% | 90% | 91% |
| OR Quart 2 | 2.4 | 1.0 | 1.5 | 2.9 | 5.3 | 2.2 | 3.4 | 1.0 | 2.9 |
| p Value | 0.16 | 0.98 | 0.51 | 0.071 | 0.13 | 0.18 | 0.10 | 0.99 | 0.23 |
| 95% CI of OR Quart2 | 0.70 | 0.14 | 0.42 | 0.91 | 0.60 | 0.69 | 0.78 | 0.062 | 0.50 |
| | 8.2 | 7.5 | 5.7 | 8.9 | 47 | 7.1 | 14 | 17 | 17 |
| OR Quart 3 | 1.8 | 3.3 | 1.0 | 1.2 | 3.1 | 1.7 | 2.3 | 6.7 | 3.7 |
| p Value | 0.35 | 0.15 | 1.0 | 0.80 | 0.34 | 0.37 | 0.28 | 0.085 | 0.14 |
| 95% CI of OR Quart3 | 0.52 | 0.64 | 0.25 | 0.34 | 0.31 | 0.52 | 0.51 | 0.77 | 0.66 |
| | 6.5 | 17 | 4.0 | 4.0 | 30 | 5.7 | 10 | 57 | 20 |
| OR Quart 4 | 3.7 | 1.0 | 3.6 | 6.1 | 5.3 | 5.0 | 4.0 | 1.0 | 5.5 |
| p Value | 0.034 | 0.98 | 0.043 | 0.0018 | 0.13 | 0.0061 | 0.060 | 0.99 | 0.046 |
| 95% CI of OR Quart4 | 1.1 | 0.14 | 1.0 | 2.0 | 0.60 | 1.6 | 0.95 | 0.062 | 1.0 |
| | 12 | 7.5 | 12 | 19 | 47 | 16 | 17 | 17 | 29 |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 0.00240 | 1.00E-9 | 1.00E-9 |
| Average | 0.00715 | 0.0102 | 0.00715 | 0.0133 | 0.00715 | 0.0150 |
| Stdev | 0.0203 | 0.0434 | 0.0203 | 0.0446 | 0.0203 | 0.0622 |
| p(t-test) | | 0.60 | | 0.30 | | 0.34 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 79 | 36 | 79 | 41 | 79 | 23 |
| n (Patient) | 72 | 36 | 72 | 41 | 72 | 23 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 1.00E-9 | 1.00E-9 | 0.00507 | 1.00E-9 | 1.00E-9 |
| Average | 0.00582 | 0.0263 | 0.00582 | 0.0316 | 0.00582 | 0.0334 |
| Stdev | 0.0146 | 0.0783 | 0.0146 | 0.0849 | 0.0146 | 0.0938 |
| p(t-test) | | 0.0040 | | 6.0E-4 | | 7.1E-4 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 187 | 11 | 187 | 11 | 187 | 10 |
| n (Patient) | 127 | 11 | 127 | 11 | 127 | 10 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.00131 | 1.00E-9 | 0.00131 | 0.00202 | 0.00131 | 1.00E-9 |
| Average | 0.00817 | 0.00387 | 0.00817 | 0.0104 | 0.00817 | 0.00218 |
| Stdev | 0.0213 | 0.00693 | 0.0213 | 0.0213 | 0.0213 | 0.00328 |
| p(t-test) | | 0.28 | | 0.60 | | 0.25 |
| Min | 1.00E-9 | 1.00E-9 | 1.00E-9 | 1.00E-9 | 1.00E-9 | 1.00E-9 |
| Max | 0.114 | 0.0300 | 0.114 | 0.108 | 0.114 | 0.00903 |
| n (Samp) | 70 | 30 | 70 | 40 | 70 | 17 |
| n (Patient) | 56 | 30 | 56 | 40 | 56 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.44 | 0.45 | 0.61 | 0.62 | 0.57 | 0.47 | 0.51 | 0.43 |
| SE | 0.059 | 0.092 | 0.064 | 0.055 | 0.093 | 0.058 | 0.069 | 0.094 | 0.080 |
| p | 0.65 | 0.52 | 0.43 | 0.038 | 0.21 | 0.22 | 0.72 | 0.91 | 0.35 |
| nCohort 1 | 79 | 187 | 70 | 79 | 187 | 70 | 79 | 187 | 70 |
| nCohort 2 | 36 | 11 | 30 | 41 | 11 | 40 | 23 | 10 | 17 |
| Cutoff 1 | 0 | 0 | 0 | 0 | 0 | 0 | 0 | 0 | 0 |
| Sens 1 | 100% | 100% | 100% | 100% | 100% | 100% | 100% | 100% | 100% |
| Spec 1 | 0% | 0% | 0% | 0% | 0% | 0% | 0% | 0% | 0% |
| Cutoff 2 | 0 | 0 | 0 | 0 | 0 | 0 | 0 | 0 | 0 |
| Sens 2 | 100% | 100% | 100% | 100% | 100% | 100% | 100% | 100% | 100% |
| Spec 2 | 0% | 0% | 0% | 0% | 0% | 0% | 0% | 0% | 0% |
| Cutoff 3 | 0 | 0 | 0 | 0 | 0 | 0 | 0 | 0 | 0 |
| Sens 3 | 100% | 100% | 100% | 100% | 100% | 100% | 100% | 100% | 100% |
| Spec 3 | 0% | 0% | 0% | 0% | 0% | 0% | 0% | 0% | 0% |
| Cutoff 4 | 0.00274 | 0.00377 | 0.00377 | 0.00274 | 0.00377 | 0.00377 | 0.00274 | 0.00377 | 0.00377 |
| Sens 4 | 33% | 27% | 33% | 49% | 64% | 42% | 30% | 40% | 24% |
| Spec 4 | 71% | 71% | 71% | 71% | 71% | 71% | 71% | 71% | 71% |
| Cutoff 5 | 0.00507 | 0.00819 | 0.00813 | 0.00507 | 0.00819 | 0.00813 | 0.00507 | 0.00819 | 0.00813 |
| Sens 5 | 28% | 27% | 17% | 37% | 27% | 35% | 26% | 30% | 12% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 5 | 81% | 82% | 80% | 81% | 82% | 80% | 81% | 82% | 80% |
| Cutoff 6 | 0.0166 | 0.0136 | 0.0165 | 0.0166 | 0.0136 | 0.0165 | 0.0166 | 0.0136 | 0.0165 |
| Sens 6 | 6% | 18% | 7% | 17% | 18% | 18% | 4% | 20% | 0% |
| Spec 6 | 91% | 90% | 90% | 91% | 90% | 90% | 91% | 90% | 90% |
| OR Quart 2 | 0.12 | 0 | 0.24 | 6.0 | 0 | 1.1 | 1.1 | 0.48 | 0.71 |
| p Value | 0.011 | na | 0.057 | 0.012 | na | 0.84 | 0.94 | 0.41 | 0.68 |
| 95% CI of | 0.024 | na | 0.056 | 1.5 | na | 0.36 | 0.29 | 0.084 | 0.14 |
| OR Quart2 | 0.61 | na | 1.0 | 24 | na | 3.5 | 3.8 | 2.7 | 3.6 |
| OR Quart 3 | 2.0 | 1.4 | 1.2 | 5.2 | 1.0 | 1.2 | 2.4 | 0 | 1.7 |
| p Value | 0.19 | 0.70 | 0.77 | 0.021 | 1.0 | 0.77 | 0.14 | na | 0.47 |
| 95% CI of | 0.71 | 0.29 | 0.38 | 1.3 | 0.24 | 0.38 | 0.74 | na | 0.40 |
| OR Quart3 | 5.7 | 6.4 | 3.7 | 21 | 4.2 | 3.8 | 8.1 | na | 7.1 |
| OR Quart 4 | 0.55 | 1.4 | 0.84 | 9.0 | 0.72 | 2.4 | 0 | 0.98 | 1.1 |
| p Value | 0.30 | 0.68 | 0.77 | 0.0020 | 0.68 | 0.13 | na | 0.98 | 0.94 |
| 95% CI of | 0.17 | 0.30 | 0.26 | 2.2 | 0.15 | 0.78 | na | 0.23 | 0.23 |
| OR Quart4 | 1.7 | 6.6 | 2.7 | 36 | 3.4 | 7.2 | na | 4.2 | 4.9 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Apolipoprotein A-II**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 54100 | 49700 | 54100 | 45500 | 54100 | 43300 |
| Average | 55400 | 59300 | 55400 | 51900 | 55400 | 46600 |
| Stdev | 29200 | 19900 | 29200 | 32000 | 29200 | 33800 |
| p(t-test) | | 0.57 | | 0.57 | | 0.26 |
| Min | 5450 | 32800 | 5450 | 10700 | 5450 | 1810 |
| Max | 253000 | 97000 | 253000 | 176000 | 253000 | 152000 |
| n (Samp) | 230 | 19 | 230 | 26 | 230 | 15 |
| n (Patient) | 158 | 19 | 158 | 26 | 158 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52600 | 51800 | 52600 | 45500 | 52600 | 43200 |
| Average | 54900 | 58300 | 54900 | 51000 | 54900 | 47000 |
| Stdev | 29800 | 21200 | 29800 | 31800 | 29800 | 35000 |
| p(t-test) | | 0.62 | | 0.54 | | 0.35 |
| Min | 7680 | 23300 | 7680 | 10700 | 7680 | 1810 |
| Max | 253000 | 97000 | 253000 | 176000 | 253000 | 152000 |
| n (Samp) | 201 | 19 | 201 | 26 | 201 | 14 |
| n (Patient) | 133 | 19 | 133 | 26 | 133 | 14 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | nd | 0.56 | 0.44 | nd | 0.43 | 0.36 | nd | 0.37 |
| SE | 0.071 | nd | 0.071 | 0.061 | nd | 0.062 | 0.079 | nd | 0.082 |
| p | 0.38 | nd | 0.40 | 0.30 | nd | 0.24 | 0.069 | nd | 0.11 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 1 | 230 | nd | 201 | 230 | nd | 201 | 230 | nd | 201 |
| nCohort 2 | 19 | nd | 19 | 26 | nd | 26 | 15 | nd | 14 |
| Cutoff 1 | 43600 | nd | 43600 | 35400 | nd | 35000 | 36000 | nd | 36000 |
| Sens 1 | 74% | nd | 74% | 73% | nd | 73% | 73% | nd | 71% |
| Spec 1 | 35% | nd | 36% | 21% | nd | 21% | 21% | nd | 21% |
| Cutoff 2 | 43000 | nd | 40500 | 30600 | nd | 30600 | 35900 | nd | 31700 |
| Sens 2 | 84% | nd | 84% | 81% | nd | 81% | 80% | nd | 86% |
| Spec 2 | 34% | nd | 30% | 17% | nd | 18% | 21% | nd | 19% |
| Cutoff 3 | 40200 | nd | 32500 | 28300 | nd | 26600 | 7970 | nd | 7970 |
| Sens 3 | 95% | nd | 95% | 92% | nd | 92% | 93% | nd | 93% |
| Spec 3 | 30% | nd | 20% | 16% | nd | 14% | 1% | nd | 1% |
| Cutoff 4 | 64400 | nd | 64800 | 64400 | nd | 64800 | 64400 | nd | 64800 |
| Sens 4 | 42% | nd | 42% | 19% | nd | 15% | 13% | nd | 14% |
| Spec 4 | 70% | nd | 70% | 70% | nd | 70% | 70% | nd | 70% |
| Cutoff 5 | 72600 | nd | 72300 | 72600 | nd | 72300 | 72600 | nd | 72300 |
| Sens 5 | 26% | nd | 26% | 12% | nd | 12% | 13% | nd | 14% |
| Spec 5 | 80% | nd | 80% | 80% | nd | 80% | 80% | nd | 80% |
| Cutoff 6 | 87100 | nd | 83600 | 87100 | nd | 83600 | 87100 | nd | 83600 |
| Sens 6 | 16% | nd | 16% | 8% | nd | 8% | 7% | nd | 7% |
| Spec 6 | 90% | nd | 90% | 90% | nd | 90% | 90% | nd | 90% |
| OR Quart 2 | 12 | nd | 5.2 | 1.2 | nd | 1.9 | 0 | nd | 0 |
| p Value | 0.021 | nd | 0.041 | 0.75 | nd | 0.35 | na | nd | na |
| 95% CI of OR Quart2 | 1.5 95 | nd | 1.1 25 | 0.35 4.2 | nd | 0.51 6.7 | na na | nd | na na |
| OR Quart 3 | 1.0 | nd | 0.49 | 1.2 | nd | 1.6 | 3.9 | nd | 3.9 |
| p Value | 1.0 | nd | 0.57 | 0.75 | nd | 0.51 | 0.099 | nd | 0.10 |
| 95% CI of OR Quart3 | 0.061 16 | nd | 0.043 5.6 | 0.35 4.2 | nd | 0.42 5.8 | 0.77 20 | nd | 0.77 20 |
| OR Quart 4 | 7.6 | nd | 3.9 | 1.9 | nd | 2.5 | 3.3 | nd | 2.7 |
| p Value | 0.061 | nd | 0.10 | 0.26 | nd | 0.14 | 0.16 | nd | 0.25 |
| 95% CI of OR Quart4 | 0.91 64 | nd | 0.77 20 | 0.61 6.1 | nd | 0.73 8.8 | 0.63 17 | nd | 0.50 15 |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 70.5 | 58.2 | nd | nd | nd | nd |
| Average | 90.5 | 69.4 | nd | nd | nd | nd |
| Stdev | 59.1 | 45.7 | nd | nd | nd | nd |
| p(t-test) | | 0.31 | nd | nd | nd | nd |
| Min | 20.6 | 36.3 | nd | nd | nd | nd |
| Max | 326 | 188 | nd | nd | nd | nd |
| n (Samp) | 60 | 9 | nd | nd | nd | nd |
| n (Patient) | 37 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.3 | 58.2 | nd | nd | nd | nd |
| Average | 91.4 | 69.4 | nd | nd | nd | nd |
| Stdev | 54.9 | 45.7 | nd | nd | nd | nd |
| p(t-test) | | 0.27 | nd | nd | nd | nd |
| Min | 33.8 | 36.3 | nd | nd | nd | nd |
| Max | 271 | 188 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 45 | 9 | nd | nd | nd | nd |
| n (Patient) | 26 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | 0.31 | nd | nd | nd | nd | nd | nd |
| SE | 0.10 | nd | 0.10 | nd | nd | nd | nd | nd | nd |
| p | 0.089 | nd | 0.066 | nd | nd | nd | nd | nd | nd |
| nCohort 1 | 60 | nd | 45 | nd | nd | nd | nd | nd | nd |
| nCohort 2 | 9 | nd | 9 | nd | nd | nd | nd | nd | nd |
| Cutoff 1 | 45.8 | nd | 45.8 | nd | nd | nd | nd | nd | nd |
| Sens 1 | 78% | nd | 78% | nd | nd | nd | nd | nd | nd |
| Spec 1 | 15% | nd | 11% | nd | nd | nd | nd | nd | nd |
| Cutoff 2 | 42.1 | nd | 42.0 | nd | nd | nd | nd | nd | nd |
| Sens 2 | 89% | nd | 89% | nd | nd | nd | nd | nd | nd |
| Spec 2 | 13% | nd | 9% | nd | nd | nd | nd | nd | nd |
| Cutoff 3 | 35.1 | nd | 33.8 | nd | nd | nd | nd | nd | nd |
| Sens 3 | 100% | nd | 100% | nd | nd | nd | nd | nd | nd |
| Spec 3 | 5% | nd | 2% | nd | nd | nd | nd | nd | nd |
| Cutoff 4 | 95.6 | nd | 95.9 | nd | nd | nd | nd | nd | nd |
| Sens 4 | 11% | nd | 11% | nd | nd | nd | nd | nd | nd |
| Spec 4 | 70% | nd | 73% | nd | nd | nd | nd | nd | nd |
| Cutoff 5 | 110 | nd | 103 | nd | nd | nd | nd | nd | nd |
| Sens 5 | 11% | nd | 11% | nd | nd | nd | nd | nd | nd |
| Spec 5 | 80% | nd | 80% | nd | nd | nd | nd | nd | nd |
| Cutoff 6 | 147 | nd | 186 | nd | nd | nd | nd | nd | nd |
| Sens 6 | 11% | nd | 11% | nd | nd | nd | nd | nd | nd |
| Spec 6 | 90% | nd | 91% | nd | nd | nd | nd | nd | nd |
| OR Quart 2 | 1.1 | nd | 1.1 | nd | nd | nd | nd | nd | nd |
| p Value | 0.97 | nd | 0.96 | nd | nd | nd | nd | nd | nd |
| 95% CI of OR Quart2 | 0.061 18 | nd | 0.061 19 | nd | nd | nd | nd | nd | nd |
| OR Quart 3 | 5.2 | nd | 5.2 | nd | nd | nd | nd | nd | nd |
| p Value | 0.16 | nd | 0.17 | nd | nd | nd | nd | nd | nd |
| 95% CI of OR Quart3 | 0.52 53 | nd | 0.50 54 | nd | nd | nd | nd | nd | nd |
| OR Quart 4 | 3.6 | nd | 3.9 | nd | nd | nd | nd | nd | nd |
| p Value | 0.29 | nd | 0.27 | nd | nd | nd | nd | nd | nd |
| 95% CI of OR Quart4 | 0.34 39 | nd | 0.35 43 | nd | nd | nd | nd | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 7.75 | nd | nd | nd | nd |
| Average | 42.0 | 23.4 | nd | nd | nd | nd |
| Stdev | 59.4 | 35.2 | nd | nd | nd | nd |
| p(t-test) | | 0.36 | nd | nd | nd | nd |
| Min | 0.400 | 3.79 | nd | nd | nd | nd |
| Max | 366 | 114 | nd | nd | nd | nd |
| n (Samp) | 82 | 9 | nd | nd | nd | nd |
| n (Patient) | 59 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.9 | 7.75 | nd | nd | nd | nd |
| Average | 46.6 | 23.4 | nd | nd | nd | nd |
| Stdev | 65.1 | 35.2 | nd | nd | nd | nd |
| p(t-test) | | 0.30 | nd | nd | nd | nd |
| Min | 0.400 | 3.79 | nd | nd | nd | nd |
| Max | 366 | 114 | nd | nd | nd | nd |
| n (Samp) | 64 | 9 | nd | nd | nd | nd |
| n (Patient) | 46 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.32 | nd | 0.28 | nd | nd | nd | nd | nd | nd |
| SE | 0.10 | nd | 0.10 | nd | nd | nd | nd | nd | nd |
| p | 0.085 | nd | 0.032 | nd | nd | nd | nd | nd | nd |
| nCohort 1 | 82 | nd | 64 | nd | nd | nd | nd | nd | nd |
| nCohort 2 | 9 | nd | 9 | nd | nd | nd | nd | nd | nd |
| Cutoff 1 | 4.69 | nd | 4.69 | nd | nd | nd | nd | nd | nd |
| Sens 1 | 78% | nd | 78% | nd | nd | nd | nd | nd | nd |
| Spec 1 | 9% | nd | 3% | nd | nd | nd | nd | nd | nd |
| Cutoff 2 | 4.59 | nd | 4.59 | nd | nd | nd | nd | nd | nd |
| Sens 2 | 89% | nd | 89% | nd | nd | nd | nd | nd | nd |
| Spec 2 | 9% | nd | 3% | nd | nd | nd | nd | nd | nd |
| Cutoff 3 | 3.65 | nd | 0.400 | nd | nd | nd | nd | nd | nd |
| Sens 3 | 100% | nd | 100% | nd | nd | nd | nd | nd | nd |
| Spec 3 | 6% | nd | 2% | nd | nd | nd | nd | nd | nd |
| Cutoff 4 | 43.6 | nd | 45.3 | nd | nd | nd | nd | nd | nd |
| Sens 4 | 11% | nd | 11% | nd | nd | nd | nd | nd | nd |
| Spec 4 | 71% | nd | 70% | nd | nd | nd | nd | nd | nd |
| Cutoff 5 | 52.2 | nd | 54.5 | nd | nd | nd | nd | nd | nd |
| Sens 5 | 11% | nd | 11% | nd | nd | nd | nd | nd | nd |
| Spec 5 | 80% | nd | 81% | nd | nd | nd | nd | nd | nd |
| Cutoff 6 | 87.8 | nd | 106 | nd | nd | nd | nd | nd | nd |
| Sens 6 | 11% | nd | 11% | nd | nd | nd | nd | nd | nd |
| Spec 6 | 90% | nd | 91% | nd | nd | nd | nd | nd | nd |
| OR Quart 2 | 2.1 | nd | 2.2 | nd | nd | nd | nd | nd | nd |
| p Value | 0.56 | nd | 0.52 | nd | nd | nd | nd | nd | nd |
| 95% CI of OR Quart2 | 0.18 25 | nd | 0.19 27 | nd | nd | nd | nd | nd | nd |
| OR Quart 3 | 1.0 | nd | 1.1 | nd | nd | nd | nd | nd | nd |
| p Value | 1.0 | nd | 0.97 | nd | nd | nd | nd | nd | nd |
| 95% CI of OR Quart3 | 0.059 17 | nd | 0.061 18 | nd | nd | nd | nd | nd | nd |
| OR Quart 4 | 6.5 | nd | 6.9 | nd | nd | nd | nd | nd | nd |
| p Value | 0.10 | nd | 0.094 | nd | nd | nd | nd | nd | nd |
| 95% CI of OR Quart4 | 0.69 61 | nd | 0.72 67 | nd | nd | nd | nd | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 77700 | 123000 | 77700 | 103000 | 77700 | 117000 |
| Average | 113000 | 130000 | 113000 | 122000 | 113000 | 146000 |
| Stdev | 97300 | 57100 | 97300 | 77800 | 97300 | 81700 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.64 | | 0.70 | | 0.26 |
| Min | 14500 | 51600 | 14500 | 3320 | 14500 | 50500 |
| Max | 621000 | 231000 | 621000 | 340000 | 621000 | 285000 |
| n (Samp) | 123 | 7 | 123 | 20 | 123 | 12 |
| n (Patient) | 97 | 7 | 97 | 20 | 97 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 86600 | 95800 | 86600 | 105000 |
| Average | nd | nd | 123000 | 105000 | 123000 | 128000 |
| Stdev | nd | nd | 101000 | 52100 | 101000 | 80100 |
| p(t-test) | nd | nd | | 0.44 | | 0.88 |
| Min | nd | nd | 25800 | 3320 | 25800 | 50500 |
| Max | nd | nd | 621000 | 209000 | 621000 | 277000 |
| n (Samp) | nd | nd | 104 | 20 | 104 | 10 |
| n (Patient) | nd | nd | 81 | 20 | 81 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | nd | nd | 0.60 | nd | 0.54 | 0.69 | nd | 0.57 |
| SE | 0.12 | nd | nd | 0.071 | nd | 0.072 | 0.088 | nd | 0.098 |
| p | 0.14 | nd | nd | 0.16 | nd | 0.54 | 0.031 | nd | 0.47 |
| nCohort 1 | 123 | nd | nd | 123 | nd | 104 | 123 | nd | 104 |
| nCohort 2 | 7 | nd | nd | 20 | nd | 20 | 12 | nd | 10 |
| Cutoff 1 | 108000 | nd | nd | 81200 | nd | 85700 | 100000 | nd | 79100 |
| Sens 1 | 71% | nd | nd | 70% | nd | 70% | 75% | nd | 70% |
| Spec 1 | 70% | nd | nd | 53% | nd | 50% | 67% | nd | 45% |
| Cutoff 2 | 91400 | nd | nd | 69300 | nd | 70100 | 79100 | nd | 75400 |
| Sens 2 | 86% | nd | nd | 80% | nd | 80% | 83% | nd | 80% |
| Spec 2 | 63% | nd | nd | 45% | nd | 38% | 52% | nd | 40% |
| Cutoff 3 | 51000 | nd | nd | 54100 | nd | 63600 | 75400 | nd | 54100 |
| Sens 3 | 100% | nd | nd | 90% | nd | 90% | 92% | nd | 90% |
| Spec 3 | 20% | nd | nd | 24% | nd | 33% | 48% | nd | 18% |
| Cutoff 4 | 110000 | nd | nd | 110000 | nd | 114000 | 110000 | nd | 114000 |
| Sens 4 | 57% | nd | nd | 40% | nd | 30% | 50% | nd | 40% |
| Spec 4 | 71% | nd | nd | 71% | nd | 70% | 71% | nd | 70% |
| Cutoff 5 | 165000 | nd | nd | 165000 | nd | 184000 | 165000 | nd | 184000 |
| Sens 5 | 29% | nd | nd | 25% | nd | 10% | 25% | nd | 20% |
| Spec 5 | 80% | nd | nd | 80% | nd | 81% | 80% | nd | 81% |
| Cutoff 6 | 248000 | nd | nd | 248000 | nd | 257000 | 248000 | nd | 257000 |
| Sens 6 | 0% | nd | nd | 5% | nd | 0% | 25% | nd | 20% |
| Spec 6 | 90% | nd | nd | 90% | nd | 90% | 90% | nd | 90% |
| OR Quart 2 | 0 | nd | nd | 0.97 | nd | 3.5 | 2.0 | nd | 0.96 |
| p Value | na | nd | nd | 0.97 | nd | 0.15 | 0.58 | nd | 0.97 |
| 95% CI of OR Quart2 | na | nd | nd | 0.18 | nd | 0.64 | 0.17 | nd | 0.13 |
| | na | nd | nd | 5.2 | nd | 19 | 23 | nd | 7.4 |
| OR Quart 3 | 4.4 | nd | nd | 3.0 | nd | 5.0 | 6.9 | nd | 2.2 |
| p Value | 0.19 | nd | nd | 0.12 | nd | 0.054 | 0.083 | nd | 0.40 |
| 95% CI of OR Quart3 | 0.47 | nd | nd | 0.74 | nd | 0.98 | 0.78 | nd | 0.36 |
| | 42 | nd | nd | 13 | nd | 26 | 60 | nd | 13 |
| OR Quart 4 | 2.0 | nd | nd | 2.1 | nd | 2.1 | 3.1 | nd | 0.96 |
| p Value | 0.58 | nd | nd | 0.31 | nd | 0.40 | 0.34 | nd | 0.97 |
| 95% CI of OR Quart4 | 0.17 | nd | nd | 0.49 | nd | 0.36 | 0.31 | nd | 0.13 |
| | 23 | nd | nd | 9.3 | nd | 13 | 31 | nd | 7.4 |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 178 | 415 | nd | nd | nd | nd |
| Average | 989 | 2040 | nd | nd | nd | nd |
| Stdev | 1850 | 2760 | nd | nd | nd | nd |
| p(t-test) | | 0.13 | nd | nd | nd | nd |
| Min | 0.116 | 55.2 | nd | nd | nd | nd |
| Max | 6840 | 6400 | nd | nd | nd | nd |
| n (Samp) | 80 | 9 | nd | nd | nd | nd |
| n (Patient) | 58 | 9 | nd | nd | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 182 | 415 | nd | nd | nd | nd |
| Average | 1090 | 2040 | nd | nd | nd | nd |
| Stdev | 2000 | 2760 | nd | nd | nd | nd |
| p(t-test) | | 0.21 | nd | nd | nd | nd |
| Min | 0.116 | 55.2 | nd | nd | nd | nd |
| Max | 6840 | 6400 | nd | nd | nd | nd |
| n (Samp) | 62 | 9 | nd | nd | nd | nd |
| n (Patient) | 45 | 9 | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.56 | nd | nd | nd | nd | nd | nd |
| SE | 0.10 | nd | 0.11 | nd | nd | nd | nd | nd | nd |
| p | 0.44 | nd | 0.55 | nd | nd | nd | nd | nd | nd |
| nCohort 1 | 80 | nd | 62 | nd | nd | nd | nd | nd | nd |
| nCohort 2 | 9 | nd | 9 | nd | nd | nd | nd | nd | nd |
| Cutoff 1 | 82.7 | nd | 82.7 | nd | nd | nd | nd | nd | nd |
| Sens 1 | 78% | nd | 78% | nd | nd | nd | nd | nd | nd |
| Spec 1 | 20% | nd | 19% | nd | nd | nd | nd | nd | nd |
| Cutoff 2 | 61.7 | nd | 55.2 | nd | nd | nd | nd | nd | nd |
| Sens 2 | 89% | nd | 89% | nd | nd | nd | nd | nd | nd |
| Spec 2 | 18% | nd | 16% | nd | nd | nd | nd | nd | nd |
| Cutoff 3 | 53.6 | nd | 52.0 | nd | nd | nd | nd | nd | nd |
| Sens 3 | 100% | nd | 100% | nd | nd | nd | nd | nd | nd |
| Spec 3 | 15% | nd | 15% | nd | nd | nd | nd | nd | nd |
| Cutoff 4 | 429 | nd | 470 | nd | nd | nd | nd | nd | nd |
| Sens 4 | 44% | nd | 44% | nd | nd | nd | nd | nd | nd |
| Spec 4 | 70% | nd | 71% | nd | nd | nd | nd | nd | nd |
| Cutoff 5 | 1300 | nd | 1410 | nd | nd | nd | nd | nd | nd |
| Sens 5 | 33% | nd | 33% | nd | nd | nd | nd | nd | nd |
| Spec 5 | 80% | nd | 81% | nd | nd | nd | nd | nd | nd |
| Cutoff 6 | 3790 | nd | 5160 | nd | nd | nd | nd | nd | nd |
| Sens 6 | 33% | nd | 22% | nd | nd | nd | nd | nd | nd |
| Spec 6 | 90% | nd | 90% | nd | nd | nd | nd | nd | nd |
| OR Quart 2 | 0.30 | nd | 0.27 | nd | nd | nd | nd | nd | nd |
| p Value | 0.32 | nd | 0.29 | nd | nd | nd | nd | nd | nd |
| 95% CI of | 0.029 | nd | 0.026 | nd | nd | nd | nd | nd | nd |
| OR Quart2 | 3.2 | nd | 2.9 | nd | nd | nd | nd | nd | nd |
| OR Quart 3 | 0.30 | nd | 0.58 | nd | nd | nd | nd | nd | nd |
| p Value | 0.32 | nd | 0.58 | nd | nd | nd | nd | nd | nd |
| 95% CI of | 0.029 | nd | 0.085 | nd | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart3 | 3.2 | nd | 4.0 | nd | nd | nd | nd | nd | nd |
| OR Quart 4 | 1.3 | nd | 0.93 | nd | nd | nd | nd | nd | nd |
| p Value | 0.73 | nd | 0.94 | nd | nd | nd | nd | nd | nd |
| 95% CI of | 0.26 | nd | 0.16 | nd | nd | nd | nd | nd | nd |
| OR Quart4 | 6.8 | nd | 5.4 | nd | nd | nd | nd | nd | nd |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.71 | 2.13 | 1.71 | 2.32 | 1.71 | 2.36 |
| Average | 2.73 | 2.72 | 2.73 | 2.84 | 2.73 | 2.41 |
| Stdev | 3.35 | 2.76 | 3.35 | 2.87 | 3.35 | 1.73 |
| p(t-test) | | 0.99 | | 0.86 | | 0.69 |
| Min | 0.183 | 0.324 | 0.183 | 0.245 | 0.183 | 0.355 |
| Max | 33.6 | 14.1 | 33.6 | 16.6 | 33.6 | 7.86 |
| n (Samp) | 434 | 27 | 434 | 34 | 434 | 17 |
| n (Patient) | 173 | 27 | 173 | 34 | 173 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.74 | 2.90 | 1.74 | 3.41 | 1.74 | 2.55 |
| Average | 2.76 | 4.92 | 2.76 | 2.99 | 2.76 | 3.08 |
| Stdev | 3.28 | 6.27 | 3.28 | 1.97 | 3.28 | 1.69 |
| p(t-test) | | 0.11 | | 0.84 | | 0.80 |
| Min | 0.183 | 0.324 | 0.183 | 0.245 | 0.183 | 0.355 |
| Max | 33.6 | 17.1 | 33.6 | 5.37 | 33.6 | 5.44 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.77 | 2.36 | 1.77 | 2.32 | 1.77 | 1.93 |
| Average | 2.89 | 2.85 | 2.89 | 2.92 | 2.89 | 2.26 |
| Stdev | 3.55 | 2.71 | 3.55 | 2.91 | 3.55 | 1.79 |
| p(t-test) | | 0.95 | | 0.97 | | 0.47 |
| Min | 0.183 | 0.491 | 0.183 | 0.710 | 0.183 | 0.324 |
| Max | 33.6 | 14.1 | 33.6 | 16.6 | 33.6 | 7.86 |
| n (Samp) | 359 | 27 | 359 | 32 | 359 | 17 |
| n (Patient) | 139 | 27 | 139 | 32 | 139 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.55 | 0.55 | 0.58 | 0.55 | 0.55 | 0.65 | 0.48 |
| SE | 0.058 | 0.12 | 0.059 | 0.053 | 0.10 | 0.054 | 0.073 | 0.11 | 0.072 |
| p | 0.56 | 0.57 | 0.43 | 0.35 | 0.42 | 0.37 | 0.54 | 0.20 | 0.81 |
| nCohort 1 | 434 | 535 | 359 | 434 | 535 | 359 | 434 | 535 | 359 |
| nCohort 2 | 27 | 6 | 27 | 34 | 9 | 32 | 17 | 7 | 17 |
| Cutoff 1 | 1.26 | 0.825 | 1.49 | 1.23 | 1.22 | 1.27 | 1.68 | 2.44 | 1.62 |
| Sens 1 | 70% | 83% | 70% | 71% | 78% | 72% | 71% | 71% | 71% |
| Spec 1 | 35% | 16% | 41% | 34% | 32% | 34% | 48% | 66% | 45% |
| Cutoff 2 | 1.04 | 0.825 | 1.09 | 0.971 | 0.562 | 1.06 | 1.04 | 2.28 | 0.679 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 2 | 81% | 83% | 81% | 82% | 89% | 81% | 82% | 86% | 82% |
| Spec 2 | 28% | 16% | 28% | 25% | 7% | 27% | 28% | 63% | 10% |
| Cutoff 3 | 0.768 | 0.314 | 0.883 | 0.795 | 0.241 | 0.872 | 0.611 | 0.314 | 0.562 |
| Sens 3 | 93% | 100% | 93% | 91% | 100% | 91% | 94% | 100% | 94% |
| Spec 3 | 15% | 2% | 18% | 17% | 1% | 18% | 10% | 2% | 6% |
| Cutoff 4 | 2.71 | 2.71 | 3.17 | 2.71 | 2.71 | 3.17 | 2.71 | 2.71 | 3.17 |
| Sens 4 | 26% | 50% | 22% | 38% | 56% | 34% | 29% | 43% | 12% |
| Spec 4 | 70% | 70% | 70% | 70% | 70% | 70% | 70% | 70% | 70% |
| Cutoff 5 | 3.99 | 3.88 | 4.22 | 3.99 | 3.88 | 4.22 | 3.99 | 3.88 | 4.22 |
| Sens 5 | 11% | 33% | 11% | 18% | 33% | 16% | 12% | 29% | 12% |
| Spec 5 | 80% | 80% | 80% | 80% | 80% | 80% | 80% | 80% | 80% |
| Cutoff 6 | 5.44 | 5.46 | 5.97 | 5.44 | 5.46 | 5.97 | 5.44 | 5.46 | 5.97 |
| Sens 6 | 7% | 17% | 7% | 12% | 0% | 3% | 6% | 0% | 6% |
| Spec 6 | 90% | 90% | 90% | 90% | 90% | 90% | 90% | 90% | 90% |
| OR Quart 2 | 1.2 | 0 | 1.5 | 0.85 | 0.50 | 0.99 | 0.99 | 0 | 4.3 |
| p Value | 0.76 | na | 0.53 | 0.78 | 0.57 | 0.99 | 0.99 | na | 0.071 |
| 95% CI of OR Quart2 | 0.36 4.1 | na na | 0.41 5.6 | 0.28 2.6 | 0.044 5.5 | 0.31 3.2 | 0.20 5.0 | na na | 0.88 21 |
| OR Quart 3 | 2.1 | 0.50 | 3.0 | 1.8 | 0.50 | 2.3 | 3.1 | 3.0 | 1.5 |
| p Value | 0.19 | 0.57 | 0.070 | 0.24 | 0.57 | 0.10 | 0.092 | 0.34 | 0.65 |
| 95% CI of OR Quart3 | 0.69 6.3 | 0.044 5.5 | 0.91 9.7 | 0.68 4.7 | 0.044 5.5 | 0.84 6.4 | 0.83 12 | 0.31 30 | 0.25 9.3 |
| OR Quart 4 | 1.2 | 1.5 | 1.5 | 1.3 | 2.6 | 1.2 | 0.65 | 3.0 | 2.0 |
| p Value | 0.77 | 0.66 | 0.53 | 0.61 | 0.27 | 0.79 | 0.65 | 0.34 | 0.42 |
| 95% CI of OR Quart4 | 0.36 4.0 | 0.25 9.1 | 0.41 5.6 | 0.47 3.6 | 0.49 13 | 0.38 3.6 | 0.11 4.0 | 0.31 29 | 0.37 11 |

## Myoglobin

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 28.5 | 65.8 | 28.5 | 80.9 | 28.5 | 82.0 |
| Average | 82.5 | 171 | 82.5 | 252 | 82.5 | 221 |
| Stdev | 182 | 372 | 182 | 471 | 182 | 396 |
| p(t-test) |  | 0.024 |  | 1.2E-5 |  | 0.0040 |
| Min | 3.55 | 4.22 | 3.55 | 3.96 | 3.55 | 5.12 |
| Max | 2130 | 1880 | 2130 | 1880 | 2130 | 1310 |
| n (Samp) | 434 | 27 | 434 | 34 | 434 | 17 |
| n (Patient) | 173 | 27 | 173 | 34 | 173 | 17 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 32.7 | 142 | 32.7 | 125 | 32.7 | 151 |
| Average | 87.4 | 640 | 87.4 | 653 | 87.4 | 396 |
| Stdev | 195 | 849 | 195 | 793 | 195 | 429 |
| p(t-test) |  | 3.2E-10 |  | 3.3E-14 |  | 5.1E-5 |
| Min | 3.55 | 35.2 | 3.55 | 13.0 | 3.55 | 17.5 |
| Max | 2130 | 1880 | 2130 | 1880 | 2130 | 1180 |
| n (Samp) | 535 | 6 | 535 | 9 | 535 | 7 |
| n (Patient) | 207 | 6 | 207 | 9 | 207 | 7 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 30.3 | 56.9 | 30.3 | 86.9 | 30.3 | 79.1 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 83.3 | 103 | 83.3 | 171 | 83.3 | 261 |
| Stdev | 185 | 149 | 185 | 306 | 185 | 518 |
| p(t-test) | | 0.59 | | 0.016 | | 7.1E-4 |
| Min | 4.60 | 4.22 | 4.60 | 3.96 | 4.60 | 5.12 |
| Max | 2130 | 646 | 2130 | 1410 | 2130 | 1880 |
| n (Samp) | 359 | 27 | 359 | 32 | 359 | 17 |
| n (Patient) | 139 | 27 | 139 | 32 | 139 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.83 | 0.64 | 0.66 | 0.70 | 0.67 | 0.64 | 0.81 | 0.65 |
| SE | 0.059 | 0.10 | 0.059 | 0.053 | 0.098 | 0.054 | 0.074 | 0.099 | 0.074 |
| p | 0.0056 | 0.0015 | 0.015 | 0.0030 | 0.041 | 0.0022 | 0.050 | 0.0018 | 0.040 |
| nCohort 1 | 434 | 535 | 359 | 434 | 535 | 359 | 434 | 535 | 359 |
| nCohort 2 | 27 | 6 | 27 | 34 | 9 | 32 | 17 | 7 | 17 |
| Cutoff 1 | 42.7 | 66.9 | 42.7 | 34.8 | 31.4 | 38.6 | 34.8 | 101 | 34.8 |
| Sens 1 | 70% | 83% | 70% | 71% | 78% | 72% | 71% | 71% | 71% |
| Spec 1 | 64% | 73% | 63% | 57% | 50% | 59% | 57% | 82% | 55% |
| Cutoff 2 | 22.9 | 66.9 | 22.9 | 16.2 | 19.2 | 18.9 | 17.5 | 87.6 | 18.7 |
| Sens 2 | 81% | 83% | 81% | 82% | 89% | 81% | 82% | 86% | 82% |
| Spec 2 | 40% | 73% | 39% | 25% | 30% | 31% | 27% | 78% | 30% |
| Cutoff 3 | 18.3 | 34.8 | 18.3 | 12.9 | 12.9 | 12.9 | 11.1 | 17.4 | 11.1 |
| Sens 3 | 93% | 100% | 93% | 91% | 100% | 91% | 94% | 100% | 94% |
| Spec 3 | 30% | 53% | 30% | 18% | 17% | 18% | 13% | 25% | 12% |
| Cutoff 4 | 56.0 | 60.1 | 57.8 | 56.0 | 60.1 | 57.8 | 56.0 | 60.1 | 57.8 |
| Sens 4 | 52% | 83% | 48% | 62% | 56% | 69% | 65% | 86% | 65% |
| Spec 4 | 70% | 70% | 70% | 70% | 70% | 70% | 70% | 70% | 70% |
| Cutoff 5 | 93.4 | 95.1 | 95.1 | 93.4 | 95.1 | 95.1 | 93.4 | 95.1 | 95.1 |
| Sens 5 | 33% | 67% | 22% | 44% | 56% | 47% | 35% | 71% | 35% |
| Spec 5 | 80% | 80% | 80% | 80% | 80% | 80% | 80% | 80% | 80% |
| Cutoff 6 | 182 | 180 | 188 | 182 | 180 | 188 | 182 | 180 | 188 |
| Sens 6 | 15% | 33% | 7% | 24% | 44% | 19% | 18% | 43% | 18% |
| Spec 6 | 90% | 90% | 90% | 90% | 90% | 90% | 90% | 90% | 90% |
| OR Quart 2 | 2.6 | >0 | 2.6 | 0.27 | 2.0 | 0.32 | 1.5 | 0 | 1.0 |
| p Value | 0.27 | <na | 0.27 | 0.11 | 0.57 | 0.16 | 0.66 | na | 1.0 |
| 95% CI of OR Quart2 | 0.49 / 14 | >na / na | 0.48 / 13 | 0.056 / 1.3 | 0.18 / 22 | 0.062 / 1.6 | 0.25 / 9.2 | na / na | 0.14 / 7.3 |
| OR Quart 3 | 4.8 | >2.0 | 4.9 | 1.0 | 1.0 | 1.2 | 0.99 | 0 | 2.0 |
| p Value | 0.048 | <0.57 | 0.047 | 1.0 | 1.0 | 0.79 | 0.99 | na | 0.42 |
| 95% CI of OR Quart3 | 1.0 / 23 | >0.18 / na | 1.0 / 23 | 0.34 / 2.9 | 0.062 / 16 | 0.38 / 3.6 | 0.14 / 7.2 | na / na | 0.37 / 11 |
| OR Quart 4 | 5.9 | >4.1 | 6.0 | 2.9 | 5.2 | 3.2 | 5.3 | 6.2 | 4.9 |
| p Value | 0.023 | <0.21 | 0.022 | 0.024 | 0.14 | 0.020 | 0.033 | 0.094 | 0.047 |
| 95% CI of OR Quart4 | 1.3 / 27 | >0.45 / na | 1.3 / 28 | 1.1 / 7.1 | 0.59 / 45 | 1.2 / 8.5 | 1.1 / 25 | 0.73 / 52 | 1.0 / 23 |

## Mucin-16

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.34 | 1.72 | 1.34 | 1.78 | 1.34 | 2.02 |
| Average | 4.05 | 40.6 | 4.05 | 23.6 | 4.05 | 19.4 |
| Stdev | 10.9 | 98.2 | 10.9 | 66.0 | 10.9 | 61.0 |
| p(t-test) | | 4.2E-6 | | 2.0E-4 | | 0.0027 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.117 | 1.12 | 0.117 | 0.191 | 0.117 | 0.549 |
| Max | 131 | 263 | 131 | 253 | 131 | 248 |
| n (Samp) | 196 | 7 | 196 | 22 | 196 | 16 |
| n (Patient) | 130 | 7 | 130 | 22 | 130 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.46 | 2.02 | 1.46 | 3.28 |
| Average | nd | nd | 4.51 | 27.2 | 4.51 | 23.4 |
| Stdev | nd | nd | 11.7 | 70.6 | 11.7 | 67.5 |
| p(t-test) | nd | nd | | 1.8E-4 | | 0.0018 |
| Min | nd | nd | 0.122 | 0.191 | 0.122 | 0.549 |
| Max | nd | nd | 131 | 253 | 131 | 248 |
| n (Samp) | nd | nd | 170 | 19 | 170 | 13 |
| n (Patient) | nd | nd | 108 | 19 | 108 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | nd | nd | 0.60 | nd | 0.59 | 0.64 | nd | 0.66 |
| SE | 0.11 | nd | nd | 0.067 | nd | 0.072 | 0.077 | nd | 0.085 |
| p | 0.16 | nd | nd | 0.12 | nd | 0.23 | 0.078 | nd | 0.059 |
| nCohort 1 | 196 | nd | nd | 196 | nd | 170 | 196 | nd | 170 |
| nCohort 2 | 7 | nd | nd | 22 | nd | 19 | 16 | nd | 13 |
| Cutoff 1 | 1.33 | nd | nd | 1.09 | nd | 1.12 | 1.23 | nd | 1.28 |
| Sens 1 | 71% | nd | nd | 73% | nd | 74% | 75% | nd | 77% |
| Spec 1 | 50% | nd | nd | 41% | nd | 39% | 46% | nd | 44% |
| Cutoff 2 | 1.23 | nd | nd | 1.01 | nd | 0.633 | 1.01 | nd | 1.01 |
| Sens 2 | 86% | nd | nd | 82% | nd | 84% | 81% | nd | 85% |
| Spec 2 | 46% | nd | nd | 38% | nd | 22% | 38% | nd | 35% |
| Cutoff 3 | 1.09 | nd | nd | 0.464 | nd | 0.424 | 0.761 | nd | 0.821 |
| Sens 3 | 100% | nd | nd | 91% | nd | 95% | 94% | nd | 92% |
| Spec 3 | 41% | nd | nd | 14% | nd | 12% | 29% | nd | 32% |
| Cutoff 4 | 2.77 | nd | nd | 2.77 | nd | 3.55 | 2.77 | nd | 3.55 |
| Sens 4 | 43% | nd | nd | 41% | nd | 37% | 44% | nd | 46% |
| Spec 4 | 70% | nd | nd | 70% | nd | 70% | 70% | nd | 70% |
| Cutoff 5 | 4.41 | nd | nd | 4.41 | nd | 4.88 | 4.41 | nd | 4.88 |
| Sens 5 | 29% | nd | nd | 32% | nd | 32% | 38% | nd | 38% |
| Spec 5 | 80% | nd | nd | 80% | nd | 80% | 80% | nd | 80% |
| Cutoff 6 | 6.95 | nd | nd | 6.95 | nd | 7.65 | 6.95 | nd | 7.65 |
| Sens 6 | 29% | nd | nd | 27% | nd | 32% | 31% | nd | 38% |
| Spec 6 | 90% | nd | nd | 90% | nd | 90% | 90% | nd | 90% |
| OR Quart 2 | >2.0 | nd | nd | 0.98 | nd | 1.0 | 6.6 | nd | 3.1 |
| p Value | <0.57 | nd | nd | 0.98 | nd | 1.0 | 0.085 | nd | 0.34 |
| 95% CI of OR Quart2 | >0.18 na | nd | nd | 0.23 4.1 | nd | 0.23 4.3 | 0.77 57 | nd | 0.31 31 |
| OR Quart 3 | >3.1 | nd | nd | 1.6 | nd | 1.0 | 3.1 | nd | 3.1 |
| p Value | <0.33 | nd | nd | 0.51 | nd | 1.0 | 0.33 | nd | 0.34 |
| 95% CI of OR Quart3 | >0.31 na | nd | nd | 0.42 5.9 | nd | 0.23 4.3 | 0.31 31 | nd | 0.31 31 |
| OR Quart 4 | >2.0 | nd | nd | 2.1 | nd | 1.8 | 6.6 | nd | 6.6 |
| p Value | <0.57 | nd | nd | 0.24 | nd | 0.36 | 0.085 | nd | 0.087 |
| 95% CI of OR Quart4 | >0.18 na | nd | nd | 0.60 7.5 | nd | 0.50 6.7 | 0.77 57 | nd | 0.76 57 |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.00E-9 | 0.00692 | 1.00E-9 | 0.00181 | 1.00E-9 | 0.00572 |
| Average | 0.00543 | 0.0417 | 0.00543 | 0.0242 | 0.00543 | 0.0256 |
| Stdev | 0.0150 | 0.0972 | 0.0150 | 0.0668 | 0.0150 | 0.0736 |
| p(t-test) | | 4.5E-5 | | 0.0018 | | 0.0020 |
| Min | 1.00E-9 | 1.00E-9 | 1.00E-9 | 1.00E-9 | 1.00E-9 | 1.00E-9 |
| Max | 0.114 | 0.262 | 0.114 | 0.286 | 0.114 | 0.300 |
| n (Samp) | 189 | 7 | 189 | 18 | 189 | 16 |
| n (Patient) | 125 | 7 | 125 | 18 | 125 | 16 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.000420 | 0.00142 | 0.000420 | 0.00220 |
| Average | nd | nd | 0.00637 | 0.0151 | 0.00637 | 0.00604 |
| Stdev | nd | nd | 0.0161 | 0.0277 | 0.0161 | 0.00762 |
| p(t-test) | nd | nd | | 0.053 | | 0.94 |
| Min | nd | nd | 1.00E-9 | 1.00E-9 | 1.00E-9 | 1.00E-9 |
| Max | nd | nd | 0.114 | 0.108 | 0.114 | 0.0222 |
| n (Samp) | nd | nd | 162 | 17 | 162 | 13 |
| n (Patient) | nd | nd | 101 | 17 | 101 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | nd | nd | 0.61 | nd | 0.59 | 0.65 | nd | 0.57 |
| SE | 0.12 | nd | nd | 0.073 | nd | 0.076 | 0.077 | nd | 0.086 |
| p | 0.25 | nd | nd | 0.12 | nd | 0.25 | 0.060 | nd | 0.38 |
| nCohort 1 | 189 | nd | nd | 189 | nd | 162 | 189 | nd | 162 |
| nCohort 2 | 7 | nd | nd | 18 | nd | 17 | 16 | nd | 13 |
| Cutoff 1 | 0 | nd | nd | 0 | nd | 0 | 0 | nd | 0 |
| Sens 1 | 100% | nd | nd | 100% | nd | 100% | 100% | nd | 100% |
| Spec 1 | 0% | nd | nd | 0% | nd | 0% | 0% | nd | 0% |
| Cutoff 2 | 0 | nd | nd | 0 | nd | 0 | 0 | nd | 0 |
| Sens 2 | 100% | nd | nd | 100% | nd | 100% | 100% | nd | 100% |
| Spec 2 | 0% | nd | nd | 0% | nd | 0% | 0% | nd | 0% |
| Cutoff 3 | 0 | nd | nd | 0 | nd | 0 | 0 | nd | 0 |
| Sens 3 | 100% | nd | nd | 100% | nd | 100% | 100% | nd | 100% |
| Spec 3 | 0% | nd | nd | 0% | nd | 0% | 0% | nd | 0% |
| Cutoff 4 | 0.00367 | nd | nd | 0.00367 | nd | 0.00444 | 0.00367 | nd | 0.00444 |
| Sens 4 | 57% | nd | nd | 44% | nd | 47% | 50% | nd | 38% |
| Spec 4 | 70% | nd | nd | 70% | nd | 70% | 70% | nd | 70% |
| Cutoff 5 | 0.00692 | nd | nd | 0.00692 | nd | 0.00819 | 0.00692 | nd | 0.00819 |
| Sens 5 | 43% | nd | nd | 39% | nd | 35% | 50% | nd | 31% |
| Spec 5 | 81% | nd | nd | 81% | nd | 82% | 81% | nd | 82% |
| Cutoff 6 | 0.0124 | nd | nd | 0.0124 | nd | 0.0136 | 0.0124 | nd | 0.0136 |
| Sens 6 | 29% | nd | nd | 28% | nd | 29% | 31% | nd | 23% |
| Spec 6 | 91% | nd | nd | 91% | nd | 90% | 91% | nd | 90% |
| OR Quart 2 | >3.2 | nd | nd | >7.9 | nd | 1.3 | >5.5 | nd | 4.2 |
| p Value | <0.32 | nd | nd | <0.057 | nd | 0.72 | <0.12 | nd | 0.21 |
| 95% CI of OR Quart2 | >0.32 | nd | nd | >0.94 | nd | 0.28 | >0.62 | nd | 0.45 |
| | na | nd | nd | na | nd | 6.3 | na | nd | 39 |
| OR Quart 3 | >0 | nd | nd | >4.2 | nd | 0.98 | >3.2 | nd | 3.1 |
| p Value | <na | nd | nd | <0.20 | nd | 0.98 | <0.32 | nd | 0.34 |
| 95% CI of OR Quart3 | >na | nd | nd | >0.46 | nd | 0.19 | >0.32 | nd | 0.31 |
| | na | nd | nd | na | nd | 5.1 | na | nd | 31 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | >4.4 | nd | nd | >7.9 | nd | 2.5 | >9.3 | nd | 5.4 |
| p Value | <0.20 | nd | nd | <0.057 | nd | 0.20 | <0.039 | nd | 0.13 |
| 95% CI of | >0.47 | nd | nd | >0.94 | nd | 0.61 | >1.1 | nd | 0.60 |
| OR Quart4 | na | nd | nd | na | nd | 10 | na | nd | 48 |

## Cellular tumor antigen p53

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 1.00E-9 | 1.00E-9 | 1.00E-9 | 1.00E-9 | 1.00E-9 |
| Average | 0.0690 | 0.289 | 0.0690 | 0.143 | 0.0690 | 0.172 |
| Stdev | 0.419 | 0.698 | 0.419 | 0.551 | 0.419 | 0.595 |
| p(t-test) |  | 0.19 |  | 0.49 |  | 0.36 |
| Min | 1.00E-9 | 1.00E-9 | 1.00E-9 | 1.00E-9 | 1.00E-9 | 1.00E-9 |
| Max | 3.70 | 1.87 | 3.70 | 2.35 | 3.70 | 2.39 |
| n (Samp) | 189 | 7 | 189 | 18 | 189 | 16 |
| n (Patient) | 125 | 7 | 125 | 18 | 125 | 16 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | nd | nd | 1.00E-9 | 1.00E-9 | 1.00E-9 | 1.00E-9 |
| Average | nd | nd | 0.0804 | 0.0131 | 0.0804 | 0.0283 |
| Stdev | nd | nd | 0.452 | 0.0316 | 0.452 | 0.0790 |
| p(t-test) | nd | nd |  | 0.54 |  | 0.68 |
| Min | nd | nd | 1.00E-9 | 1.00E-9 | 1.00E-9 | 1.00E-9 |
| Max | nd | nd | 3.70 | 0.117 | 3.70 | 0.285 |
| n (Samp) | nd | nd | 162 | 17 | 162 | 13 |
| n (Patient) | nd | nd | 101 | 17 | 101 | 13 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.64 | nd | nd | 0.62 | nd | 0.59 | 0.54 | nd | 0.52 |
| SE | 0.11 | nd | nd | 0.073 | nd | 0.076 | 0.077 | nd | 0.084 |
| p | 0.21 | nd | nd | 0.091 | nd | 0.23 | 0.57 | nd | 0.80 |
| nCohort 1 | 189 | nd | nd | 189 | nd | 162 | 189 | nd | 162 |
| nCohort 2 | 7 | nd | nd | 18 | nd | 17 | 16 | nd | 13 |
| Cutoff 1 | 0 | nd | nd | 0 | nd | 0 | 0 | nd | 0 |
| Sens 1 | 100% | nd | nd | 100% | nd | 100% | 100% | nd | 100% |
| Spec 1 | 0% | nd | nd | 0% | nd | 0% | 0% | nd | 0% |
| Cutoff 2 | 0 | nd | nd | 0 | nd | 0 | 0 | nd | 0 |
| Sens 2 | 100% | nd | nd | 100% | nd | 100% | 100% | nd | 100% |
| Spec 2 | 0% | nd | nd | 0% | nd | 0% | 0% | nd | 0% |
| Cutoff 3 | 0 | nd | nd | 0 | nd | 0 | 0 | nd | 0 |
| Sens 3 | 100% | nd | nd | 100% | nd | 100% | 100% | nd | 100% |
| Spec 3 | 0% | nd | nd | 0% | nd | 0% | 0% | nd | 0% |
| Cutoff 4 | 1.00E-9 | nd | nd | 1.00E-9 | nd | 1.00E-9 | 1.00E-9 | nd | 1.00E-9 |
| Sens 4 | 43% | nd | nd | 44% | nd | 41% | 25% | nd | 23% |
| Spec 4 | 81% | nd | nd | 81% | nd | 80% | 81% | nd | 80% |
| Cutoff 5 | 1.00E-9 | nd | nd | 1.00E-9 | nd | 0.000790 | 1.00E-9 | nd | 0.000790 |
| Sens 5 | 43% | nd | nd | 44% | nd | 35% | 25% | nd | 23% |
| Spec 5 | 81% | nd | nd | 81% | nd | 81% | 81% | nd | 81% |
| Cutoff 6 | 0.0225 | nd | nd | 0.0225 | nd | 0.0267 | 0.0225 | nd | 0.0267 |
| Sens 6 | 43% | nd | nd | 17% | nd | 12% | 25% | nd | 15% |
| Spec 6 | 90% | nd | nd | 90% | nd | 90% | 90% | nd | 90% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 | 3.1 | nd | nd | 10 | nd | >13 | >16 | nd | >13 |
| p Value | 0.33 | nd | nd | 0.029 | nd | <0.018 | <0.0096 | nd | <0.018 |
| 95% CI of | 0.31 | nd | nd | 1.3 | nd | >1.5 | >2.0 | nd | >1.5 |
| OR Quart2 | 31 | nd | nd | 86 | nd | na | na | nd | na |
| OR Quart 3 | 0 | nd | nd | 0 | nd | >0 | >0 | nd | >0 |
| p Value | na | nd | nd | na | nd | <na | <na | nd | <na |
| 95% CI of | na | nd | nd | na | nd | >na | >na | nd | >na |
| OR Quart3 | na | nd | nd | na | nd | na | na | nd | na |
| OR Quart 4 | 3.1 | nd | nd | 9.1 | nd | >8.1 | >4.2 | nd | >3.1 |
| p Value | 0.33 | nd | nd | 0.041 | nd | <0.055 | <0.20 | nd | <0.33 |
| 95% CI of | 0.31 | nd | nd | 1.1 | nd | >0.95 | >0.46 | nd | >0.31 |
| OR Quart4 | 31 | nd | nd | 76 | nd | na | na | nd | na |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Apolipoprotein A-II**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 57100 | 48800 | nd | nd | 57100 | 45400 |
| Average | 57200 | 51600 | nd | nd | 57000 | 46300 |
| Stdev | 36400 | 24300 | nd | nd | 39000 | 21400 |
| p(t-test) |  | 0.53 | nd | nd |  | 0.34 |
| Min | 7970 | 1810 | nd | nd | 7970 | 1810 |
| Max | 251000 | 100000 | nd | nd | 251000 | 75500 |
| n (Samp) | 50 | 19 | nd | nd | 41 | 14 |
| n (Patient) | 50 | 19 | nd | nd | 41 | 14 |

|  | At Enrollment | | |
|---|---|---|---|
|  | sCr or UO | sCr only | UO only |
| AUC | 0.47 | nd | 0.44 |
| SE | 0.079 | nd | 0.091 |
| p | 0.69 | nd | 0.49 |
| nCohort 1 | 50 | nd | 41 |
| nCohort 2 | 19 | nd | 14 |
| Cutoff 1 | 37400 | nd | 32500 |
| Sens 1 | 74% | nd | 71% |
| Spec 1 | 26% | nd | 24% |
| Cutoff 2 | 28800 | nd | 27600 |
| Sens 2 | 84% | nd | 86% |
| Spec 2 | 18% | nd | 22% |
| Cutoff 3 | 23900 | nd | 23900 |
| Sens 3 | 95% | nd | 93% |
| Spec 3 | 16% | nd | 20% |
| Cutoff 4 | 64400 | nd | 66800 |
| Sens 4 | 26% | nd | 21% |
| Spec 4 | 70% | nd | 71% |
| Cutoff 5 | 72300 | nd | 72300 |
| Sens 5 | 21% | nd | 14% |
| Spec 5 | 80% | nd | 80% |
| Cutoff 6 | 83600 | nd | 85700 |

|  | At Enrollment | | |
|  | sCr or UO | sCr only | UO only |
| --- | --- | --- | --- |
| Sens 6 | 11% | nd | 0% |
| Spec 6 | 90% | nd | 90% |
| OR Quart 2 | 0.80 | nd | 1.0 |
| p Value | 0.77 | nd | 1.0 |
| 95% CI of | 0.17 | nd | 0.16 |
| OR Quart2 | 3.7 | nd | 6.1 |
| OR Quart 3 | 1.1 | nd | 1.5 |
| p Value | 0.91 | nd | 0.66 |
| 95% CI of | 0.25 | nd | 0.26 |
| OR Quart3 | 4.7 | nd | 8.2 |
| OR Quart 4 | 1.1 | nd | 1.6 |
| p Value | 0.91 | nd | 0.58 |
| 95% CI of | 0.25 | nd | 0.29 |
| OR Quart4 | 4.7 | nd | 9.3 |

## Myoglobin

|  | sCr or UO | | sCr only | | UO only | |
|  | Cohort 1 | Cohort 2 | Cohort 1 | Cohort 2 | Cohort 1 | Cohort 2 |
| --- | --- | --- | --- | --- | --- | --- |
| Median | 40.1 | 64.7 | nd | nd | 35.8 | 79.1 |
| Average | 139 | 182 | nd | nd | 132 | 88.9 |
| Stdev | 314 | 344 | nd | nd | 329 | 98.2 |
| p(t-test) |  | 0.60 | nd | nd |  | 0.60 |
| Min | 7.19 | 4.16 | nd | nd | 7.19 | 4.16 |
| Max | 2130 | 1310 | nd | nd | 2130 | 397 |
| n (Samp) | 54 | 23 | nd | nd | 46 | 17 |
| n (Patient) | 54 | 23 | nd | nd | 46 | 17 |

|  | At Enrollment | | |
|  | sCr or UO | sCr only | UO only |
| --- | --- | --- | --- |
| AUC | 0.55 | nd | 0.54 |
| SE | 0.073 | nd | 0.083 |
| p | 0.48 | nd | 0.66 |
| nCohort 1 | 54 | nd | 46 |
| nCohort 2 | 23 | nd | 17 |
| Cutoff 1 | 30.3 | nd | 34.2 |
| Sens 1 | 74% | nd | 71% |
| Spec 1 | 43% | nd | 50% |
| Cutoff 2 | 17.8 | nd | 15.3 |
| Sens 2 | 83% | nd | 82% |
| Spec 2 | 19% | nd | 13% |
| Cutoff 3 | 12.9 | nd | 4.16 |
| Sens 3 | 91% | nd | 94% |
| Spec 3 | 13% | nd | 0% |
| Cutoff 4 | 91.7 | nd | 90.0 |
| Sens 4 | 35% | nd | 41% |
| Spec 4 | 70% | nd | 72% |
| Cutoff 5 | 189 | nd | 141 |
| Sens 5 | 17% | nd | 18% |
| Spec 5 | 81% | nd | 80% |
| Cutoff 6 | 328 | nd | 315 |
| Sens 6 | 13% | nd | 6% |
| Spec 6 | 91% | nd | 91% |
| OR Quart 2 | 0.41 | nd | 0.29 |
| p Value | 0.26 | nd | 0.18 |

|  | At Enrollment | | |
|---|---|---|---|
|  | sCr or UO | sCr only | UO only |
| 95% CI of | 0.085 | nd | 0.046 |
| OR Quart2 | 1.9 | nd | 1.8 |
| OR Quart 3 | 1.6 | nd | 0.91 |
| p Value | 0.50 | nd | 0.90 |
| 95% CI of | 0.42 | nd | 0.20 |
| OR Quart3 | 5.9 | nd | 4.1 |
| OR Quart 4 | 0.93 | nd | 0.91 |
| p Value | 0.91 | nd | 0.90 |
| 95% CI of | 0.24 | nd | 0.20 |
| OR Quart4 | 3.6 | nd | 4.1 |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

## Apolipoprotein A-II

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 52600 | 43600 | 52600 | 41100 | 52600 | 57700 |
| Average | 52700 | 57800 | 52700 | 57100 | 52700 | 72600 |
| Stdev | 22500 | 48900 | 22500 | 51100 | 22500 | 58900 |
| p(t-test) | | 0.55 | | 0.62 | | 0.068 |
| Min | 5450 | 10700 | 5450 | 10700 | 5450 | 9430 |
| Max | 105000 | 176000 | 105000 | 176000 | 105000 | 176000 |
| n (Samp) | 97 | 11 | 97 | 10 | 97 | 6 |
| n (Patient) | 97 | 11 | 97 | 10 | 97 | 6 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 53400 | 57800 | 53400 | 52600 | 53400 | 57700 |
| Average | 53900 | 66500 | 53900 | 64700 | 53900 | 72600 |
| Stdev | 20600 | 54800 | 20600 | 54700 | 20600 | 58900 |
| p(t-test) | | 0.18 | | 0.25 | | 0.073 |
| Min | 8680 | 10700 | 8680 | 10700 | 8680 | 9430 |
| Max | 123000 | 176000 | 123000 | 176000 | 123000 | 176000 |
| n (Samp) | 84 | 8 | 84 | 8 | 84 | 6 |
| n (Patient) | 84 | 8 | 84 | 8 | 84 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | 0.53 | 0.45 | nd | 0.51 | 0.57 | nd | 0.56 |
| SE | 0.094 | nd | 0.11 | 0.098 | nd | 0.11 | 0.13 | nd | 0.13 |
| p | 0.69 | nd | 0.78 | 0.61 | nd | 0.94 | 0.59 | nd | 0.66 |
| nCohort 1 | 97 | nd | 84 | 97 | nd | 84 | 97 | nd | 84 |
| nCohort 2 | 11 | nd | 8 | 10 | nd | 8 | 6 | nd | 6 |
| Cutoff 1 | 37600 | nd | 37600 | 37600 | nd | 37600 | 37600 | nd | 37600 |
| Sens 1 | 73% | nd | 75% | 70% | nd | 75% | 83% | nd | 83% |
| Spec 1 | 27% | nd | 19% | 27% | nd | 19% | 27% | nd | 19% |
| Cutoff 2 | 14000 | nd | 10700 | 14000 | nd | 10700 | 37600 | nd | 37600 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 2 | 82% | nd | 88% | 80% | nd | 88% | 83% | nd | 83% |
| Spec 2 | 4% | nd | 1% | 4% | nd | 1% | 27% | nd | 19% |
| Cutoff 3 | 10700 | nd | 8680 | 10700 | nd | 8680 | 8680 | nd | 8680 |
| Sens 3 | 91% | nd | 100% | 90% | nd | 100% | 100% | nd | 100% |
| Spec 3 | 2% | nd | 1% | 2% | nd | 1% | 2% | nd | 1% |
| Cutoff 4 | 61600 | nd | 61700 | 61600 | nd | 61700 | 61600 | nd | 61700 |
| Sens 4 | 36% | nd | 50% | 40% | nd | 50% | 50% | nd | 50% |
| Spec 4 | 70% | nd | 70% | 70% | nd | 70% | 70% | nd | 70% |
| Cutoff 5 | 70000 | nd | 69100 | 70000 | nd | 69100 | 70000 | nd | 69100 |
| Sens 5 | 36% | nd | 50% | 30% | nd | 38% | 50% | nd | 50% |
| Spec 5 | 80% | nd | 81% | 80% | nd | 81% | 80% | nd | 81% |
| Cutoff 6 | 82900 | nd | 77900 | 82900 | nd | 77900 | 82900 | nd | 77900 |
| Sens 6 | 18% | nd | 25% | 20% | nd | 25% | 33% | nd | 33% |
| Spec 6 | 91% | nd | 90% | 91% | nd | 90% | 91% | nd | 90% |
| OR Quart 2 | 0 | nd | 0.30 | 0.31 | nd | 0.30 | 2.0 | nd | 0.45 |
| p Value | na | nd | 0.32 | 0.32 | nd | 0.32 | 0.58 | nd | 0.53 |
| 95% CI of OR Quart2 | na | nd | 0.029 | 0.030 | nd | 0.029 | 0.17 | nd | 0.038 |
| | na | nd | 3.2 | 3.2 | nd | 3.2 | 24 | nd | 5.4 |
| OR Quart 3 | 1.0 | nd | 0 | 1.0 | nd | 0.30 | 0 | nd | 0 |
| p Value | 1.0 | nd | na | 1.0 | nd | 0.32 | na | nd | na |
| 95% CI of OR Quart3 | 0.22 | nd | na | 0.18 | nd | 0.029 | na | nd | na |
| | 4.5 | nd | na | 5.5 | nd | 3.2 | na | nd | na |
| OR Quart 4 | 0.72 | nd | 1.4 | 1.0 | nd | 1.0 | 3.1 | nd | 1.5 |
| p Value | 0.69 | nd | 0.68 | 0.96 | nd | 1.0 | 0.34 | nd | 0.67 |
| 95% CI of OR Quart4 | 0.14 | nd | 0.28 | 0.19 | nd | 0.18 | 0.30 | nd | 0.23 |
| | 3.6 | nd | 7.1 | 5.7 | nd | 5.6 | 32 | nd | 10.0 |

## Caspase-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 91.6 | 138 | 91.6 | 90.7 | 91.6 | 83.0 |
| Average | 108 | 154 | 108 | 121 | 108 | 101 |
| Stdev | 74.7 | 96.4 | 74.7 | 71.9 | 74.7 | 53.3 |
| p(t-test) | | 0.17 | | 0.69 | | 0.83 |
| Min | 33.8 | 44.7 | 33.8 | 44.7 | 33.8 | 44.7 |
| Max | 326 | 328 | 326 | 241 | 326 | 192 |
| n (Samp) | 26 | 8 | 26 | 7 | 26 | 6 |
| n (Patient) | 26 | 8 | 26 | 7 | 26 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | nd | nd | 0.56 | nd | nd | 0.51 | nd | nd |
| SE | 0.12 | nd | nd | 0.13 | nd | nd | 0.13 | nd | nd |
| p | 0.15 | nd | nd | 0.62 | nd | nd | 0.94 | nd | nd |
| nCohort 1 | 26 | nd | nd | 26 | nd | nd | 26 | nd | nd |
| nCohort 2 | 8 | nd | nd | 7 | nd | nd | 6 | nd | nd |
| Cutoff 1 | 71.0 | nd | nd | 70.7 | nd | nd | 66.1 | nd | nd |
| Sens 1 | 75% | nd | nd | 71% | nd | nd | 83% | nd | nd |
| Spec 1 | 38% | nd | nd | 38% | nd | nd | 31% | nd | nd |
| Cutoff 2 | 70.7 | nd | nd | 66.1 | nd | nd | 66.1 | nd | nd |
| Sens 2 | 88% | nd | nd | 86% | nd | nd | 83% | nd | nd |
| Spec 2 | 38% | nd | nd | 31% | nd | nd | 31% | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 42.1 | nd | nd | 42.1 | nd | nd | 42.1 | nd | nd |
| Sens 3 | 100% | nd | nd | 100% | nd | nd | 100% | nd | nd |
| Spec 3 | 15% | nd | nd | 15% | nd | nd | 15% | nd | nd |
| Cutoff 4 | 103 | nd | nd | 103 | nd | nd | 103 | nd | nd |
| Sens 4 | 62% | nd | nd | 43% | nd | nd | 33% | nd | nd |
| Spec 4 | 73% | nd | nd | 73% | nd | nd | 73% | nd | nd |
| Cutoff 5 | 121 | nd | nd | 121 | nd | nd | 121 | nd | nd |
| Sens 5 | 62% | nd | nd | 43% | nd | nd | 33% | nd | nd |
| Spec 5 | 81% | nd | nd | 81% | nd | nd | 81% | nd | nd |
| Cutoff 6 | 254 | nd | nd | 254 | nd | nd | 254 | nd | nd |
| Sens 6 | 12% | nd | nd | 0% | nd | nd | 0% | nd | nd |
| Spec 6 | 92% | nd | nd | 92% | nd | nd | 92% | nd | nd |
| OR Quart 2 | 2.0 | nd | nd | 2.3 | nd | nd | 2.3 | nd | nd |
| p Value | 0.60 | nd | nd | 0.53 | nd | nd | 0.53 | nd | nd |
| 95% CI of | 0.15 | nd | nd | 0.17 | nd | nd | 0.17 | nd | nd |
| OR Quart2 | 27 | nd | nd | 33 | nd | nd | 33 | nd | nd |
| OR Quart 3 | 0 | nd | nd | 1.0 | nd | nd | 1.0 | nd | nd |
| p Value | na | nd | nd | 1.0 | nd | nd | 1.0 | nd | nd |
| 95% CI of | na | nd | nd | 0.052 | nd | nd | 0.052 | nd | nd |
| OR Quart3 | na | nd | nd | 19 | nd | nd | 19 | nd | nd |
| OR Quart 4 | 8.8 | nd | nd | 3.5 | nd | nd | 2.3 | nd | nd |
| p Value | 0.086 | nd | nd | 0.33 | nd | nd | 0.53 | nd | nd |
| 95% CI of | 0.74 | nd | nd | 0.28 | nd | nd | 0.17 | nd | nd |
| OR Quart4 | 100 | nd | nd | 43 | nd | nd | 33 | nd | nd |

## Caspase-9

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.7 | 12.2 | 28.7 | 6.83 | 28.7 | 7.20 |
| Average | 50.0 | 16.5 | 50.0 | 13.7 | 50.0 | 14.2 |
| Stdev | 69.0 | 14.3 | 69.0 | 15.0 | 69.0 | 16.2 |
| p(t-test) | | 0.16 | | 0.18 | | 0.22 |
| Min | 2.58 | 4.32 | 2.58 | 3.79 | 2.58 | 3.79 |
| Max | 366 | 46.4 | 366 | 46.4 | 366 | 46.4 |
| n (Samp) | 37 | 9 | 37 | 7 | 37 | 6 |
| n (Patient) | 37 | 9 | 37 | 7 | 37 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.29 | nd | nd | 0.22 | nd | nd | 0.23 | nd | nd |
| SE | 0.10 | nd | nd | 0.11 | nd | nd | 0.12 | nd | nd |
| p | 0.040 | nd | nd | 0.012 | nd | nd | 0.024 | nd | nd |
| nCohort 1 | 37 | nd | nd | 37 | nd | nd | 37 | nd | nd |
| nCohort 2 | 9 | nd | nd | 7 | nd | nd | 6 | nd | nd |
| Cutoff 1 | 6.05 | nd | nd | 6.05 | nd | nd | 4.34 | nd | nd |
| Sens 1 | 78% | nd | nd | 71% | nd | nd | 83% | nd | nd |
| Spec 1 | 11% | nd | nd | 11% | nd | nd | 8% | nd | nd |
| Cutoff 2 | 4.34 | nd | nd | 4.34 | nd | nd | 4.34 | nd | nd |
| Sens 2 | 89% | nd | nd | 86% | nd | nd | 83% | nd | nd |
| Spec 2 | 8% | nd | nd | 8% | nd | nd | 8% | nd | nd |
| Cutoff 3 | 3.30 | nd | nd | 3.30 | nd | nd | 3.30 | nd | nd |
| Sens 3 | 100% | nd | nd | 100% | nd | nd | 100% | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 3 | 5% | nd | nd | 5% | nd | nd | 5% | nd | nd |
| Cutoff 4 | 50.7 | nd | nd | 50.7 | nd | nd | 50.7 | nd | nd |
| Sens 4 | 0% | nd | nd | 0% | nd | nd | 0% | nd | nd |
| Spec 4 | 70% | nd | nd | 70% | nd | nd | 70% | nd | nd |
| Cutoff 5 | 71.4 | nd | nd | 71.4 | nd | nd | 71.4 | nd | nd |
| Sens 5 | 0% | nd | nd | 0% | nd | nd | 0% | nd | nd |
| Spec 5 | 81% | nd | nd | 81% | nd | nd | 81% | nd | nd |
| Cutoff 6 | 106 | nd | nd | 106 | nd | nd | 106 | nd | nd |
| Sens 6 | 0% | nd | nd | 0% | nd | nd | 0% | nd | nd |
| Spec 6 | 92% | nd | nd | 92% | nd | nd | 92% | nd | nd |
| OR Quart 2 | >4.5 | nd | nd | >1.1 | nd | nd | >1.1 | nd | nd |
| p Value | <0.23 | nd | nd | <0.95 | nd | nd | <0.95 | nd | nd |
| 95% CI of OR Quart2 | >0.39 na | nd | nd | >0.060 na | nd | nd | >0.060 na | nd | nd |
| OR Quart 3 | >2.4 | nd | nd | >2.4 | nd | nd | >1.1 | nd | nd |
| p Value | <0.50 | nd | nd | <0.49 | nd | nd | <0.95 | nd | nd |
| 95% CI of OR Quart3 | >0.19 na | nd | nd | >0.19 na | nd | nd | >0.060 na | nd | nd |
| OR Quart 4 | >6.9 | nd | nd | >6.3 | nd | nd | >7.3 | nd | nd |
| p Value | <0.11 | nd | nd | <0.13 | nd | nd | <0.10 | nd | nd |
| 95% CI of OR Quart4 | >0.63 na | nd | nd | >0.58 na | nd | nd | >0.66 na | nd | nd |

## Cadherin-1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72700 | 112000 | 72700 | 112000 | 72700 | 162000 |
| Average | 103000 | 157000 | 103000 | 154000 | 103000 | 174000 |
| Stdev | 108000 | 103000 | 108000 | 105000 | 108000 | 96900 |
| p(t-test) | | 0.13 | | 0.15 | | 0.13 |
| Min | 14500 | 38300 | 14500 | 38300 | 14500 | 50500 |
| Max | 621000 | 340000 | 621000 | 340000 | 621000 | 285000 |
| n (Samp) | 50 | 12 | 50 | 12 | 50 | 6 |
| n (Patient) | 50 | 12 | 50 | 12 | 50 | 6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 77600 | 151000 | 77600 | 132000 | nd | nd |
| Average | 110000 | 170000 | 110000 | 163000 | nd | nd |
| Stdev | 96000 | 127000 | 96000 | 132000 | nd | nd |
| p(t-test) | | 0.15 | | 0.20 | nd | nd |
| Min | 14500 | 38300 | 14500 | 38300 | nd | nd |
| Max | 621000 | 340000 | 621000 | 340000 | nd | nd |
| n (Samp) | 96 | 6 | 96 | 6 | nd | nd |
| n (Patient) | 96 | 6 | 96 | 6 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 80200 | 162000 | 80200 | 162000 | 80200 | 162000 |
| Average | 114000 | 170000 | 114000 | 170000 | 114000 | 174000 |
| Stdev | 117000 | 86300 | 117000 | 86300 | 117000 | 96900 |
| p(t-test) | | 0.21 | | 0.21 | | 0.24 |
| Min | 39700 | 64400 | 39700 | 64400 | 39700 | 50500 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 621000 | 285000 | 621000 | 285000 | 621000 | 285000 |
| n (Samp) | 44 | 8 | 44 | 8 | 44 | 6 |
| n (Patient) | 44 | 8 | 44 | 8 | 44 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.62 | 0.77 | 0.66 | 0.56 | 0.77 | 0.77 | nd | 0.74 |
| SE | 0.092 | 0.13 | 0.10 | 0.093 | 0.13 | 0.10 | 0.12 | nd | 0.12 |
| p | 0.038 | 0.35 | 0.0087 | 0.094 | 0.65 | 0.0087 | 0.021 | nd | 0.050 |
| nCohort 1 | 50 | 96 | 44 | 50 | 96 | 44 | 50 | nd | 44 |
| nCohort 2 | 12 | 6 | 8 | 12 | 6 | 8 | 6 | nd | 6 |
| Cutoff 1 | 88900 | 53300 | 105000 | 62800 | 53300 | 105000 | 105000 | nd | 105000 |
| Sens 1 | 75% | 83% | 75% | 75% | 83% | 75% | 83% | nd | 83% |
| Spec 1 | 62% | 22% | 75% | 42% | 22% | 75% | 76% | nd | 75% |
| Cutoff 2 | 62800 | 53300 | 88900 | 54100 | 53300 | 88900 | 105000 | nd | 105000 |
| Sens 2 | 83% | 83% | 88% | 83% | 83% | 88% | 83% | nd | 83% |
| Spec 2 | 42% | 22% | 59% | 24% | 22% | 59% | 76% | nd | 75% |
| Cutoff 3 | 52600 | 38100 | 62800 | 52600 | 38100 | 62800 | 48100 | nd | 47600 |
| Sens 3 | 92% | 100% | 100% | 92% | 100% | 100% | 100% | nd | 100% |
| Spec 3 | 22% | 6% | 34% | 22% | 6% | 34% | 20% | nd | 11% |
| Cutoff 4 | 97000 | 108000 | 97500 | 97000 | 108000 | 97500 | 97000 | nd | 97500 |
| Sens 4 | 58% | 50% | 75% | 58% | 50% | 75% | 83% | nd | 83% |
| Spec 4 | 70% | 71% | 70% | 70% | 71% | 70% | 70% | nd | 70% |
| Cutoff 5 | 114000 | 151000 | 131000 | 114000 | 151000 | 131000 | 114000 | nd | 131000 |
| Sens 5 | 50% | 50% | 50% | 50% | 50% | 50% | 67% | nd | 50% |
| Spec 5 | 80% | 80% | 82% | 80% | 80% | 82% | 80% | nd | 82% |
| Cutoff 6 | 153000 | 242000 | 153000 | 153000 | 242000 | 153000 | 153000 | nd | 153000 |
| Sens 6 | 42% | 33% | 50% | 42% | 33% | 50% | 50% | nd | 50% |
| Spec 6 | 90% | 91% | 91% | 90% | 91% | 91% | 90% | nd | 91% |
| OR Quart 2 | 0.43 | 0 | >1.1 | 0.27 | 0 | >1.1 | 0 | nd | 0 |
| p Value | 0.51 | na | <0.96 | 0.28 | na | <0.96 | na | nd | na |
| 95% CI of | 0.035 | na | >0.061 | 0.025 | na | >0.061 | na | nd | na |
| OR Quart2 | 5.3 | na | na | 2.9 | na | na | na | nd | na |
| OR Quart 3 | 1.6 | 0.48 | >3.9 | 0.62 | 0 | >3.9 | 1.0 | nd | 1.0 |
| p Value | 0.63 | 0.56 | <0.27 | 0.63 | na | <0.27 | 1.0 | nd | 1.0 |
| 95% CI of | 0.23 | 0.041 | >0.35 | 0.087 | na | >0.35 | 0.056 | nd | 0.055 |
| OR Quart3 | 11 | 5.7 | na | 4.3 | na | na | 18 | nd | 18 |
| OR Quart 4 | 3.9 | 1.5 | >5.8 | 2.4 | 0.96 | >5.8 | 5.2 | nd | 4.9 |
| p Value | 0.14 | 0.67 | <0.14 | 0.29 | 0.96 | <0.14 | 0.17 | nd | 0.19 |
| 95% CI of | 0.64 | 0.23 | >0.55 | 0.47 | 0.17 | >0.55 | 0.50 | nd | 0.46 |
| OR Quart4 | 24 | 9.8 | na | 12 | 5.3 | na | 54 | nd | 52 |

## Cyclin-dependent kinase inhibitor 1

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 212 | 243 | 212 | 255 | 212 | 299 |
| Average | 1210 | 333 | 1210 | 363 | 1210 | 402 |
| Stdev | 1930 | 337 | 1930 | 383 | 1930 | 405 |
| p(t-test) | | 0.19 | | 0.26 | | 0.32 |
| Min | 42.3 | 73.9 | 42.3 | 68.7 | 42.3 | 68.7 |
| Max | 6840 | 1190 | 6840 | 1190 | 6840 | 1190 |
| n (Samp) | 37 | 9 | 37 | 7 | 37 | 6 |
| n (Patient) | 37 | 9 | 37 | 7 | 37 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | nd | nd | 0.45 | nd | nd | 0.47 | nd | nd |
| SE | 0.11 | nd | nd | 0.12 | nd | nd | 0.13 | nd | nd |
| p | 0.71 | nd | nd | 0.68 | nd | nd | 0.81 | nd | nd |
| nCohort 1 | 37 | nd | nd | 37 | nd | nd | 37 | nd | nd |
| nCohort 2 | 9 | nd | nd | 7 | nd | nd | 6 | nd | nd |
| Cutoff 1 | 138 | nd | nd | 138 | nd | nd | 138 | nd | nd |
| Sens 1 | 78% | nd | nd | 71% | nd | nd | 83% | nd | nd |
| Spec 1 | 38% | nd | nd | 38% | nd | nd | 38% | nd | nd |
| Cutoff 2 | 125 | nd | nd | 125 | nd | nd | 138 | nd | nd |
| Sens 2 | 89% | nd | nd | 86% | nd | nd | 83% | nd | nd |
| Spec 2 | 32% | nd | nd | 32% | nd | nd | 38% | nd | nd |
| Cutoff 3 | 73.1 | nd | nd | 61.7 | nd | nd | 61.7 | nd | nd |
| Sens 3 | 100% | nd | nd | 100% | nd | nd | 100% | nd | nd |
| Spec 3 | 14% | nd | nd | 11% | nd | nd | 11% | nd | nd |
| Cutoff 4 | 866 | nd | nd | 866 | nd | nd | 866 | nd | nd |
| Sens 4 | 11% | nd | nd | 14% | nd | nd | 17% | nd | nd |
| Spec 4 | 70% | nd | nd | 70% | nd | nd | 70% | nd | nd |
| Cutoff 5 | 1760 | nd | nd | 1760 | nd | nd | 1760 | nd | nd |
| Sens 5 | 0% | nd | nd | 0% | nd | nd | 0% | nd | nd |
| Spec 5 | 81% | nd | nd | 81% | nd | nd | 81% | nd | nd |
| Cutoff 6 | 5160 | nd | nd | 5160 | nd | nd | 5160 | nd | nd |
| Sens 6 | 0% | nd | nd | 0% | nd | nd | 0% | nd | nd |
| Spec 6 | 92% | nd | nd | 92% | nd | nd | 92% | nd | nd |
| OR Quart 2 | 6.3 | nd | nd | 3.8 | nd | nd | 3.8 | nd | nd |
| p Value | 0.13 | nd | nd | 0.29 | nd | nd | 0.29 | nd | nd |
| 95% CI of OR Quart2 | 0.58 68 | nd | nd | 0.32 43 | nd | nd | 0.32 43 | nd | nd |
| OR Quart 3 | 3.7 | nd | nd | 2.2 | nd | nd | 1.0 | nd | nd |
| p Value | 0.29 | nd | nd | 0.54 | nd | nd | 1.0 | nd | nd |
| 95% CI of OR Quart3 | 0.32 42 | nd | nd | 0.17 29 | nd | nd | 0.055 18 | nd | nd |
| OR Quart 4 | 1.1 | nd | nd | 1.0 | nd | nd | 1.1 | nd | nd |
| p Value | 0.95 | nd | nd | 1.0 | nd | nd | 0.94 | nd | nd |
| 95% CI of OR Quart4 | 0.060 20 | nd | nd | 0.055 18 | nd | nd | 0.060 20 | nd | nd |

## Carcinoembryonic antigen-related cell adhesion molecule 5

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.72 | 3.47 | 1.72 | 3.47 | 1.72 | 2.26 |
| Average | 2.52 | 4.00 | 2.52 | 3.11 | 2.52 | 2.81 |
| Stdev | 2.90 | 3.87 | 2.90 | 1.92 | 2.90 | 1.94 |
| p(t-test) | | 0.065 | | 0.42 | | 0.77 |
| Min | 0.183 | 0.726 | 0.183 | 0.563 | 0.183 | 0.726 |
| Max | 20.8 | 17.1 | 20.8 | 5.94 | 20.8 | 5.94 |
| n (Samp) | 110 | 17 | 110 | 17 | 110 | 9 |
| n (Patient) | 110 | 17 | 110 | 17 | 110 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.88 | 2.35 | 1.88 | 2.35 | nd | nd |
| Average | 3.12 | 2.81 | 3.12 | 2.75 | nd | nd |
| Stdev | 4.09 | 2.17 | 4.09 | 2.23 | nd | nd |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.83 | | 0.80 | nd | nd |
| Min | 0.183 | 0.726 | 0.183 | 0.563 | nd | nd |
| Max | 33.6 | 5.44 | 33.6 | 5.44 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.71 | 5.01 | 1.71 | 3.72 | 1.71 | 3.47 |
| Average | 2.73 | 5.08 | 2.73 | 3.75 | 2.73 | 3.38 |
| Stdev | 3.23 | 4.31 | 3.23 | 1.66 | 3.23 | 1.82 |
| p(t-test) | | 0.030 | | 0.30 | | 0.60 |
| Min | 0.183 | 1.30 | 0.183 | 1.30 | 0.183 | 1.30 |
| Max | 20.8 | 17.1 | 20.8 | 5.94 | 20.8 | 5.94 |
| n (Samp) | 89 | 11 | 89 | 11 | 89 | 7 |
| n (Patient) | 89 | 11 | 89 | 11 | 89 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.50 | 0.78 | 0.63 | 0.48 | 0.75 | 0.60 | nd | 0.70 |
| SE | 0.076 | 0.10 | 0.086 | 0.077 | 0.11 | 0.088 | 0.10 | nd | 0.11 |
| p | 0.033 | 1.00 | 0.0012 | 0.086 | 0.83 | 0.0047 | 0.34 | nd | 0.085 |
| nCohort 1 | 110 | 180 | 89 | 110 | 180 | 89 | 110 | nd | 89 |
| nCohort 2 | 17 | 8 | 11 | 17 | 8 | 11 | 9 | nd | 7 |
| Cutoff 1 | 1.44 | 0.826 | 2.71 | 1.44 | 0.710 | 2.52 | 1.28 | nd | 2.23 |
| Sens 1 | 71% | 75% | 73% | 71% | 75% | 73% | 78% | nd | 71% |
| Spec 1 | 45% | 16% | 73% | 45% | 12% | 72% | 40% | nd | 64% |
| Cutoff 2 | 0.886 | 0.825 | 2.52 | 0.886 | 0.649 | 2.23 | 0.886 | nd | 1.49 |
| Sens 2 | 82% | 88% | 82% | 82% | 88% | 82% | 89% | nd | 86% |
| Spec 2 | 25% | 16% | 72% | 25% | 8% | 64% | 25% | nd | 46% |
| Cutoff 3 | 0.825 | 0.710 | 1.49 | 0.649 | 0.504 | 1.49 | 0.710 | nd | 1.28 |
| Sens 3 | 94% | 100% | 91% | 94% | 100% | 91% | 100% | nd | 100% |
| Spec 3 | 23% | 12% | 46% | 14% | 6% | 46% | 17% | nd | 40% |
| Cutoff 4 | 2.42 | 2.71 | 2.52 | 2.42 | 2.71 | 2.52 | 2.42 | nd | 2.52 |
| Sens 4 | 65% | 50% | 82% | 59% | 50% | 73% | 44% | nd | 57% |
| Spec 4 | 70% | 70% | 72% | 70% | 70% | 72% | 70% | nd | 72% |
| Cutoff 5 | 3.25 | 3.88 | 3.74 | 3.25 | 3.88 | 3.74 | 3.25 | nd | 3.74 |
| Sens 5 | 53% | 38% | 55% | 53% | 38% | 45% | 44% | nd | 29% |
| Spec 5 | 80% | 80% | 81% | 80% | 80% | 81% | 80% | nd | 81% |
| Cutoff 6 | 5.56 | 6.28 | 6.77 | 5.56 | 6.28 | 6.77 | 5.56 | nd | 6.77 |
| Sens 6 | 12% | 0% | 9% | 6% | 0% | 0% | 11% | nd | 0% |
| Spec 6 | 90% | 90% | 91% | 90% | 90% | 91% | 90% | nd | 91% |
| OR Quart 2 | 0.97 | 0 | >2.2 | 0.97 | 0 | >2.2 | 3.1 | nd | >2.2 |
| p Value | 0.97 | na | <0.54 | 0.97 | na | <0.54 | 0.34 | nd | <0.54 |
| 95% CI of | 0.18 | na | >0.18 | 0.18 | na | >0.18 | 0.30 | nd | >0.18 |
| OR Quart2 | 5.2 | na | na | 5.2 | na | na | 32 | nd | na |
| OR Quart 3 | 0.62 | 0 | >2.2 | 0.62 | 0 | >2.2 | 0.97 | nd | >1.0 |
| p Value | 0.62 | na | <0.54 | 0.62 | na | <0.54 | 0.98 | nd | <0.98 |
| 95% CI of | 0.097 | na | >0.18 | 0.097 | na | >0.18 | 0.058 | nd | >0.062 |
| OR Quart3 | 4.0 | na | na | 4.0 | na | na | 16 | nd | na |
| OR Quart 4 | 3.7 | 1.0 | >9.7 | 3.7 | 1.0 | >9.7 | 4.3 | nd | >4.8 |
| p Value | 0.073 | 1.0 | <0.041 | 0.073 | 1.0 | <0.041 | 0.20 | nd | <0.18 |
| 95% CI of | 0.88 | 0.23 | >1.1 | 0.88 | 0.23 | >1.1 | 0.45 | nd | >0.50 |
| OR Quart4 | 15 | 4.3 | na | 15 | 4.3 | na | 41 | nd | na |

## Myoglobin

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 48.8 | 222 | 48.8 | 145 | 48.8 | 122 |
| Average | 109 | 574 | 109 | 498 | 109 | 407 |
| Stdev | 211 | 653 | 211 | 658 | 211 | 480 |
| p(t-test) | | 4.0E-8 | | 3.3E-6 | | 4.7E-4 |
| Min | 5.55 | 16.2 | 5.55 | 16.2 | 5.55 | 16.2 |
| Max | 1720 | 1880 | 1720 | 1880 | 1720 | 1180 |
| n (Samp) | 110 | 17 | 110 | 17 | 110 | 9 |
| n (Patient) | 110 | 17 | 110 | 17 | 110 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 56.7 | 475 | 56.7 | 475 | nd | nd |
| Average | 143 | 694 | 143 | 683 | nd | nd |
| Stdev | 283 | 718 | 283 | 729 | nd | nd |
| p(t-test) | | 2.1E-6 | | 3.5E-6 | nd | nd |
| Min | 5.55 | 16.2 | 5.55 | 16.2 | nd | nd |
| Max | 2130 | 1880 | 2130 | 1880 | nd | nd |
| n (Samp) | 180 | 8 | 180 | 8 | nd | nd |
| n (Patient) | 180 | 8 | 180 | 8 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 46.2 | 222 | 46.2 | 145 | 46.2 | 122 |
| Average | 120 | 547 | 120 | 438 | 120 | 352 |
| Stdev | 223 | 581 | 223 | 574 | 223 | 423 |
| p(t-test) | | 6.9E-6 | | 5.8E-4 | | 0.016 |
| Min | 5.55 | 86.7 | 5.55 | 69.7 | 5.55 | 69.7 |
| Max | 1720 | 1660 | 1720 | 1660 | 1720 | 1160 |
| n (Samp) | 89 | 11 | 89 | 11 | 89 | 7 |
| n (Patient) | 89 | 11 | 89 | 11 | 89 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.75 | 0.86 | 0.76 | 0.68 | 0.82 | 0.76 | nd | 0.79 |
| SE | 0.064 | 0.10 | 0.072 | 0.071 | 0.11 | 0.080 | 0.095 | nd | 0.10 |
| p | 5.2E-7 | 0.016 | 3.9E-7 | 3.1E-4 | 0.096 | 8.3E-5 | 0.0061 | nd | 0.0048 |
| nCohort 1 | 110 | 180 | 89 | 110 | 180 | 89 | 110 | nd | 89 |
| nCohort 2 | 17 | 8 | 11 | 17 | 8 | 11 | 9 | nd | 7 |
| Cutoff 1 | 124 | 101 | 138 | 101 | 30.6 | 105 | 79.5 | nd | 87.6 |
| Sens 1 | 71% | 75% | 73% | 71% | 75% | 73% | 78% | nd | 71% |
| Spec 1 | 82% | 69% | 80% | 77% | 32% | 75% | 71% | nd | 69% |
| Cutoff 2 | 101 | 34.3 | 124 | 66.9 | 19.2 | 101 | 66.9 | nd | 79.5 |
| Sens 2 | 82% | 88% | 82% | 82% | 88% | 82% | 89% | nd | 86% |
| Spec 2 | 77% | 35% | 78% | 62% | 19% | 74% | 62% | nd | 67% |
| Cutoff 3 | 33.6 | 15.7 | 108 | 19.2 | 15.7 | 87.6 | 15.0 | nd | 66.9 |
| Sens 3 | 94% | 100% | 91% | 94% | 100% | 91% | 100% | nd | 100% |
| Spec 3 | 38% | 12% | 76% | 21% | 12% | 69% | 14% | nd | 61% |
| Cutoff 4 | 78.9 | 105 | 97.8 | 78.9 | 105 | 97.8 | 78.9 | nd | 97.8 |
| Sens 4 | 88% | 62% | 91% | 76% | 62% | 82% | 78% | nd | 57% |
| Spec 4 | 70% | 70% | 71% | 70% | 70% | 71% | 70% | nd | 71% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 5 | 112 | 150 | 146 | 112 | 150 | 146 | 112 | nd | 146 |
| Sens 5 | 71% | 62% | 64% | 59% | 62% | 45% | 56% | nd | 43% |
| Spec 5 | 80% | 80% | 81% | 80% | 80% | 81% | 80% | nd | 81% |
| Cutoff 6 | 212 | 315 | 323 | 212 | 315 | 323 | 212 | nd | 323 |
| Sens 6 | 53% | 50% | 36% | 41% | 50% | 27% | 44% | nd | 29% |
| Spec 6 | 90% | 90% | 91% | 90% | 90% | 91% | 90% | nd | 91% |
| OR Quart 2 | 0.97 | 1.0 | >0 | 0.47 | 0.49 | >0 | 0 | nd | >0 |
| p Value | 0.98 | 1.0 | <na | 0.54 | 0.56 | <na | na | nd | <na |
| 95% CI of | 0.058 | 0.061 | >na | 0.040 | 0.043 | >na | na | nd | >na |
| OR Quart2 | 16 | 16 | na | 5.4 | 5.6 | na | na | nd | na |
| OR Quart 3 | 3.1 | 1.0 | >4.8 | 2.1 | 0 | >6.2 | 3.1 | nd | >4.8 |
| p Value | 0.34 | 1.0 | <0.18 | 0.42 | na | <0.11 | 0.34 | nd | <0.18 |
| 95% CI of | 0.30 | 0.061 | >0.49 | 0.35 | na | >0.67 | 0.30 | nd | >0.50 |
| OR Quart3 | 32 | 16 | na | 12 | na | na | 32 | nd | na |
| OR Quart 4 | 18 | 5.5 | >9.7 | 6.6 | 2.7 | >7.9 | 5.6 | nd | >3.4 |
| p Value | 0.0075 | 0.13 | <0.041 | 0.022 | 0.25 | <0.066 | 0.13 | nd | <0.30 |
| 95% CI of | 2.2 | 0.61 | >1.1 | 1.3 | 0.49 | >0.88 | 0.61 | nd | >0.33 |
| OR Quart4 | 150 | 49 | na | 33 | 15 | na | 51 | nd | na |

## Mucin-16

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.17 | 8.40 | 1.17 | 7.52 | nd | nd |
| Average | 3.77 | 31.2 | 3.77 | 30.6 | nd | nd |
| Stdev | 15.1 | 67.4 | 15.1 | 67.6 | nd | nd |
| p(t-test) |  | 0.0035 |  | 0.0043 | nd | nd |
| Min | 0.117 | 2.02 | 0.117 | 2.02 | nd | nd |
| Max | 131 | 198 | 131 | 198 | nd | nd |
| n (Samp) | 75 | 8 | 75 | 8 | nd | nd |
| n (Patient) | 75 | 8 | 75 | 8 | nd | nd |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.36 | 7.47 | 1.36 | 7.47 | nd | nd |
| Average | 4.38 | 38.3 | 4.38 | 38.3 | nd | nd |
| Stdev | 16.6 | 78.2 | 16.6 | 78.2 | nd | nd |
| p(t-test) |  | 0.0042 |  | 0.0042 | nd | nd |
| Min | 0.122 | 2.02 | 0.122 | 2.02 | nd | nd |
| Max | 131 | 198 | 131 | 198 | nd | nd |
| n (Samp) | 62 | 6 | 62 | 6 | nd | nd |
| n (Patient) | 62 | 6 | 62 | 6 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.89 | nd | 0.85 | 0.88 | nd | 0.85 | nd | nd | nd |
| SE | 0.079 | nd | 0.100 | 0.080 | nd | 0.100 | nd | nd | nd |
| p | 9.8E-7 | nd | 3.7E-4 | 1.6E-6 | nd | 3.7E-4 | nd | nd | nd |
| nCohort 1 | 75 | nd | 62 | 75 | nd | 62 | nd | nd | nd |
| nCohort 2 | 8 | nd | 6 | 8 | nd | 6 | nd | nd | nd |
| Cutoff 1 | 4.24 | nd | 2.09 | 4.24 | nd | 2.09 | nd | nd | nd |
| Sens 1 | 75% | nd | 83% | 75% | nd | 83% | nd | nd | nd |
| Spec 1 | 88% | nd | 68% | 88% | nd | 68% | nd | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 2 | 2.09 | nd | 2.09 | 2.09 | nd | 2.09 | nd | nd | nd |
| Sens 2 | 88% | nd | 83% | 88% | nd | 83% | nd | nd | nd |
| Spec 2 | 69% | nd | 68% | 69% | nd | 68% | nd | nd | nd |
| Cutoff 3 | 1.96 | nd | 1.96 | 1.96 | nd | 1.96 | nd | nd | nd |
| Sens 3 | 100% | nd | 100% | 100% | nd | 100% | nd | nd | nd |
| Spec 3 | 67% | nd | 66% | 67% | nd | 66% | nd | nd | nd |
| Cutoff 4 | 2.20 | nd | 2.56 | 2.20 | nd | 2.56 | nd | nd | nd |
| Sens 4 | 75% | nd | 67% | 75% | nd | 67% | nd | nd | nd |
| Spec 4 | 72% | nd | 71% | 72% | nd | 71% | nd | nd | nd |
| Cutoff 5 | 2.98 | nd | 3.41 | 2.98 | nd | 3.41 | nd | nd | nd |
| Sens 5 | 75% | nd | 67% | 75% | nd | 67% | nd | nd | nd |
| Spec 5 | 80% | nd | 81% | 80% | nd | 81% | nd | nd | nd |
| Cutoff 6 | 5.21 | nd | 6.93 | 5.21 | nd | 6.93 | nd | nd | nd |
| Sens 6 | 62% | nd | 50% | 62% | nd | 50% | nd | nd | nd |
| Spec 6 | 91% | nd | 90% | 91% | nd | 90% | nd | nd | nd |
| OR Quart 2 | >0 | nd | >0 | >0 | nd | >0 | nd | nd | nd |
| p Value | <na | nd | <na | <na | nd | <na | nd | nd | nd |
| 95% CI of OR Quart2 | >na na | nd | >na na | >na na | nd | >na na | nd | nd | nd |
| OR Quart 3 | >2.1 | nd | >2.3 | >2.1 | nd | >2.3 | nd | nd | nd |
| p Value | <0.56 | nd | <0.52 | <0.56 | nd | <0.52 | nd | nd | nd |
| 95% CI of OR Quart3 | >0.18 na | nd | >0.19 na | >0.18 na | nd | >0.19 na | nd | nd | nd |
| OR Quart 4 | >8.0 | nd | >5.2 | >8.0 | nd | >5.2 | nd | nd | nd |
| p Value | <0.066 | nd | <0.16 | <0.066 | nd | <0.16 | nd | nd | nd |
| 95% CI of OR Quart4 | >0.87 na | nd | >0.52 na | >0.87 na | nd | >0.52 na | nd | nd | nd |

## Tumor necrosis factor receptor superfamily member 10B

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.00E-9 | 0.0138 | 1.00E-9 | 0.0129 | nd | nd |
| Average | 0.00697 | 0.0265 | 0.00697 | 0.0259 | nd | nd |
| Stdev | 0.0202 | 0.0355 | 0.0202 | 0.0359 | nd | nd |
| p(t-test) |  | 0.020 |  | 0.024 | nd | nd |
| Min | 1.00E-9 | 1.00E-9 | 1.00E-9 | 1.00E-9 | nd | nd |
| Max | 0.114 | 0.108 | 0.114 | 0.108 | nd | nd |
| n (Samp) | 72 | 8 | 72 | 8 | nd | nd |
| n (Patient) | 72 | 8 | 72 | 8 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.81 | nd | nd | 0.79 | nd | nd | nd | nd | nd |
| SE | 0.095 | nd | nd | 0.098 | nd | nd | nd | nd | nd |
| p | 0.0012 | nd | nd | 0.0031 | nd | nd | nd | nd | nd |
| nCohort 1 | 72 | nd | nd | 72 | nd | nd | nd | nd | nd |
| nCohort 2 | 8 | nd | nd | 8 | nd | nd | nd | nd | nd |
| Cutoff 1 | 0.00462 | nd | nd | 0.00438 | nd | nd | nd | nd | nd |
| Sens 1 | 75% | nd | nd | 75% | nd | nd | nd | nd | nd |
| Spec 1 | 79% | nd | nd | 79% | nd | nd | nd | nd | nd |
| Cutoff 2 | 0.00438 | nd | nd | 0.00207 | nd | nd | nd | nd | nd |
| Sens 2 | 88% | nd | nd | 88% | nd | nd | nd | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 2 | 79% | nd | nd | 65% | nd | nd | nd | nd | nd |
| Cutoff 3 | 0 | nd | nd | 0 | nd | nd | nd | nd | nd |
| Sens 3 | 100% | nd | nd | 100% | nd | nd | nd | nd | nd |
| Spec 3 | 0% | nd | nd | 0% | nd | nd | nd | nd | nd |
| Cutoff 4 | 0.00274 | nd | nd | 0.00274 | nd | nd | nd | nd | nd |
| Sens 4 | 88% | nd | nd | 75% | nd | nd | nd | nd | nd |
| Spec 4 | 71% | nd | nd | 71% | nd | nd | nd | nd | nd |
| Cutoff 5 | 0.00507 | nd | nd | 0.00507 | nd | nd | nd | nd | nd |
| Sens 5 | 62% | nd | nd | 50% | nd | nd | nd | nd | nd |
| Spec 5 | 81% | nd | nd | 81% | nd | nd | nd | nd | nd |
| Cutoff 6 | 0.0124 | nd | nd | 0.0124 | nd | nd | nd | nd | nd |
| Sens 6 | 50% | nd | nd | 50% | nd | nd | nd | nd | nd |
| Spec 6 | 90% | nd | nd | 90% | nd | nd | nd | nd | nd |
| OR Quart 2 | >1.1 | nd | nd | >1.1 | nd | nd | nd | nd | nd |
| p Value | <0.97 | nd | nd | <0.97 | nd | nd | nd | nd | nd |
| 95% CI of | >0.061 | nd | nd | >0.061 | nd | nd | nd | nd | nd |
| OR Quart2 | na | nd | nd | na | nd | nd | nd | nd | nd |
| OR Quart 3 | >2.2 | nd | nd | >2.2 | nd | nd | nd | nd | nd |
| p Value | <0.53 | nd | nd | <0.53 | nd | nd | nd | nd | nd |
| 95% CI of | >0.19 | nd | nd | >0.19 | nd | nd | nd | nd | nd |
| OR Quart3 | na | nd | nd | na | nd | nd | nd | nd | nd |
| OR Quart 4 | >6.7 | nd | nd | >6.7 | nd | nd | nd | nd | nd |
| p Value | <0.098 | nd | nd | <0.098 | nd | nd | nd | nd | nd |
| 95% CI of | >0.70 | nd | nd | >0.70 | nd | nd | nd | nd | nd |
| OR Quart4 | na | nd | nd | na | nd | nd | nd | nd | nd |

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

**Claims**

1. A method for evaluating renal status in a subject, comprising:

    performing one or more assaysconfigured to detect one or more biomarkers, the one or more biomarkers comprising Tumor necrosis factor receptor superfamily member 10B, on a body fluid sample obtained from the subject to provide an assay result; and
    correlating the assay result(s) to the renal status of the subject.

2. A method according to claim 1, wherein the one or more biomarkers further comprise one or more of Cadherin-16, Caspase-9, Bcl2 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53.

3. A method according to claim 1 or 2, wherein said correlation step comprises

(i) correlating the assay result(s) to one or more of risk stratification, diagnosis, staging, classifying and monitoring of the renal status of the subject or

(ii) assigning a likelihood of one or more future changes in renal status to the subject based on the assay result(s).

4. A method according to claim 1 or 2, wherein said one or more future changes in renal status comprise one or more of a future injury to renal function, future reduced renal function, future improvement in renal function, and future acute renal failure (ARF).

5. A method according to claim 1 or 2, wherein the subject is not in acute renal failure.

6. A method according to claim 1 or 2, wherein the subject has not experienced a 1.5-fold or greater increase in serum creatinine over a baseline value determined prior to the time at which the body fluid sample is obtained, or

wherein the subject has a urine output of at least 0.5 ml/kg/hr over the 12 hours preceding the time at which the body fluid sample is obtained, or

wherein the subject has not experienced an increase of 0.3 mg/dL or greater in serum creatinine over a baseline value determined prior to the time at which the body fluid sample is obtained, or

wherein the subject (i) has not experienced a 1.5-fold or greater increase in serum creatinine over a baseline value determined prior to the time at which the body fluid sample is obtained,

(ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

7. A method according to claim 1 or 2, wherein the subject is in RIFLE stage 0 or R.

8. A method according to claim 7, wherein

(a) the subject is in RIFLE stage 0, and said correlating step comprises assigning a likelihood that the subject will reach RIFLE stage R, I or F within 72 hours, within 48 hours or within 24 hours, or

(b) the subject is in RIFLE stage 0 or R, and said correlating step comprises assigning a likelihood that the subject will reach RIFLE stage I or F within 72 hours, within 48 hours or within 24 hours.

9. A method according to claim 1 or 2, wherein the subject is in RIFLE stage 0, R, or I, and said correlating step comprises assigning a likelihood that the subject will reach RIFLE stage F within 72 hours,

wherein the subject is optionally in RIFLE stage I, and said correlating step comprises assigning a likelihood that the subject will reach RIFLE stage F within 72 hours, within 48 hours or within 24 hours.

10. A method according to claim 1 or 2, wherein said assay result(s) comprise a measured urine or plasma concentration of Tumor necrosis factor receptor superfamily member 10B, and optionally one or more of:

a measured urine or plasma concentration of Cadherin-16,
a measured urine or plasma concentration of Caspase-9,
a measured urine or plasma concentration of Bcl2 antagonist of cell death,
a measured urine or plasma concentration of Caspase-1,
a measured urine or plasma concentration of Cadherin-1,
a measured urine or plasma concentration of Poly [ADP-ribose] polymerase 1,
a measured urine or plasma concentration of Cyclin-dependent kinase inhibitor 1,
a measured urine or plasma concentration of Cadherin-5,
a measured urine or plasma concentration of Myoglobin,
a measured urine or plasma concentration of Apolipoprotein A-II,
a measured urine or plasma concentration of Mucin-16,
a measured urine or plasma concentration of Carcinoembryonic antigen-related cell adhesion molecule 5, or
a measured urine or plasma concentration of Cellular tumor antigen p53 and said correlation step comprises comparing each measured concentration to a corresponding threshold concentration, and

(A) for a positive going marker, assigning an increased likelihood of progression to a worsening RIFLE stage to the subject, relative to the subject's current RIFLE stage, when the measured concentration is above the threshold, or assigning a decreased likelihood of progressing to a worsening RIFLE stage to the subject, relative to the subject's current RIFLE stage, when the measured concentration is below the threshold, or

for a negative going marker, assigning a decreased likelihood of progression to a worsening RIFLE stage to the subject, relative to the subject's current RIFLE stage, when the measured concentration is above the threshold, or assigning an increased likelihood of progressing to a worsening RIFLE stage to the subject, relative to the subject's current RIFLE stage, when the measured concentration is below the threshold, or (B) for a positive going marker, assigning an increased likelihood of progressing to a need for renal replacement therapy to the subject when the measured concentration is above the threshold, or assigning a decreased likelihood of progressing to a need for renal replacement therapy when the measured concentration is below the threshold, or

for a negative going marker, assigning a decreased likelihood of progressing to a need for renal replacement therapy to the subject when the measured concentration is above the threshold, or assigning an increased likelihood of progressing to a need for renal replacement therapy when the measured concentration is below the threshold.

11. A method according to claim 5, wherein said assay result(s) comprise a measured urine or plasma concentration of Tumor necrosis factor receptor superfamily member 10B, and optionally one or more of:

> a measured urine or plasma concentration of Cadherin-16,
> a measured urine or plasma concentration of Caspase-9,
> a measured urine or plasma concentration of Bc12 antagonist of cell death,
> a measured urine or plasma concentration of Caspase-1,
> a measured urine or plasma concentration of Cadherin-1,
> a measured urine or plasma concentration of Poly [ADP-ribose] polymerase 1,
> a measured urine or plasma concentration of Cyclin-dependent kinase inhibitor 1,
> a measured urine or plasma concentration of Cadherin-5,
> a measured urine or plasma concentration of Myoglobin,
> a measured urine or plasma concentration of Apolipoprotein A-II,
> a measured urine or plasma concentration of Mucin-16,
> a measured urine or plasma concentration of Carcinoembryonic antigen-related cell adhesion molecule 5, or
> a measured urine or plasma concentration of Cellular tumor antigen p53 and said correlation step comprises comparing each measured concentration to a corresponding threshold concentration, and
> for a positive going marker, assigning an increased likelihood of progressing to acute renal failure when the measured concentration is above the threshold, or assigning a decreased likelihood of progressing to acute renal failure to the subject when the measured concentration is below the threshold, or
> for a negative going marker, assigning a decreased likelihood of progressing to acute renal failure when the measured concentration is above the threshold, or assigning an increased likelihood of progressing to acute renal failure to the subject when the measured concentration is below the threshold.

12. A method according to claim 8, wherein said assay result(s) comprise a measured urine or plasma concentration of Tumor necrosis factor receptor superfamily member 10B, and optionally one or more of:

> a measured urine or plasma concentration of Cadherin-16,
> a measured urine or plasma concentration of Caspase-9,
> a measured urine or plasma concentration of Bc12 antagonist of cell death,
> a measured urine or plasma concentration of Caspase-1,
> a measured urine or plasma concentration of Cadherin-1,
> a measured urine or plasma concentration of Poly [ADP-ribose] polymerase 1,
> a measured urine or plasma concentration of Cyclin-dependent kinase inhibitor 1,
> a measured urine or plasma concentration of Cadherin-5,
> a measured urine or plasma concentration of Myoglobin,
> a measured urine or plasma concentration of Apolipoprotein A-II,
> a measured urine or plasma concentration of Mucin-16,
> a measured urine or plasma concentration of Carcinoembryonic antigen-related cell adhesion molecule 5, or
> a measured urine or plasma concentration of Cellular tumor antigen p53 and said correlation step comprises comparing each measured concentration to a corresponding threshold concentration, and
> for a positive going marker, assigning an increased likelihood of progressing to RIFLE stage R, I or F to the subject, when the measured concentration is above the threshold, or assigning a decreased likelihood of progressing to RIFLE stage R, I or F to the subject when the measured concentration is below the threshold, or for a negative going marker, assigning a decreased likelihood of progressing to RIFLE stage R, I or F to the subject, when the measured concentration is above the threshold, or assigning an increased likelihood of progressing to RIFLE stage R, I or F to the subject when the measured concentration is below the threshold.

13. A method according to claim 9, wherein said assay result(s) comprise a measured urine or plasma concentration of Tumor necrosis factor receptor superfamily member 10B, and optionally one or more of:

> a measured urine or plasma concentration of Cadherin-16,
> a measured urine or plasma concentration of Caspase-9,
> a measured urine or plasma concentration of Bc12 antagonist of cell death,
> a measured urine or plasma concentration of Caspase-1,
> a measured urine or plasma concentration of Cadherin-1,
> a measured urine or plasma concentration of Poly [ADP-ribose] polymerase 1,
> a measured urine or plasma concentration of Cyclin-dependent kinase inhibitor 1,
> a measured urine or plasma concentration of Cadherin-5,
> a measured urine or plasma concentration of Myoglobin,
> a measured urine or plasma concentration of Apolipoprotein A-II,
> a measured urine or plasma concentration of Mucin-16,
> a measured urine or plasma concentration of Carcinoembryonic antigen-related cell adhesion molecule 5, or
> a measured urine or plasma concentration of Cellular tumor antigen p53 and said correlation step comprises comparing each measured concentration to a corresponding threshold concentration, and
> for a positive going marker, assigning an increased likelihood of progressing to RIFLE stage F to the subject, when the measured concentration is above the threshold, or assigning a decreased likelihood of progressing to RIFLE stage F to the subject when the measured concentration is below the threshold, or
> for a negative going marker, assigning a decreased likelihood of progressing to RIFLE stage F to the subject, when the measured concentration is above the threshold, or assigning an increased likelihood of progressing to RIFLE stage F to the subject when the measured concentration is below the threshold

14. A method according to claim 1 or 2, wherein the subject is selected for evaluation of renal status (i) based on the pre-existence in the subject of one or more known risk factors for prerenal, intrinsic renal, or postrenal ARF, or based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

15. The use of one or more biomarkers comprising Tumor necrosis factor receptor superfamily member 10B, and optionally one or more of Cadherin-16, Caspase-9, Bc12 antagonist of cell death, Caspase-1, Cadherin-1, Poly [ADP-ribose] polymerase 1, Cyclin-dependent kinase inhibitor 1, Cadherin-5, Myoglobin, Apolipoprotein A-II, Mucin-16, Carcinoembryonic antigen-related cell adhesion molecule 5, and Cellular tumor antigen p53 for

> (i) the diagnosis, risk stratification, prognosis, classifying and monitoring of renal status of a subject not in acute renal failure or
> (ii) for assigning an increased likelihood of progressing to a worsening RIFLE stage to a subject, relative to the subject's current RIFLE stage.

**Patentansprüche**

1. Verfahren zur Evaluierung des Nierenstatus eines Subjekts, umfassend:

> Durchführen einer oder mehrerer Untersuchung(en), die dafür konfiguriert sind, einen oder mehrere Biomarker, wobei der eine oder die mehreren Biomarker Mitglied 10B der Tumor-Nekrose-Faktor-Rezeptor-Superfamilie umfasst, in einer Körperflüssigkeitsprobe, die von dem Subjekt erhalten wurde, nachzuweisen, um ein Untersuchungsergebnis bereitzustellen;
> und

Korrelieren des/der Untersuchungsergebnisse(s) mit dem Nierenstatus des Subjekts.

2. Verfahren nach Anspruch 1, wobei der eine oder die mehreren Biomarker ferner einen oder mehrere aus Cadherin-16, Caspase-9, Bc12 Zelltodantagonist, Caspase-1, Cadherin-1, Poly[ADP-ribose] Polymerase 1, Cyclin-abhängigen Kinase Inhibitor 1, Cadherin-5, Myoglobin, Apolipoprotein A-II, Mucin-16, carcinoembryonalen Antigen-ähnlichen Zelladhäsions-Molekül 5 und zellulären Tumorantigen p53 umfasst.

3. Verfahren nach Anspruch 1 oder 2, wobei der Korrelationsschritt umfasst,

(i) Korrelieren des/der Untersuchungsergebnis(se) mit einem oder mehreren aus Risiko-Stratifizierung, Diagnose, Einstufen (Staging), Klassifizieren und Überwachen des Nierenstatus des Subjekts oder
(ii) Zuweisen einer Wahrscheinlichkeit, basierend auf dem/den Untersuchungsergebnis(sen), von einer oder mehreren zukünftigen Veränderung(en) des Nierenstatus zu dem Subjekt.

4. Verfahren nach Anspruch 1 oder 2, wobei die eine oder die mehreren zukünftige(n) Veränderung(en) des Nierenstatus ein oder mehrere aus einer zukünftigen Schädigung der Nierenfunktion, zukünftiger reduzierter Nierenfunktion, zukünftiger Verbesserung der Nierenfunktion und zukünftiges akutes Nierenversagen (ARF) umfasst.

5. Verfahren nach Anspruch 1 oder 2, wobei das Subjekt kein akutes Nierenversagen hat.

6. Verfahren nach Anspruch 1 oder 2, wobei das Subjekt keine 1,5-fache oder größere Zunahme des Serum-Kreatinins über einen Basiswert, der vor dem Zeitpunkt, bei dem die Körperflüssigkeitsprobe erhalten wurde, bestimmt wurde, erfahren hat, oder
wobei das Subjekt eine Urinausscheidung von mindestens 0,5 ml/kg/h über 12 Stunden vor dem Zeitpunkt, an dem die Körperflüssigkeitsprobe erhalten wurde, hat oder
wobei das Subjekt keine Zunahme von 0,3 mg/dL oder mehr des Serum-Kreatinins über einen Basiswert, der vor dem Zeitpunkt, an dem die Körperflüssigkeitsprobe erhalten wurde, bestimmt wurde, erfahren hat oder
wobei das Subjekt (i) keine 1,5-fache oder größere Zunahme des Serum-Kreatinins über einen Basiswert, der vor dem Zeitpunkt, an dem die Körperflüssigkeitsprobe erhalten wurde, bestimmt wurde, erfahren hat (ii) eine Urinausscheidung von mindestens 0,5 ml/kg/h über 12 Stunden vor dem Zeitpunkt, an dem die Körperflüssigkeitsprobe erhalten wurde, hat und (iii) keine Zunahme von 0,3 mg/dL oder mehr des Serum-Kreatinins über einen Basiswert, der vor dem Zeitpunkt, an dem die Körperflüssigkeitsprobe erhalten wurde, bestimmt wurde, erfahren hat.

7. Verfahren nach Anspruch 1 oder 2, wobei das Subjekt sich in RIFLE Stadium 0 oder R befindet.

8. Verfahren nach Anspruch 7, wobei

(a) das Subjekt sich in RIFLE Stadium 0 befindet, und der Korrelationsschritt das Zuweisen einer Wahrscheinlichkeit, dass das Subjekt RIFLE Stadium R, I oder F innerhalb von 72 Stunden, innerhalb von 48 Stunden oder innerhalb, 24 Stunden erreichen wird, umfasst oder
(b) das Subjekt sich in RIFLE Stadium 0 oder R befindet, und der Korrelationsschritt das Zuweisen einer Wahrscheinlichkeit, dass das Subjekt RIFLE Stadium I oder F innerhalb von 72 Stunden innerhalb von 48 Stunden oder innerhalb von 24 Stunden erreichen wird, umfasst.

9. Verfahren nach Anspruch 1 oder 2, wobei das Subjekt in RIFLE Stadium 0, R oder I ist, und der Korrelationsschritt das Zuweisen einer Wahrscheinlichkeit, dass das Subjekt RIFLE Stadium F innerhalb von 72 Stunden erreichen wird, umfasst
wobei das Subjekt sich gegebenenfalls in RIFLE Stadium I befindet und der Korrelationsschritt das Zuweisen einer Wahrscheinlichkeit, dass das Subjekt RIFLE Stadium F innerhalb von 72 Stunden innerhalb von 48 Stunden oder innerhalb von 24 Stunden erreichen wird, umfasst.

10. Verfahren nach Anspruch 1 oder 2, wobei das/die Untersuchungsergebnis(se) eine gemessene Urin- oder Plasmakonzentration von Mitglied 10B der Tumor-Nekrose-Faktor-Rezeptor-Superfamilie umfasst, und gegebenenfalls ein oder mehrere aus:

einer gemessenen Urin- oder Plasmakonzentration von Cadherin-16,
einer gemessenen Urin- oder Plasmakonzentration von Caspase-9,
einer gemessenen Urin- oder Plasmakonzentration von dem Bc12 Zelltodantagonist,

einer gemessenen Urin- oder Plasmakonzentration von Caspase-1,

einer gemessenen Urin- oder Plasmakonzentration von Cadherin-1,

einer gemessenen Urin- oder Plasmakonzentration von Poly [ADP-ribose] Polymerase 1,

einer gemessenen Urin- oder Plasmakonzentration von dem Cyclin-abhängigen Kinase Inhibitor 1,

einer gemessenen Urin- oder Plasmakonzentration von Cadherin-5,

einer gemessenen Urin- oder Plasmakonzentration von Myoglobin,

einer gemessenen Urin- oder Plasma-Konzentration von Apolipoprotein A-II,

einer gemessenen Urin- oder Plasmakonzentration von Mucin-16,

einer gemessenen Urin- oder Plasmakonzentration von dem carcinoembryonalen Antigen-ähnlichen Zelladhäsions-Molekül 5 oder

einer gemessenen Urin- oder Plasmakonzentration von zellulärem Tumorantigen p53

und der Korrelationsschritt das Vergleichen jeder gemessenen Konzentration mit einer entsprechenden Schwellenwertkonzentration umfasst und

(A) bei einem positiver werdenden Marker, wenn die gemessene Konzentration über dem Schwellenwert liegt, das Zuweisen einer erhöhten Wahrscheinlichkeit einer Entwicklung einer Verschlechterung des RIFLE Stadiums zu dem Subjekt, im Vergleich zu dem aktuellen RIFLE Stadium des Subjekts, oder das Zuweisen einer verringerten Wahrscheinlichkeit einer Entwicklung einer Verschlechterung des RIFLE Stadiums zu dem Subjekt, im Vergleich zu dem aktuellen RIFLE Stadium des Subjekts, wenn die gemessene Konzentration unter dem Schwellenwert liegt, oder bei einem negativer werdenden Marker, wenn die gemessene Konzentration über dem Schwellenwert liegt, das Zuweisen einer verringerten Wahrscheinlichkeit einer Entwicklung einer Verschlechterung des RIFLE Stadiums zu dem Subjekt, im Vergleich zu dem aktuellen RIFLE Stadium des Subjekts, oder das Zuweisen einer erhöhten Wahrscheinlichkeit einer Entwicklung einer Verschlechterung des RIFLE Stadiums zu dem Subjekt, im Vergleich zu dem aktuellen RIFLE Stadium des Subjekts, wenn die gemessene Konzentration unter dem Schwellenwert liegt, oder

(B) bei einem positiver werdenden Marker, wenn die gemessene Konzentration über dem Schwellenwert liegt, das Zuweisen einer erhöhten Wahrscheinlichkeit einer Entwicklung des Bedarfs einer Nierenersatztherapie zu dem Subjekt, oder das Zuweisen einer verringerten Wahrscheinlichkeit einer Entwicklung des Bedarfs einer Nierenersatztherapie, wenn die gemessene Konzentration unter dem Schwellenwert liegt, oder

bei einem negativer werdenden Marker, wenn die gemessene Konzentration über dem Schwellenwert liegt, das Zuweisen einer verringerten Wahrscheinlichkeit einer Entwicklung des Bedarfs einer Nierenersatztherapie zu dem Subjekt oder das Zuweisen einer erhöhten Wahrscheinlichkeit einer Entwicklung des Bedarfs einer Nierenersatztherapie, wenn die gemessene Konzentration unter dem Schwellenwert liegt.

11. Verfahren nach Anspruch 5, wobei das/die Untersuchungsergebnis(se) eine gemessene Urin- oder Plasmakonzentration von Mitglied 10B der Tumor-Nekrose-Faktor-Rezeptor-Superfamilie, umfasst und gegebenenfalls ein oder mehrere aus:

einer gemessenen Urin- oder Plasmakonzentration von Cadherin-16,

einer gemessenen Urin- oder Plasmakonzentration von Caspase-9,

einer gemessenen Urin- oder Plasmakonzentration von dem Bc12 Zelltodantagonist,

einer gemessenen Urin- oder Plasmakonzentration von Caspase-1,

einer gemessenen Urin- oder Plasmakonzentration von Cadherin-1,

einer gemessenen Urin- oder Plasmakonzentration von Poly [ADP-ribose] Polymerase 1,

einer gemessenen Urin- oder Plasmakonzentration von dem Cyclin-abhängigen Kinase Inhibitor 1,

einer gemessenen Urin- oder Plasmakonzentration von Cadherin-5,

einer gemessenen Urin- oder Plasmakonzentration von Myoglobin,

einer gemessenen Urin- oder Plasma-Konzentration von Apolipoprotein A-II,

einer gemessenen Urin- oder Plasmakonzentration von Mucin-16,

einer gemessenen Urin- oder Plasmakonzentration von dem carcinoembryonalen Antigen-ähnlichen Zelladhäsions-Molekül 5 oder

einer gemessenen Urin- oder Plasmakonzentration von zellulärem Tumorantigen p53

und der Korrelationsschritt das Vergleichen jeder gemessenen Konzentration mit einer entsprechenden Schwellenwertkonzentration umfasst, und

bei einem positiver werdenden Marker, wenn die gemessene Konzentration über dem Schwellenwert liegt, das Zuweisen einer erhöhten Wahrscheinlichkeit der Entwicklung eines akuten Nierenversagens oder das Zuweisen einer verringerten Wahrscheinlichkeit der Entwicklung eines akuten Nierenversagens zu dem Subjekt, wenn die gemessene Konzentration unter dem Schwellenwert liegt, oder
bei einem negativer werdenden Marker, wenn die gemessene Konzentration über dem Schwellenwert liegt, das Zuweisen einer verringerten Wahrscheinlichkeit der Entwicklung eines akuten Nierenversagens oder das Zuweisen einer erhöhten Wahrscheinlichkeit der Entwicklung eines akuten Nierenversagens zu dem Subjekt, wenn die gemessene Konzentration unter dem Schwellenwert liegt.

12. Verfahren nach Anspruch 8, wobei das/die Untersuchungsergebnis(se) eine gemessene Urin- oder Plasmakonzentration von Mitglied 10B der Tumor-Nekrose-Faktor-Rezeptor-Superfamilie,
und gegebenenfalls ein oder mehrere aus:

einer gemessenen Urin- oder Plasmakonzentration von Cadherin-16,
einer gemessenen Urin- oder Plasmakonzentration von Caspase-9,
einer gemessenen Urin- oder Plasmakonzentration von dem Bc12 Zelltodantagonist,
einer gemessenen Urin- oder Plasmakonzentration von Caspase-1,
einer gemessenen Urin- oder Plasmakonzentration von Cadherin-1,
einer gemessenen Urin- oder Plasmakonzentration von Poly [ADP-ribose] Polymerase 1,
einer gemessenen Urin- oder Plasmakonzentration von dem Cyclin-abhängigen Kinase Inhibitor 1,
einer gemessenen Urin- oder Plasmakonzentration von Cadherin-5,
einer gemessenen Urin- oder Plasmakonzentration von Myoglobin,
einer gemessenen Urin- oder Plasma-Konzentration von Apolipoprotein A-II,
einer gemessenen Urin- oder Plasmakonzentration von Mucin-16,
einer gemessenen Urin- oder Plasmakonzentration von dem carcinoembryonalen Antigen-ähnlichen Zelladhäsions-Molekül 5 oder
einer gemessenen Urin- oder Plasmakonzentration von zellulärem Tumorantigen p53
und der Korrelationsschritt das Vergleichen jeder gemessenen Konzentration mit einer entsprechenden Schwellenwertkonzentration umfasst, und
bei einem positiver werdenden Marker, wenn die gemessene Konzentration über dem Schwellenwert liegt, das Zuweisen einer erhöhten Wahrscheinlichkeit einer Entwicklung zu RIFLE Stadium R, I oder F zu dem Subjekt, oder das Zuweisen einer verringerten Wahrscheinlichkeit einer Entwicklung zu RIFLE Stadium R, I oder F zu dem Subjekt, wenn die gemessene Konzentration unter dem Schwellenwert liegt, oder bei einem negativer werdenden Marker, wenn die gemessene Konzentration über dem Schwellenwert liegt, das Zuweisen einer verringerten Wahrscheinlichkeit einer Entwicklung zu RIFLE Stadium R, I oder F zu dem Subjekt, oder das Zuweisen einer erhöhten Wahrscheinlichkeit einer Entwicklung zu RIFLE Stadium R, I oder F zu dem Subjekt, wenn die gemessene Konzentration unter dem Schwellenwert liegt.

13. Verfahren nach Anspruch 9, wobei das/die Untersuchungsergebnis(se) eine gemessene Konzentration der Urin- oder Plasmakonzentration von Mitglied 10B der Tumor-Nekrose-Faktor-Rezeptor-Superfamilie umfasst
und gegebenenfalls ein oder mehrere aus:

einer gemessenen Urin- oder Plasmakonzentration von Cadherin-16,
einer gemessenen Urin- oder Plasmakonzentration von Caspase-9,
einer gemessenen Urin- oder Plasmakonzentration von dem Bc12 Zelltodantagonist,
einer gemessenen Urin- oder Plasmakonzentration von Caspase-1,
einer gemessenen Urin- oder Plasmakonzentration von Cadherin-1,
einer gemessenen Urin- oder Plasmakonzentration von Poly [ADP-ribose] Polymerase 1,
einer gemessenen Urin- oder Plasmakonzentration von dem Cyclin-abhängigen Kinase Inhibitor 1,
einer gemessenen Urin- oder Plasmakonzentration von Cadherin-5,
einer gemessenen Urin- oder Plasmakonzentration von Myoglobin,
einer gemessenen Urin- oder Plasma-Konzentration von Apolipoprotein A-II,
einer gemessenen Urin- oder Plasmakonzentration von Mucin-16,
einer gemessenen Urin- oder Plasmakonzentration von dem carcinoembryonalen Antigen-ähnlichen Zelladhäsions-Molekül 5 oder
einer gemessenen Urin- oder Plasmakonzentration von zellulärem Tumorantigen p53
und der Korrelationsschritt das Vergleichen jeder gemessenen Konzentration mit einer entsprechenden Schwellenwertkonzentration umfasst, und
bei einem positiver werdenden Marker, wenn die gemessene Konzentration über dem Schwellenwert liegt, das Zuweisen einer erhöhten Wahrscheinlichkeit einer Entwicklung zu RIFLE Stadium F zu dem Subjekt, oder das Zuweisen einer verringerten Wahrscheinlichkeit einer Entwicklung zu RIFLE Stadium F zu dem Subjekt, wenn die gemessene Konzentration unter dem Schwellenwert liegt oder

bei einem negativer werdenden Marker, wenn die gemessene Konzentration über dem Schwellenwert liegt, das Zuweisen einer verringerten Wahrscheinlichkeit einer Entwicklung zu RIFLE Stadium F zu dem Subjekt oder das Zuweisen einer erhöhten Wahrscheinlichkeit einer Entwicklung zu RIFLE Stadium F zu dem Subjekt, wenn die gemessene Konzentration unter dem Schwellenwert liegt.

14. Verfahren nach Anspruch 1 oder 2, wobei das Subjekt zum Evaluieren des Nierenstatus ausgewählt wird

(i) auf der Grundlage des vorherigen Bestehens von einem oder mehreren bekannten Risikofaktoren des Subjekts für prärenales, immanent-renales, oder postrenales ARF, oder auf der Grundlage einer bestehenden Diagnose von einem oder mehreren aus kongestiver Herzinsuffizienz, Präeklampsie, Eklampsie, Diabetes mellitus, Hypertonie, Koronare Herzkrankheit, Proteinurie, Niereninsuffizienz, einer glomerulären Filtration unterhalb des normalen Bereichs, Zirrhose, Serum-Kreatinin oberhalb des normalen Bereichs, Sepsis, Schädigung der Nierenfunktion, verminderte Nierenfunktion oder ARF, oder auf der Grundlage eines laufenden oder vorangegangenen größeren gefäßchirurgischen Eingriffs, Koronararterien-Bypassoperation oder einer anderen Herzchirurgie, oder auf der Grundlage einer Exposition zu NSAIDs, Cyclosporinen, Tacrolimus, Aminoglycosiden, Foscarnet, Ethylenglykol, Hämoglobin, Myoglobin, Ifosfamid, Schwermetallen, Methotrexat, röntgendichten Kontrastmitteln oder Streptozotocin ausgewählt wird.

15. Verwendung von einem oder mehreren Biomarkern, die Mitglied 10B der Tumor-Nekrose-Faktor-Rezeptor-Superfamilie umfassen und gegebenenfalls einem oder mehreren aus Cadherin-16, Caspase-9, Bc12 Zelltodantagonist, Caspase-1, Cadherin-1, Poly[ADP-ribose] Polymerase 1, Cyclin-abhängigen Kinase Inhibitor 1, Cadherin-5, Myoglobin, Apolipoprotein A-II, Mucin-16, carcinoembryonalen Antigen-ähnlichen Zelladhäsions-Molekül 5, und zellulären Tumorantigen p53 zur

(i) Diagnose, Risiko-Stratifizierung, Prognose, Klassifizieren und Überwachen des Nierenstatus eines Subjekts, das kein akutes Nierenversagen hat, oder zum
(ii) Zuweisen einer erhöhten Wahrscheinlichkeit einer Entwicklung einer Verschlechterung des RIFLE Stadiums zu einem Subjekt, im Vergleich zu dem aktuellen RIFLE Stadium des Subjekts.

## Revendications

1. Procédé d'évaluation de l'état des reins chez un sujet, comprenant :

la réalisation d'un ou de plusieurs dosages configurés pour détecter un ou plusieurs marqueurs biologiques, l'un ou les plusieurs marqueurs biologiques comprenant le membre 10B de la superfamille des récepteurs du facteur de nécrose tumorale, sur un échantillon de fluide corporel obtenu d'un sujet pour fournir un résultat de dosage ; et
la corrélation du/des résultat(s) de dosage à l'état des reins du sujet.

2. Procédé selon la revendication 1, dans lequel l'un ou les plusieurs marqueurs biologiques comprennent en outre un ou plusieurs parmi la cadhérine-16, la caspase-9, l'antagoniste de Bc12 spécifique de l'apoptose cellulaire, la caspase-1, la cadhérine-1, la poly[ADP-ribose]polymérase 1, l'inhibiteur 1 de kinase dépendant de la cycline, la cadhérine-5, la myoglobine, l'apolipoprotéine A-II, la mucine-16, la molécule 5 d'adhésion cellulaire liée à l'antigène carcino-embryonnaire, et l'antigène p53 spécifique des tumeurs cellulaires.

3. Procédé selon la revendication 1 ou la revendication 2, dans lequel ladite étape de corrélation comprend

(i) la corrélation du/des résultat(s) de dosage à un ou plusieurs parmi une stratification des risques, un diagnostic, une stadification, une classification et une surveillance de l'état des reins du sujet ou
(ii) l'assignation d'une probabilité d'un ou de plusieurs changements futurs de l'état des reins chez le sujet, en fonction du/des résultat(s) de dosage.

4. Procédé selon la revendication 1 ou la revendication 2, dans lequel lesdits un ou plusieurs changements futurs de l'état des reins comprennent un ou plusieurs parmi une lésion future de la fonction rénale, une fonction rénale réduite future, une amélioration future de la fonction rénale, et une insuffisance rénale aiguë (IRA) future.

**5.** Procédé selon la revendication 1 ou la revendication 2, dans lequel le sujet n'est pas atteint d'une insuffisance rénale aiguë.

**6.** Procédé selon la revendication 1 ou la revendication 2, dans lequel le sujet n'a pas connu d'augmentation d'au moins 1,5 fois de la créatinine sérique par rapport à une valeur de référence déterminée avant le moment auquel l'échantillon de fluide corporel est obtenu, ou

dans lequel le sujet à une production d'urine d'au moins 0,5 ml / kg / heure sur les 12 heures qui précèdent le moment auquel l'échantillon de fluide corporel est obtenu, ou

dans lequel le sujet n'a pas connu d'augmentation d'au moins 0,3 mg / dl de créatinine sérique par rapport à une valeur de référence déterminée avant le moment auquel l'échantillon de fluide corporel est obtenu, ou

dans lequel le sujet (i) n'a pas connu d'augmentation d'au moins 1,5 fois de la créatinine sérique par rapport à une valeur de référence déterminée avant le moment auquel l'échantillon de fluide corporel est obtenu, (ii) a une production d'urine d'au moins 0,5 ml / kg / heure sur les 12 heures qui précèdent le moment auquel l'échantillon de fluide corporel est obtenu, et (iii) n'a pas connu d'augmentation d'au moins 0,3 mg / dl de créatinine sérique par rapport à une valeur de référence déterminée avant le moment auquel l'échantillon de fluide corporel est obtenu.

**7.** Procédé selon la revendication 1 ou la revendication 2, dans lequel le sujet est dans la classe 0 ou R de RIFLE.

**8.** Procédé selon la revendication 7, dans lequel

(a) le sujet est dans la classe 0 de RIFLE, et ladite étape de corrélation comprend l'assignation d'une probabilité que le sujet atteindra la classe R, I ou F de RIFLE dans les 72 heures, dans les 48 heures ou dans les 24 heures, ou
(b) le sujet est dans la classe 0 ou R de RIFLE, et ladite étape de corrélation comprend l'assignation d'une probabilité que le sujet atteindra la classe I ou F de RIFLE dans les 72 heures, dans les 48 heures ou dans les 24 heures.

**9.** Procédé selon la revendication 1 ou la revendication 2, dans lequel le sujet est dans la classe 0, R, ou I de RIFLE et ladite étape de corrélation comprend l'assignation d'une probabilité que le sujet atteindra la classe F de RIFLE dans les 72 heures,

dans lequel le sujet est éventuellement dans la classe I de RIFLE, et ladite étape de corrélation comprend l'assignation d'une probabilité que le sujet atteindra la classe F de RIFLE dans les 72 heures, dans les 48 heures ou dans les 24 heures.

**10.** Procédé selon la revendication 1 ou la revendication 2, dans lequel le (s) dit (s) résultat(s) de dosage comprennent une concentration d'urine ou de plasma mesurée du membre 10B de la superfamille des récepteurs du facteur de nécrose tumorale, et éventuellement une ou plusieurs parmi :

une concentration d'urine ou de plasma mesurée de cadhérine-16,
une concentration d'urine ou de plasma mesurée de caspase-9,
une concentration d'urine ou de plasma mesurée d'antagoniste de Bc12 spécifique de l'apoptose cellulaire,
une concentration d'urine ou de plasma mesurée de caspase-1,
une concentration d'urine ou de plasma mesurée de cadhérine-1,
une concentration d'urine ou de plasma mesurée de poly[ADP-ribose]polymérase 1,
une concentration d'urine ou de plasma mesurée d'inhibiteur 1 de kinase dépendant de la cycline,
une concentration d'urine ou de plasma mesurée de cadhérine-5,
une concentration d'urine ou de plasma mesurée de myoglobine,
une concentration d'urine ou de plasma mesurée d'apolipoprotéine A-II,
une concentration d'urine ou de plasma mesurée de mucine-16,
une concentration d'urine ou de plasma mesurée de molécule 5 d'adhésion cellulaire liée à l'antigène carcino-embryonnaire, ou
une concentration d'urine ou de plasma mesurée d'antigène p53 spécifique des tumeurs cellulaires
et ladite étape de corrélation comprend la comparaison de chaque concentration mesurée à une concentration seuil correspondante, et

(A) pour un marqueur de sens positif, l'assignation d'une probabilité augmentée de progression du sujet vers une classe RIFLE plus grave, par rapport à la classe RIFLE actuelle du sujet, quand la concentration mesurée est supérieure au seuil, ou l'assignation d'une probabilité diminuée de progression du sujet vers une classe RIFLE plus grave, par rapport à la classe RIFLE actuelle du sujet, quand la concentration mesurée est inférieure au seuil, ou

pour un marqueur de sens négatif, l'assignation d'une probabilité diminuée de progression du sujet vers une classe RIFLE plus grave, par rapport à la classe RIFLE actuelle du sujet, quand la concentration mesurée est supérieure au seuil, ou l'assignation d'une probabilité augmentée de progression du sujet vers une classe RIFLE plus grave, par rapport à la classe RIFLE actuelle du sujet, quand la concentration mesurée est inférieure au seuil, ou

(B) pour un marqueur de sens positif, l'assignation d'une probabilité augmentée de progression vers un besoin de traitement rénal substitutif pour le sujet quand la concentration mesurée est supérieure au seuil, ou l'assignation d'une probabilité diminuée de progression vers un besoin de traitement rénal substitutif quand la concentration mesurée est inférieure au seuil, ou

pour un marqueur de sens négatif, l'assignation d'une probabilité diminuée de progression vers un besoin de traitement rénal substitutif pour le sujet quand la concentration mesurée est supérieure au seuil, ou l'assignation d'une probabilité augmentée de progression vers un besoin de traitement rénal substitutif quand la concentration mesurée est inférieure au seuil.

**11.** Procédé selon la revendication 5, dans lequel le(s)dit(s) résultat(s) de dosage comprennent une concentration d'urine ou de plasma mesurée du membre 10B de la superfamille des récepteurs du facteur de nécrose tumorale, et éventuellement une ou plusieurs parmi :

une concentration d'urine ou de plasma mesurée de cadhérine-16,
une concentration d'urine ou de plasma mesurée de caspase-9,
une concentration d'urine ou de plasma mesurée d'antagoniste de Bcl2 spécifique de l'apoptose cellulaire,
une concentration d'urine ou de plasma mesurée de caspase-1,
une concentration d'urine ou de plasma mesurée de cadhérine-1,
une concentration d'urine ou de plasma mesurée de poly[ADP-ribose]polymérase 1,
une concentration d'urine ou de plasma mesurée d'inhibiteur 1 de kinase dépendant de la cycline,
une concentration d'urine ou de plasma mesurée de cadhérine-5,
une concentration d'urine ou de plasma mesurée de myoglobine,
une concentration d'urine ou de plasma mesurée d'apolipoprotéine A-II,
une concentration d'urine ou de plasma mesurée de mucine-16,
une concentration d'urine ou de plasma mesurée de molécule 5 d'adhésion cellulaire liée à l'antigène carcino-embryonnaire, ou
une concentration d'urine ou de plasma mesurée d'antigène p53 spécifique des tumeurs cellulaires
et ladite étape de corrélation comprend la comparaison de chaque concentration mesurée à une concentration seuil correspondante, et
pour un marqueur de sens positif, l'assignation d'une probabilité augmentée de progression vers une insuffisance rénale aiguë quand la concentration mesurée est supérieure au seuil, ou l'assignation d'une probabilité diminuée de progression du sujet vers une insuffisance rénale aiguë quand la concentration mesurée est inférieure au seuil, ou
pour un marqueur de sens négatif, l'assignation d'une probabilité diminuée de progression vers une insuffisance rénale aiguë quand la concentration mesurée est supérieure au seuil, ou l'assignation d'une probabilité augmentée de progression du sujet vers une insuffisance rénale aiguë quand la concentration mesurée est inférieure au seuil.

**12.** Procédé selon la revendication 8, dans lequel le(s)dit(s) résultat(s) de dosage comprennent une concentration d'urine ou de plasma mesurée du membre 10B de la superfamille des récepteurs du facteur de nécrose tumorale, et éventuellement une ou plusieurs parmi :

une concentration d'urine ou de plasma mesurée de cadhérine-16,
une concentration d'urine ou de plasma mesurée de caspase-9,
une concentration d'urine ou de plasma mesurée d'antagoniste de Bc12 spécifique de l'apoptose cellulaire,
une concentration d'urine ou de plasma mesurée de caspase-1,
une concentration d'urine ou de plasma mesurée de cadhérine-1,
une concentration d'urine ou de plasma mesurée de poly[ADP-ribose]polymérase 1,
une concentration d'urine ou de plasma mesurée d'inhibiteur 1 de kinase dépendant de la cycline,
une concentration d'urine ou de plasma mesurée de cadhérine-5,
une concentration d'urine ou de plasma mesurée de myoglobine,
une concentration d'urine ou de plasma mesurée d'apolipoprotéine A-II,

une concentration d'urine ou de plasma mesurée de mucine-16,

une concentration d'urine ou de plasma mesurée de molécule 5 d'adhésion cellulaire liée à l'antigène carcino-embryonnaire, ou

une concentration d'urine ou de plasma mesurée d'antigène p53 spécifique des tumeurs cellulaires

et ladite étape de corrélation comprend la comparaison de chaque concentration mesurée à une concentration seuil correspondante, et

pour un marqueur de sens positif, l'assignation d'une probabilité augmentée de progression du sujet vers la classe R, I ou F de RIFLE, quand la concentration mesurée est supérieure au seuil, ou l'assignation d'une probabilité diminuée de progression du sujet vers la classe R, I ou F de RIFLE quand la concentration mesurée est inférieure au seuil, ou

pour un marqueur de sens négatif, l'assignation d'une probabilité diminuée de progression du sujet vers la classe R, I ou F de RIFLE, quand la concentration mesurée est supérieure au seuil, ou l'assignation d'une probabilité augmentée de progression du sujet vers la classe R, I ou F de RIFLE quand la concentration mesurée est inférieure au seuil.

13. Procédé selon la revendication 9, dans lequel le(s)dit(s) résultat(s) de dosage comprennent une concentration d'urine ou de plasma mesurée du membre 10B de la superfamille des récepteurs du facteur de nécrose tumorale, et éventuellement une ou plusieurs parmi :

une concentration d'urine ou de plasma mesurée de cadhérine-16,

une concentration d'urine ou de plasma mesurée de caspase-9,

une concentration d'urine ou de plasma mesurée d'antagoniste de Bc12 spécifique de l'apoptose cellulaire,

une concentration d'urine ou de plasma mesurée de caspase-1,

une concentration d'urine ou de plasma mesurée de cadhérine-1,

une concentration d'urine ou de plasma mesurée de poly[ADP-ribose]polymérase 1,

une concentration d'urine ou de plasma mesurée d'inhibiteur 1 de kinase dépendant de la cycline,

une concentration d'urine ou de plasma mesurée de cadhérine-5,

une concentration d'urine ou de plasma mesurée de myoglobine,

une concentration d'urine ou de plasma mesurée d'apolipoprotéine A-II,

une concentration d'urine ou de plasma mesurée de mucine-16,

une concentration d'urine ou de plasma mesurée de molécule 5 d'adhésion cellulaire liée à l'antigène carcino-embryonnaire, ou

une concentration d'urine ou de plasma mesurée d'antigène p53 spécifique des tumeurs cellulaires

et ladite étape de corrélation comprend la comparaison de chaque concentration mesurée à une concentration seuil correspondante, et

pour un marqueur de sens positif, l'assignation d'une probabilité augmentée de progression du sujet vers la classe F de RIFLE, quand la concentration mesurée est supérieure au seuil, ou l'assignation d'une probabilité diminuée de progression du sujet vers la classe F de RIFLE quand la concentration mesurée est inférieure au seuil, ou pour un marqueur de sens négatif, l'assignation d'une probabilité diminuée de progression du sujet vers la classe F de RIFLE, quand la concentration mesurée est supérieure au seuil, ou l'assignation d'une probabilité augmentée de progression du sujet vers la classe F de RIFLE quand la concentration mesurée est inférieure au seuil.

14. Procédé selon la revendication 1 ou la revendication 2, dans lequel le sujet est sélectionné pour une évaluation de l'état des reins (i) en fonction de la préexistence chez le sujet d'un ou de plusieurs facteurs de risque connus pour une IRA pré-rénale, rénale intrinsèque, ou post-rénale, ou

en fonction d'un diagnostic existant d'un(e) ou de plusieurs parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, de la créatinine sérique supérieure à la plage normale, une sepsie, une lésion de la fonction rénale, une fonction rénale réduite, ou une IRA, ou en fonction du fait de subir ou d'avoir subi une intervention chirurgicale vasculaire majeure, un pontage aorto-coronarien, ou une autre intervention cardiaque, ou en fonction de l'exposition à des AINS, à des cyclosporines, au tacrolimus, à des aminoglycosides, au foscarnet, à de l'éthylèneglycol, à l'hémoglobine, à la myoglobine, à l'ifosfamide, à des métaux lourds, au méthotrexate, à des agents de contraste radio-opaques, ou à de la streptozotocine.

15. Utilisation d'un ou de plusieurs marqueurs biologiques comprenant le membre 10B de la superfamille des récepteurs du facteur de nécrose tumorale, et éventuellement un ou plusieurs parmi la cadhérine-16, la caspase-9, l'antagoniste de Bc12 spécifique de l'apoptose cellulaire, la caspase-1, la cadhérine-1, la poly[ADP-ribose]polymérase 1, l'inhibiteur 1 de kinase dépendant de la cycline, la cadhérine-5, la myoglobine, l'apolipoprotéine A-II, la mucine-16, la molécule 5 d'adhésion cellulaire liée à l'antigène carcino-embryonnaire, et l'antigène p53 spécifique des tumeurs cellulaires pour

(i) le diagnostic, la stratification des risques, le pronostic, la classification et la surveillance de l'état des reins d'un sujet n'étant pas atteint d'insuffisance rénale aiguë ou

(ii) pour l'assignation d'une probabilité augmentée de progression du sujet vers une classe de RIFLE plus grave par rapport à la classe de RIFLE actuelle du sujet.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007248989 D1
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- PRAUGHT ; SHLIPAK. *Curr Opin Nephrol Hypertens,* 2005, vol. 14, 265-270
- CHERTOW et al. *J Am Soc Nephrol,* 2005, vol. 16, 3365-3370
- LASSNIGG. *J Am Soc Nephrol,* 2004, vol. 15, 1597-1605
- BELLOMO et al. *Crit Care.,* 2004, vol. 8 (4), R204-12
- KELLUM. *Crit. Care Med.,* 2008, vol. 36, S141-45
- RICCI et al. *Kidney Int.,* 2008, vol. 73, 538-546
- MEHTA et al. *Crit. Care,* 2007, vol. 11, R31
- MCCOLLOUGH et al. *Rev Cardiovasc Med.,* 2006, vol. 7 (4), 177-197
- THE IMMUNOASSAY HANDBOOK. The Immunoassay Handbook. Stockton Press, 1994
- Fundamental Immunology. Raven Press, 1993
- WILSON. *J. Immunol. Methods,* 1994, vol. 175, 267-273
- YARMUSH. *J. Biochem. Biophys. Methods,* 1992, vol. 25, 85-97
- WARD et al. *Nature,* 1989, vol. 341, 544-546
- VAN ERP et al. *J. Immunoassay,* 1991, vol. 12, 425-43
- NELSON ; GRISWOLD. *Comput. Methods Programs Biomed.,* 1988, vol. 27, 65-8
- CWIRLA et al. *Proc. Natl. Acad. Sci. USA,* vol. 87, 6378-82
- DEVLIN et al. *Science,* 1990, vol. 249, 404-6
- SCOTT ; SMITH. *Science,* 1990, vol. 249, 386-88
- FISCHER et al. *Intensive Care Med.,* 2003, vol. 29, 1043-51
- BAGSHAW et al. *Nephrol. Dial. Transplant.,* 2008, vol. 23, 1203-1210
- Merck Manual of Diagnosis and Therapy. Merck Research Laboratories, 1999
- WIJEYSUNDERA et al. *JAMA,* 2007, vol. 297, 1801-9
- HANLEY, J. A. ; MCNEIL, B.J. The meaning and use of the area under a receiver operating characteristic (ROC) curve. *Radiology,* 1982, vol. 143, 29-36